# EUROPÄISCHE PATENTSCHRIFT

(11) **EP 0 960 100 B1**
(45) Veröffentlichungstag und Bekanntmachung des Hinweises auf die Patenterteilung: **09.07.2003**
(21) Anmeldenummer: 97954936.7
(22) Anmeldetag: 19.12.1997
(51) Int. Cl.: C07D 231/20, C07D 231/22, C07D 231/24, A01N 43/56

(54) **SUBSTITUIERTE 4-BENZOYL-PYRAZOLE**
SUBSTITUTED 4-BENZOYL-PYRAZOLES
4-BENZOYLE-PYRAZOLES SUBSTITUES

(30) Priorität: 03.01.1997 DE 19700096
(43) Veröffentlichungstag der Anmeldung: 01.12.1999
(73) Patentinhaber: BASF AKTIENGESELLSCHAFT, 67056 Ludwigshafen (DE)
(72) Erfinder: HILL, Regina, Luise, D-67346 Speyer (DE); KARDORFF, Uwe, D-68159 Mannheim (DE); RACK, Michael, D-69123 Heidelberg (DE); GÖTZ, Norbert, D-67547 Worms (DE); BAUMANN, Ernst, D-67373 Dudenhofen (DE); VON DEYN, Wolfgang, D-67435 Neustadt (DE); ENGEL, Stefan, D-65510 Idstein (DE); MAYER, Guido, D-67433 Neustadt (DE); OTTEN, Martina, D-67069 Ludwigshafen (DE); REINHEIMER, Joachim, D-67063 Ludwigshafen (DE); WITSCHEL, Matthias, D-67061 Ludwigshafen (DE); MISSLITZ, Ulf, D-67433 Neustadt (DE); WALTER, Helmut, D-67283 Obrigheim (DE); WESTPHALEN, Karl-Otto, D-67346 Speyer (DE)
(86) Internationale Anmeldenummer: EP9707210
(87) Internationale Veröffentlichungsnummer: WO98029392

(56) Entgegenhaltungen:
- EP-A- 0 203 428
- EP-A- 0 282 944
- EP-A- 0 344 775
- DE-A- 2 513 750
- GB-A- 2 122 188
- US-A- 4 643 757
- PATENT ABSTRACTS OF JAPAN vol. 6, no. 24 (C-92) [902] , 12.Februar 1982 & JP 56 147772 A (ISHIHARA SANGYO K.K.), 16.November 1981,

## Beschreibung

Die vorliegende Erfindung betrifft substituierte 4-Benzoyl-pyrazole der Formel I 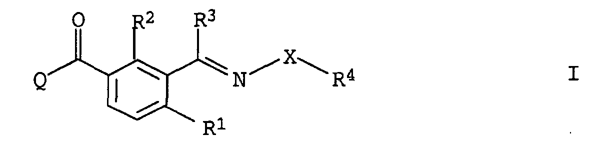 in der die Variablen folgende Bedeutung haben:
- R¹, R²: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁶;
- R³: Wasserstoff, Cyano, C₁-C₆-Alkyl, C₁-C₆-Halogenalkyl, -OR⁶, -SR⁶ oder -NR⁶R⁸;
- R⁴: Wasserstoff, C₁-C₆-Alkyl, C₃-C₆-Cycloalkyl, C₃-C₆-Alkenyl oder C₃-C₆-Alkinyl, wobei die vier letztgenannten Substituenten partiell oder vollständig halogeniert sein können und/oder eine bis drei der folgenden Gruppen tragen können:
Hydroxy, Mercapto, Amino, Cyano, R⁸, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy und Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
und wobei der voranstehende Heterocyclyl- bzw. Heterocyclyloxy-Rest ein bis drei Heteroatome ausgewählt aus der Gruppe Sauerstoff, Stickstoff und Schwefel enthält, 3-7-gliedrig, mono- oder polycyclisch ist und gesättigt oder partiell ungesättigt ist;
und wobei der voranstehend genannte Hetaryl- bzw. Hetaryloxy-Rest neben Kohlenstoffringgliedern zusätzlich ein bis vier Stickstoffatome oder ein bis drei Stickstoffatome und ein Sauerstoff- oder ein Schwefelatom, oder ein Sauerstoff-, oder ein Schwefelatom enthält und aromatisch ist;
- X: Sauerstoff oder NR⁷;
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁶: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁷: Wasserstoff oder C₁-C₆-Alkyl;
- R⁸: C₁-C₆-Alkyl, C₁-C₆-Halogenalkyl, C₃-C₆-Alkenyl oder C₃-C₆-Alkinyl;
- Q: ein in 4-Stellung verknüpftes Pyrazol der Formel II 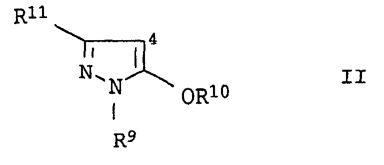 wobei
R⁹ für C₁-C₆-Alkyl, C₁-C₆-Halogenalkyl, Phenyl oder Phenyl, das partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁰ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenylsulfonyl, wobei die vier letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹¹ für Wasserstoff, C₁-C₆-Alkyl oder C₁-C₆-Halogenalkyl; stehen;
sowie deren landwirtschaftlich brauchbaren Salze.

Außerdem betrifft die Erfindung Verfahren und Zwischenprodukte zur Herstellung von Verbindungen der Formel I, Mittel welche diese enthalten, sowie die Verwendung der Verbindungen der Formel I und diese enthaltende Mittel zur Schadpflanzenbekämpfung.

Aus der Literatur, beispielsweise aus EP-A 282 944 sind 4-Benzoyl-pyrazole bekannt.

Die herbiziden Eigenschaften der bisher bekannten Verbindungen sowie die Verträglichkeiten gegenüber Kulturpflanzen können jedoch nur bedingt befriedigen. Es lag daher dieser Erfindung die Aufgabe zugrunde, neue, insbesondere herbizid wirksame, Verbindungen mit verbesserten Eigenschaften zu finden.

Demgemäß wurden die 4-Benzoyl-pyrazole der Formel I sowie deren herbizide Wirkung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I enthalten eine Kohlenstoff-Stickstoff-Doppelbindung und liegen daher als E-Isomere oder Z-Isomere oder als E/Z-Isomerengemische vor. Weiterhin können die Verbindungen der Formel I weitere Kohlenstoff- bzw. Kohlenstoff-Stickstoff-Doppelbindungen enthalten. Gegenstand der Erfindung sind sowohl die reinen geometrischen Isomere als auch Gemische hiervon.

Die Verbindungen der Formel I können je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen, insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Die für die Substituenten R¹-R¹¹ oder als Reste an Phenyl-, Hetaryl- und Heterocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Alkyliminooxy-, Alkoxyamino-, Alkylsulfonyl-, Halogenalkylsulfonyl, Alkylcarbonyl-, Halogenalkylcarbonyl, Alkoxycarbonyl-, Alkoxyalkoxycarbonyl-, Alkenyl-, Cycloalkenyl-, Alkinyl-Teile können geradkettig oder verzweigt sein. Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, Propyl, 1-Methylethyl, Butyl, 1-Methylpropyl, 2-Methylpropyl und 1,1-Dimethylethyl;
- C₁-C₄-Alkyl, sowie die Alkylteile von C₁-C₄-Alkylcarbonyl: C₂-C₄-Alkyl, wie voranstehend genannt sowie Methyl;
- C₂-C₆-Alkyl, sowie die Alkylteile von C₁-C₆-Alkoxy-C₂-C₆alkyl: C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl und 1-Ethyl-3-methylpropyl;
- C₁-C₆-Alkyl, sowie die Alkylteile von C₁-C₆-Alkoxy-C₁-C₆alkyl und C₁-C₆-Alkylcarbonyl: C₂-C₆-Alkyl, wie voranstehend genannt, sowie Methyl;
- C₁-C₄-Halogenalkyl: einen C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl und Nonafluorbutyl;
- C₁-C₆-Halogenalkyl, sowie die Halogenalkylteile von C₁-C₆-Halogenalkylcarbonyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl und Dodecafluorhexyl;
- C₁-C₄-Alkoxy, sowie die Alkoxyteile von C₁-C₄-Alkoxyamino, C₁-C₄-Alkoxy-C₂-C₆-alkoxycarbonyl und C₁-C₄-Alkoxycarbonyl: Methoxy, Ethoxy, Propoxy, 1-Methylethoxy, Butoxy, 1-Methylpropoxy, 2-Methylpropoxy und 1,1-Dimethylethoxy;
- C₁-C₆-Alkoxy, sowie die Alkoxyteile von C₁-C₆-Alkoxy-C₁-C₆alkyl, C₁-C₆-Alkoxy-C₂-C₆-alkyl, C₁-C₄-Alkoxy-C₂-C₆-alkoxycarbonyl und C₁-C₆-Alkoxycarbonyl: C₁-C₄-Alkoxy wie voranstehend genannt, sowie Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy und 1-Ethyl-2-methylpropoxy;
- C₁-C₄-Halogenalkoxy: einen C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₄-Alkylsulfonyl (C₁-C₄-Alkyl-S(=O)₂-): Methylsulfonyl, Ethylsulfonyl, Propylsulfonyl, 1-Methylethylsulfonyl, Butylsulfonyl, 1-Methylpropylsulfonyl, 2-Methylpropylsulfonyl und 1,1-Dimethylethylsulfonyl;
- C₁-C₆-Alkylsulfonyl: C₁-C₄-Alkylsulfonyl wie voranstehend genannt, sowie Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsulfonyl;
- C₁-C₆-Halogenalkylsulfonyl: einen C₁-C₆-Alkylsulfonylrest wie voranstehend genannt, der partiell oder vollständig durch Fluor, Chlor, Brom und/oder Iod substituiert ist, also Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-Iodpentylsulfonyl, 6-Fluorhexylsulfonyl, 6-Bromhexylsulfonyl, 6-Iodhexylsulfonyl und Dodecafluorhexylsulfonyl;
- C₁-C₄-Alkyliminooxy: Methyliminooxy, Ethyliminooxy, 1-Propyliminooxy, 2-Propyliminooxy, 1-Butyliminoox und 2-Butyliminooxy;
- C₃-C₆-Alkenyl: Prop-1-en-1-yl, Prop-2-en-1-yl, 1-Methylethenyl, Buten-1-yl, Buten-2-yl, Buten-3-yl, 2-Methylprop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, l-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethylbut-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methylprop-1-en-1-yl und 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl und 4-Methyl-pent-2-in-5-yl;
- C₂-C₆-Alkinyl: C₃-C₆-Alkinyl, wie voranstehend genannt, sowie Ethinyl:
- C₃-C₆-Cycloalkyl: Cyclopropyl, Cyclobutyl, Cyclopentyl und Cyclohexyl;
- C₄-C₆-Cycloalkenyl: Cyclobuten-1-yl, Cyclobuten-3-yl, Cyclopenten-1-yl, Cyclopenten-3-yl, Cyclopenten-4-yl, Cyclohexen-1-yl, Cyclohexen-3-yl und Cyclohexen-4-yl;
- Heterocyclyl, sowie die Heterocyclylreste in Heterocyclyloxy: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, Oxetan-3-yl, Thietan-3-yl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dioxan-2-yl, 1,3-Dithian-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 1,3-Dithiolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl,
- Hetaryl, sowie die Hetarylreste in Hetaryloxy:
   aromatische mono- oder polycyclische Reste, welche neben Kohlenstoffringgliedern zusätzlich ein bis vier Stickstoffatome oder ein bis drei Stickstoffatome und ein Sauerstoffoder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol- 2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate.

Alle Phenyl-, Hetaryl- und Heterocyclylringe sind vorzugsweise unsubstituiert oder tragen ein bis drei Halogenatome und/oder einen oder zwei Reste aus folgender Gruppe: Nitro, Cyano, Methyl, Trifluormethyl, Methoxy, Trifluormethoxy oder Methoxycarbonyl.

In Hinblick auf die Verwendung der erfindungsgemäßen Verbindungen der Formel I als Herbizide haben die Variablen vorzugsweise folgende Bedeutungen, und zwar jeweils für sich allein oder in Kombination:
- R¹: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁶;
besonders bevorzugt Nitro, Halogen wie z.B. Fluor, Chlor oder Brom, C₁-C₆-Halogenalkyl, -OR⁵ oder -SO₂R⁶ wie z.B. Methylsulfonyl, Ethylsulfonyl oder Difluormethylsulfonyl;
insbesondere bevorzugt Nitro, Fluor, Chlor, Brom, Trifluormethyl, Methoxy, Ethoxy, Methylsulfonyl, Ethylsulfonyl oder Difluormethylsulfonyl;
- R²: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁶;
besonders bevorzugt Wasserstoff, Nitro, Halogen wie z.B. Fluor, Chlor oder Brom, C₁-C₆-Alkyl wie z.B. Methyl oder Ethyl, C₁-C₆-Halogenalkyl, -OR⁵ oder -SO₂R⁶ wie z.B. Methylsulfonyl, Ethylsulfonyl oder Difluormethylsulfonyl; insbesondere bevorzugt Nitro, Fluor, Chlor, Brom, Methyl, Ethyl, Trifluormethyl, Methoxy, Ethoxy, Methylsulfonyl, Ethylsulfonyl oder Difluormethylsulfonyl;
- R³: Wasserstoff, Cyano, C₁-C₆-Alkyl, C₁-C₆-Halogenalkyl oder -OR⁶;
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy oder Hetaryloxy, wobei die acht letztgenannten Reste ihrerseits partiell oder vollständig halogeniert sein können und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
besonders bevorzugt C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können Hydroxy, Mercapto, Amino, Cyano, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy oder Hetaryloxy, wobei die acht letztgenannten Reste ihrerseits partiell oder vollständig halogeniert sein können und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
und wobei jeweils der voranstehend genannte Heterocyclyl-bzw. Heterocylyloxy-Rest ein bis drei Heteroatome ausgewählt aus der Gruppe Sauerstoff, Stickstoff- oder Schwefel enthält, 3-7-gliedrig, mono- oder polycyclisch ist und gesättigt oder partiell ungesättigt ist;
und wobei der voranstehend genannte Hetaryl- bzw. Hetaryloxy-Rest neben Kohlenstoffgliedern zusätzlich ein- bis vier Stickstoffatome oder ein bis drei Stickstoffatome und ein Sauerstoff- oder ein Schwefelatom, oder ein Sauerstoff- oder ein Schwefelatom enthält und aromatisch ist;
- X: Sauerstoff oder NH;
- n: 0 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl; besonders bevorzugt Methyl, Ethyl, Trifluormethyl, Difluormethyl, Methoxyethyl, Allyl oder Propargyl;
- R⁶: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
besonders bevorzugt Methyl, Ethyl, Trifluormethyl, Difluormethyl, Methoxyethyl, Allyl oder Propargyl;
- R⁷: Wasserstoff oder C₁-C₆-Alkyl;
- R⁸: C₁-C₆-Alkyl, C₁-C₆-Halogenalkyl, C₃-C₆-Alkenyl oder C₃-C₆-Alkinyl;
- R⁹: C₁-C₆-Alkyl oder C₁-C₆-Halogenalkyl; besonders bevorzugt Methyl, Ethyl, Propyl, Isopropyl, Butyl oder Isobutyl;
- R¹⁰: Wasserstoff, C₁-C₆-Alkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonylmethyl, oder Phenylsulfonyl, wobei der Phenylring der zwei letztgenannten Substituenten partiell oder vollständig halogeniert sein kann und/oder einen bis drei der folgenden Reste tragen kann: Nitro, Cyano, C₁-C₄-Alkyl, C₁-C₄-Halogenalkyl, C₁-C₄-Alkoxy, C₁-C₄-Halogenalkoxy;
- R¹¹: Wasserstoff, C₁-C₆-Alkyl oder C₁-C₆-Halogenalkyl; besonders bevorzugt Wasserstoff, Methyl, Ethyl oder Trifluormethyl.

Außerordentlich bevorzugt sind die Verbindungen der Formel I in der die Variablen R¹ bis R³, Q und X die oben genannte Bedeutung haben und
- R⁴: Wasserstoff, C₁-C₆-Alkyl, C₃-C₆-Cycloalkyl, C₃-C₆-Alkenyl oder C₃-C₆-Alkinyl, wobei die 4 letztgenannten Substituenten partiell oder vollständig halogeniert sein können und/oder eine bis drei der folgenden Gruppen tragen können: Hydroxy, Mercapto, Amino, Cyano, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy oder Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
und wobei jeweils der voranstehend genannte Heterocyclyl-bzw. Heterocyclyloxy-Rest ein bis drei Heteroatome ausgewählt aus der Gruppe Sauerstoff, Stickstoff oder Schwefel enthält, 3-7-gliedrig, mono- oder polycyclisch ist und gesättigt oder partiell ungesättigt ist;
und wobei der voranstehend genannte Heteroyl- bzw. Hetaryloxy-Rest neben Kohlenstoffgliedern zusätzlich ein bis vier Stickstoffatome oder ein bis drei Stickstoffatome und ein Sauerstoff- oder ein Schwefelatom, oder ein Sauerstoff- oder ein Schwefelatom enthält und aromatisch ist;
bedeutet.

Insbesonders außerordentlich bevorzugt sind die Verbindungen I1 (≙ I mit R¹ = Cl, R⁹ = CH₃ und R¹⁰ und R¹¹ = H, insbesondere die Verbindungen der Tabelle 1. ≡

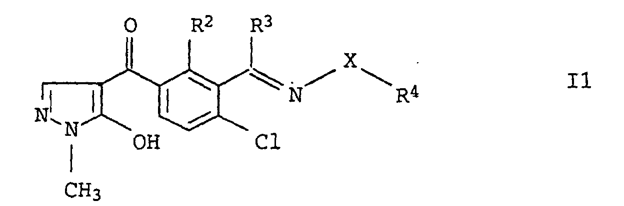

**Tabelle 1**

| Nr. | R² | R³ | R⁴ | X |
|---|---|---|---|---|
| I1.001 | Cl | H | CH₃ | O |
| I1.002 | Cl | H | C₂H₅ | O |
| I1.003 | Cl | H | CH₂-C≡CH | O |
| I1.004 | Cl | CH₃ | CH₃ | O |
| I1.005 | Cl | CH₃ | C₂H₅ | O |
| I1.006 | Cl | CH₃ | CH₂-C≡CH | O |
| I1.007 | Cl | C₂H₅ | CH₃ | O |
| I1.008 | Cl | C₂H₅ | C₂H₅ | O |
| I1.009 | Cl | C₂H₅ | CH₂-C≡CH | O |
| I1.010 | Cl | OCH₃ | CH₃ | O |
| I1.011 | Cl | OCH₃ | C₂H₅ | O |
| I1.012 | Cl | OCH₃ | CH₂-C≡CH | O |
| I1.013 | Cl | OC₂H₅ | CH₃ | O |
| I1.014 | Cl | OC₂H₅ | C₂H₅ | O |
| I1.015 | Cl | OC₂H₅ | CH₂-C≡CH | O |
| I1.016 | Cl | H | CH₃ | NH |
| I1.017 | Cl | H | C₂H₅ | NH |
| I1.018 | Cl | H | CH₂-C≡CH | NH |
| I1.019 | Cl | CH₃ | CH₃ | NH |
| I1.020 | Cl | CH₃ | C₂H₅ | NH |
| I1.021 | Cl | CH₃ | CH₂-C≡CH | NH |
| I1.022 | Cl | C₂H₅ | CH₃ | NH |
| I1.023 | Cl | C₂H₅ | C₂H₅ | NH |
| I1.024 | Cl | C₂H₅ | CH₂-C≡CH | NH |
| I1.025 | Cl | OCH₃ | CH₃ | NH |
| I1.026 | Cl | OCH₃ | C₂H₅ | NH |
| I1.027 | Cl | OCH₃ | CH₂-C≡CH | NH |
| I1.028 | Cl | OC₂H₅ | CH₃ | NH |
| I1.029 | Cl | OC₂H₅ | C₂H₅ | NH |
| I1.030 | Cl | OC₂H₅ | CH₂-C≡CH | NH |
| I1.031 | CH₃ | H | CH₃ | O |
| I1.032 | CH₃ | H | C₂H₅ | O |
| I1.033 | CH₃ | H | CH₂-C≡CH | O |
| I1.034 | CH₃ | CH₃ | CH₃ | O |
| I1.035 | CH₃ | CH₃ | C₂H₅ | O |
| I1.036 | CH₃ | CH₃ | CH₂-C≡CH | O |
| I1.037 | CH₃ | C₂H₅ | CH₃ | O |
| I1.038 | CH₃ | C₂H₅ | C₂H₅ | O |
| I1.039 | CH₃ | C₂H₅ | CH₂-C=CH | O |
| I1.040 | CH₃ | OCH₃ | CH₃ | O |
| I1.041 | CH₃ | OCH₃ | C₂H₅ | O |
| I1.042 | CH₃ | OCH₃ | CH₂-C ≡CH | O |
| I1.043 | CH₃ | OC₂H₅ | CH₃ | O |
| I1.044 | CH₃ | OC₂H₅ | C₂H₅ | O |
| I1.045 | CH₃ | OC₂H₅ | CH₂-C≡CH | O |
| I1.046 | CH₃ | H | CH₃ | NH |
| I1.047 | CH₃ | H | C₂H₅ | NH |
| I1.048 | CH₃ | H | CH₂-C≡CH | NH |
| I1.049 | CH₃ | CH₃ | CH₃ | NH |
| I1.050 | CH₃ | CH₃ | C₂H₅ | NH |
| I1.051 | CH₃ | CH₃ | CH₂-C≡CH | NH |
| I1.052 | CH₃ | C₂H₅ | CH₃ | NH |
| I1.053 | CH₃ | C₂H₅ | C₂H₅ | NH |
| I1.054 | CH₃ | C₂H₅ | CH₂-C≡CH | NH |
| I1.055 | CH₃ | OCH₃ | CH₃ | NH |
| I1.056 | CH₃ | OCH₃ | C₂H₅ | NH |
| I1.057 | CH₃ | OCH₃ | CH₂-C≡CH | NH |
| I1.058 | CH₃ | OC₂H₅ | CH₃ | NH |
| I1.059 | CH₃ | OC₂H₅ | C₂H₅ | NH |
| I1.060 | CH₃ | OC₂H₅ | CH₂-C≡CH | NH |
| I1.061 | OCH₃ | H | CH₃ | O |
| I1.062 | OCH₃ | H | C₂H₅ | O |
| I1.063 | OCH₃ | H | CH₂-C≡CH | O |
| I1.064 | OCH₃ | CH₃ | CH₃ | O |
| I1.065 | OCH₃ | CH₃ | C₂H₅ | O |
| I1.066 | OCH₃ | CH₃ | CH₂-C≡CH | O |
| I1.067 | OCH₃ | C₂H₅ | CH₃ | O |
| I1.068 | OCH₃ | C₂H₅ | C₂H₅ | O |
| I1.069 | OCH₃ | C₂H₅ | CH₂-C≡CH | O |
| I1.070 | OCH₃ | OCH₃ | CH₃ | O |
| I1.071 | OCH₃ | OCH₃ | C₂H₅ | O |
| I1.072 | OCH₃ | OCH₃ | CH₂-C≡CH | O |
| I1.073 | OCH₃ | OC₂H₅ | CH₃ | O |
| I1.074 | OCH₃ | OC₂H₅ | C₂H₅ | O |
| I1.075 | OCH₃ | OC₂H₅ | CH₂-C≡CH | O |
| I1.076 | OCH₃ | H | CH₃ | NH |
| I1.077 | OCH₃ | H | C₂H₅ | NH |
| I1.078 | OCH₃ | H | CH₂-C≡CH | NH |
| I1.079 | OCH₃ | CH₃ | CH₃ | NH |
| I1.080 | OCH₃ | CH₃ | C₂H₅ | NH |
| I1.081 | OCH₃ | CH₃ | CH₂-C≡CH | NH |
| I1.082 | OCH₃ | C₂H₅ | CH₃ | NH |
| I1.083 | OCH₃ | C₂H₅ | C₂H₅ | NH |
| I1.084 | OCH₃ | C₂H₅ | CH₂-C≡CH | NH |
| I1.085 | OCH₃ | OCH₃ | CH₃ | NH |
| I1.086 | OCH₃ | OCH₃ | C₂H₅ | NH |
| I1.087 | OCH₃ | OCH₃ | CH₂-C≡CH | NH |
| I1.088 | OCH₃ | OC₂H₅ | CH₃ | NH |
| I1.089 | OCH₃ | OC₂H₅ | C₂H₅ | NH |
| I1.090 | OCH₃ | OC₂H₅ | CH₂-C≡CH | NH |
| I1.091 | CF₃ | H | CH₃ | O |
| I1.092 | CF₃ | H | C₂H₅ | O |
| I1.093 | CF₃ | H | CH₂-C≡CH | O |
| I1.094 | CF₃ | CH₃ | CH₃ | O |
| I1.095 | CF₃ | CH₃ | C₂H₅ | O |
| I1.096 | CF₃ | CH₃ | CH₂-C≡CH | O |
| I1.097 | CF₃ | C₂H₅ | CH₃ | O |
| I1.098 | CF₃ | C₂H₅ | C₂H₅ | O |
| I1.099 | CF₃ | C₂H₅ | C≡CH | O |
| I1.100 | CF₃ | OCH₃ | CH₃ | O |
| I1.101 | CF₃ | OCH₃ | C₂H₅ | O |
| I1.102 | CF₃ | OCH₃ | CH₂-C≡CH | O |
| I1.103 | CF₃ | OC₂H₅ | CH₃ | O |
| I1.104 | CF₃ | OC₂H₅ | C₂H₅ | O |
| I1.105 | CF₃ | OC₂H₅ | CH₂-C≡CH | O |
| I1.106 | CF₃ | H | CH₃ | NH |
| I1.107 | CF₃ | H | C₂H₅ | NH |
| I1.108 | CF₃ | H | CH₂-C≡CH | NH |
| I1.109 | CF₃ | CH₃ | CH₃ | NH |
| I1.110 | CF₃ | CH₃ | C₂H₅ | NH |
| I1.111 | CF₃ | CH₃ | CH₂-C≡CH | NH |
| I1.112 | CF₃ | C₂H₅ | CH₃ | NH |
| I1.113 | CF₃ | C₂H₅ | C₂H₅ | NH |
| I1.114 | CF₃ | C₂H₅ | CH₂-C≡CH | NH |
| I1.115 | CF₃ | OCH₃ | CH₃ | NH |
| I1.116 | CF₃ | OCH₃ | C₂H₅ | NH |
| I1.117 | CF₃ | OCH₃ | CH₂-C≡CH | NH |
| I1.118 | CF₃ | OC₂H₅ | CH₃ | NH |
| I1.119 | CF₃ | OC₂H₅ | C₂H₅ | NH |
| I1.120 | CF₃ | OC₂H₅ | CH₂-C≡CH | NH |
| I1.121 | SO₂CH₃ | H | CH₃ | O |
| I1.122 | SO₂CH₃ | H | C₂H₅ | O |
| I1.123 | SO₂CH₃ | H | CH₂-C≡CH | O |
| I1.124 | SO₂CH₃ | CH₃ | CH₃ | O |
| I1.125 | SO₂CH₃ | CH₃ | C₂H₅ | O |
| I1.126 | SO₂CH₃ | CH₃ | CH₂-C≡CH | O |
| I1.127 | SO₂CH₃ | C₂H₅ | CH₃ | O |
| I1.128 | SO₂CH₃ | C₂H₅ | C₂H₅ | O |
| I1.129 | SO₂CH₃ | C₂H₅ | CH₂-C≡CH | O |
| I1.130 | SO₂CH₃ | OCH₃ | CH₃ | O |
| I1.131 | SO₂CH₃ | OCH₃ | C₂H₅ | O |
| I1.132 | SO₂CH₃ | OCH₃ | CH₂-C≡CH | O |
| I1.133 | SO₂CH₃ | OC₂H₅ | CH₃ | O |
| I1.134 | SO₂CH₃ | OC₂H₅ | C₂H₅ | O |
| I1.135 | SO₂CH₃ | OC₂H₅ | CH₂-C≡CH | O |
| I1.136 | SO₂CH₃ | H | CH₃ | NH |
| I1.137 | SO₂CH₃ | H | C₂H₅ | NH |
| I1.138 | SO₂CH₃ | H | CH₂-C≡CH | NH |
| I1.139 | SO₂CH₃ | CH₃ | CH₃ | NH |
| I1.140 | SO₂CH₃ | CH₃ | C₂H₅ | NH |
| I1.141 | SO₂CH₃ | CH₃ | CH₂-C≡CH | NH |
| I1.142 | SO₂CH₃ | C₂H₅ | CH₃ | NH |
| I1.143 | SO₂CH₃ | C₂H₅ | C₂H₅ | NH |
| I1.144 | SO₂CH₃ | C₂H₅ | CH₂-C≡CH | NH |
| I1.145 | SO₂CH₃ | OCH₃ | CH₃ | NH |
| I1.146 | SO₂CH₃ | OCH₃ | C₂H₅ | NH |
| I1.147 | SO₂CH₃ | OCH₃ | CH₂-C≡CH | NH |
| I1.148 | SO₂CH₃ | OC₂H₅ | CH₃ | NH |
| I1.149 | SO₂CH₃ | OC₂H₅ | C₂H₅ | NH |
| I1.150 | SO₂CH₃ | OC₂H₅ | CH₂-C≡CH | NH |
| I1.151 | NO₂ | H | CH₃ | O |
| I1.152 | NO₂ | H | C₂H₅ | O |
| I1.153 | NO₂ | H | CH₂-C≡CH | O |
| I1.154 | NO₂ | CH₃ | CH₃ | O |
| I1.155 | NO₂ | CH₃ | C₂H₅ | O |
| I1.156 | NO₂ | CH₃ | CH₂-C≡CH | O |
| I1.157 | NO₂ | C₂H₅ | CH₃ | O |
| I1.158 | NO₂ | C₂H₅ | C₂H₅ | O |
| I1.159 | NO₂ | C₂H₅ | CH₂-C≡CH | O |
| I1.160 | NO₂ | OCH₃ | CH₃ | O |
| I1.161 | NO₂ | OCH₃ | C₂H₅ | O |
| I1.162 | NO₂ | OCH₃ | CH₂-C≡CH | O |
| I1.163 | NO₂ | OC₂H₅ | CH₃ | O |
| I1.164 | NO₂ | OC₂H₅ | C₂H₅ | O |
| I1.165 | NO₂ | OC₂H₅ | CH₂-C≡CH | O |
| I1.166 | NO₂ | H | CH₃ | NH |
| I1.167 | NO₂ | H | C₂H₅ | NH |
| I1.168 | NO₂ | H | CH₂-C≡CH | NH |
| I1.169 | NO₂ | CH₃ | CH₃ | NH |
| I1.170 | NO₂ | CH₃ | C₂H₅ | NH |
| I1.171 | NO₂ | CH₃ | CH₂-C≡CH | NH |
| I1.172 | NO₂ | C₂H₅ | CH₃ | NH |
| I1.173 | NO₂ | C₂H₅ | C₂H₅ | NH |
| I1.174 | NO₂ | C₂H₅ | CH₂-C≡CH | NH |
| I1.175 | NO₂ | OCH₃ | CH₃ | NH |
| I1.176 | NO₂ | OCH₃ | C₂H₅ | NH |
| I1.177 | NO₂ | OCH₃ | CH₂-C≡CH | NH |
| I1.178 | NO₂ | OC₂H₅ | CH₃ | NH |
| I1.179 | NO₂ | OC₂H₅ | C₂H₅ | NH |
| I1.180 | NO₂ | OC₂H₅ | CH₂-C≡CH | NH |

Des weiteren sind die folgenden 4-Benzoyl-pyrazole der Formel I insbesonders außerordentlich bevorzugt:
- die Verbindungen I2, insbesondere die Verbindungen I2.001-I2.180, die sich von den entsprechenden Verbindungen I1.001-I1.180 dadurch unterscheiden, daß R⁹ für Ethyl steht: 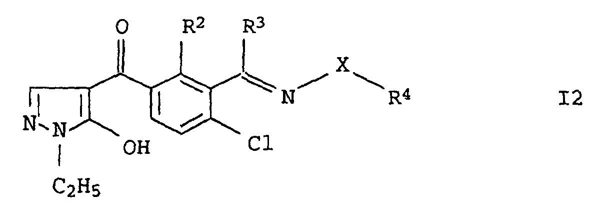
- die Verbindungen I3, insbesondere die Verbindungen I3.001-I3.180, die sich von den entsprechenden Verbindungen I1.001-I1.180 dadurch unterscheiden, daß R⁹ für n-Propyl steht: 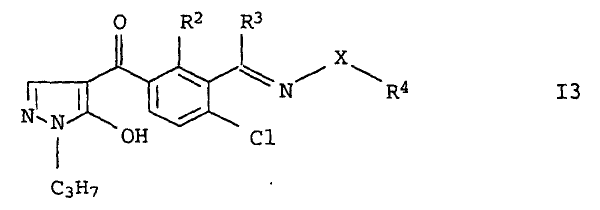
- die Verbindungen I4, insbesondere die Verbindungen I4.001-I4.180, die sich von den entsprechenden Verbindungen I1.001-I1.180 dadurch unterscheiden, daß R⁹ für n-Butyl stehen: 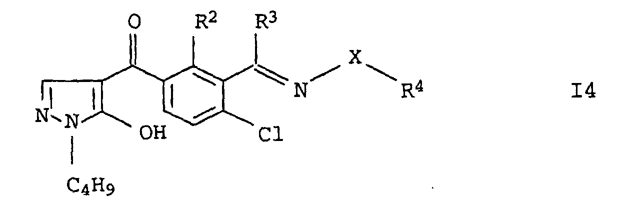
- die Verbindungen I5, insbesondere die Verbindungen I5.001-I5.180, die sich von den entsprechenden Verbindungen I1.001-I1.180 dadurch unterscheiden, daß R⁹ für iso-Butyl steht: 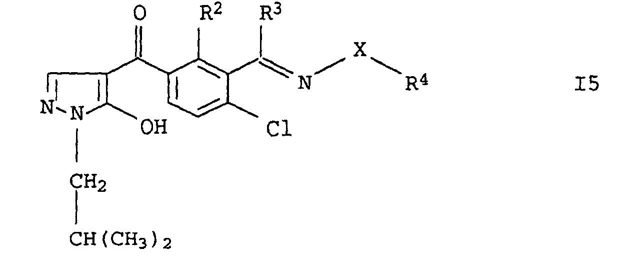
- die Verbindungen I6, insbesondere die Verbindungen I6.001-I6.180, die sich von den entsprechenden Verbindungen I1.001-I1.180 dadurch unterscheiden, daß R¹⁰ für Methyl steht: 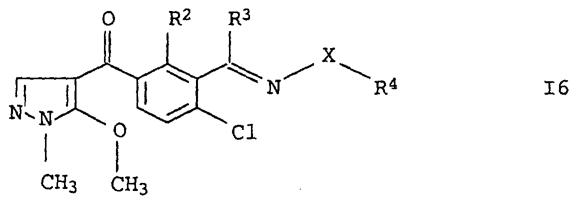
- die Verbindungen I7, insbesondere die Verbindungen I7.001-I7.180, die sich von den entsprechenden Verbindungen I1.001-I1.180 dadurch unterscheiden, daß R⁹ für Ethyl und R¹⁰ für Methyl stehen: 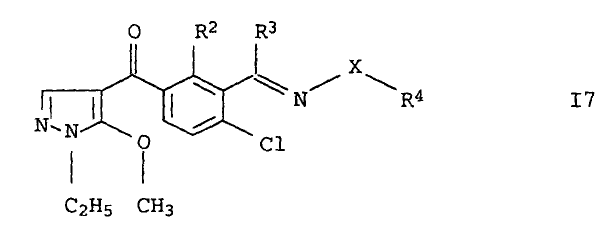
- die Verbindungen I8, insbesondere die Verbindungen I8.001-I8.180, die sich von den entsprechenden Verbindungen I1.001-I1.180 dadurch unterscheiden, daß R⁹ für n-Propyl und R¹⁰ für Methyl stehen: 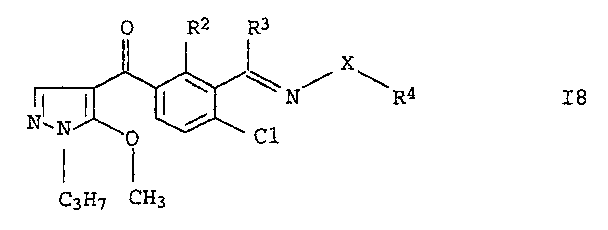
- die Verbindungen I9, insbesondere die Verbindungen I9.001-I9.180, die sich von den entsprechenden Verbindungen I1.001-I1.180 dadurch unterscheiden, daß R⁹ für n-Butyl und R¹⁰ für Methyl stehen: 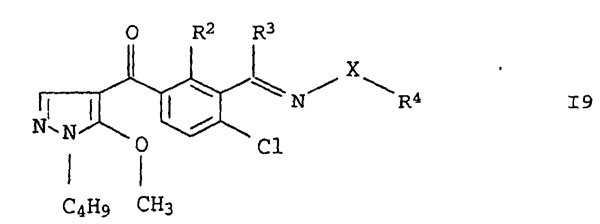
- die Verbindungen I10, insbesondere die Verbindungen I10.001-I10.180, die sich von den entsprechenden Verbindungen I1.001-I1.180 dadurch unterscheiden, daß R⁹ für iso-Butyl und R¹⁰ für Methyl stehen: 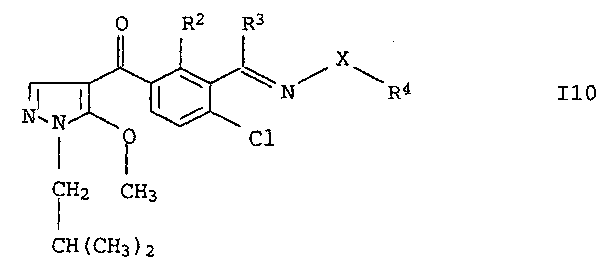
- die Verbindungen I11, insbesondere die Verbindungen I11.001-I11.180, die sich von den entsprechenden Verbindungen I1.001-I1.180 dadurch unterscheiden, daß R¹⁰ für Ethyl steht: 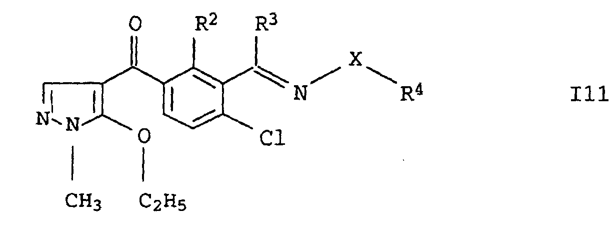
- die Verbindungen I12, insbesondere die Verbindungen I12.001-I12.180, die sich von den entsprechenden Verbindungen I1.001-I1.180 dadurch unterscheiden, daß R⁹ und R¹⁰ für Ethyl stehen: 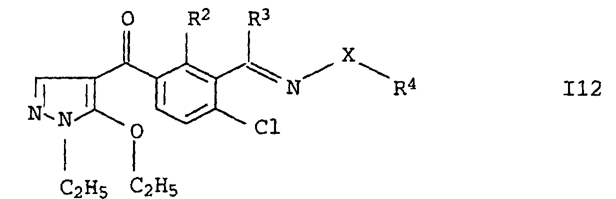
- die Verbindungen I13, insbesondere die Verbindungen I13.001-I13.180, die sich von den entsprechenden Verbindungen I1.001-I1.180 dadurch unterscheiden, daß R⁹ für Propyl und R¹⁰ für Ethyl stehen: 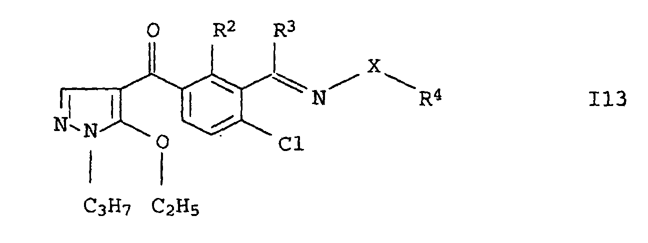
- die Verbindungen I14, insbesondere die Verbindungen I14.001-I14.180, die sich von den entsprechenden Verbindungen I1.001-I1.180 dadurch unterscheiden, daß R⁹ für n-Butyl und R¹⁰ für Ethyl stehen: 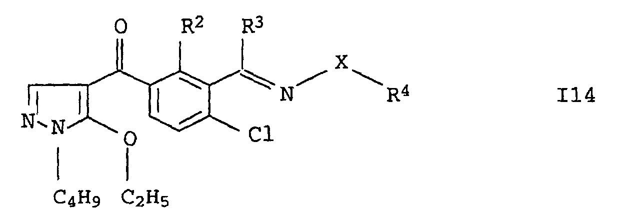
- die Verbindungen I15, insbesondere die Verbindungen I15.001-I15.180, die sich von den entsprechenden Verbindungen I1.001-I1.180 dadurch unterscheiden, daß R⁹ für iso-Butyl und R¹⁰ für Ethyl stehen: 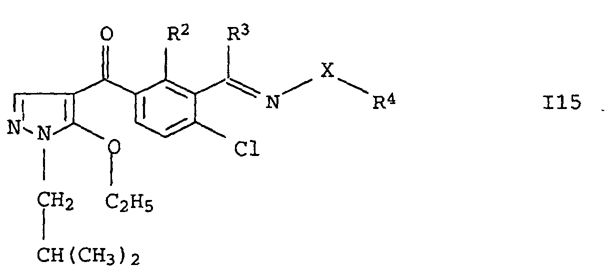
- die Verbindungen I16, insbesondere die Verbindungen I16.001-I16.180, die sich von den entsprechenden Verbindungen I1.001-I1.180 dadurch unterscheiden, daß R¹⁰ für n-Propyl steht: 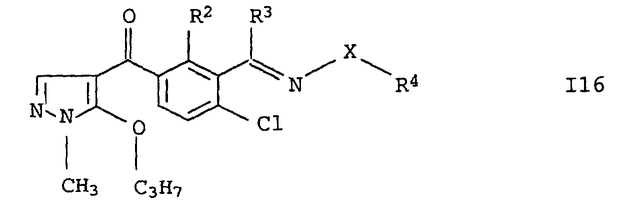
- die Verbindungen I17, insbesondere die Verbindungen I17.001-I17.180, die sich von den entsprechenden Verbindungen I1.001-I1.180 dadurch unterscheiden, daß R⁹ für Ethyl und R¹⁰ für n-Propyl stehen: 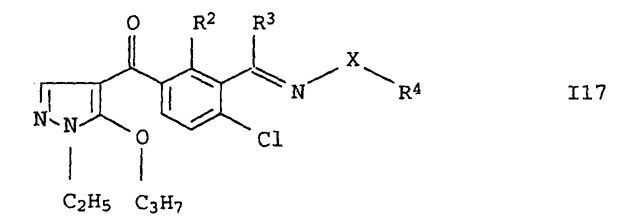
- die Verbindungen I18, insbesondere die Verbindungen I18.001-I18.180, die sich von den entsprechenden Verbindungen I1.001-I1.180 dadurch unterscheiden, daß R⁹ und R¹⁰ für n-Propyl stehen: 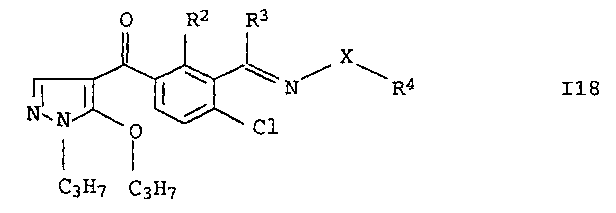
- die Verbindungen I19, insbesondere die Verbindungen I19.001-I19.180, die sich von den entsprechenden Verbindungen I1.001-I1.180 dadurch unterscheiden, daß R⁹ für n-Butyl und R¹⁰ für n-Propyl stehen: 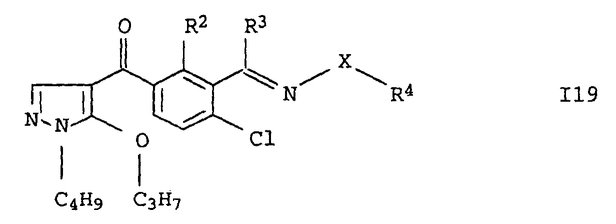
- die Verbindungen I20, insbesondere die Verbindungen I20.001-I20.180, die sich von den entsprechenden Verbindungen I1.001-I1.180 dadurch unterscheiden, daß R⁹ für iso-Butyl und R¹⁰ für n-Propyl stehen: 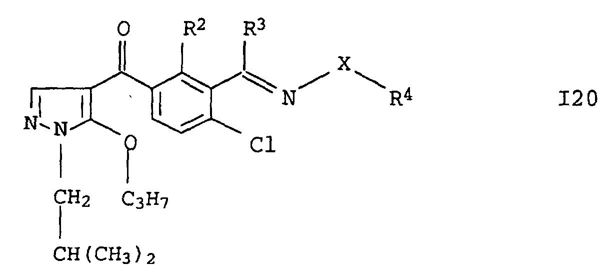
- die Verbindungen I21, insbesondere die Verbindungen I21.001-I21.180, die sich von den entsprechenden Verbindungen I1.001-I1.180 dadurch unterscheiden, daß R¹⁰ für iso-Propyl steht: 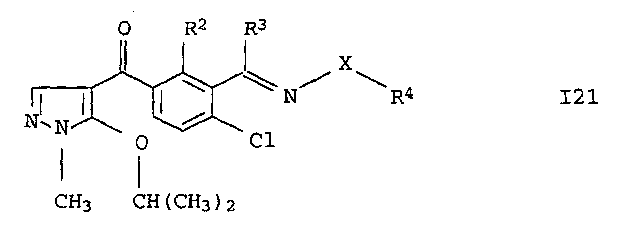
- die Verbindungen I22, insbesondere die Verbindungen I22.001-I22.180, die sich von den entsprechenden Verbindungen I1.001-I1.180 dadurch unterscheiden, daß R⁹ für Ethyl und R¹⁰ für iso-Propyl stehen: 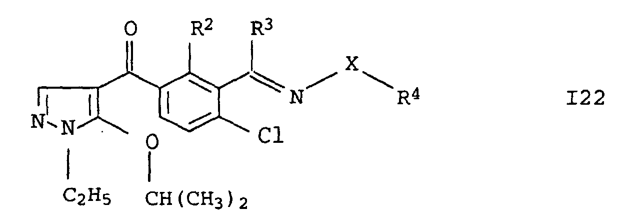
- die Verbindungen I23, insbesondere die Verbindungen I23.001-I23.180, die sich von den entsprechenden Verbindungen I1.001-I1.180 dadurch unterscheiden, daß R⁹ für n-Propyl und R¹⁰ für iso-Propyl stehen: 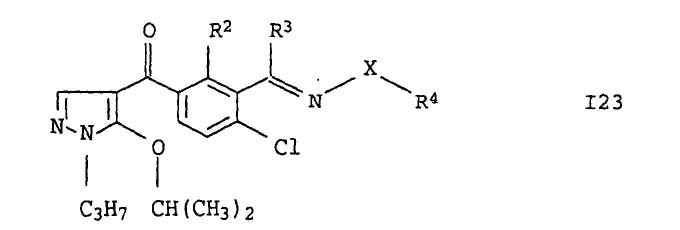
- die Verbindungen I24, insbesondere die Verbindungen I24.001-I24.180, die sich von den entsprechenden Verbindungen I1.001-I1.180 dadurch unterscheiden, daß R⁹ für n-Butyl und R¹⁰ für iso-Propyl stehen: 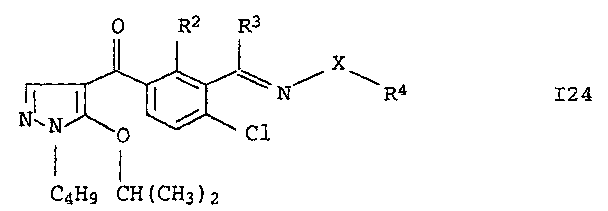
- die Verbindungen I25, insbesondere die Verbindungen I25.001-I25.180, die sich von den entsprechenden Verbindungen I1.001-I1.180 dadurch unterscheiden, daß R⁹ für iso-Butyl und R¹⁰ für iso-Propyl stehen: 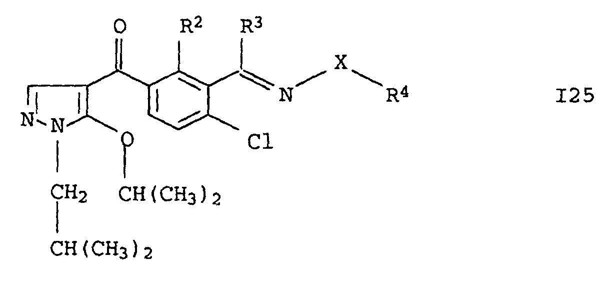
- die Verbindungen I26, insbesondere die Verbindungen I26.001-I26.180, die sich von den entsprechenden Verbindungen I1.001-I1.180 dadurch unterscheiden, daß R¹⁰ für n-Butyl steht: 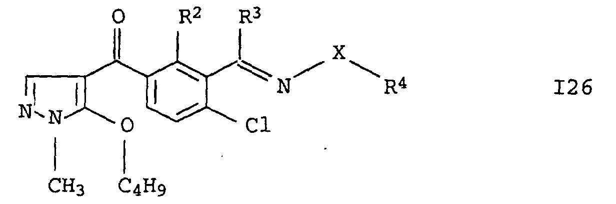
- die Verbindungen I27, insbesondere die Verbindungen I27.001-I27.180, die sich von den entsprechenden Verbindungen I1.001-I1.180 dadurch unterscheiden, daß R⁹ für Ethyl und R¹⁰ für n-Butyl stehen: 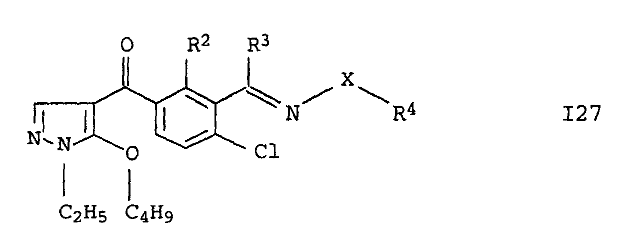
- die Verbindungen I28, insbesondere die Verbindungen I28.001-I28.180, die sich von den entsprechenden Verbindungen I1.001-I1.180 dadurch unterscheiden, daß R⁹ für n-Propyl und R¹⁰ für n-Butyl stehen: 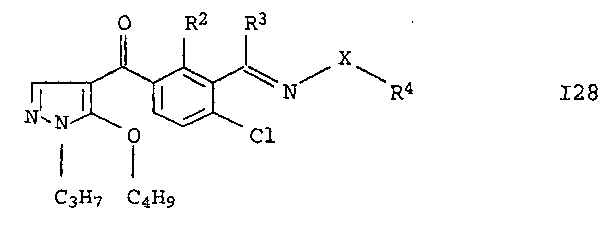
- die Verbindungen I29, insbesondere die Verbindungen I29.001-I29.180, die sich von den entsprechenden Verbindungen I1.001-I1.180 dadurch unterscheiden, daß R⁹ und R¹⁰ für n-Butyl stehen: 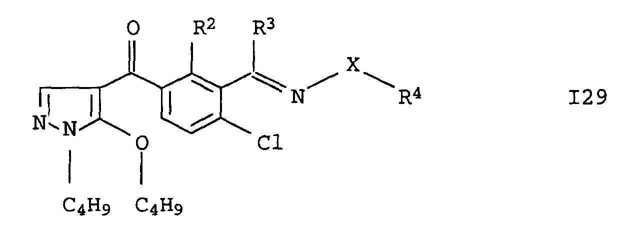
- die Verbindungen I30, insbesondere die Verbindungen I30.001-I30.180, die sich von den entsprechenden Verbindungen I1.001-I1.180 dadurch unterscheiden, daß R⁹ für iso-Butyl und R¹⁰ für n-Butyl stehen: 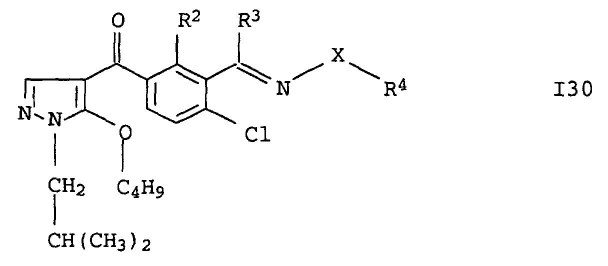
- die Verbindungen I31, insbesondere die Verbindungen I31.001-I31.180, die sich von den entsprechenden Verbindungen I1.001-I1.180 dadurch unterscheiden, daß R¹⁰ für sec-Butyl steht: 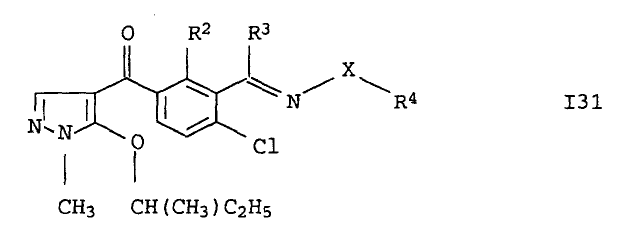
- die Verbindungen I32, insbesondere die Verbindungen I32.001-I32.180, die sich von den entsprechenden Verbindungen I1.001-I1.180 dadurch unterscheiden, daß R⁹ für Ethyl und R¹⁰ sec-Butyl für stehen: 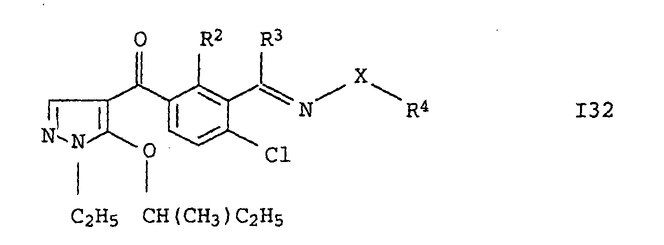
- die Verbindungen I33, insbesondere die Verbindungen I33.001-I33.180, die sich von den entsprechenden Verbindungen I1.001-I1.180 dadurch unterscheiden, daß R⁹ für n-Propyl und R¹⁰ für sec-Butyl stehen: 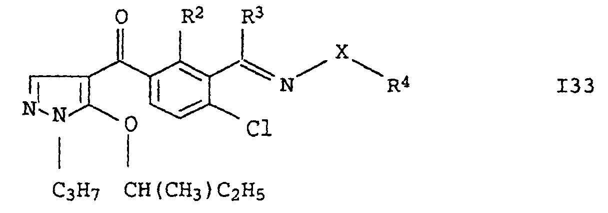
- die Verbindungen I34, insbesondere die Verbindungen I34.001-I34.180, die sich von den entsprechenden Verbindungen I1.001-I1.180 dadurch unterscheiden, daß R⁹ für n-Butyl und R¹⁰ für sec-Butyl stehen: 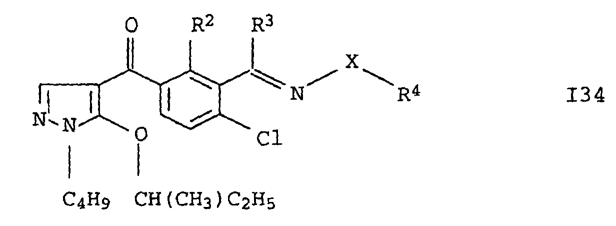
- die Verbindungen I35, insbesondere die Verbindungen I35.001-I35.180, die sich von den entsprechenden Verbindungen I1.001-I1.180 dadurch unterscheiden, daß R⁹ für iso-Butyl und R¹⁰ für sec-Butyl stehen: 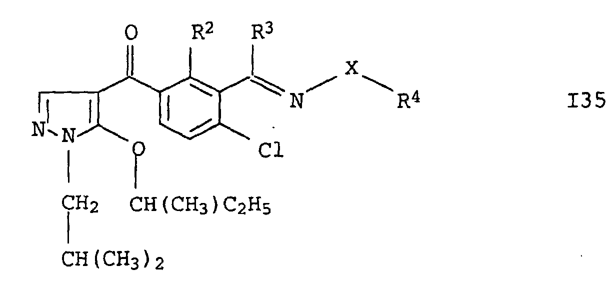
- die Verbindungen I36, insbesondere die Verbindungen I36.001-I36.180, die sich von den entsprechenden Verbindungen I1.001-I1.180 dadurch unterscheiden, daß R¹⁰ für iso-Butyl steht: 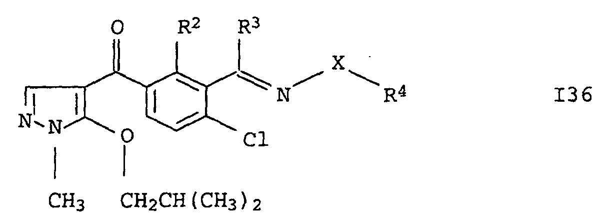
- die Verbindungen I37, insbesondere die Verbindungen I37.001-I37.180, die sich von den entsprechenden Verbindungen I1.001-I1.180 dadurch unterscheiden, daß R⁹ für Ethyl und R¹⁰ für iso-Butyl stehen: 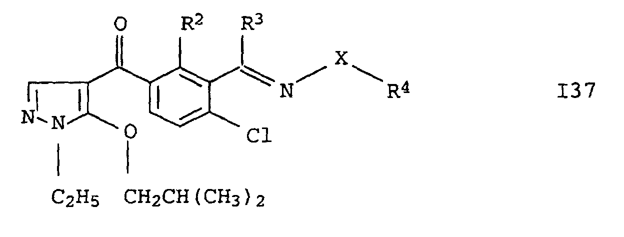
- die Verbindungen I38, insbesondere die Verbindungen I38.001-I38.180, die sich von den entsprechenden Verbindungen I1.001-I1.180 dadurch unterscheiden, daß R⁹ für n-Propyl und R¹⁰ für iso-Butyl stehen: 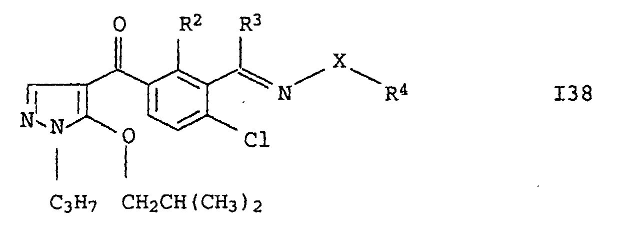
- die Verbindungen I39, insbesondere die Verbindungen I39.001-I39.180, die sich von den entsprechenden Verbindungen I1.001-I1.180 dadurch unterscheiden, daß R⁹ für n-Butyl und R¹⁰ für iso-Butyl stehen: 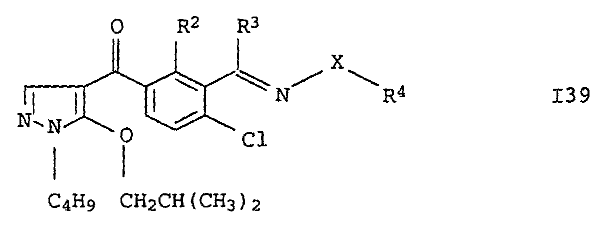
- die Verbindungen I40, insbesondere die Verbindungen I40.001-I40.180, die sich von den entsprechenden Verbindungen I1.001-I1.180 dadurch unterscheiden, daß R⁹ und R¹⁰ für iso-Butyl stehen: 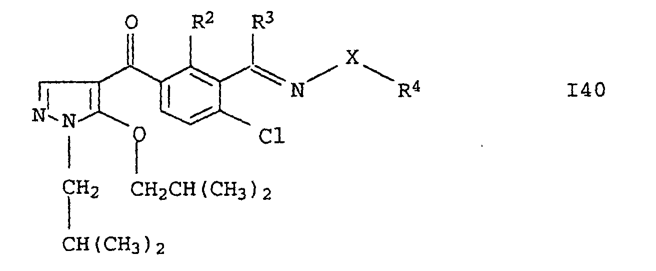
- die Verbindungen I41, insbesondere die Verbindungen I41.001-I41.180, die sich von den entsprechenden Verbindungen I1.001-I1.180 dadurch unterscheiden, daß R¹⁰ für Methylcarbonyl steht: 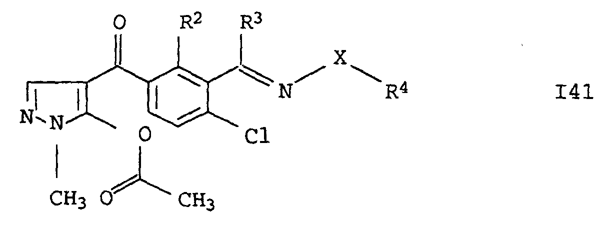
- die Verbindungen I42, insbesondere die Verbindungen I42.001-I42.180, die sich von den entsprechenden Verbindungen I1.001-I1.180 dadurch unterscheiden, daß R⁹ für Ethyl und für R¹⁰ Methylcarbonyl stehen: 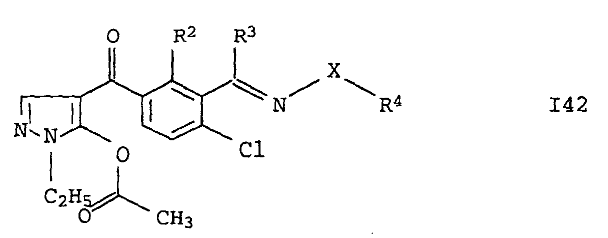
- die Verbindungen I43, insbesondere die Verbindungen I43.001-I43.180, die sich von den entsprechenden Verbindungen I1.001-I1.180 dadurch unterscheiden, daß R⁹ für Propyl und R¹⁰ für Methylcarbonyl stehen: 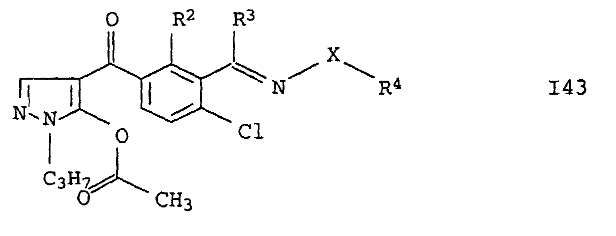
- die Verbindungen I44, insbesondere die Verbindungen I44.001-I44.180, die sich von den entsprechenden Verbindungen I1.001-I1.180 dadurch unterscheiden, daß R⁹ für n-Butyl und R¹⁰ für Methylcarbonyl stehen: 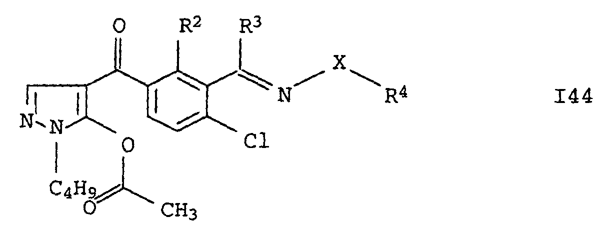
- die Verbindungen I45, insbesondere die Verbindungen I45.001-I45.180, die sich von den entsprechenden Verbindungen I1.001-I1.180 dadurch unterscheiden, daß R⁹ für iso-Butyl und R¹⁰ für Methylcarbonyl stehen: 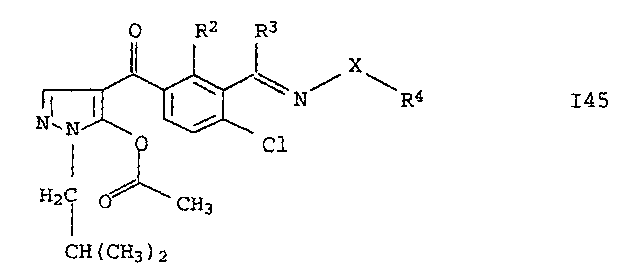
- die Verbindungen I46, insbesondere die Verbindungen I46.001-I46.180, die sich von den entsprechenden Verbindungen I1.001-I1.180 dadurch unterscheiden, daß R¹⁰ für Ethylcarbonyl steht: 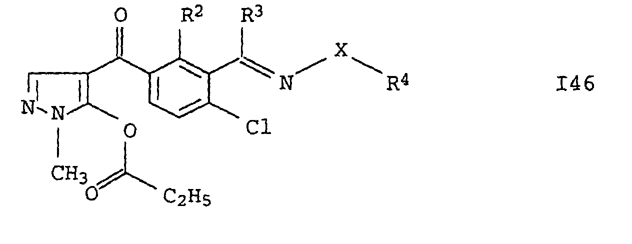
- die Verbindungen I47, insbesondere die Verbindungen I47.001-I47.180, die sich von den entsprechenden Verbindungen I1.001-I1.180 dadurch unterscheiden, daß R⁹ für Ethyl und R¹⁰ für Ethylcarbonyl stehen: 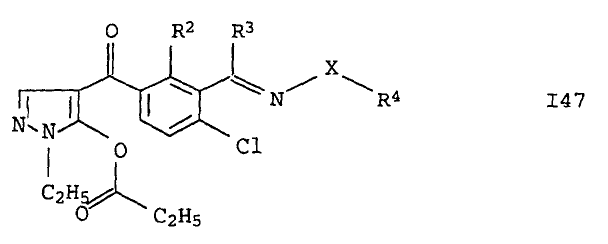
- die Verbindungen I48, insbesondere die Verbindungen I48.001-I48.180, die sich von den entsprechenden Verbindungen I1.001-I1.180 dadurch unterscheiden, daß R⁹ für Propyl und R¹⁰ für Ethylcarbonyl stehen: 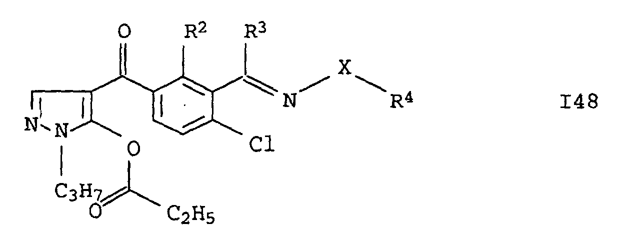
- die Verbindungen I49, insbesondere die Verbindungen I49.001-I49.180, die sich von den entsprechenden Verbindungen I1.001-I1.180 dadurch unterscheiden, daß R⁹ für n-Butyl und R¹⁰ für Ethylcarbonyl stehen: 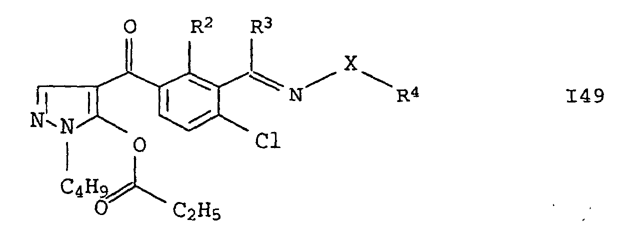
- die Verbindungen I50, insbesondere die Verbindungen I50.001-I50.180, die sich von den entsprechenden Verbindungen I1.001-I1.180 dadurch unterscheiden, daß R⁹ für iso-Butyl und R¹⁰ für Ethylcarbonyl stehen: 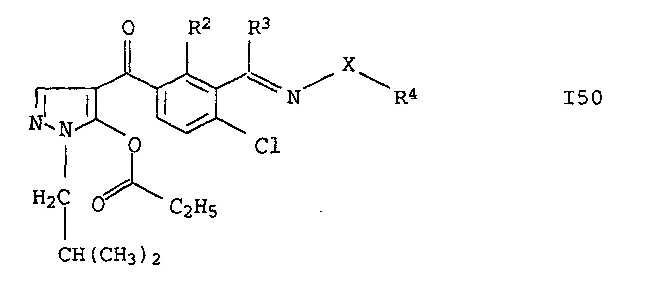
- die Verbindungen I51, insbesondere die Verbindungen I51.001-I51.180, die sich von den entsprechenden Verbindungen I1.001-I1.180 dadurch unterscheiden, daß R¹⁰ für n-Propylcarbonyl steht: 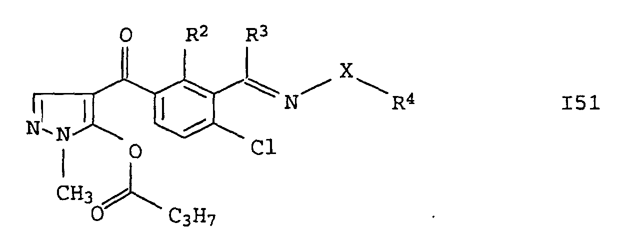
- die Verbindungen I52, insbesondere die Verbindungen I52.001-I52.180, die sich von den entsprechenden Verbindungen I1.001-I1.180 dadurch unterscheiden, daß R⁹ für Ethyl und R¹⁰ für n-Propylcarbonyl stehen: 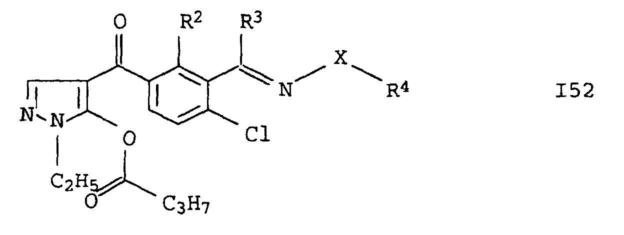
- die Verbindungen I53, insbesondere die Verbindungen I53.001-I53.180, die sich von den entsprechenden Verbindungen I1.001-I1.180 dadurch unterscheiden, daß R⁹ für Propyl und R¹⁰ für n-Propylcarbonyl stehen: 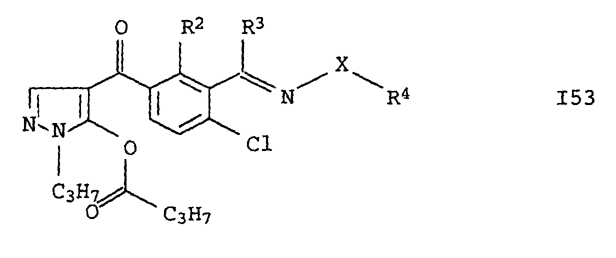
- die Verbindungen I54, insbesondere die Verbindungen I54.001-I54.180, die sich von den entsprechenden Verbindungen I1.001-I1.180 dadurch unterscheiden, daß R⁹ für n-Butyl und R¹⁰ für n-Propylcarbonyl stehen: 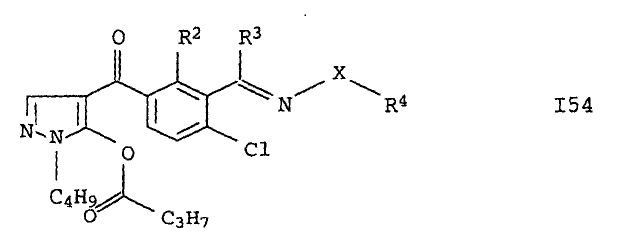
- die Verbindungen I55, insbesondere die Verbindungen I55.001-I55.180, die sich von den entsprechenden Verbindungen I1.001-I1.180 dadurch unterscheiden, daß R⁹ für iso-Butyl und R¹⁰ für n-Propylcarbonyl stehen: 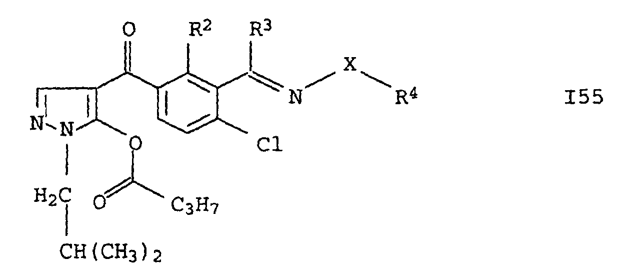
- die Verbindungen I56, insbesondere die Verbindungen I56.001-I56.180, die sich von den entsprechenden Verbindungen I1.001-I1.180 dadurch unterscheiden, daß R¹⁰ für Trifluormethylcarbonyl steht: 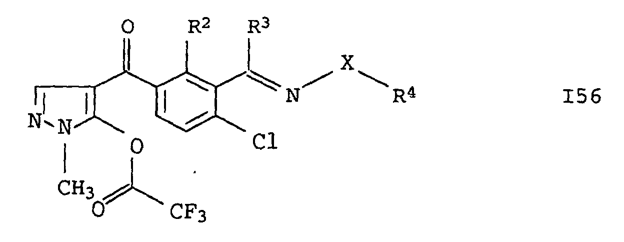
- die Verbindungen I57, insbesondere die Verbindungen I57.001-I57.180, die sich von den entsprechenden Verbindungen I1.001-I1.180 dadurch unterscheiden, daß R⁹ für Ethyl und für R¹⁰ Trifluormethylcarbonyl stehen: 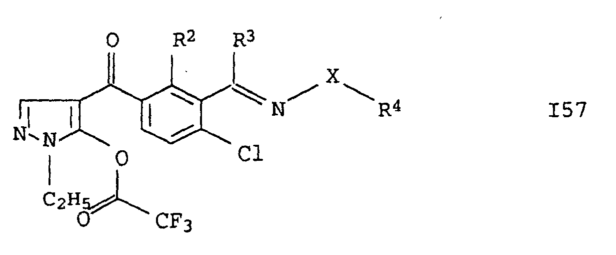
- die Verbindungen I58, insbesondere die Verbindungen I58.001-I58.180, die sich von den entsprechenden Verbindungen I1.001-I1.180 dadurch unterscheiden, daß R⁹ für n-Propyl und und R¹⁰ für Trifluormethylcarbonyl stehen: 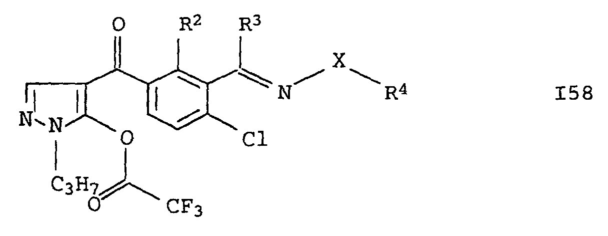
- die Verbindungen I59, insbesondere die Verbindungen I59.001-I59.180, die sich von den entsprechenden Verbindungen I1.001-I1.180 dadurch unterscheiden, daß R⁹ für n-Butyl und R¹⁰ für Trifluormethylcarbonyl stehen: 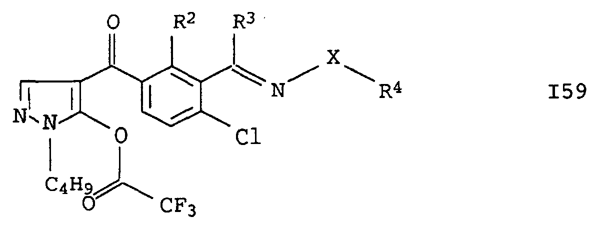
- die Verbindungen I60, insbesondere die Verbindungen I60.001-I60.180, die sich von den entsprechenden Verbindungen I1.001-I1.180 dadurch unterscheiden, daß R⁹ für iso-Butyl und R¹⁰ für Trifluormethylcarbonyl stehen: 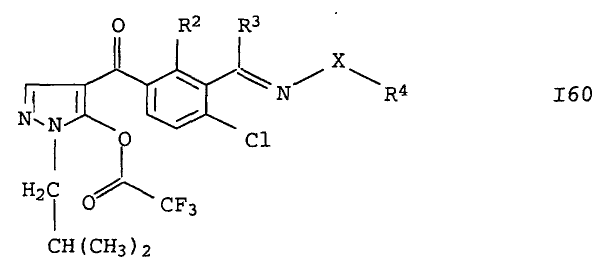
- die Verbindungen I61, insbesondere die Verbindungen I61.001-I61.180, die sich von den entsprechenden Verbindungen I1.001-I1.180 dadurch unterscheiden, daß R¹⁰ für Methylsulfonyl steht: 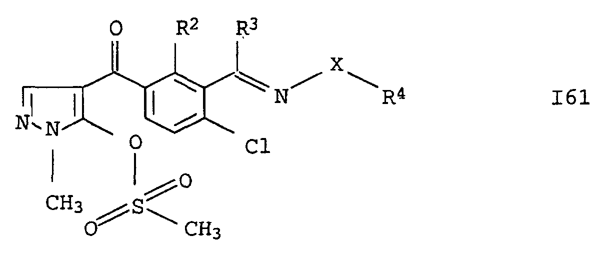
- die Verbindungen I62, insbesondere die Verbindungen I62.001-I62.180, die sich von den entsprechenden Verbindungen I1.001-I1.180 dadurch unterscheiden, daß R⁹ für Ethyl und für R¹⁰ Methylsulfonyl stehen: 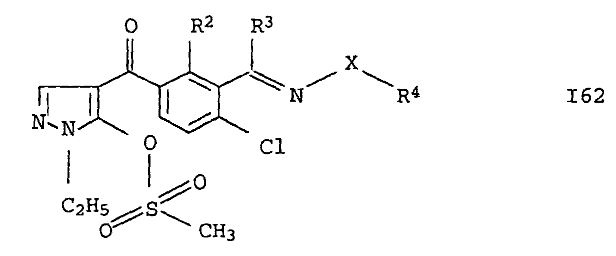
- die Verbindungen I63, insbesondere die Verbindungen I63.001-I63.180, die sich von den entsprechenden Verbindungen I1.001-I1.180 dadurch unterscheiden, daß R⁹ für n-Propyl und R¹⁰ für Methylsulfonyl stehen: 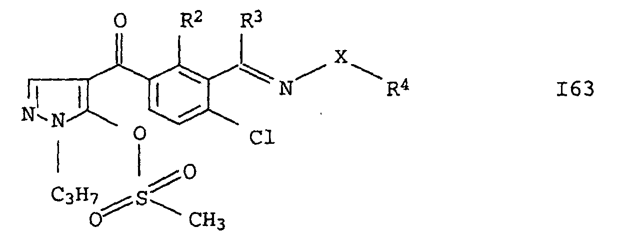
- die Verbindungen I64, insbesondere die Verbindungen I64.001-I64.180, die sich von den entsprechenden Verbindungen I1.001-I1.180 dadurch unterscheiden, daß R⁹ für n-Butyl und R¹⁰ für Methylsulfonyl stehen: 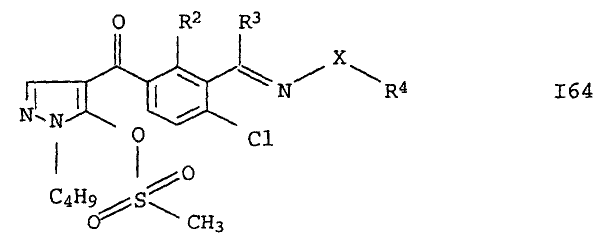
- die Verbindungen I65, insbesondere die Verbindungen I65.001-I65.180, die sich von den entsprechenden Verbindungen I1.001-I1.180 dadurch unterscheiden, daß R⁹ für iso-Butyl und R¹⁰ für Methylsulfonyl stehen: 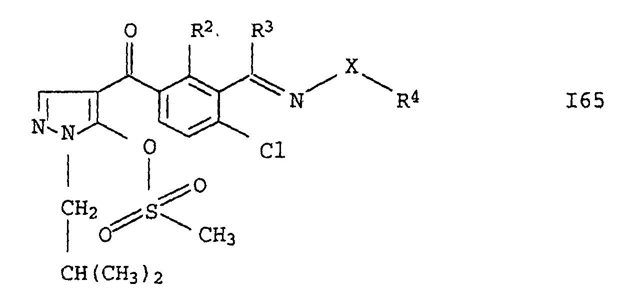
- die Verbindungen I66, insbesondere die Verbindungen I66.001-I66.180, die sich von den entsprechenden Verbindungen I1.001-I1.180 dadurch unterscheiden, daß R¹⁰ für Ethylsulfonyl steht: 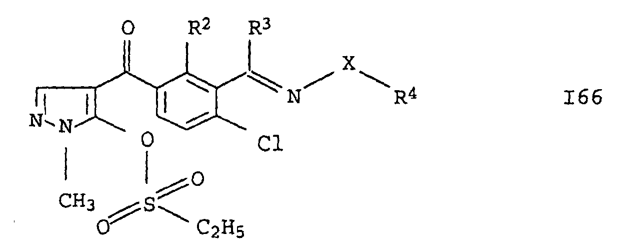
- die Verbindungen I67, insbesondere die Verbindungen I67.001-I67.180, die sich von den entsprechenden Verbindungen I1.001-I1.180 dadurch unterscheiden, daß R⁹ für Ethyl und für R¹⁰ Ethylsulfonyl stehen: 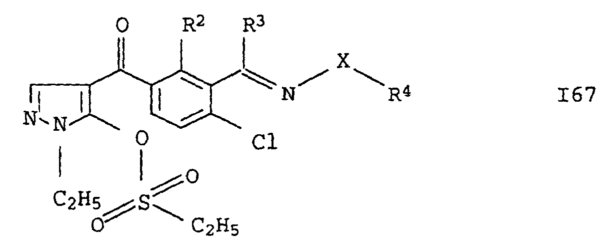
- die Verbindungen I68, insbesondere die Verbindungen I68.001-I68.180, die sich von den entsprechenden Verbindungen I1.001-I1.180 dadurch unterscheiden, daß R⁹ für Propyl und R¹⁰ für Ethylsulfonyl stehen: 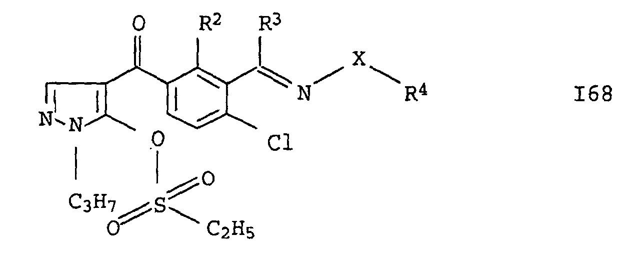
- die Verbindungen I69, insbesondere die Verbindungen I69.001-I69.180, die sich von den entsprechenden Verbindungen I1.001-I1.180 dadurch unterscheiden, daß R⁹ für n-Butyl und R¹⁰ für Ethylsulfonyl stehen: 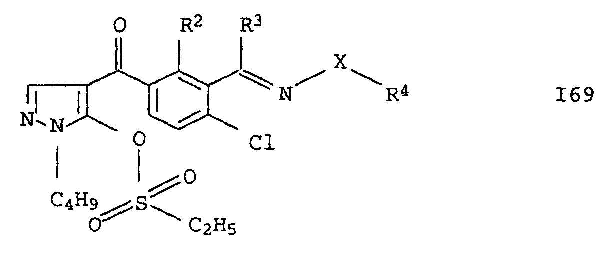
- die Verbindungen I70, insbesondere die Verbindungen I70.001-I70.180, die sich von den entsprechenden Verbindungen I1.001-I1.180 dadurch unterscheiden, daß R⁹ für iso-Butyl steht und R¹⁰ für Ethylsulfonyl stehen: 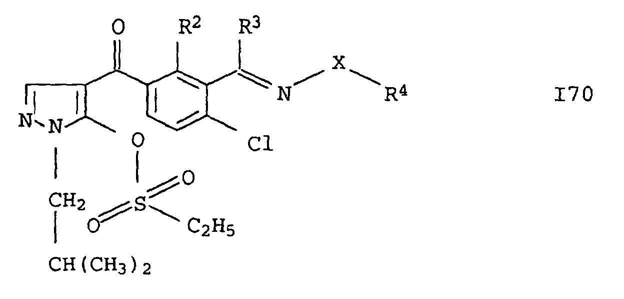
- die Verbindungen I71, insbesondere die Verbindungen I71.001-I71.180, die sich von den entsprechenden Verbindungen I1.001-I1.180 dadurch unterscheiden, daß R¹⁰ für n-Propylsulfonyl steht: 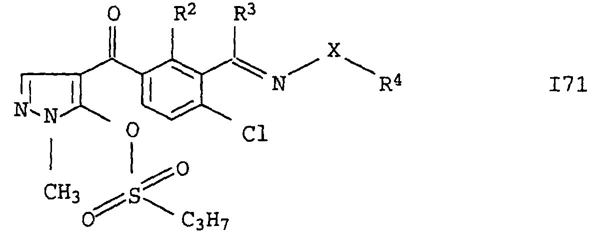
- die Verbindungen I72, insbesondere die Verbindungen I72.001-I72.180 die sich von den entsprechenden Verbindungen I1.001-I1.180 dadurch unterscheiden, daß R⁹ für Ethyl und für R¹⁰ n-Propylsulfonyl stehen: 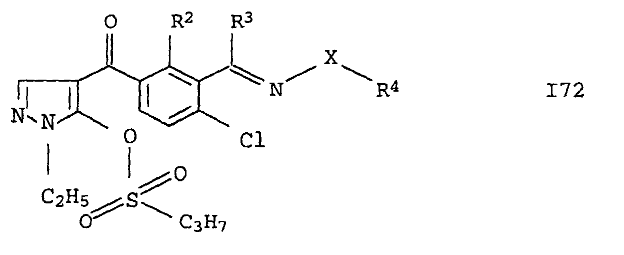
- die Verbindungen I73, insbesondere die Verbindungen I73.001-I73.180, die sich von den entsprechenden Verbindungen I1.001-I1.180 dadurch unterscheiden, daß R⁹ für n-Propyl und R¹⁰ für n-Propylsulfonyl stehen: 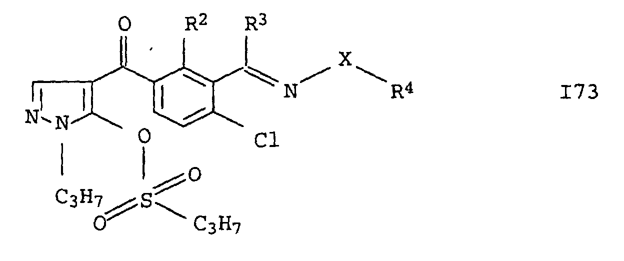
- die Verbindungen I74, insbesondere die Verbindungen I74.001-I74.180, die sich von den entsprechenden Verbindungen I1.001-I1.180 dadurch unterscheiden, daß R⁹ für n-Butyl und R¹⁰ für n-Propylsulfonyl stehen: 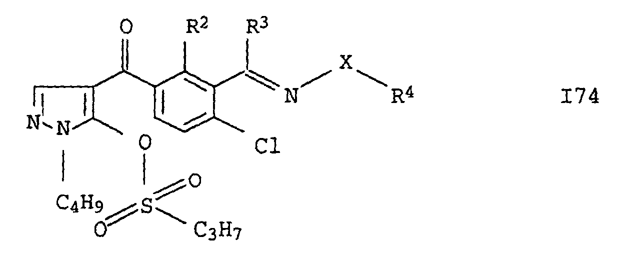
- die Verbindungen I75, insbesondere die Verbindungen I75.001-I75.180, die sich von den entsprechenden Verbindungen I1.001-I1.180 dadurch unterscheiden, daß R⁹ für iso-Butyl und R¹⁰ für n-Propylsulfonyl stehen: 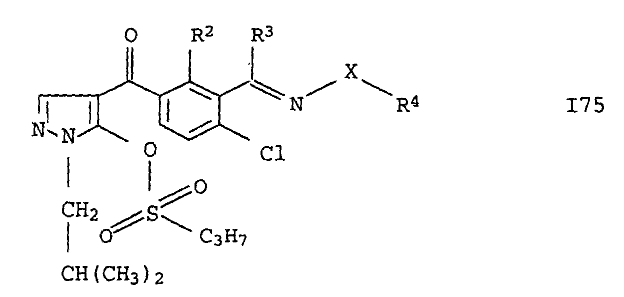
- die Verbindungen I76, insbesondere die Verbindungen I76.001-I76.180, die sich von den entsprechenden Verbindungen I1.001-I1.180 dadurch unterscheiden, daß R¹⁰ für iso-Propylsulfonyl steht: 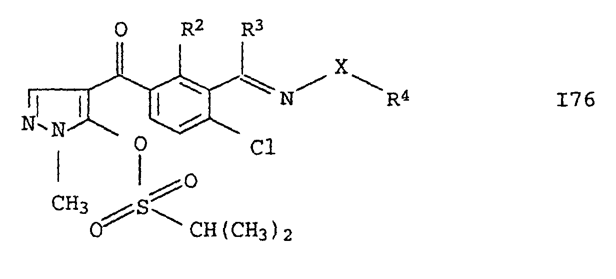
- die Verbindungen I77, insbesondere die Verbindungen I77.001-I77.180, die sich von den entsprechenden Verbindungen I1.001-I1.180 dadurch unterscheiden, daß R⁹ für Ethyl und für R¹⁰ iso-Propylsulfonyl stehen: 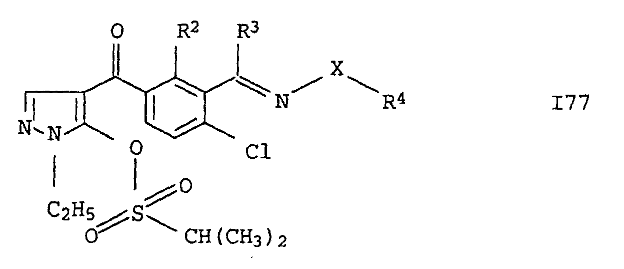
- die Verbindungen I78, insbesondere die Verbindungen I78.001-I78.180, die sich von den entsprechenden Verbindungen I1.001-I1.180 dadurch unterscheiden, daß R⁹ für Propyl und R¹⁰ für iso-Propylsulfonyl stehen: 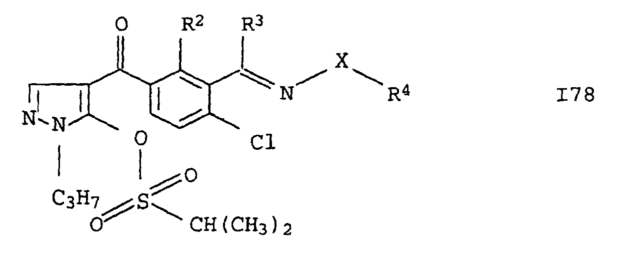
- die Verbindungen I79, insbesondere die Verbindungen I79.001-I79.180, die sich von den entsprechenden Verbindungen I1.001-I1.180 dadurch unterscheiden, daß R⁹ für n-Butyl und R¹⁰ für iso-Propylsulfonyl stehen: 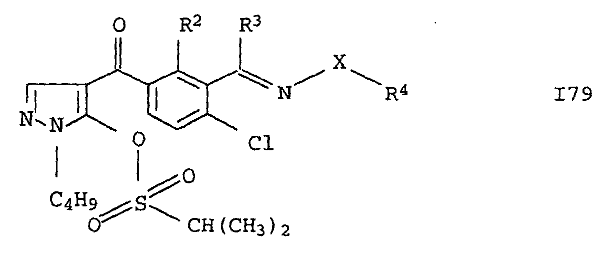
- die Verbindungen I80, insbesondere die Verbindungen I80.001-I80.180, die sich von den entsprechenden Verbindungen I1.001-I1.180 dadurch unterscheiden, daß R⁹ für iso-Butyl und R¹⁰ für iso-Propylsulfonyl stehen: 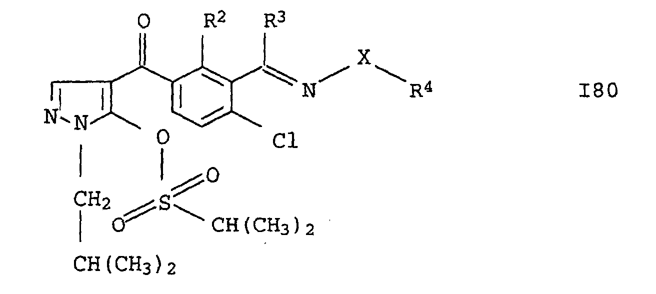
- die Verbindungen I81, insbesondere die Verbindungen I81.001-I81.180, die sich von den entsprechenden Verbindungen I1.001-I1.180 dadurch unterscheiden, daß R¹⁰ für n-Butylsulfonyl steht: 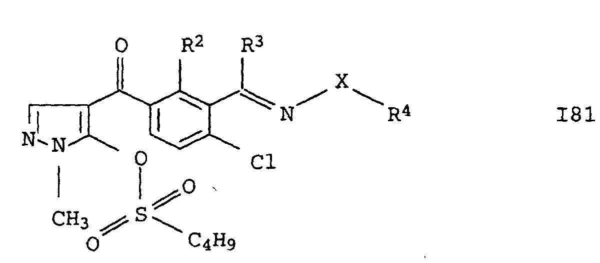
- die Verbindungen I82, insbesondere die Verbindungen I82.001-I82.180, die sich von den entsprechenden Verbindungen I1.001-I1.180 dadurch unterscheiden, daß R⁹ für Ethyl und für R¹⁰ n-Butylsulfonyl stehen: 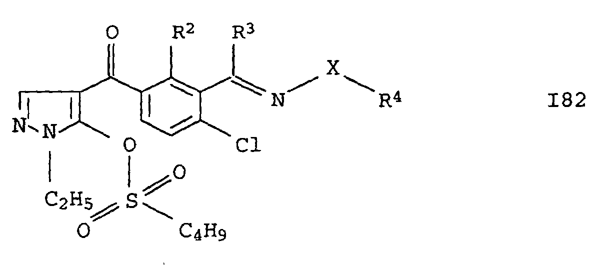
- die Verbindungen I83, insbesondere die Verbindungen I83.001-I83.180, die sich von den entsprechenden Verbindungen I1.001-I1.180 dadurch unterscheiden, daß R⁹ für n-Propyl und R¹⁰ für n-Butylsulfonyl stehen: 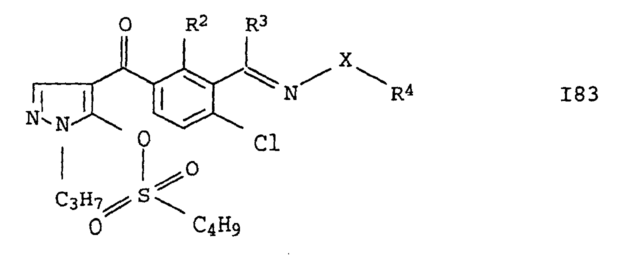
- die Verbindungen I84, insbesondere die Verbindungen I84.001-I84.180, die sich von den entsprechenden Verbindungen I1.001-I1.180 dadurch unterscheiden, daß R⁹ für n-Butyl und R¹⁰ für n-Butylsulfonyl stehen: 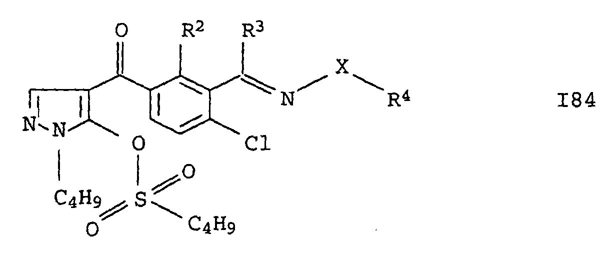
- die Verbindungen I85, insbesondere die Verbindungen I85.001-I85.180, die sich von den entsprechenden Verbindungen I1.001-I1.180 dadurch unterscheiden, daß R⁹ für iso-Butyl und R¹⁰ für n-Butylsulfonyl stehen: 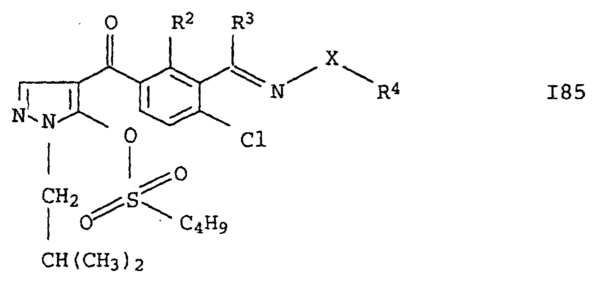
- die Verbindungen I86, insbesondere die Verbindungen I86.001-I86.180, die sich von den entsprechenden Verbindungen I1.001-I1.180 dadurch unterscheiden, daß R¹⁰ für iso-Butylsulfonyl steht: 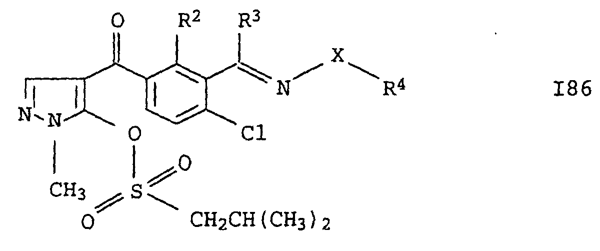
- die Verbindungen I87, insbesondere die Verbindungen I87.001-I87.180, die sich von den entsprechenden Verbindungen I1.001-I1.180 dadurch unterscheiden, daß R⁹ für Ethyl und für R¹⁰ iso-Butylsulfonyl stehen: 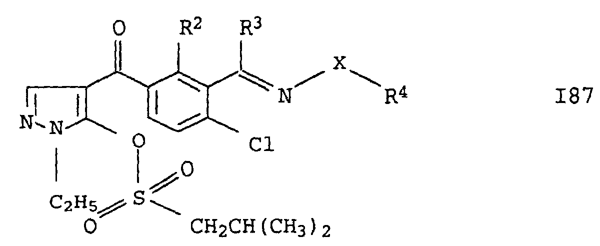
- die Verbindungen I88, insbesondere die Verbindungen I88.001-I88.180, die sich von den entsprechenden Verbindungen I1.001-I1.180 dadurch unterscheiden, daß R⁹ für n-Propyl und R¹⁰ für iso-Butylsulfonyl stehen: 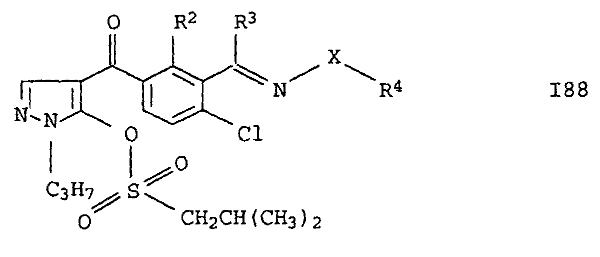
- die Verbindungen I89, insbesondere die Verbindungen I89.001-I89.180, die sich von den entsprechenden Verbindungen I1.001-I1.180 dadurch unterscheiden, daß R⁹ für n-Butyl und R¹⁰ für iso-Butylsulfonyl stehen: 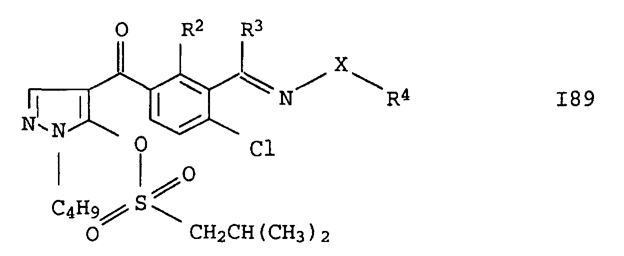
- die Verbindungen I90, insbesondere die Verbindungen I90.001-I90.180, die sich von den entsprechenden Verbindungen I1.001-I1.180 dadurch unterscheiden, daß R⁹ für iso-Butyl und R¹⁰ für iso-Butylsulfonyl stehen: 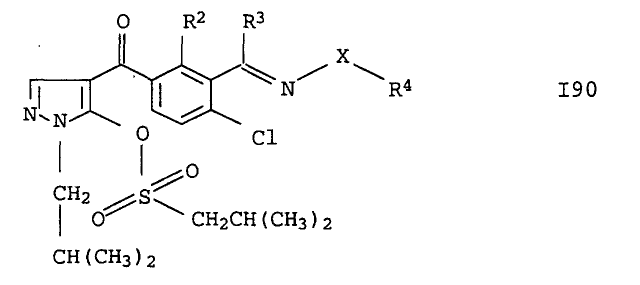
- die Verbindungen I91, insbesondere die Verbindungen I91.001-I91.180, die sich von den entsprechenden Verbindungen I1.001-I1.180 dadurch unterscheiden, daß R¹⁰ für sec-Butylsulfonyl steht: 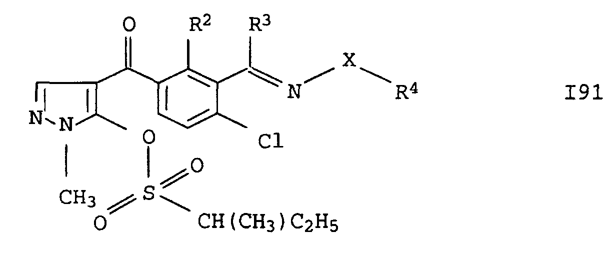
- die Verbindungen I92, insbesondere die Verbindungen I92.001-I92.180, die sich von den entsprechenden Verbindungen I1.001-I1.180 dadurch unterscheiden, daß R⁹ für Ethyl und für R¹⁰ sec-Butylsulfonyl stehen: 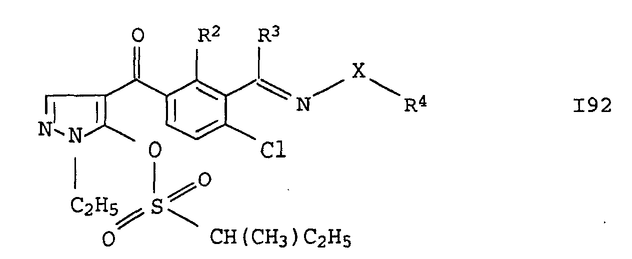
- die Verbindungen I93, insbesondere die Verbindungen I93.001-I93.180, die sich von den entsprechenden Verbindungen I1.001-I1.180 dadurch unterscheiden, daß R⁹ für n-Propyl und R¹⁰ für sec-Butylsulfonyl stehen: 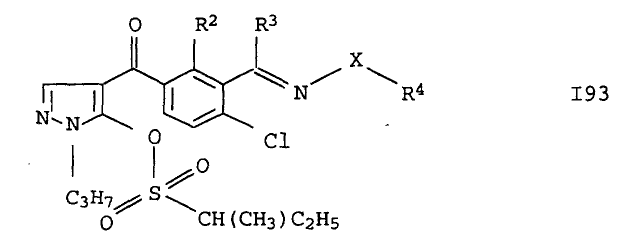
- die Verbindungen I94, insbesondere die Verbindungen I94.001-I94.180, die sich von den entsprechenden Verbindungen I1.001-I1.180 dadurch unterscheiden, daß R⁹ für n-Butyl und R¹⁰ für sec-Butylsulfonyl stehen: 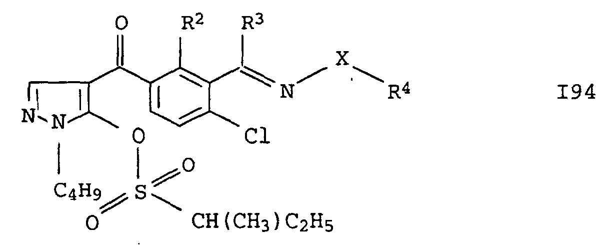
- die Verbindungen I95, insbesondere die Verbindungen I95.001-I95.180, die sich von den entsprechenden Verbindungen I1.001-I1.180 dadurch unterscheiden, daß R⁹ für iso-Butyl und R¹⁰ für sec-Butylsulfonyl stehen: 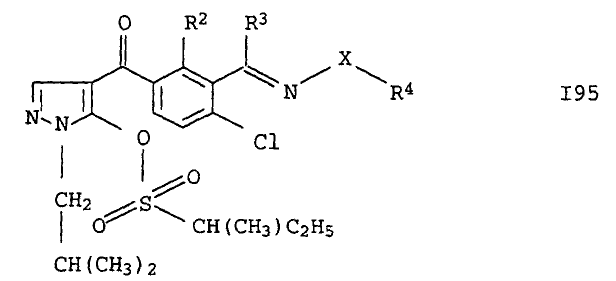
- die Verbindungen I96, insbesondere die Verbindungen I96.001-I96.180, die sich von den entsprechenden Verbindungen I1.001-I1.180 dadurch unterscheiden, daß R¹⁰ Trifluormethylsulfonyl stehen: 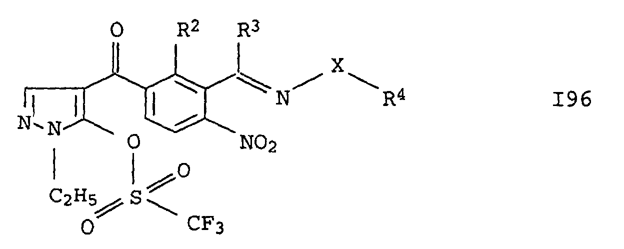
- die Verbindungen I97, insbesondere die Verbindungen I97.001-I97.180, die sich von den entsprechenden Verbindungen I1.001-I1.180 dadurch unterscheiden, daß R⁹ für Ethyl und R¹⁰ für Trifluormethylsulfonyl stehen: 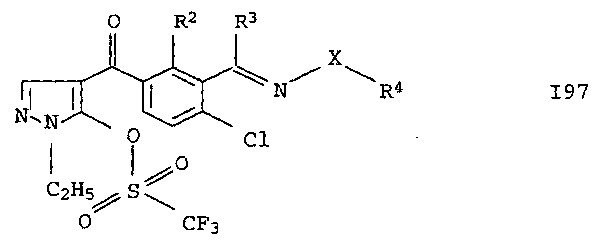
- die Verbindungen I98, insbesondere die Verbindungen I98.001-I98.180, die sich von den entsprechenden Verbindungen I1.001-I1.180 dadurch unterscheiden, daß R⁹ für n-Propyl und R¹⁰ für Trifluormethylsulfonyl stehen: 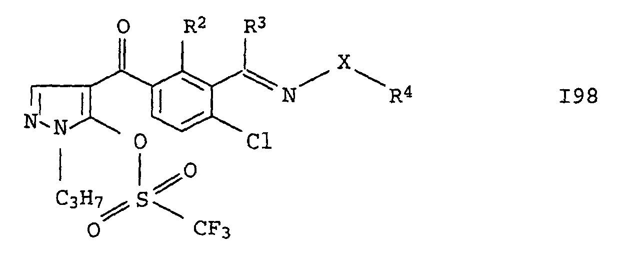
- die Verbindungen I99, insbesondere die Verbindungen I99.001-I99.180, die sich von den entsprechenden Verbindungen I1.001-I1.180 dadurch unterscheiden, daß R⁹ für n-Butyl und R¹⁰ für Trifluormethylsulfonyl stehen: 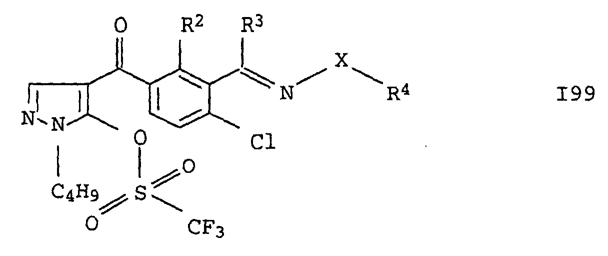
- die Verbindungen I100, insbesondere die Verbindungen I100.001-I100.180, die sich von den entsprechenden Verbindungen I1.001-I1.180 dadurch unterscheiden, daß R⁹ für iso-Butyl und R¹⁰ für Trifluormethylsulfonyl stehen: 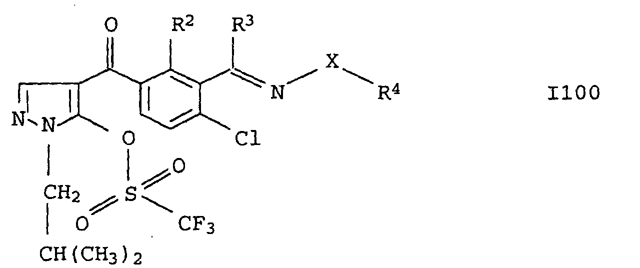
- die Verbindungen I101, insbesondere die Verbindungen I101.001-I101.180, die sich von den entsprechenden Verbindungen I1.001-I1.180 dadurch unterscheiden, daß R¹⁰ für Phenylcarbonylmethyl steht: 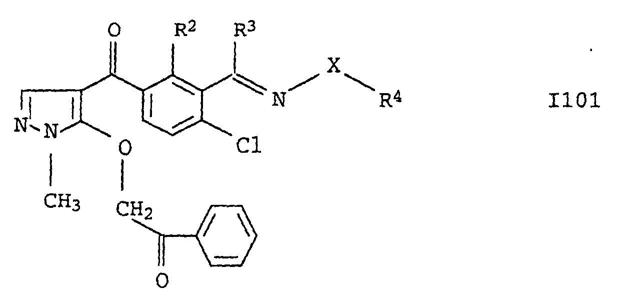
- die Verbindungen I102, insbesondere die Verbindungen I102.001-I102.180, die sich von den entsprechenden Verbindungen I1.001-I1.180 dadurch unterscheiden, daß R⁹ für Ethyl und R¹⁰ für Phenylcarbonylmethyl stehen: 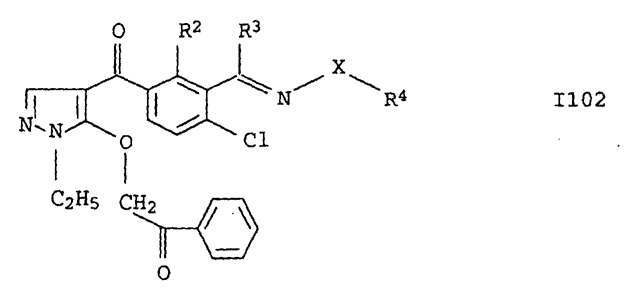
- die Verbindungen I103, insbesondere die Verbindungen I103.001-I103.180, die sich von den entsprechenden Verbindungen I1.001-I1.180 dadurch unterscheiden, daß R⁹ für n-Propyl und R¹⁰ für Phenylcarbonylmethyl stehen: 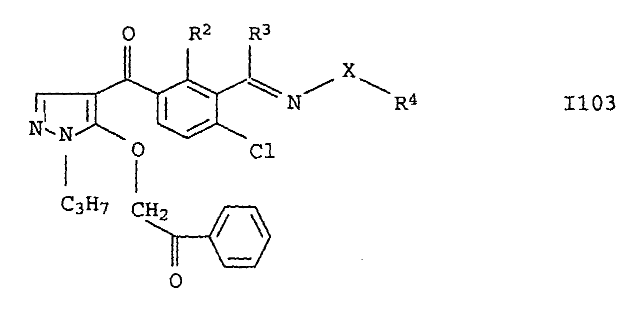
- die Verbindungen I104, insbesondere die Verbindungen I104.001-I104.180, die sich von den entsprechenden Verbindungen I1.001-I1.180 dadurch unterscheiden, daß R⁹ für n-Butyl und R¹⁰ für Phenylcarbonylmethyl stehen: 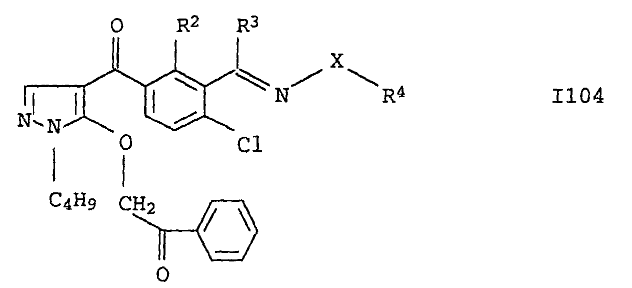
- die Verbindungen I105, insbesondere die Verbindungen I105.001-I105.180, die sich von den entsprechenden Verbindungen I1.001-I1.180 dadurch unterscheiden, daß R⁹ für iso-Butyl und R¹⁰ für Phenylcarbonylmethyl stehen: 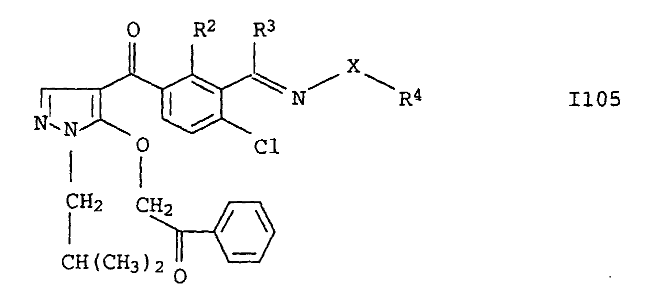
- die Verbindungen I106, insbesondere die Verbindungen I106.001-I106.180, die sich von den entsprechenden Verbindungen I1.001-I1.180 dadurch unterscheiden, daß R¹⁰ für Phenylsulfonyl steht: 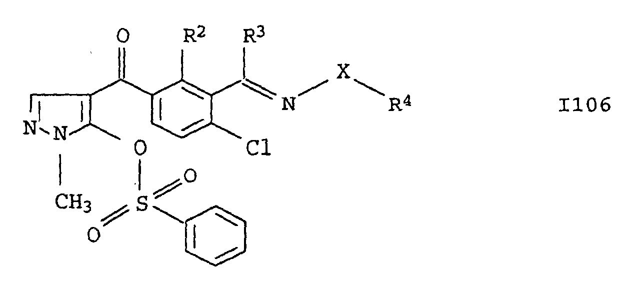
- die Verbindungen I107, insbesondere die Verbindungen I107.001-I107.180, die sich von den entsprechenden Verbindungen I1.001-I1.180 dadurch unterscheiden, daß R⁹ für Ethyl und R¹⁰ für Phenylsulfonyl stehen: 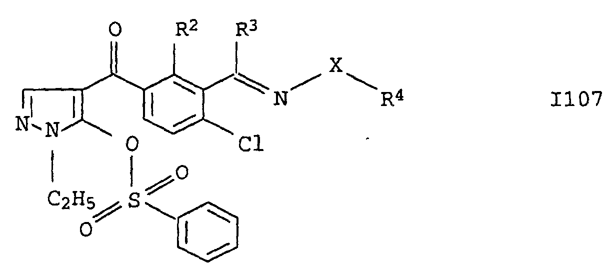
- die Verbindungen I108, insbesondere die Verbindungen I108.001-I108.180, die sich von den entsprechenden Verbindungen I1.001-I1.180 dadurch unterscheiden, daß R⁹ für n-Propyl und R¹⁰ für Phenylsulfonyl stehen: 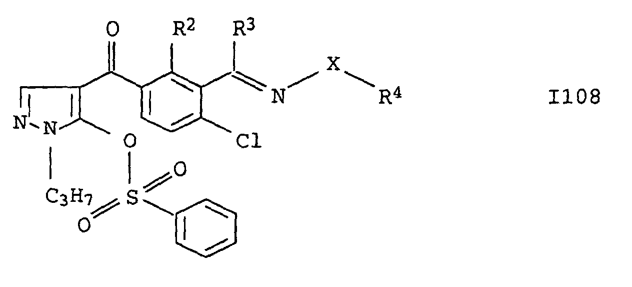
- die Verbindungen I109, insbesondere die Verbindungen I109.001-I109.180, die sich von den entsprechenden Verbindungen I1.001-I1.180 dadurch unterscheiden, daß R⁹ für n-Butyl und R¹⁰ für Phenylsulfonyl stehen: 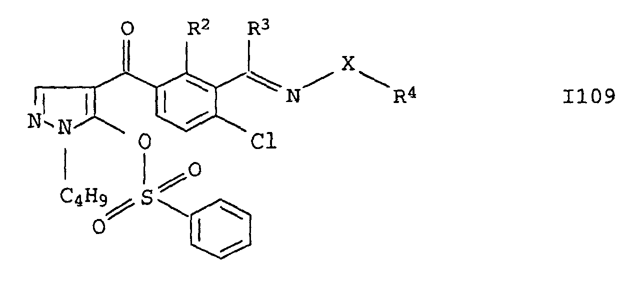
- die Verbindungen I110, insbesondere die Verbindungen I110.001-I110.180, die sich von den entsprechenden Verbindungen I1.001-I1.180 dadurch unterscheiden, daß R⁹ für iso-Butyl und R¹⁰ für Phenylsulfonyl stehen: 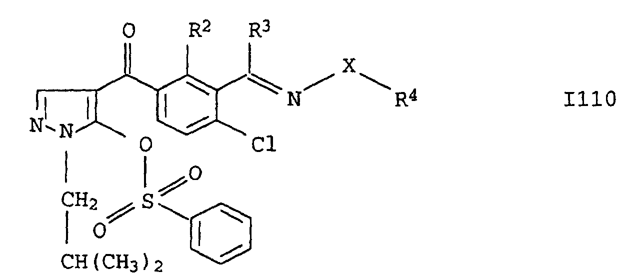
- die Verbindungen I111, insbesondere die Verbindungen I111.001-I111.180, die sich von den entsprechenden Verbindungen I1.001-I1.180 dadurch unterscheiden, daß R¹⁰ für 4-Methylphenylsulfonyl steht: 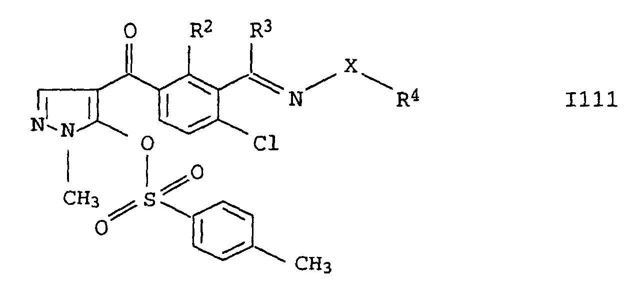
- die Verbindungen I112, insbesondere die Verbindungen I112.001-I112.180, die sich von den entsprechenden Verbindungen I1.001-I1.180 dadurch unterscheiden, daß R⁹ für Ethyl und R¹⁰ für 4-Methylphenylsulfonyl stehen: 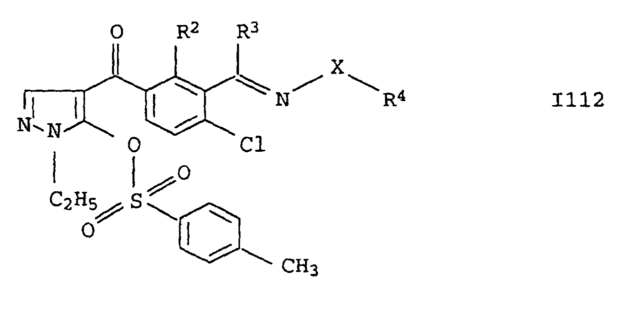
- die Verbindungen I113, insbesondere die Verbindungen I113.001-I113.180, die sich von den entsprechenden Verbindungen I1.001-I1.180 dadurch unterscheiden, daß R⁹ für n-Propyl und R¹⁰ für 4-Methylphenylsulfonyl stehen: 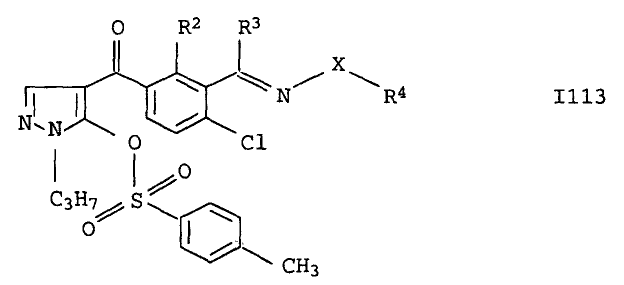
- die Verbindungen I114, insbesondere die Verbindungen I114.001-I114.180, die sich von den entsprechenden Verbindungen I1.001-I1.180 dadurch unterscheiden, daß R⁹ für n-Butyl und R¹⁰ für 4-Methylphenylsulfonyl stehen: 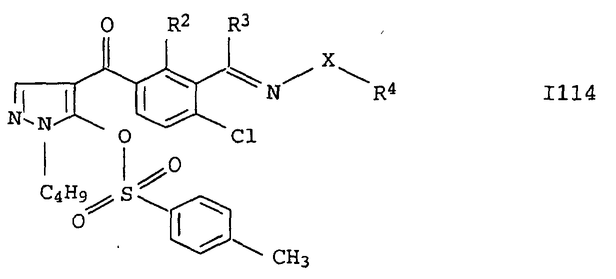
- die Verbindungen I115, insbesondere die Verbindungen I115.001-I115.180, die sich von den entsprechenden Verbindungen I1.001-I1.180 dadurch unterscheiden, daß R⁹ für iso-Butyl und R¹⁰ für 4-Methylphenylsulfonyl stehen: 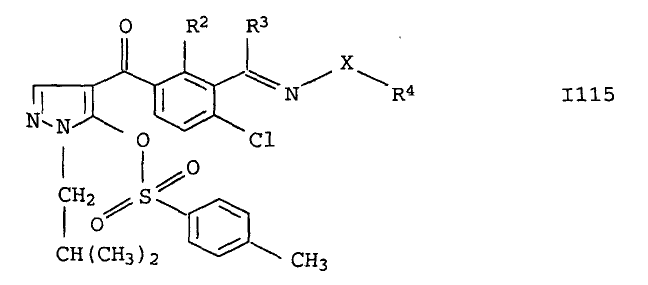
- die Verbindungen I116, insbesondere die Verbindungen I116.001-I116.180, die sich von den entsprechenden Verbindungen I1.001-I1.180 dadurch unterscheiden, daß R¹ für Nitro steht: 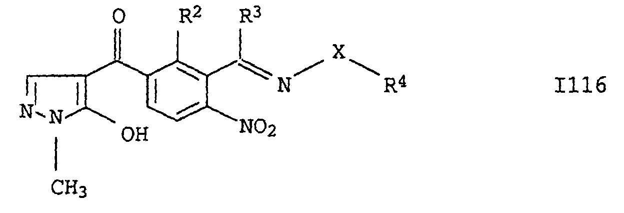
- die Verbindungen I117, insbesondere die Verbindungen I117.001-I117.180, die sich von den entsprechenden Verbindungen I1.001-I1.180 dadurch unterscheiden, daß R¹ für Nitro und R⁹ für Ethyl stehen: 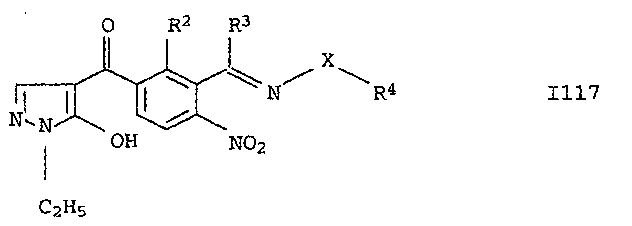
- die Verbindungen I118, insbesondere die Verbindungen I118.001-I118.180, die sich von den entsprechenden Verbindungen I1.001-I1.180 dadurch unterscheiden, daß R¹ für Nitro und R⁹ für Propyl stehen: 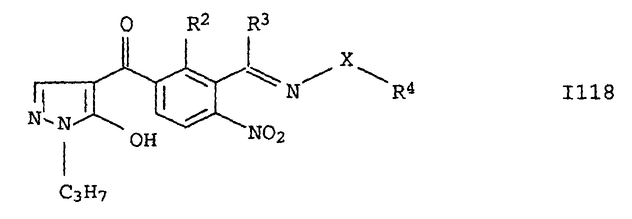
- die Verbindungen I119, insbesondere die Verbindungen I119.001-I119.180, die sich von den entsprechenden Verbindungen I1.001-I1.180 dadurch unterscheiden, daß R¹ für Nitro und R⁹ für n-Butyl stehen: 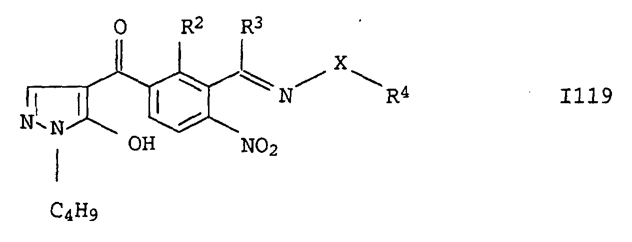
- die Verbindungen I120, insbesondere die Verbindungen I120.001-I120.180, die sich von den entsprechenden Verbindungen I1.001-I1.180 dadurch unterscheiden, daß R¹ für Nitro und R⁹ für iso-Butyl stehen: 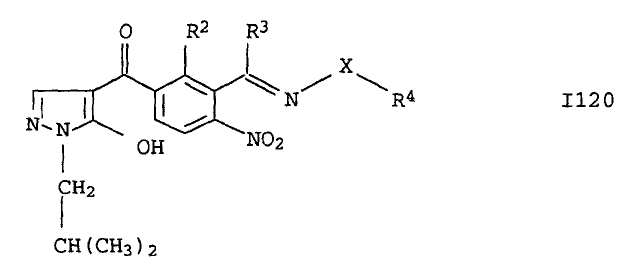
- die Verbindungen I121, insbesondere die Verbindungen I121.001-I121.180, die sich von den entsprechenden Verbindungen I1.001-I1.180 dadurch unterscheiden, daß R¹ für Nitro und R¹⁰ für Methyl stehen: 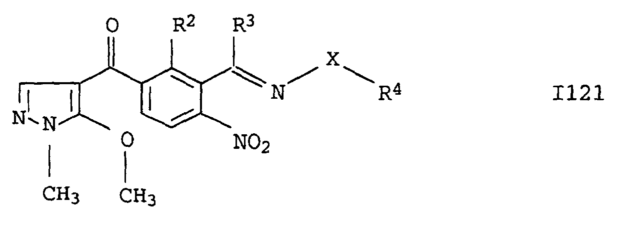
- die Verbindungen I122, insbesondere die Verbindungen I122.001-I122.180, die sich von den entsprechenden Verbindungen I1.001-I1.180 dadurch unterscheiden, daß R¹ für Nitro, R⁹ für Ethyl und R¹⁰ für Methyl stehen: 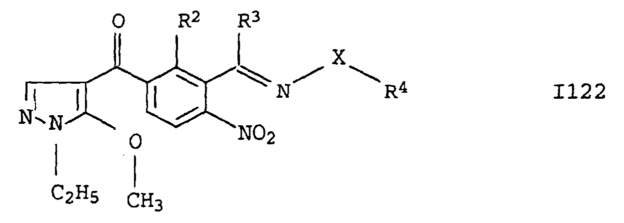
- die Verbindungen I123, insbesondere die Verbindungen I123.001-I123.180, die sich von den entsprechenden Verbindungen I1.001-I1.180 dadurch unterscheiden, daß R¹ für Nitro, R⁹ für n-Propyl und R¹⁰ für Methyl stehen: 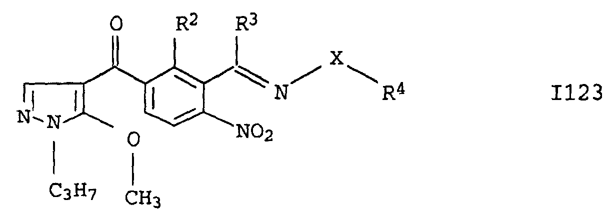
- die Verbindungen I124, insbesondere die Verbindungen I124.001-I124.180, die sich von den entsprechenden Verbindungen I1.001-I1.180 dadurch unterscheiden, daß R¹ für Nitro, R⁹ für n-Butyl und R¹⁰ für Methyl stehen: 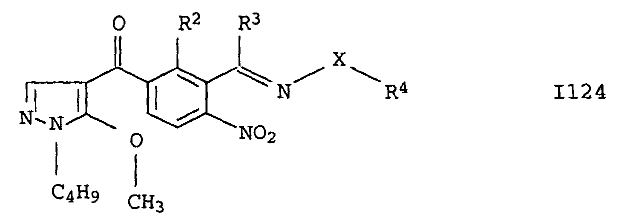
- die Verbindungen I125, insbesondere die Verbindungen I125.001-I125.180, die sich von den entsprechenden Verbindungen I1.001-I1.180 dadurch unterscheiden, daß R¹ für Nitro, R⁹ für iso-Butyl und R¹⁰ für Methyl stehen: 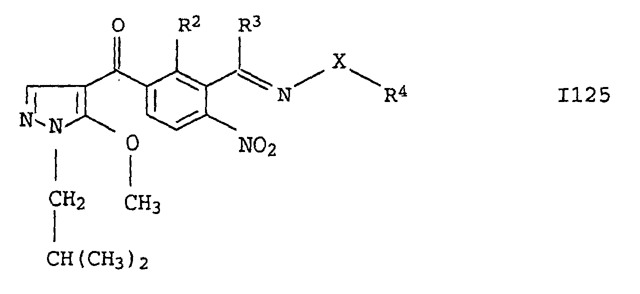
- die Verbindungen I126, insbesondere die Verbindungen I126.001-I126.180, die sich von den entsprechenden Verbindungen I1.001-I1.180 dadurch unterscheiden, daß R¹ für Nitro und R¹⁰ für Ethyl stehen: 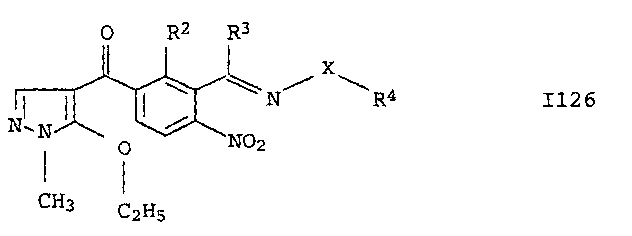
- die Verbindungen I127, insbesondere die Verbindungen I127.001-I127.180, die sich von den entsprechenden Verbindungen I1.001-I1.180 dadurch unterscheiden, daß R¹ für Nitro und R⁹ und R¹⁰ für Ethyl stehen: 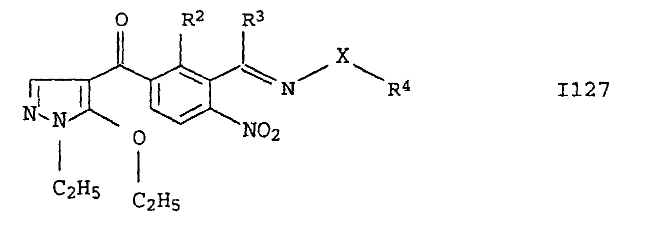
- die Verbindungen I128, insbesondere die Verbindungen I128.001-I128.180, die sich von den entsprechenden Verbindungen I1.001-I1.180 dadurch unterscheiden, daß R¹ für Nitro, R⁹ für n-Propyl und R¹⁰ für Ethyl stehen: 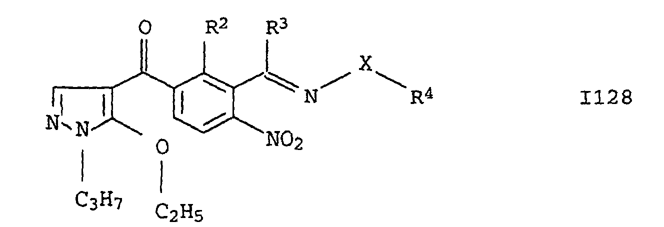
- die Verbindungen I129, insbesondere die Verbindungen I129.001-I129.180, die sich von den entsprechenden Verbindungen I1.001-I1.180 dadurch unterscheiden, daß R¹ für Nitro, R⁹ für n-Butyl und R¹⁰ für Ethyl stehen: 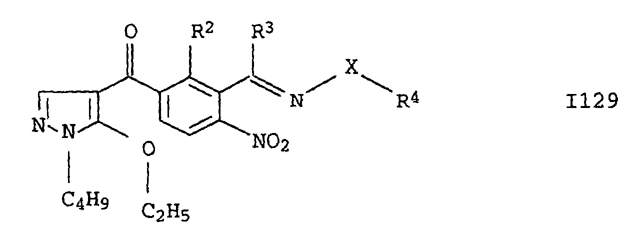
- die Verbindungen I130, insbesondere die Verbindungen I130.001-I130.180, die sich von den entsprechenden Verbindungen I1.001-I1.180 dadurch unterscheiden, daß R¹ Nitro, R⁹ für iso-Butyl und R¹⁰ für Ethyl stehen: 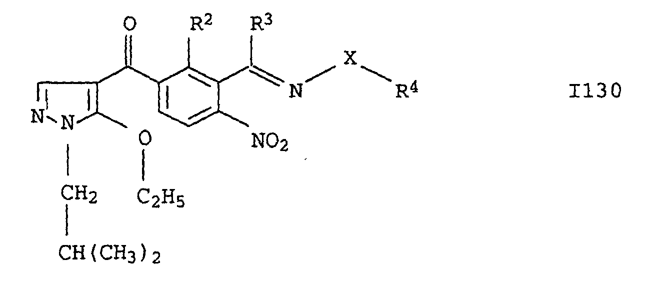
- die Verbindungen I131, insbesondere die Verbindungen I131.001-I131.180, die sich von den entsprechenden Verbindungen I1.001-I1.180 dadurch unterscheiden, daß R¹ für Nitro und R¹⁰ für n-Propyl stehen: 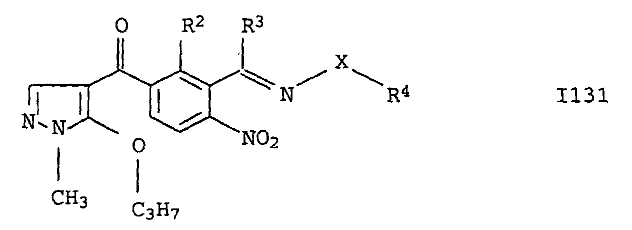
- die Verbindungen I132, insbesondere die Verbindungen I132.001-I132.180, die sich von den entsprechenden Verbindungen I1.001-I1.180 dadurch unterscheiden, daß R¹ für Nitro, R⁹ für Ethyl und R¹⁰ für n-Propyl stehen: 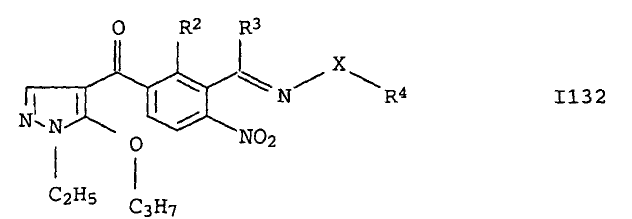
- die Verbindungen I133, insbesondere die Verbindungen I133.001-I133.180, die sich von den entsprechenden Verbindungen I1.001-I1.180 dadurch unterscheiden, daß R¹ für für Nitro, R⁹ und R¹⁰ für n-Propyl stehen: 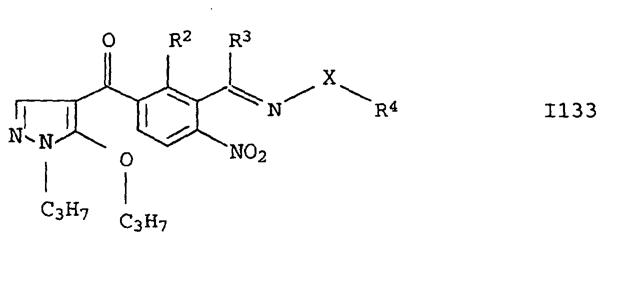
- die Verbindungen I134, insbesondere die Verbindungen I134.001-I134.180, die sich von den entsprechenden Verbindungen I1.001-I1.180 dadurch unterscheiden, daß R¹ Nitro, R⁹ für n-Butyl und R¹⁰ für n-Propyl stehen: 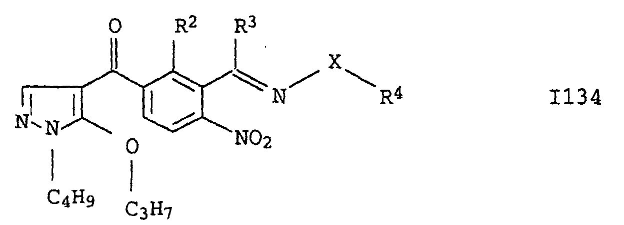
- die Verbindungen I135, insbesondere die Verbindungen I135.001-I135.180, die sich von den entsprechenden Verbindungen I1.001-I1.180 dadurch unterscheiden, daß R¹ Nitro, R⁹ für iso-Butyl und R¹⁰ für n-Propyl stehen: 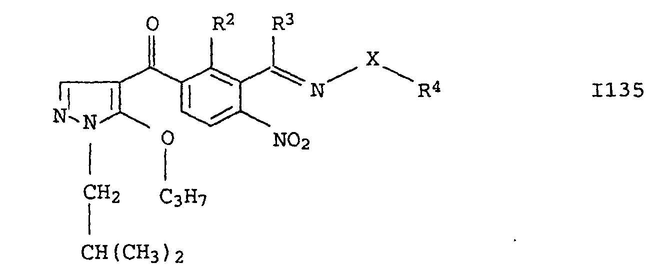
- die Verbindungen I136, insbesondere die Verbindungen I136.001-I136.180, die sich von den entsprechenden Verbindungen I1.001-I1.180 dadurch unterscheiden, daß R¹ für Nitro und R¹⁰ für iso-Propyl stehen: 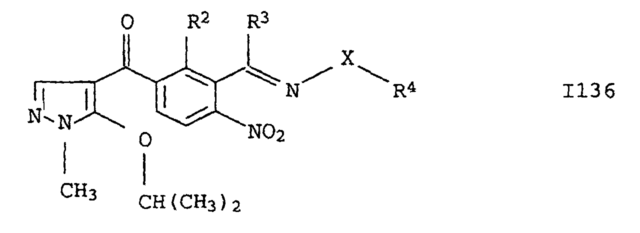
- die Verbindungen I137, insbesondere die Verbindungen I137.001-I137.180, die sich von den entsprechenden Verbindungen I1.001-I1.180 dadurch unterscheiden, daß R¹ für Nitro, R⁹ für Ethyl und R¹⁰ für iso-Propyl stehen: 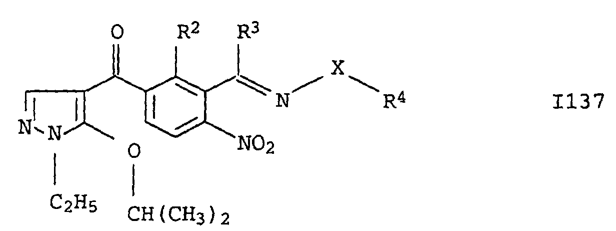
- die Verbindungen I138, insbesondere die Verbindungen I138.001-I138.180, die sich von den entsprechenden Verbindungen I1.001-I1.180 dadurch unterscheiden, daß R¹ für Nitro, R⁹ für n-Propyl und R¹⁰ für iso-Propyl stehen: 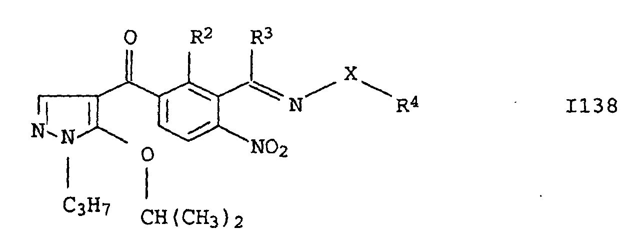
- die Verbindungen I139, insbesondere die Verbindungen I139.001-I139.180, die sich von den entsprechenden Verbindungen I1.001-I1.180 dadurch unterscheiden, daß R¹ Nitro, R⁹ für n-Butyl und R¹⁰ für iso-Propyl stehen: 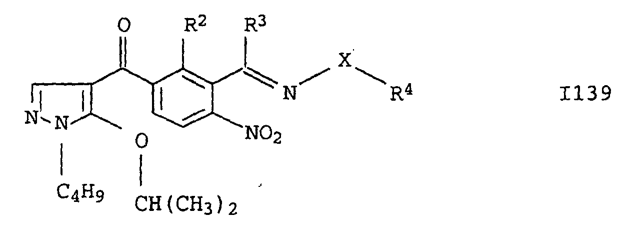
- die Verbindungen I140, insbesondere die Verbindungen I140.001-I140.180, die sich von den entsprechenden Verbindungen I1.001-I1.180 dadurch unterscheiden, daß R¹ für Nitro, R⁹ für iso-Butyl und R¹⁰ für iso-Propyl stehen: 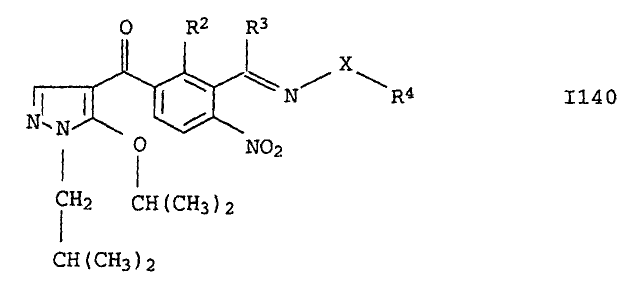
- die Verbindungen I141, insbesondere die Verbindungen I141.001-I141.180, die sich von den entsprechenden Verbindungen I1.001-I1.180 dadurch unterscheiden, daß R¹ für Nitro und R¹⁰ für n-Butyl stehen: 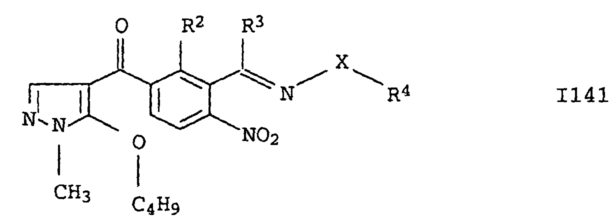
- die Verbindungen I142, insbesondere die Verbindungen I142.001-I142.180, die sich von den entsprechenden Verbindungen I1.001-I1.180 dadurch unterscheiden, daß R¹ für Nitro, R⁹ für Ethyl und R¹⁰ für n-Butyl stehen: 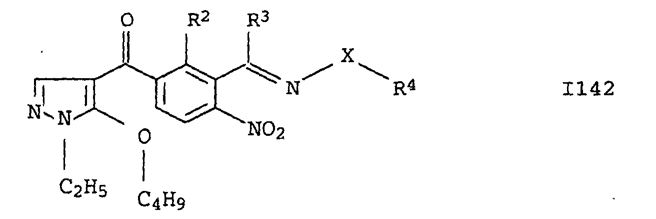
- die Verbindungen I143, insbesondere die Verbindungen I143.001-I143.180, die sich von den entsprechenden Verbindungen I1.001-I1.180 dadurch unterscheiden, daß R¹ für Nitro, R⁹ für n-Propyl und R¹⁰ für n-Butyl stehen: 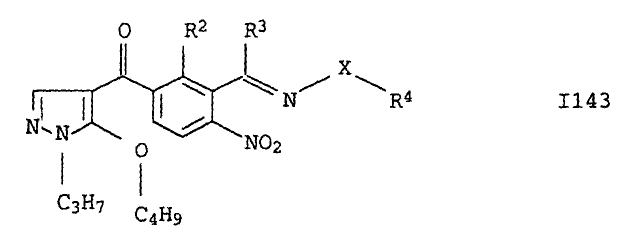
- die Verbindungen I144, insbesondere die Verbindungen I144.001-I144.180, die sich von den entsprechenden Verbindungen I1.001-I1.180 dadurch unterscheiden, daß R¹ für Nitro, R⁹ und R¹⁰ für n-Butyl stehen: 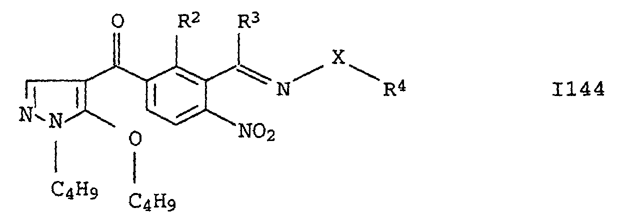
- die Verbindungen I145, insbesondere die Verbindungen I145.001-I145.180, die sich von den entsprechenden Verbindungen I1.001-I1.180 dadurch unterscheiden, daß R¹ für Nitro, R⁹ für iso-Butyl und R¹⁰ für n-Butyl stehen: 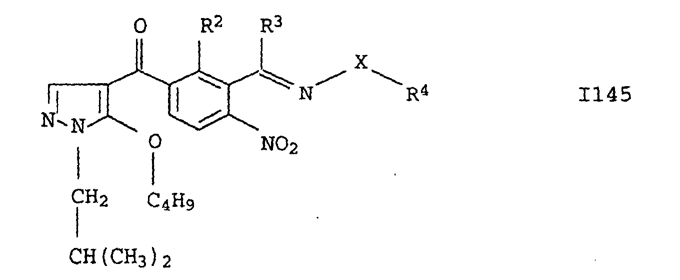
- die Verbindungen I146, insbesondere die Verbindungen I146.001-I146.180, die sich von den entsprechenden Verbindungen I1.001-I1.180 dadurch unterscheiden, daß R¹ für Nitro und R¹⁰ für sec-Butyl stehen: 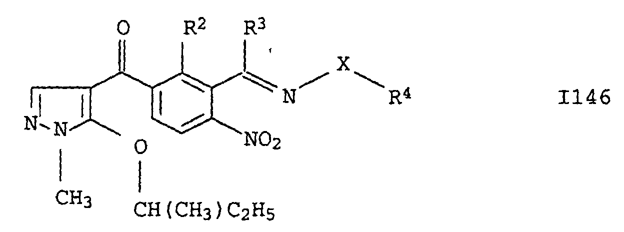
- die Verbindungen I147, insbesondere die Verbindungen I147.001-I147.180, die sich von den entsprechenden Verbindungen I1.001-I1.180 dadurch unterscheiden, daß R¹ für Nitro, R⁹ für Ethyl und R¹⁰ für sec-Butyl stehen: 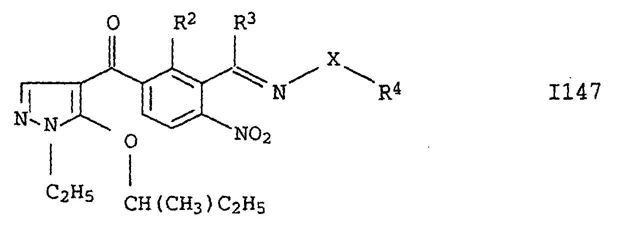
- die Verbindungen I148, insbesondere die Verbindungen I148.001-I148.180, die sich von den entsprechenden Verbindungen I1.001-I1.180 dadurch unterscheiden, daß R¹ für Nitro, R⁹ für n-Propyl und R¹⁰ für sec-Butyl stehen: 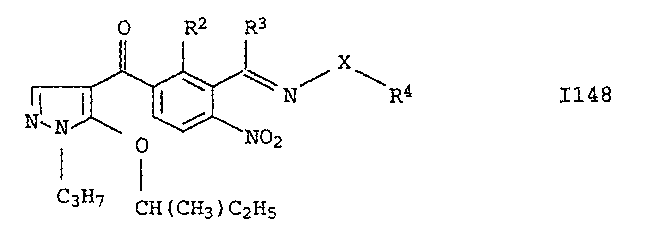
- die Verbindungen I149, insbesondere die Verbindungen I149.001-I149.180, die sich von den entsprechenden Verbindungen I1.001-I1.180 dadurch unterscheiden, daß R¹ für Nitro, R⁹ für n-Butyl und R¹⁰ für sec-Butyl stehen: 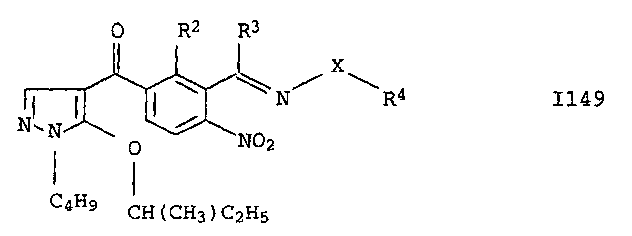
- die Verbindungen I150, insbesondere die Verbindungen I150.001-I150.180, die sich von den entsprechenden Verbindungen I1.001-I1.180 dadurch unterscheiden, daß R¹ für Nitro, R⁹ für iso-Butyl und R¹⁰ für sec-Butyl stehen: 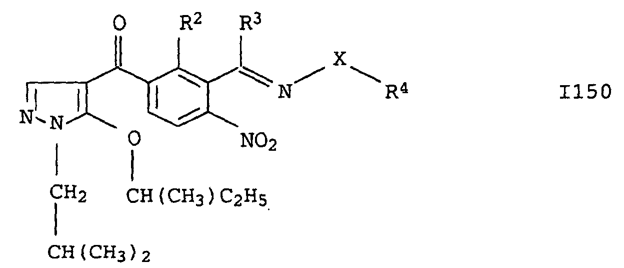
- die Verbindungen I151, insbesondere die Verbindungen I151.001-I151.180, die sich von den entsprechenden Verbindungen I1.001-I1.180 dadurch unterscheiden, daß R¹ für Nitro und R¹⁰ für iso-Butyl steht: 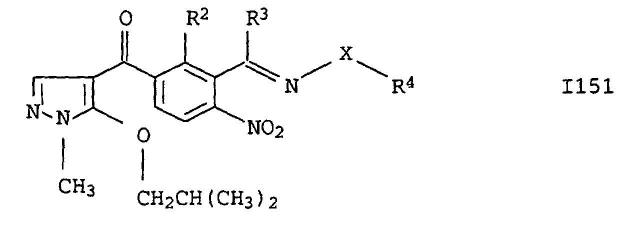
- die Verbindungen I152, insbesondere die Verbindungen I152.001-I152.180, die sich von den entsprechenden Verbindungen I1.001-I1.180 dadurch unterscheiden, daß R¹ für Nitro, R⁹ für Ethyl und R¹⁰ für iso-Butyl stehen: 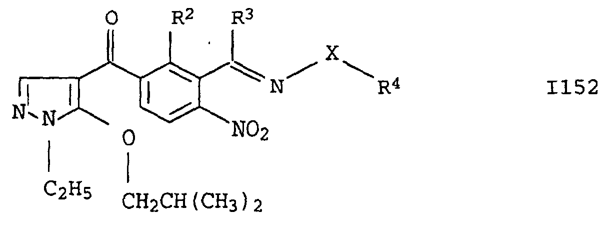
- die Verbindungen I153, insbesondere die Verbindungen I153.001-I153.180, die sich von den entsprechenden Verbindungen I1.001-I1.180 dadurch unterscheiden, daß R¹ für Nitro, R⁹ für n-Propyl und R¹⁰ für iso-Butyl stehen. 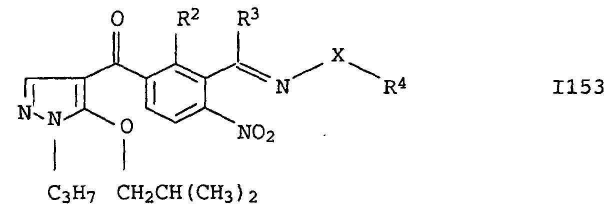
- die Verbindungen I154, insbesondere die Verbindungen I154.001-I154.180, die sich von den entsprechenden Verbindungen I1.001-I1.180 dadurch unterscheiden, daß R¹ für Nitro, R⁹ für n-Butyl und R¹⁰ für iso-Butyl stehen: 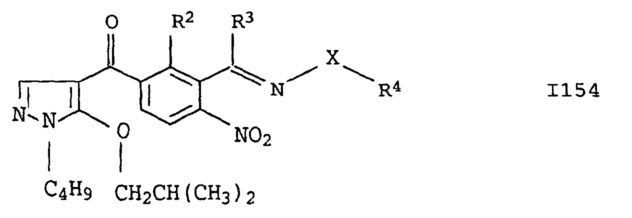
- die Verbindungen I155, insbesondere die Verbindungen I155.001-I154.180, die sich von den entsprechenden Verbindungen I1.001-I1.180 dadurch unterscheiden, daß R¹ für Nitro, R⁹ und R¹⁰ für iso-Butyl stehen: 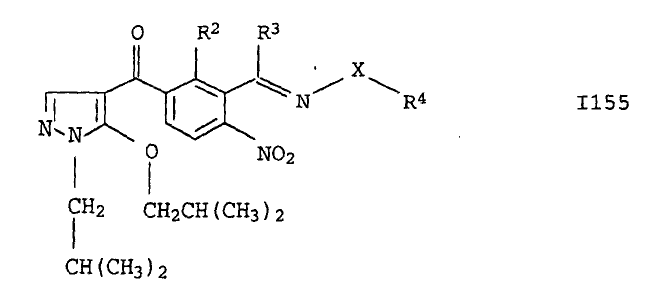
- die Verbindungen I156, insbesondere die Verbindungen I156.001-I156.180, die sich von den entsprechenden Verbindungen I1.001-I1.180 dadurch unterscheiden, daß R¹ für Nitro und R¹⁰ für Methylcarbonyl stehen: 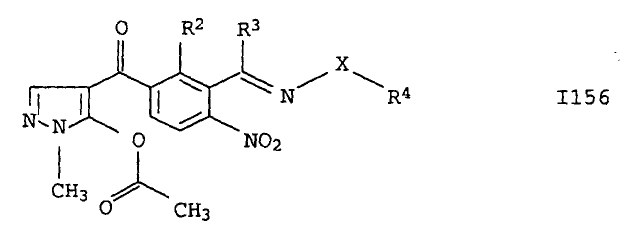
- die Verbindungen I157, insbesondere die Verbindungen I157.001-I157.180, die sich von den entsprechenden Verbindungen I1.001-I1.180 dadurch unterscheiden, daß R¹ für Nitro, R⁹ für Ethyl und R¹⁰ für Methylcarbonyl stehen: 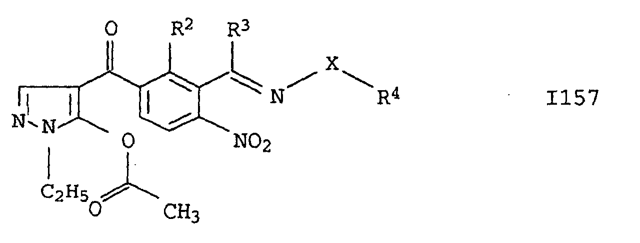
- die Verbindungen I158, insbesondere die Verbindungen I158.001-I158.180, die sich von den entsprechenden Verbindungen I1.001-I1.180 dadurch unterscheiden, daß R¹ für Nitro, R⁹ für n-Propyl und R¹⁰ für Methylcarbonyl stehen: 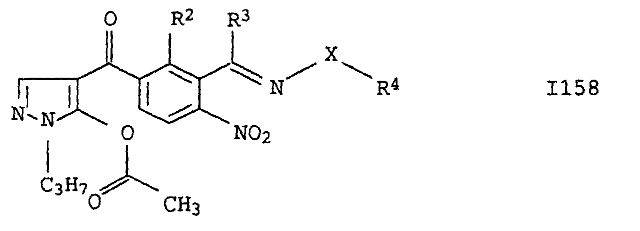
- die Verbindungen I159, insbesondere die Verbindungen I159.001-I159.180, die sich von den entsprechenden Verbindungen I1.001-I1.180 dadurch unterscheiden, daß R¹ für Nitro, R⁹ für n-Butyl und R¹⁰ für Methylcarbonyl stehen: 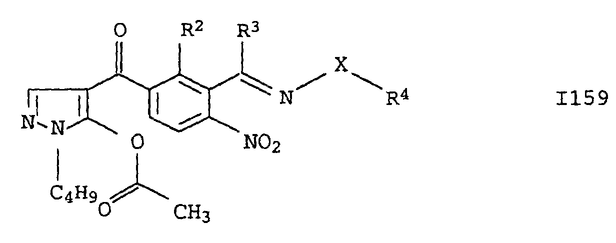
- die Verbindungen I160, insbesondere die Verbindungen I160.001-I160.180, die sich von den entsprechenden Verbindungen I1.001-I1.180 dadurch unterscheiden, daß R¹ für Nitro, R⁹ für iso-Butyl und R¹⁰ für Methylcarbonyl stehen: 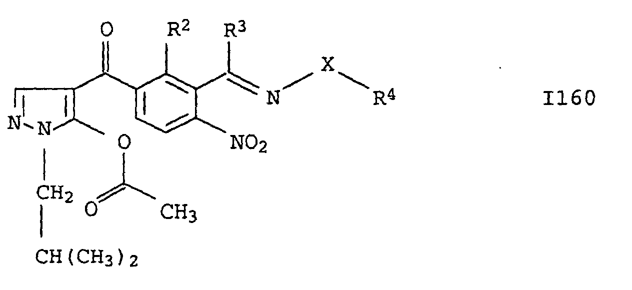
- die Verbindungen I161, insbesondere die Verbindungen I161.001-I161.180, die sich von den entsprechenden Verbindungen I1.001-I1.180 dadurch unterscheiden, daß R¹ für Nitro und R¹⁰ für Ethylcarbonyl stehen: 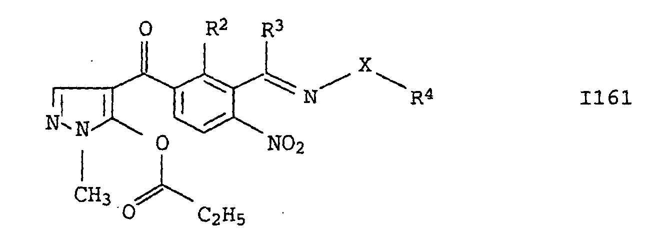
- die Verbindungen I162, insbesondere die Verbindungen I162.001-I162.180, die sich von den entsprechenden Verbindungen I1.001-I1.180 dadurch unterscheiden, daß R¹ Nitro, R⁹ für Ethyl und R¹⁰ für Ethylcarbonyl stehen: 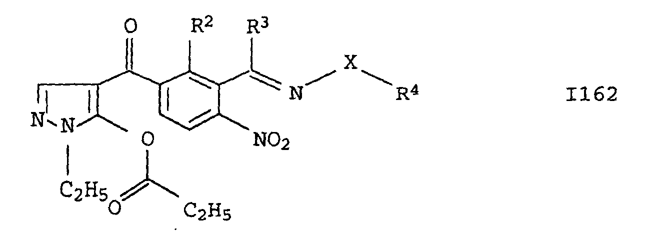
- die Verbindungen I163, insbesondere die Verbindungen I163.001-I163.180, die sich von den entsprechenden Verbindungen I1.001-I1.180 dadurch unterscheiden, daß R¹ für Nitro, R⁹ für n-Propyl und R¹⁰ für Ethylcarbonyl stehen: 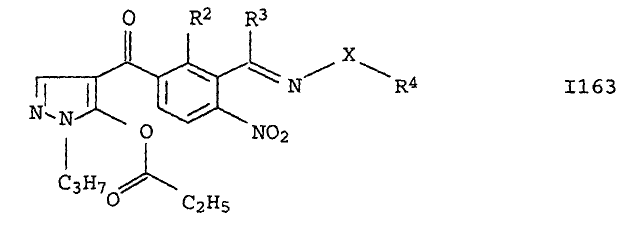
- die Verbindungen I164, insbesondere die Verbindungen I164.001-I164.180, die sich von den entsprechenden Verbindungen I1.001-I1.180 dadurch unterscheiden, daß R¹ Nitro, R⁹ für n-Butyl und R¹⁰ für Ethylcarbonyl stehen: 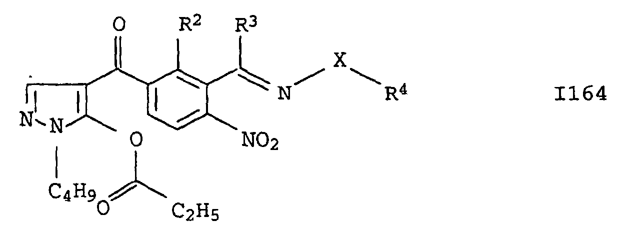
- die Verbindungen I165, insbesondere die Verbindungen I165.001-I165.180, die sich von den entsprechenden Verbindungen I1.001-I1.180 dadurch unterscheiden, daß R¹ Nitro, R⁹ für iso-Butyl und R¹⁰ für Ethylcarbonyl stehen: 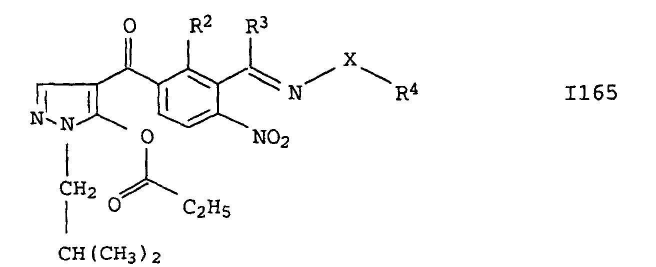
- die Verbindungen I166, insbesondere die Verbindungen I166.001-I166.180, die sich von den entsprechenden Verbindungen I1.001-I1.180 dadurch unterscheiden, daß R¹ für Nitro und R¹⁰ für n-Propylcarbonyl stehen: 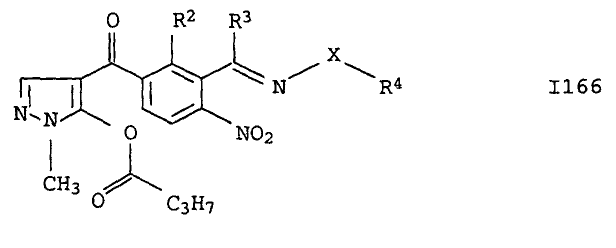
- die Verbindungen I167, insbesondere die Verbindungen I167.001-I167.180, die sich von den entsprechenden Verbindungen I1.001-I1.180 dadurch unterscheiden, daß R¹ für Nitro, R⁹ für Ethyl und R¹⁰ für n-Propylcarbonyl stehen: 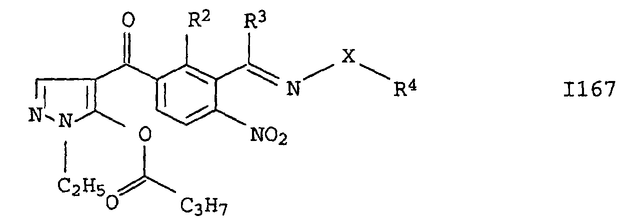
- die Verbindungen I168, insbesondere die Verbindungen I168.001-I168.180, die sich von den entsprechenden Verbindungen I1.001-I1.180 dadurch unterscheiden, daß R¹ für Nitro, R⁹ für n-Propyl und R¹⁰ für n-Propylcarbonyl stehen: 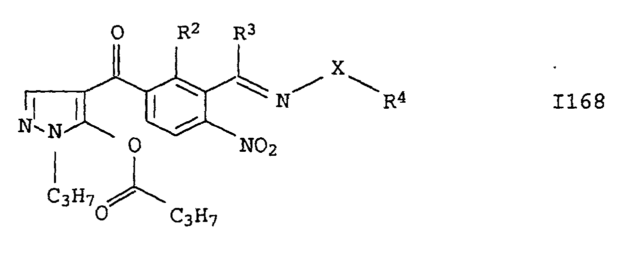
- die Verbindungen I169, insbesondere die Verbindungen I169.001-I169.180, die sich von den entsprechenden Verbindungen I1.001-I1.180 dadurch unterscheiden, daß R¹ Nitro, R⁹ für n-Butyl und R¹⁰ für n-Propylcarbonyl stehen: 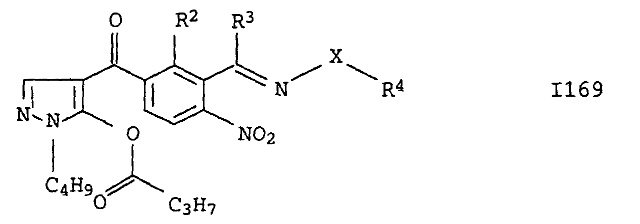
- die Verbindungen I170, insbesondere die Verbindungen I170.001-I170.180, die sich von den entsprechenden Verbindungen I1.001-I1.180 dadurch unterscheiden, daß R¹ für Nitro, R⁹ für iso-Butyl und R¹⁰ für n-Propylcarbonyl stehen: 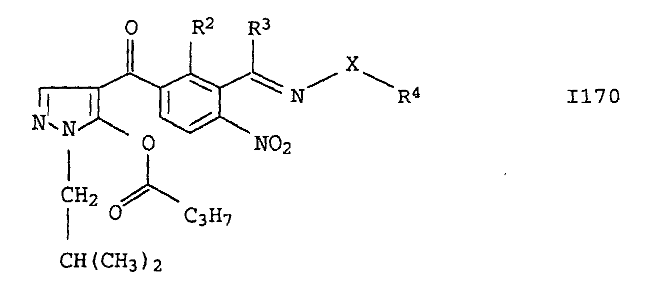
- die Verbindungen I171, insbesondere die Verbindungen I171.001-I171.180, die sich von den entsprechenden Verbindungen I1.001-I1.180 dadurch unterscheiden, daß R¹ für Nitro und R¹⁰ für Trifluormethylcarbonyl stehen: 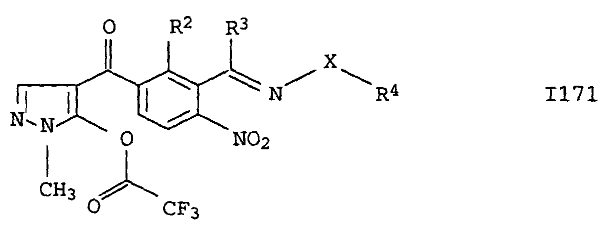
- die Verbindungen I172, insbesondere die Verbindungen I172.001-I172.180, die sich von den entsprechenden Verbindungen I1.001-I1.180 dadurch unterscheiden, daß R¹ für Nitro, R⁹ für Ethyl und R¹⁰ für Trifluormethylcarbonyl stehen: 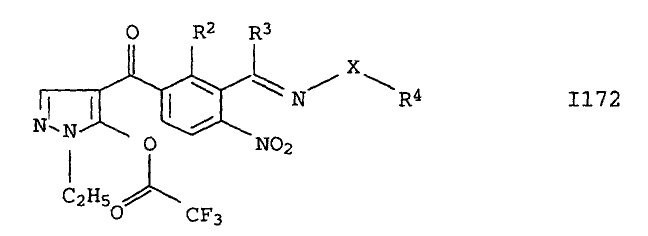
- die Verbindungen I173, insbesondere die Verbindungen I173.001-I173.180, die sich von den entsprechenden Verbindungen I1.001-I1.180 dadurch unterscheiden, daß R¹ für Nitro, R⁹ für n-Propyl und R¹⁰ für Trifluormethylcarbonyl stehen: 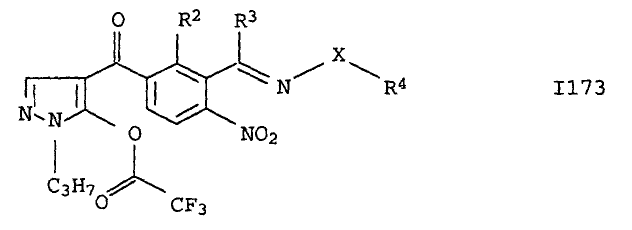
- die Verbindungen I174, insbesondere die Verbindungen I174.001-I174.180, die sich von den entsprechenden Verbindungen I1.001-I1.180 dadurch unterscheiden, daß R¹ Nitro, R⁹ für n-Butyl und R¹⁰ für Trifluormethylcarbonyl stehen: 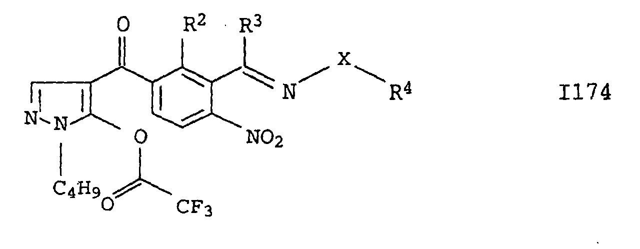
- die Verbindungen I175, insbesondere die Verbindungen I175.001-I175.180, die sich von den entsprechenden Verbindungen I1.001-I1.180 dadurch unterscheiden, daß R¹ Nitro, R⁹ für iso-Butyl und R¹⁰ für Trifluormethylcarbonyl stehen: 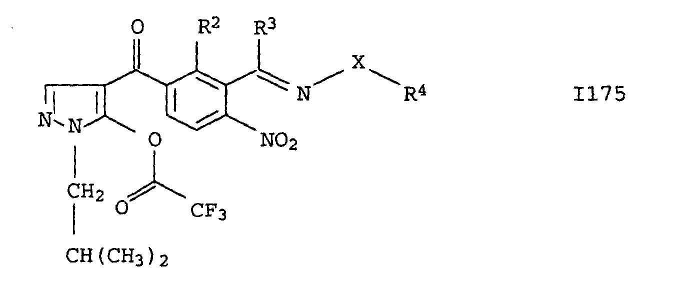
- die Verbindungen I176, insbesondere die Verbindungen I176.001-I176.180, die sich von den entsprechenden Verbindungen I1.001-I1.180 dadurch unterscheiden, daß R¹ für Nitro und R¹⁰ für Methylsulfonyl stehen: 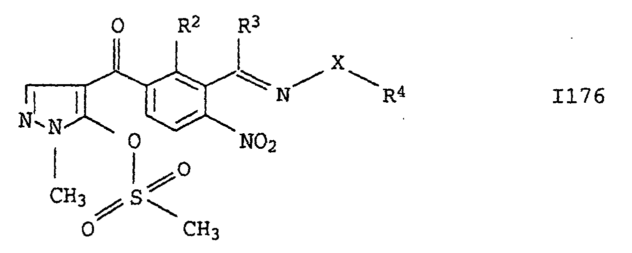
- die Verbindungen I177, insbesondere die Verbindungen I177.001-I177.180, die sich von den entsprechenden Verbindungen I1.001-I1.180 dadurch unterscheiden, daß R¹ für Nitro, R⁹ für Ethyl und R¹⁰ für Methylsulfonyl stehen: 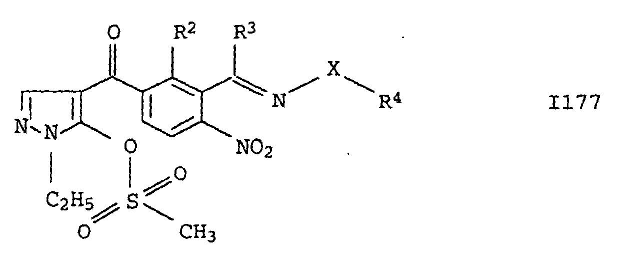
- die Verbindungen I178, insbesondere die Verbindungen I178.001-I178.180, die sich von den entsprechenden Verbindungen I1.001-I1.180 dadurch unterscheiden, daß R¹ Nitro, R⁹ für n-Propyl und R¹⁰ für Methylsulfonyl stehen: 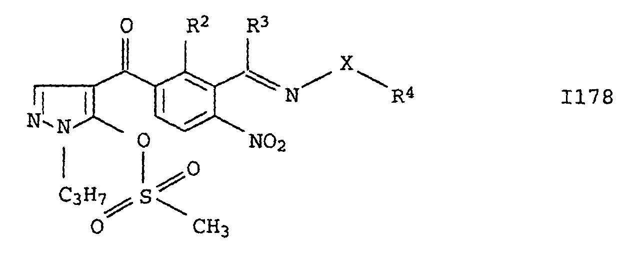
- die Verbindungen I179, insbesondere die Verbindungen I179.001-I179.180, die sich von den entsprechenden Verbindungen I1.001-I1.180 dadurch unterscheiden, daß R¹ für Nitro, R⁹ für n-Butyl und R¹⁰ für Methylsulfonyl stehen: 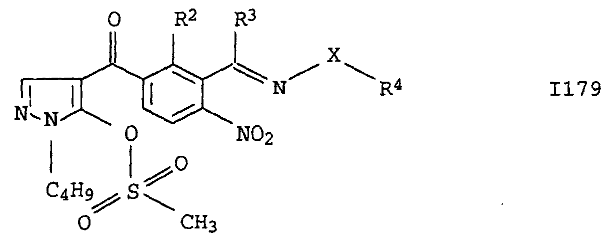
- die Verbindungen I180, insbesondere die Verbindungen I180.001-I180.180, die sich von den entsprechenden Verbindungen I1.001-I1.180 dadurch unterscheiden, daß R¹ für Nitro, R⁹ für iso-Butyl und R¹⁰ für Methylsulfonyl stehen: 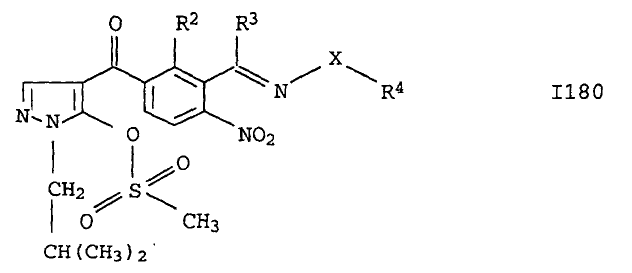
- die Verbindungen I181, insbesondere die Verbindungen I181.001-I181.180, die sich von den entsprechenden Verbindungen I1.001-I1.180 dadurch unterscheiden, daß R¹ Nitro und R¹⁰ für Ethylsulfonyl stehen: 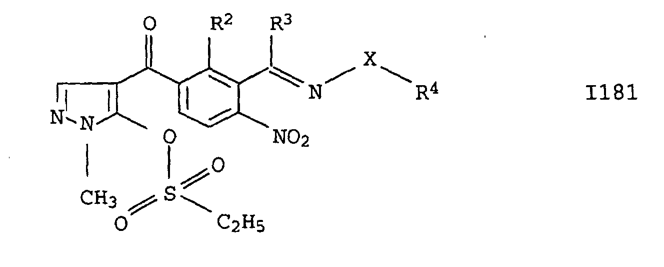
- die Verbindungen I182, insbesondere die Verbindungen I182.001-I182.180, die sich von den entsprechenden Verbindungen I1.001-I1.180 dadurch unterscheiden, daß R¹ Nitro, R⁹ für Ethyl und R¹⁰ für Ethylsulfonyl stehen: 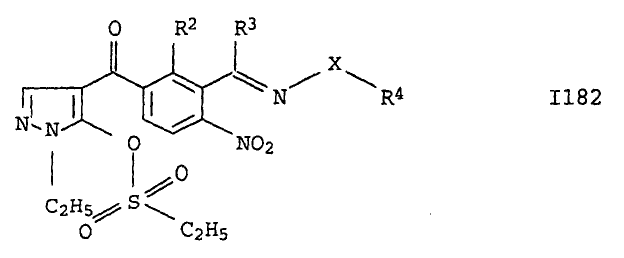
- die Verbindungen I183, insbesondere die Verbindungen I183.001-I183.180, die sich von den entsprechenden Verbindungen I1.001-I1.180 dadurch unterscheiden, daß R¹ Nitro, R⁹ für n-Propyl und R¹⁰ für Ethylsulfonyl stehen: 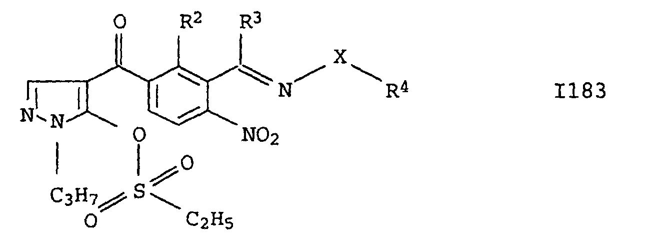
- die Verbindungen I184, insbesondere die Verbindungen I184.001-I184.180, die sich von den entsprechenden Verbindungen I1.001-I1.180 dadurch unterscheiden, daß R¹ Nitro, R⁹ für n-Butyl und R¹⁰ für Ethylsulfonyl stehen: 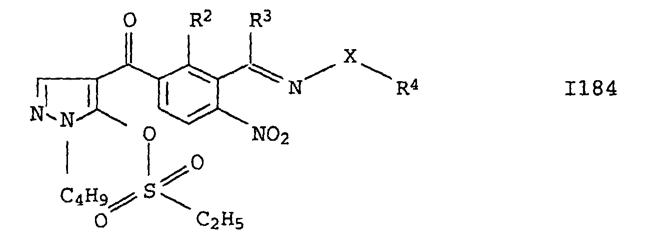
- die Verbindungen I185, insbesondere die Verbindungen I185.001-I185.180, die sich von den entsprechenden Verbindungen I1.001-I1.180 dadurch unterscheiden, daß R¹ Nitro, R⁹ für iso-Butyl und R¹⁰ für Ethylsulfonyl stehen: 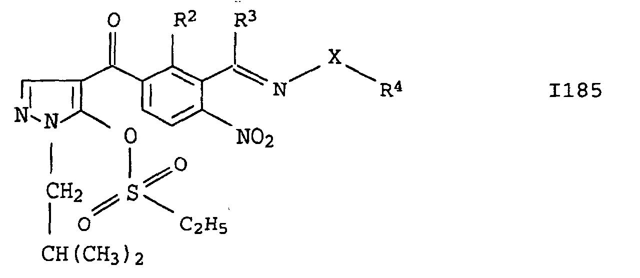
- die Verbindungen I186, insbesondere die Verbindungen I186.001-I186.180, die sich von den entsprechenden Verbindungen I1.001-I1.180 dadurch unterscheiden, daß R¹ Nitro und R¹⁰ für n-Propylsulfonyl stehen: 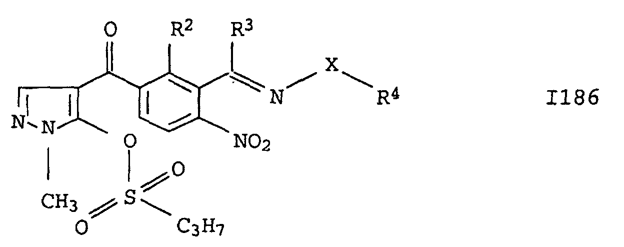
- die Verbindungen I187, insbesondere die Verbindungen I187.001-I187.180, die sich von den entsprechenden Verbindungen I1.001-I1.180 dadurch unterscheiden, daß R¹ Nitro, R⁹ für Ethyl und R¹⁰ für n-Propylsulfonyl stehen: 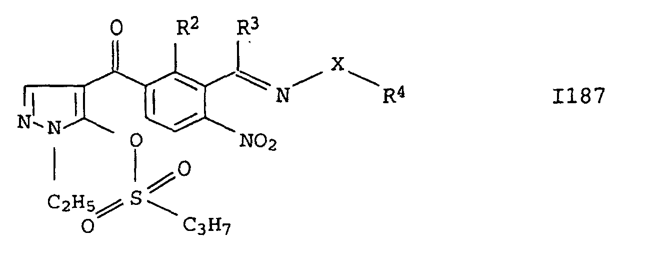
- die Verbindungen I188, insbesondere die Verbindungen I188.001-I188.180, die sich von den entsprechenden Verbindungen I1.001-I1.180 dadurch unterscheiden, daß R¹ für Nitro, R⁹ für n-Propyl und R¹⁰ für n-Propylsulfonyl stehen: 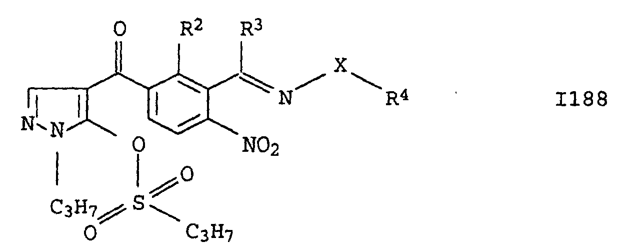
- die Verbindungen I189, insbesondere die Verbindungen I189.001-I189.189, die sich von den entsprechenden Verbindungen I1.001-I1.180 dadurch unterscheiden, daß R¹ für Nitro, R⁹ für n-Butyl und R¹⁰ für n-Propylsulfonyl stehen: 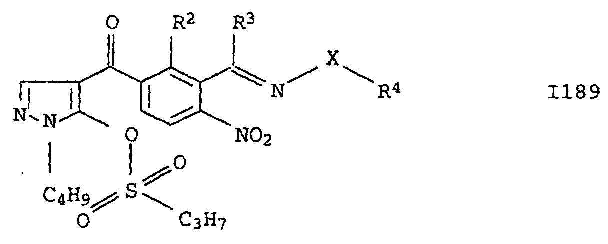
- die Verbindungen I190, insbesondere die Verbindungen I190.001-I190.180, die sich von den entsprechenden Verbindungen I1.001-I1.180 dadurch unterscheiden, daß R¹ für Nitro, R⁹ für iso-Butyl und R¹⁰ für n-Propylsulfonyl stehen: 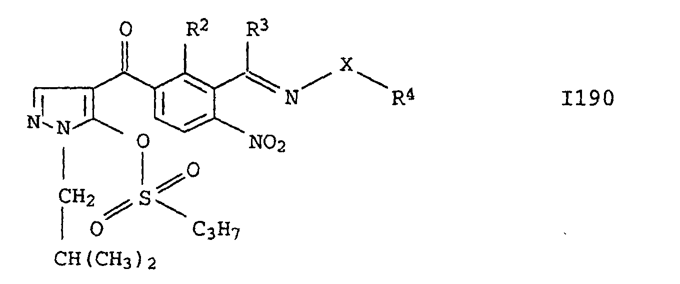
- die Verbindungen I191, insbesondere die Verbindungen I191.001-I191.180, die sich von den entsprechenden Verbindungen I1.001-I1.180 dadurch unterscheiden, daß R¹ für Nitro und R¹⁰ für iso-Propylsulfonyl stehen: 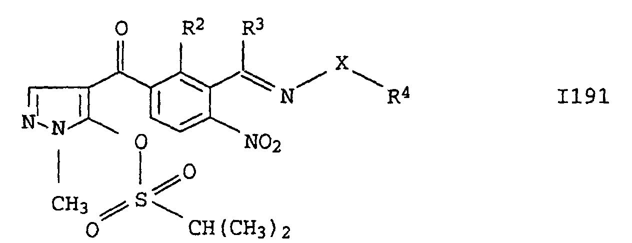
- die Verbindungen I192, insbesondere die Verbindungen I192.001-I192.180, die sich von den entsprechenden.Verbindungen I1.001-I1.180 dadurch unterscheiden, daß R¹ für Nitro, R⁹ für Ethyl und R¹⁰ für iso-Propylsulfonyl stehen: 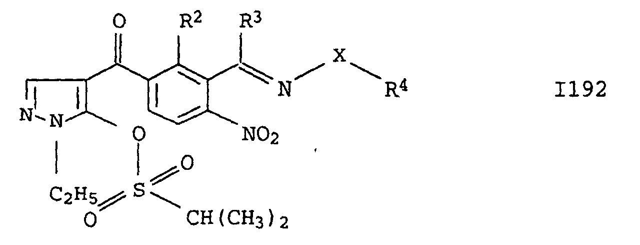
- die Verbindungen I193, insbesondere die Verbindungen I193.001-I193.180, die sich von den entsprechenden Verbindungen I1.001-I1.180 dadurch unterscheiden, daß R¹ für Nitro, R⁹ für n-Propyl und R¹⁰ für iso-Propylsulfonyl stehen: 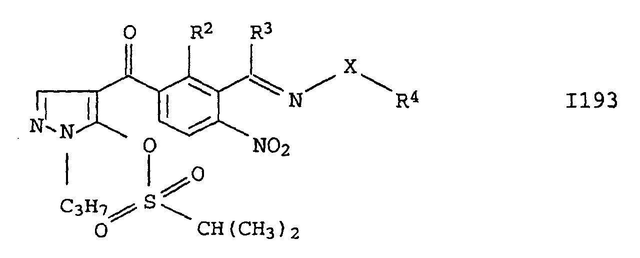
- die Verbindungen I194, insbesondere die Verbindungen I194.001-I194.180, die sich von den entsprechenden Verbindungen I1.001-I1.180 dadurch unterscheiden, daß R¹ für Nitro, R⁹ für n-Butyl und R¹⁰ für iso-Propylsulfonyl stehen: 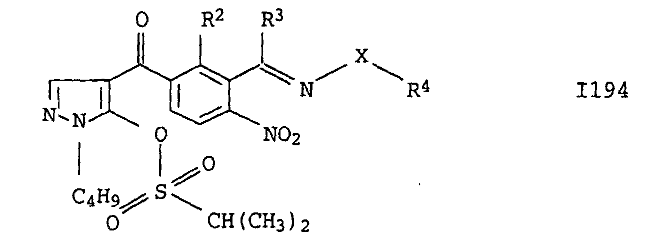
- die Verbindungen I195, insbesondere die Verbindungen I195.001-I195.180, die sich von den entsprechenden Verbindungen I1.001-I1.180 dadurch unterscheiden, daß R¹ für Nitro, R⁹ für iso-Butyl und R¹⁰ für iso-Propylsulfonyl stehen: 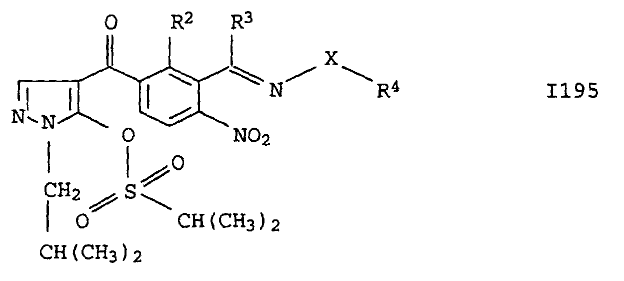
- die Verbindungen I196, insbesondere die Verbindungen I196.001-I196.180, die sich von den entsprechenden Verbindungen I1.001-I1.180 dadurch unterscheiden, daß R¹ für Nitro und R¹⁰ für n-Butylsulfonyl stehen: 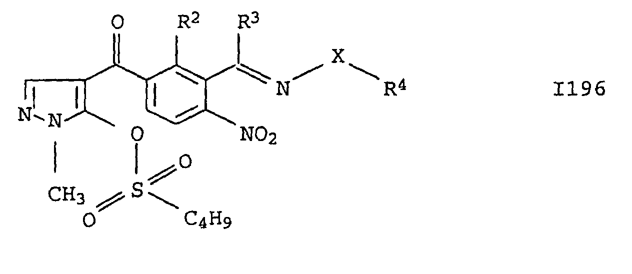
- die Verbindungen I197, insbesondere die Verbindungen I197.001-I197.180, die sich von den entsprechenden Verbindungen I1.001-I1.180 dadurch unterscheiden, daß R¹ für Nitro, R⁹ für Ethyl und R¹⁰ für n-Butylsulfonyl stehen: 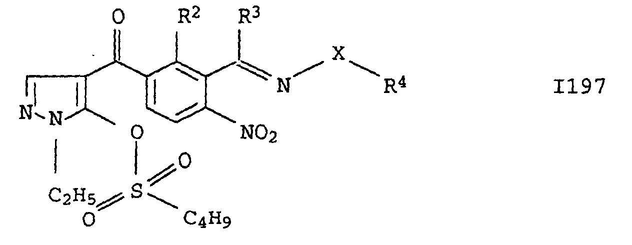
- die Verbindungen I198, insbesondere die Verbindungen I198.001-I198.180, die sich von den entsprechenden Verbindungen I1.001-I1.180 dadurch unterscheiden, daß R¹ für Nitro, R⁹ für n-Propyl und R¹⁰ für n-Butylsulfonyl stehen: 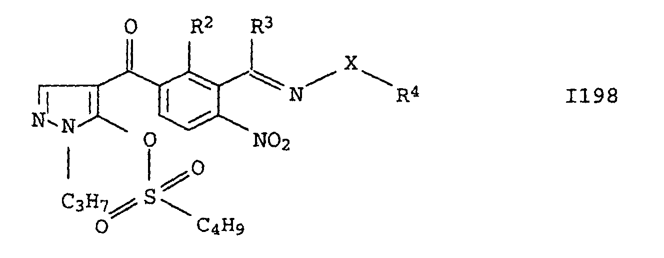
- die Verbindungen I199, insbesondere die Verbindungen I199.001-I199.180, die sich von den entsprechenden Verbindungen I1.001-I1.180 dadurch unterscheiden, daß R¹ für Nitro, R⁹ für n-Butyl und R¹⁰ für n-Butylsulfonyl stehen: 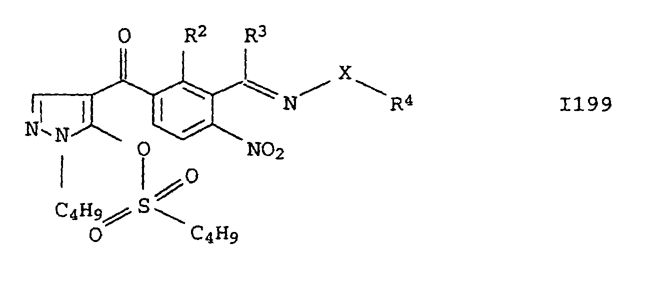
- die Verbindungen I200, insbesondere die Verbindungen I200.001-I200.180, die sich von den entsprechenden Verbindungen I1.001-I1.180 dadurch unterscheiden, daß R¹ für Nitro, R⁹ für iso-Butyl und R¹⁰ für n-Butylsulfonyl stehen: 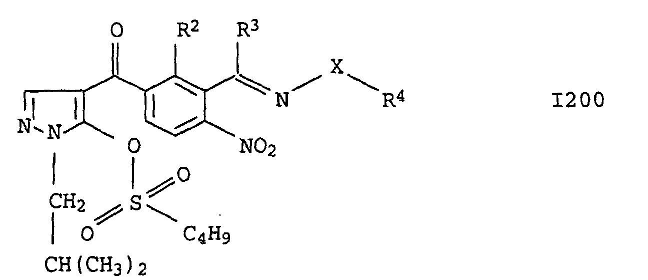
- die Verbindungen I201, insbesondere die Verbindungen I201.001-I201.180, die sich von den entsprechenden Verbindungen I1.001-I1.180 dadurch unterscheiden, daß R¹ für Nitro und R¹⁰ für iso-Butylsulfonyl stehen: 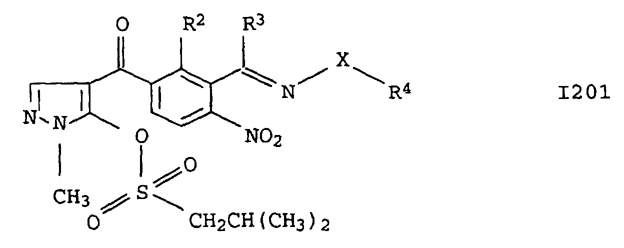
- die Verbindungen I202, insbesondere die Verbindungen I202.001-I202.180, die sich von den entsprechenden Verbindungen I1.001-I1.180 dadurch unterscheiden, daß R¹ für Nitro, R⁹ für Ethyl und R¹⁰ für iso-Butylsulfonyl stehen: 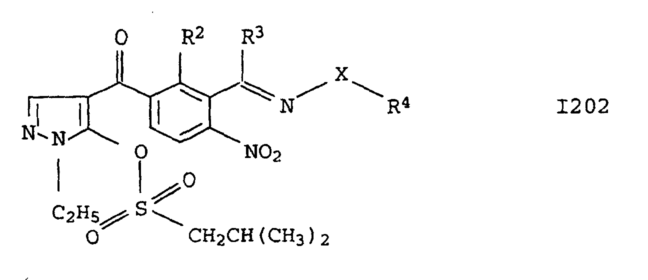
- die Verbindungen I203, insbesondere die Verbindungen I203.001-I203.180, die sich von den entsprechenden Verbindungen I1.001-I1.180 dadurch unterscheiden, daß R¹ für Nitro, R⁹ für n-Propyl und R¹⁰ für iso-Butylsulfonyl stehen: 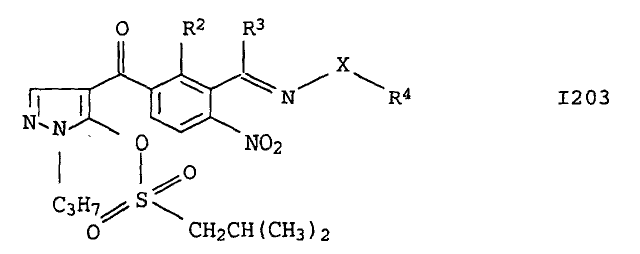
- die Verbindungen I204, insbesondere die Verbindungen I204.001-I204.180, die sich von den entsprechenden Verbindungen I1.001-I1.180 dadurch unterscheiden, daß R¹ für Nitro, R⁹ für n-Butyl und R¹⁰ für iso-Butylsulfonyl stehen: 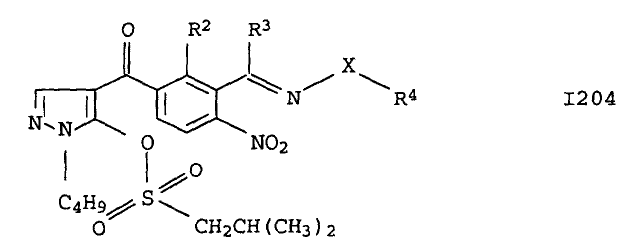
- die Verbindungen I205, insbesondere die Verbindungen I205.001-I205.180, die sich von den entsprechenden Verbindungen I1.001-I1.180 dadurch unterscheiden, daß R¹ für Nitro, R⁹ für iso-Butyl und R¹⁰ für iso-Butylsulfonyl stehen: 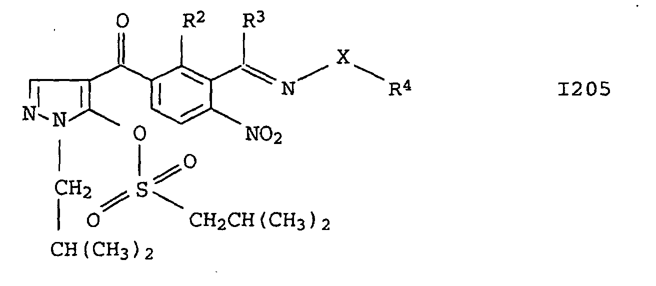
- die Verbindungen I206, insbesondere die Verbindungen I206.001-I2066.180, die sich von den entsprechenden Verbindungen I1.001-I1.180 dadurch unterscheiden, daß R¹ für Nitro und R¹⁰ für sec-Butylsulfonyl stehen: 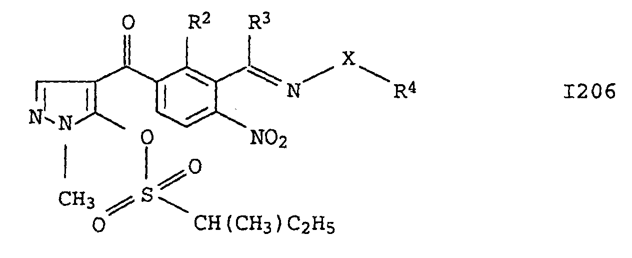
- die Verbindungen I207, insbesondere die Verbindungen I207.001-I207.180, die sich von den entsprechenden Verbindungen I1.001-I1.180 dadurch unterscheiden, daß R¹ für Nitro, R⁹ für Ethyl und R¹⁰ für sec-Butylsulfonyl stehen: 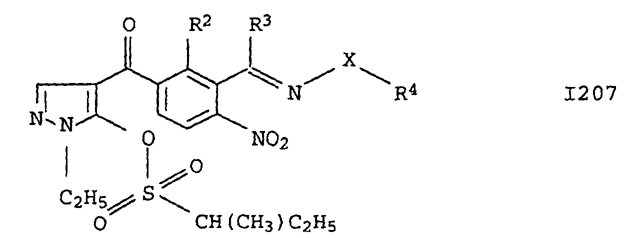
- die Verbindungen I208, insbesondere die Verbindungen I208.001-I208.180, die sich von den entsprechenden Verbindungen I1.001-I1.180 dadurch unterscheiden, daß R¹ für Nitro, R⁹ für n-Propyl und R¹⁰ für sec-Butylsulfonyl stehen: 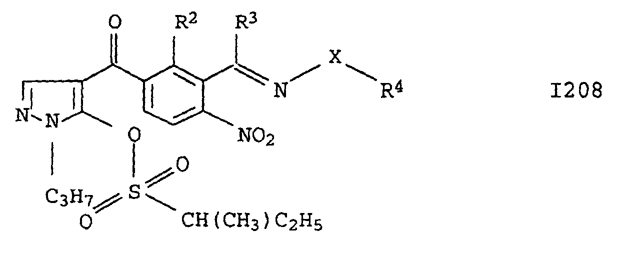
- die Verbindungen I209, insbesondere die Verbindungen I209.001-I209.180, die sich von den entsprechenden Verbindungen I1.001-I1.180 dadurch unterscheiden, daß R¹ für Nitro, R⁹ für n-Butyl und R¹⁰ für sec-Butylsulfonyl stehen: 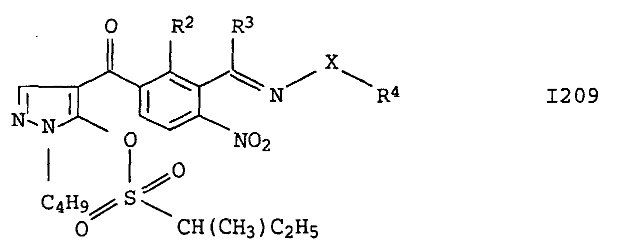
- die Verbindungen I210, insbesondere die Verbindungen I210.001-I210.180, die sich von den entsprechenden Verbindungen I1.001-I1.180 dadurch unterscheiden, daß R¹ für Nitro, R⁹ für iso-Butyl und R¹⁰ für sec-Butylsulfonyl stehen: 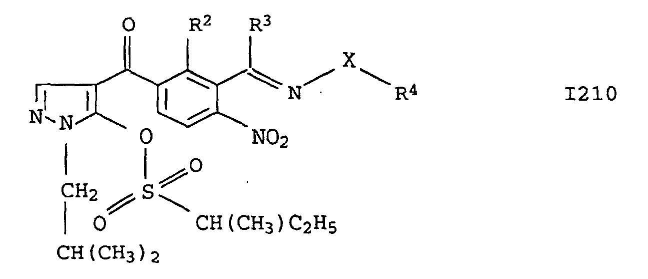
- die Verbindungen I211, insbesondere die Verbindungen I211.001-I211.180, die sich von den entsprechenden Verbindungen I1.001-I1.180 dadurch unterscheiden, daß R¹ für Nitro und R¹⁰ für Trifluormethylsulfonyl stehen: 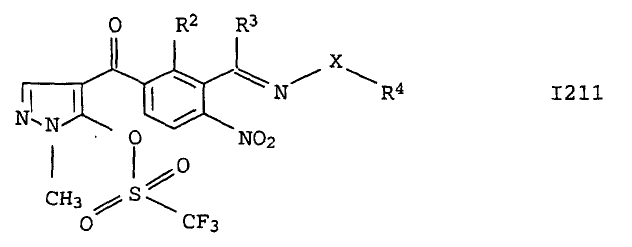
- die Verbindungen I212, insbesondere die Verbindungen I212.001-I212.180, die sich von den entsprechenden Verbindungen I1.001-I1.180 dadurch unterscheiden, daß R¹ für Nitro, R⁹ für Ethyl und R¹⁰ für Trifluormethylsulfonyl stehen: 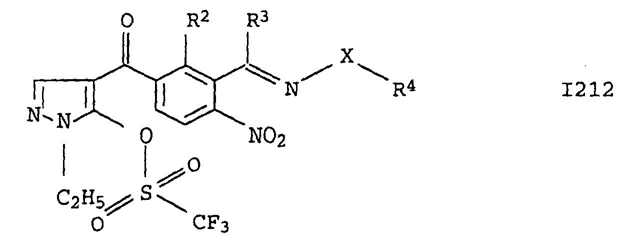
- die Verbindungen I213, insbesondere die Verbindungen I213.001-I213.180, die sich von den entsprechenden Verbindungen I1.001-I1.180 dadurch unterscheiden, daß R¹ für Nitro, R⁹ für n-Propyl und R¹⁰ für Trifluormethylsulfonyl stehen: 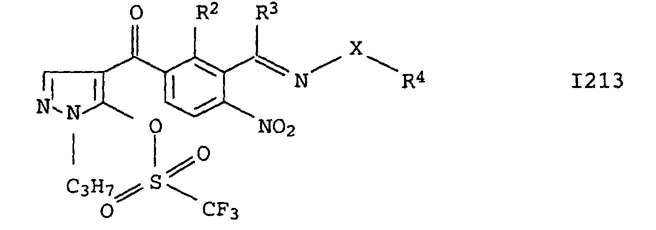
- die Verbindungen I214, insbesondere die Verbindungen I214.001-I214.180, die sich von den entsprechenden Verbindungen I1.001-I1.180 dadurch unterscheiden, daß R¹ für Nitro, R⁹ für n-Butyl und R¹⁰ für Trifluormethylsulfonyl stehen: 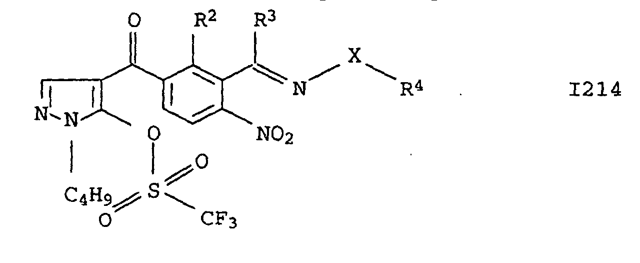
- die Verbindungen I215, insbesondere die Verbindungen I215.001-I125.180, die sich von den entsprechenden Verbindungen I1.001-I1.180 dadurch unterscheiden, daß R¹ für Nitro, R⁹ für iso-Butyl und R¹⁰ für Trifluormethylsulfonyl stehen: 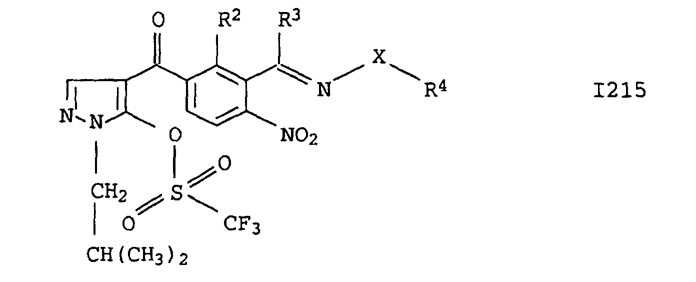
- die Verbindungen I216, insbesondere die Verbindungen I216.001-I216.180, die sich von den entsprechenden Verbindungen I1.001-I1.180 dadurch unterscheiden, daß R¹ für Nitro, R⁹ für Methyl und R¹⁰ für Phenylcarbonylmethyl stehen: 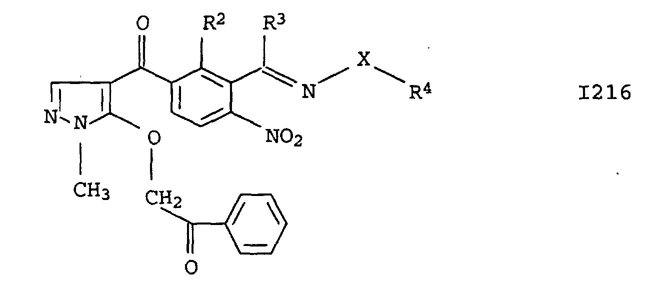
- die Verbindungen I217, insbesondere die Verbindungen I217.001-I217.180, die sich von den entsprechenden Verbindungen I1.001-I1.180 dadurch unterscheiden, daß R¹ für Nitro, R⁹ für Ethyl und R¹⁰ für Phenylcarbonylmethyl stehen: 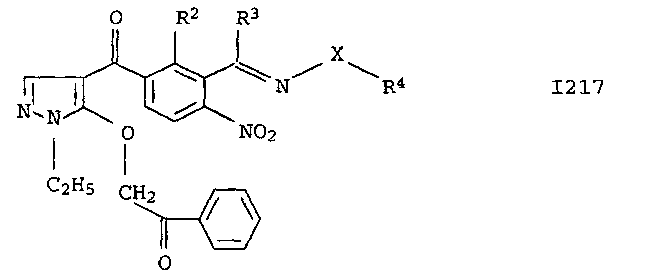
- die Verbindungen I218, insbesondere die Verbindungen I218.001-I218.180, die sich von den entsprechenden Verbindungen I1.001-I1.180 dadurch unterscheiden, daß R¹ für Nitro, R⁹ für n-Propyl und R¹⁰ für Phenylcarbonylmethyl stehen: 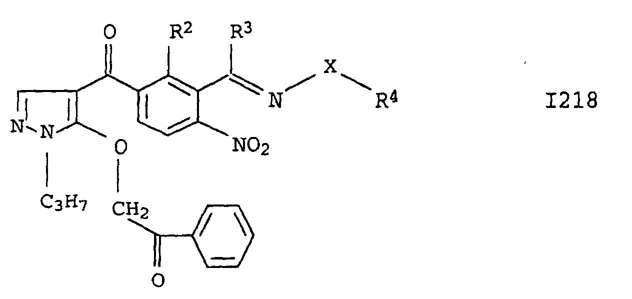
- die Verbindungen I219, insbesondere die Verbindungen I219.001-I219.180, die sich von den entsprechenden Verbindungen I1.001-I1.180 dadurch unterscheiden, daß R¹ für Nitro, R⁹ für n-Butyl und R¹⁰ für Phenylcarbonylmethyl stehen: 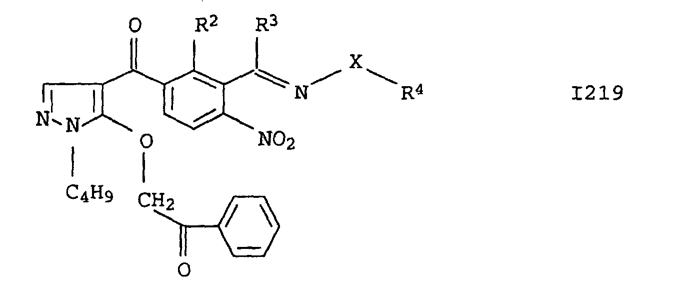
- die Verbindungen I220, insbesondere die Verbindungen I220.001-I220.180, die sich von den entsprechenden Verbindungen I1.001-I1.180 dadurch unterscheiden, daß R¹ für Nitro, R⁹ für iso-Butyl und R¹⁰ für Phenylcarbonylmethyl stehen: 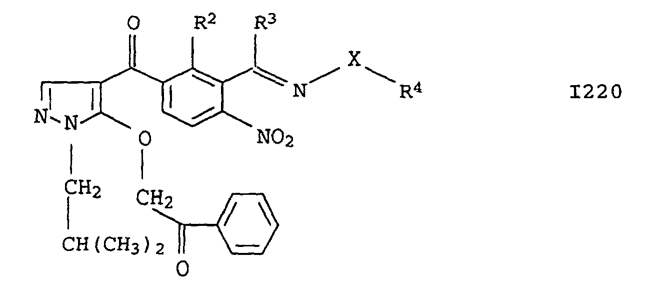
- die Verbindungen I221, insbesondere die Verbindungen I221.001-I221.180, die sich von den entsprechenden Verbindungen I1.001-I1.180 dadurch unterscheiden, daß R¹ für Nitro und R¹⁰ für Phenylsulfonyl stehen: 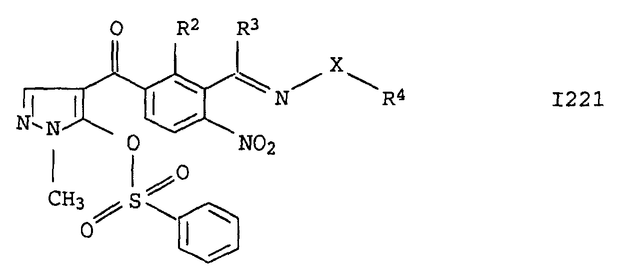
- die Verbindungen I222, insbesondere die Verbindungen I222.001-I222.180, die sich von den entsprechenden Verbindungen I1.001-I1.180 dadurch unterscheiden, daß R¹ für Nitro, R⁹ für Ethyl und R¹⁰ für Phenylsulfonyl stehen: 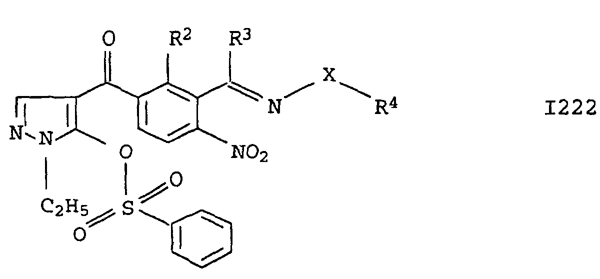
- die Verbindungen I223, insbesondere die Verbindungen I223.001-I223.180, die sich von den entsprechenden Verbindungen I1.001-I1.180 dadurch unterscheiden, daß R¹ für Nitro, R⁹ für n-Propyl und R¹⁰ für Phenylsulfonyl stehen: 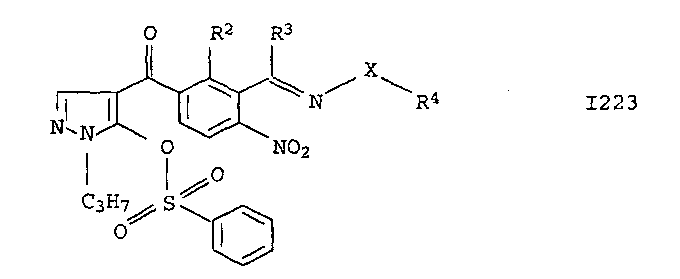
- die Verbindungen I224, insbesondere die Verbindungen I224.001-I224.180, die sich von den entsprechenden Verbindungen I1.001-I1.180 dadurch unterscheiden, daß R¹ für Nitro, R⁹ für n-Butyl und R¹⁰ für Phenylsulfonyl stehen: 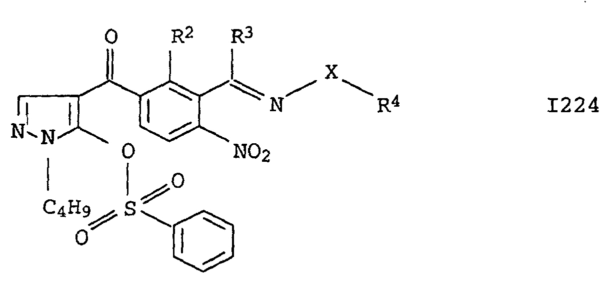
- die Verbindungen I225, insbesondere die Verbindungen I225.001-I225.180, die sich von den entsprechenden Verbindungen I1.001-I1.180 dadurch unterscheiden, daß R¹ für Nitro, R⁹ für iso-Butyl und R¹⁰ für Phenylsulfonyl stehen: 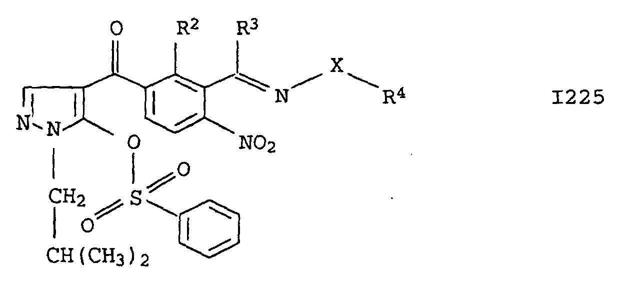
- die Verbindungen I226, insbesondere die Verbindungen I226.001-I226.180, die sich von den entsprechenden Verbindungen I1.001-I1.180 dadurch unterscheiden, daß R¹ für Nitro und R¹⁰ für 4-Methylphenylsulfonyl stehen: 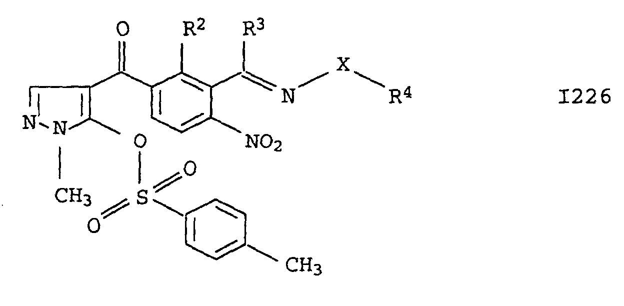
- die Verbindungen I227, insbesondere die Verbindungen I227.001-I227.180, die sich von den entsprechenden Verbindungen I1.001-I1.180 dadurch unterscheiden, daß R¹ für Nitro, R⁹ für Ethyl und R¹⁰ für 4-Methylphenylsulfonyl stehen: 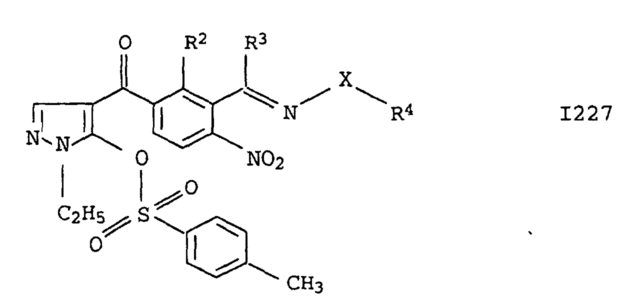
- die Verbindungen I228, insbesondere die Verbindungen I228.001-I228.180, die sich von den entsprechenden Verbindungen I1.001-I1.180 dadurch unterscheiden, daß R¹ für Nitro, R⁹ für n-Propyl und R¹⁰ für 4-Methylphenylsulfonyl stehen: 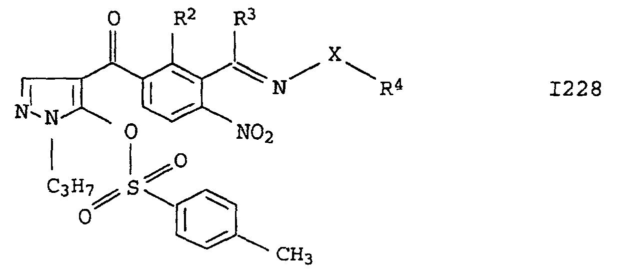
- die Verbindungen I229, insbesondere die Verbindungen I229.001-I229.180, die sich von den entsprechenden Verbindungen I1.001-I1.180 dadurch unterscheiden, daß R¹ für Nitro, R⁹ für n-Butyl und R¹⁰ für 4-Methylphenylsulfonyl stehen: 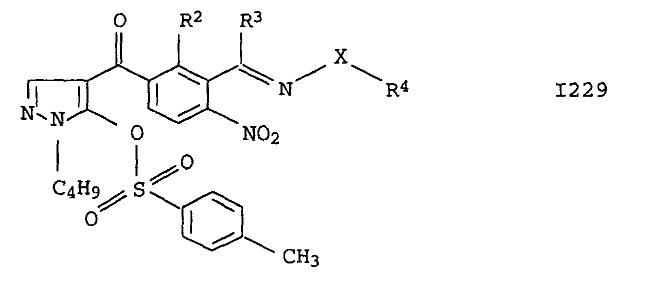
- die Verbindungen I230, insbesondere die Verbindungen I230.001-I230.180, die sich von den entsprechenden Verbindungen I1.001-I1.180 dadurch unterscheiden, daß R¹ für Nitro, R⁹ für iso-Butyl und R¹⁰ für 4-Methylphenylsulfonyl stehen: 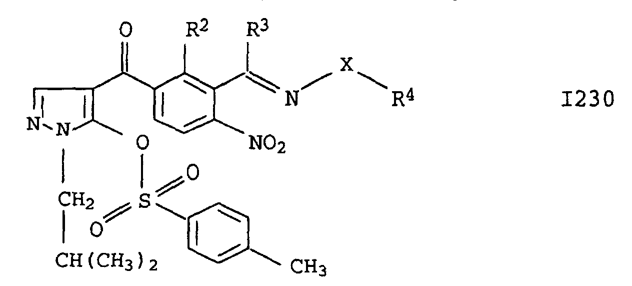
- die Verbindungen I231, insbesondere die Verbindungen I231.001-I231.180, die sich von den entsprechenden Verbindungen I1.001-I1.180 dadurch unterscheiden, daß R¹ für Methylsulfonyl steht: 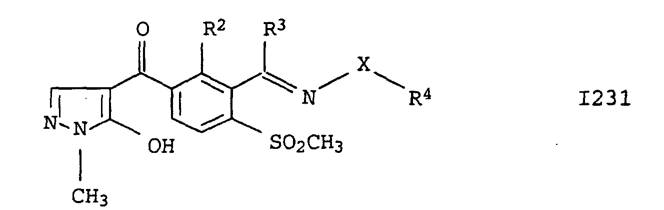
- die Verbindungen I232, insbesondere die Verbindungen I232.001-I232.180, die sich von den entsprechenden Verbindungen I1.001-I1.180 dadurch unterscheiden, daß R¹ für Methylsulfonyl und R⁹ für Ethyl stehen: 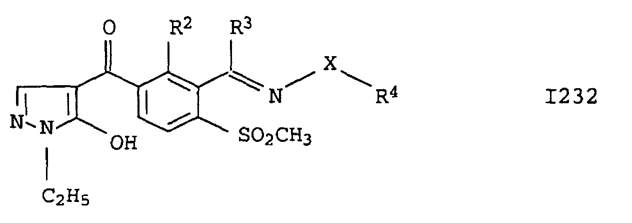
- die Verbindungen I233, insbesondere die Verbindungen I233.001-I233.180, die sich von den entsprechenden Verbindungen I1.001-I1.180 dadurch unterscheiden, daß R¹ für Methylsulfonyl und R⁹ für n-Propyl stehen: 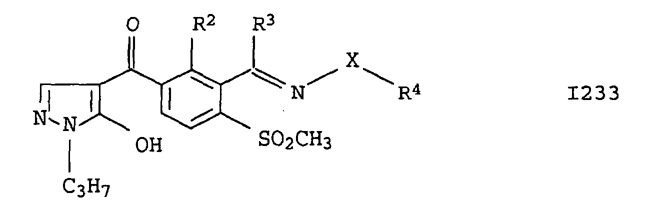
- die Verbindungen I234, insbesondere die Verbindungen I234.001-I234.180, die sich von den entsprechenden Verbindungen I1.001-I1.180 dadurch unterscheiden, daß R¹ für Methylsulfonyl und R⁹ für n-Butyl stehen: 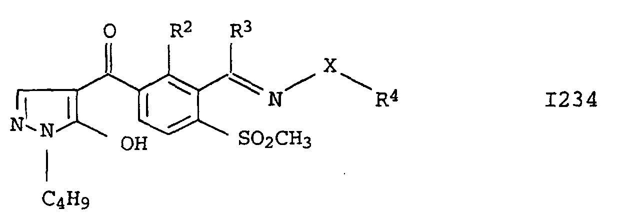
- die Verbindungen I235, insbesondere die Verbindungen I235.001-I235.180, die sich von den entsprechenden Verbindungen I1.001-I1.180 dadurch unterscheiden, daß R¹ für Methylsulfonyl und R⁹ für iso-Butyl stehen: 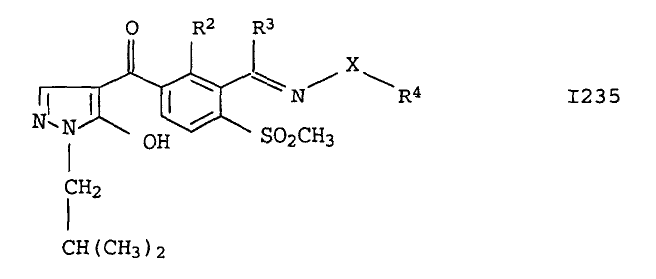
- die Verbindungen I236, insbesondere die Verbindungen I236.001-I236.180, die sich von den entsprechenden Verbindungen I1.001-I1.180 dadurch unterscheiden, daß R¹ für Methylsulfonyl und R¹⁰ für Methyl stehen: 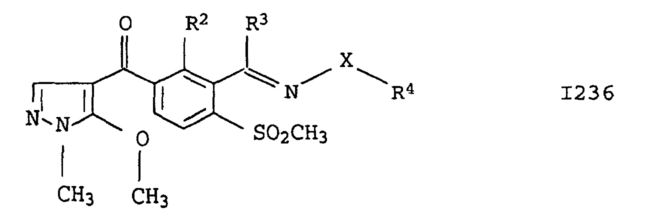
- die Verbindungen I237, insbesondere die Verbindungen I237.001-I237.180, die sich von den entsprechenden Verbindungen I1.001-I1.180 dadurch unterscheiden, daß R¹ für Methylsulfonyl, R⁹ für Ethyl und R¹⁰ für Methyl stehen: 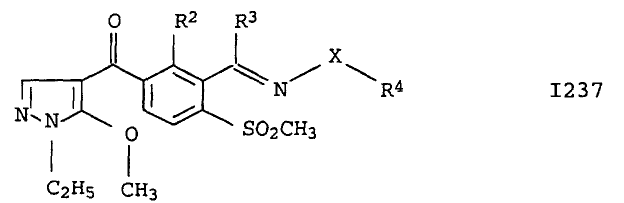
- die Verbindungen I238, insbesondere die Verbindungen I238.001-I238.180, die sich von den entsprechenden Verbindungen I1.001-I1.180 dadurch unterscheiden, daß R¹ für Methylsulfonyl, R⁹ für n-Propyl und R¹⁰ für Methyl stehen: 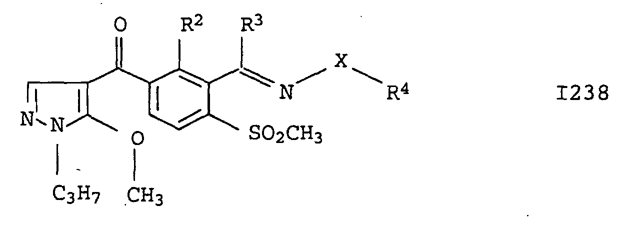
- die Verbindungen I239, insbesondere die Verbindungen I239.001-I239.180, die sich von den entsprechenden Verbindungen I1.001-I1.180 dadurch unterscheiden, daß R¹ für Methylsulfonyl, R⁹ für n-Butyl und R¹⁰ für Methyl stehen: 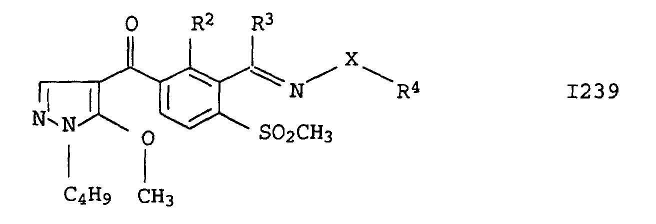
- die Verbindungen I240, insbesondere die Verbindungen I240.001-I240.180, die sich von den entsprechenden Verbindungen I1.001-I1.180 dadurch unterscheiden, daß R¹ für Methylsulfonyl, R⁹ für iso-Butyl und R¹⁰ für Methyl stehen: 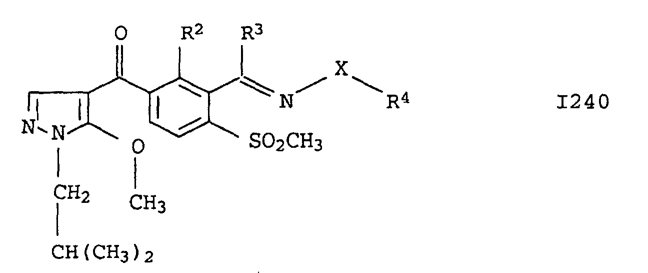
- die Verbindungen I241, insbesondere die Verbindungen I241.001-I241.180, die sich von den entsprechenden Verbindungen I1.001-I1.180 dadurch unterscheiden, daß R¹ für Methylsulfonyl und R¹⁰ für Ethyl stehen: 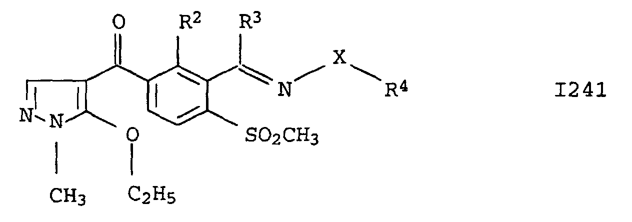
- die Verbindungen I242, insbesondere die Verbindungen I242.001-I242.180, die sich von den entsprechenden Verbindungen I1.001-I1.180 dadurch unterscheiden, daß R¹ für Methylsulfonyl, R⁹ und R¹⁰ für Ethyl stehen: 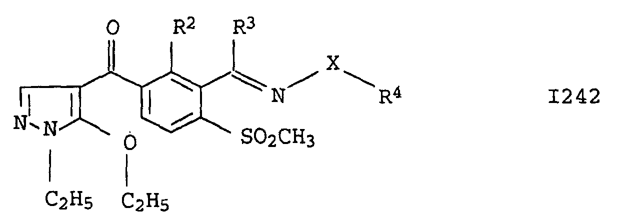
- die Verbindungen I243, insbesondere die Verbindungen I243.001-I243.180, die sich von den entsprechenden Verbindungen I1.001-I1.180 dadurch unterscheiden, daß R¹ für Methylsulfonyl, R⁹ für n-Propyl und R¹⁰ für Ethyl stehen: 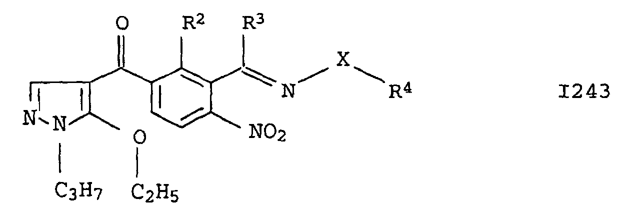
- die Verbindungen I244, insbesondere die Verbindungen I244.001-I244.180, die sich von den entsprechenden Verbindungen I1.001-I1.180 dadurch unterscheiden, daß R¹ für Methylsulfonyl, R⁹ für n-Butyl und R¹⁰ für Ethyl stehen: 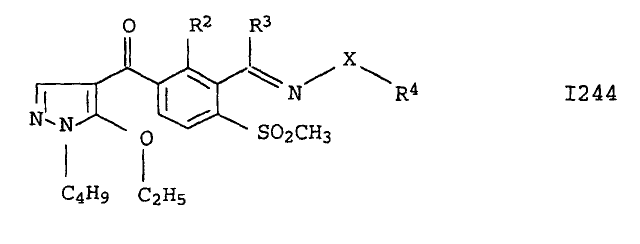
- die Verbindungen I245, insbesondere die Verbindungen I245.001-I245.180, die sich von den entsprechenden Verbindungen I1.001-I1.180 dadurch unterscheiden, daß R¹ für Methylsulfonyl, R⁹ für iso-Butyl und R¹⁰ für Ethyl stehen: 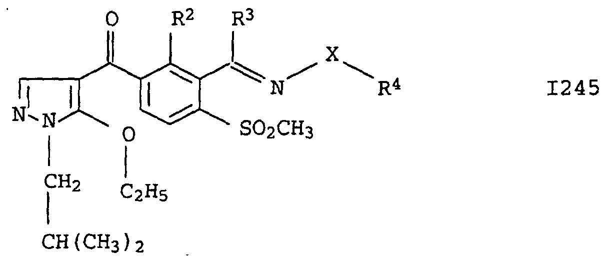
- die Verbindungen I246, insbesondere die Verbindungen I246.001-I246.180, die sich von den entsprechenden Verbindungen I1.001-I1.180 dadurch unterscheiden, daß R¹ für Methylsulfonyl und R¹⁰ für n-Propyl stehen: 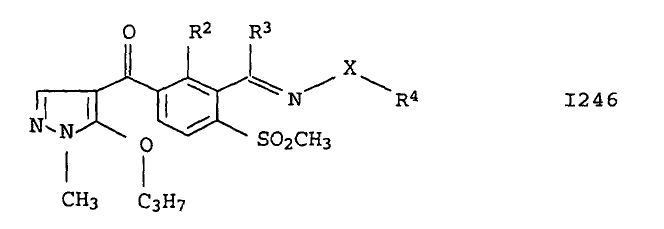
- die Verbindungen I247, insbesondere die Verbindungen I247.001-I247.180, die sich von den entsprechenden Verbindungen I1.001-I1.180 dadurch unterscheiden, daß R¹ für Methylsulfonyl, R⁹ für Ethyl und R¹⁰ für n-Propyl stehen: 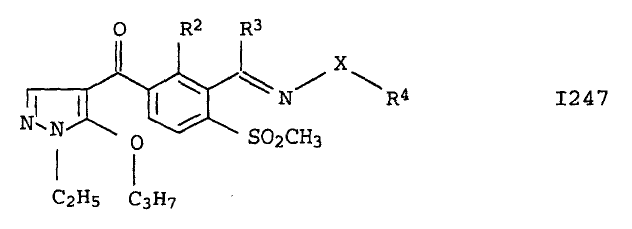
- die Verbindungen I248, insbesondere die Verbindungen I248.001-I248.180, die sich von den entsprechenden Verbindungen I1.001-I1.180 dadurch unterscheiden, daß R¹ für Methylsulfonyl, R⁹ und R¹⁰ für n-Propyl stehen: 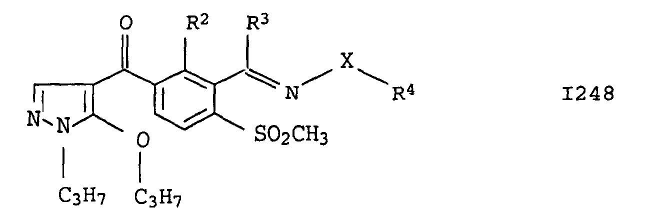
- die Verbindungen I249, insbesondere die Verbindungen I249.001-I249.180, die sich von den entsprechenden Verbindungen I1.001-I1.180 dadurch unterscheiden, daß R¹ für Methylsulfonyl, R⁹ für n-Butyl und R¹⁰ für n-Propyl stehen: 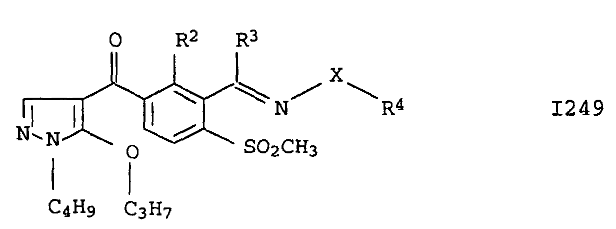
- die Verbindungen I250, insbesondere die Verbindungen I250.001-I250.180, die sich von den entsprechenden Verbindungen I1.001-I1.180 dadurch unterscheiden, daß R¹ für Methylsulfonyl, R⁹ für iso-Butyl und R¹⁰ für n-Propyl stehen: 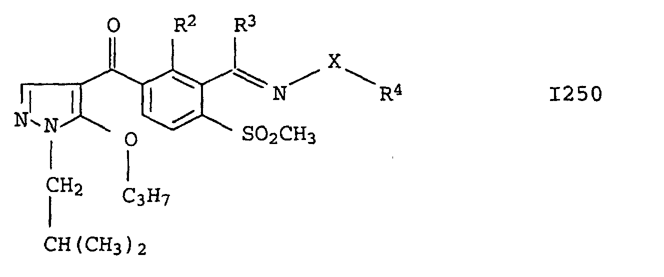
- die Verbindungen I251, insbesondere die Verbindungen I251.001-I251.180, die sich von den entsprechenden Verbindungen I1.001-I1.180 dadurch unterscheiden, daß R¹ für Methylsulfonyl und R¹⁰ für iso-Propyl stehen: 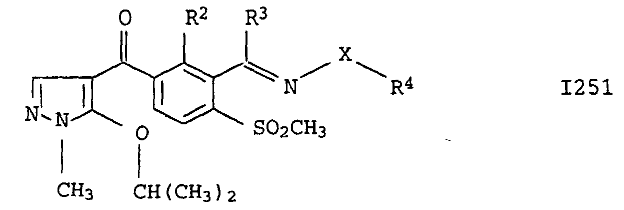
- die Verbindungen I252, insbesondere die Verbindungen I252.001-I252.180, die sich von den entsprechenden Verbindungen I1.001-I1.180 dadurch unterscheiden, daß R¹ für Methylsulfonyl, R⁹ für Ethyl und R¹⁰ für iso-Propyl stehen: 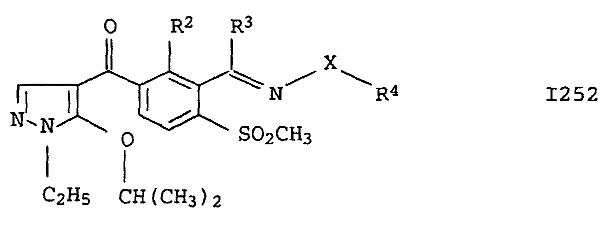
- die Verbindungen I253, insbesondere die Verbindungen I253.001-I253.180, die sich von den entsprechenden Verbindungen I1.001-I1.180 dadurch unterscheiden, daß R¹ für Methylsulfonyl, R⁹ für n-Propyl und R¹⁰ für iso-Propyl stehen: 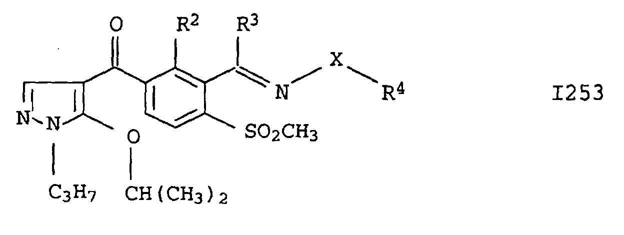
- die Verbindungen I254, insbesondere die Verbindungen I254.001-I254.180, die sich von den entsprechenden Verbindungen I1.001-I1.180 dadurch unterscheiden, daß R¹ für Methylsulfonyl, R⁹ für n-Butyl und R¹⁰ für iso-Propyl stehen: 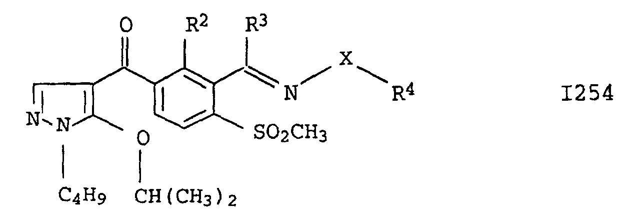
- die Verbindungen I255, insbesondere die Verbindungen I255.001-I255.180, die sich von den entsprechenden Verbindungen I1.001-I1.180 dadurch unterscheiden, daß R¹ für Methylsulfonyl, R⁹ für iso-Butyl und R¹⁰ für iso-Propyl stehen: 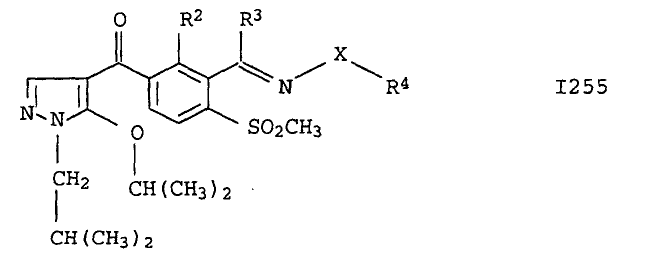
- die Verbindungen I256, insbesondere die Verbindungen I256.001-I256.180, die sich von den entsprechenden Verbindungen I1.001-I1.180 dadurch unterscheiden, daß R¹ für Methylsulfonyl und R¹⁰ für n-Butyl stehen: 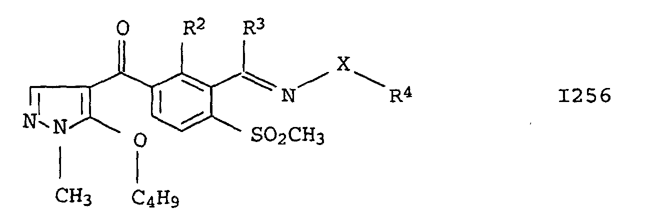
- die Verbindungen I257, insbesondere die Verbindungen I257.001-I257.180, die sich von den entsprechenden Verbindungen I1.001-I1.180 dadurch unterscheiden, daß R¹ für Methylsulfonyl, R⁹ für Ethyl und R¹⁰ für n-Butyl stehen: 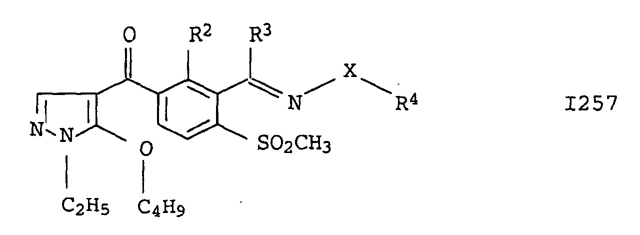
- die Verbindungen I258, insbesondere die Verbindungen I258.001-I258.180, die sich von den entsprechenden Verbindungen I1.001-I1.180 dadurch unterscheiden, daß R¹ für Methylsulfonyl, R⁹ für n-Propyl und R¹⁰ für n-Butyl stehen: 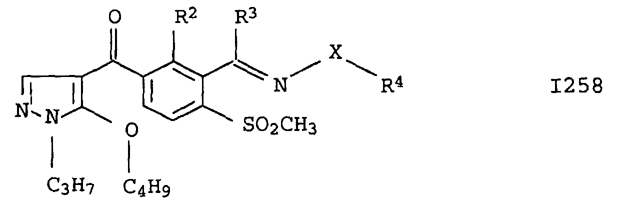
- die Verbindungen I259, insbesondere die Verbindungen I259.001-I259.180, die sich von den entsprechenden Verbindungen I1.001-I1.180 dadurch unterscheiden, daß R¹ für Methylsulfonyl, R⁹ und R¹⁰ für n-Butyl stehen: 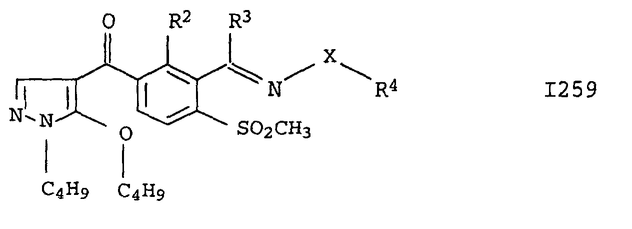
- die Verbindungen I260, insbesondere die Verbindungen I260.001-I260.180, die sich von den entsprechenden Verbindungen I1.001-I1.180 dadurch unterscheiden, daß R¹ für Methylsulfonyl, R⁹ für iso-Butyl und R¹⁰ für n-Butyl stehen: 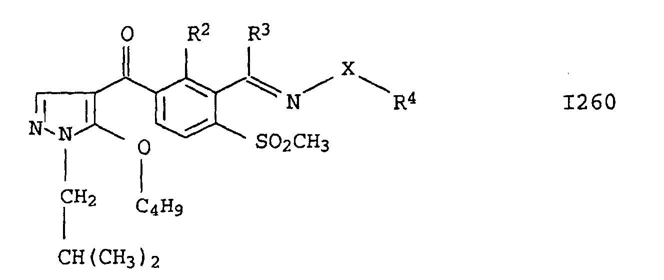
- die Verbindungen I261, insbesondere die Verbindungen I261.001-I261.180, die sich von den entsprechenden Verbindungen I1.001-I1.180 dadurch unterscheiden, daß R¹ für Methylsulfonyl und R¹⁰ für sec-Butyl stehen: 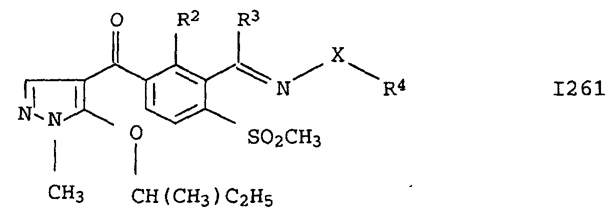
- die Verbindungen I262, insbesondere die Verbindungen I262.001-I262.180, die sich von den entsprechenden Verbindungen I1.001-I1.180 dadurch unterscheiden, daß R¹ für Methylsulfonyl, R⁹ für Ethyl und R¹⁰ für sec-Butyl stehen: 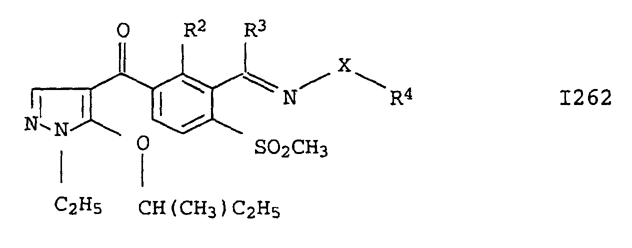
- die Verbindungen I263, insbesondere die Verbindungen I263.001-I263.180, die sich von den entsprechenden Verbindungen I1.001-I1.180 dadurch unterscheiden, daß R¹ für Methylsulfonyl, R⁹ für n-Propyl und R¹⁰ für sec-Butyl stehen: 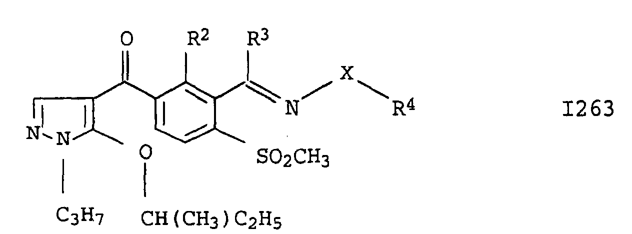
- die Verbindungen I264, insbesondere die Verbindungen I264.001-I264.180, die sich von den entsprechenden Verbindungen I1.001-I1.180 dadurch unterscheiden, daß R¹ für Methylsulfony, R⁹ für n-Butyl und R¹⁰ für sec-Butyl stehen: 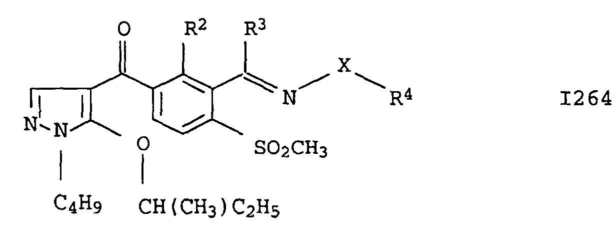
- die Verbindungen I265, insbesondere die Verbindungen I265.001-I265.180, die sich von den entsprechenden Verbindungen I1.001-I1.180 dadurch unterscheiden, daß R¹ für Methylsulfonyl, R⁹ für iso-Butyl und R¹⁰ für sec-Butyl stehen: 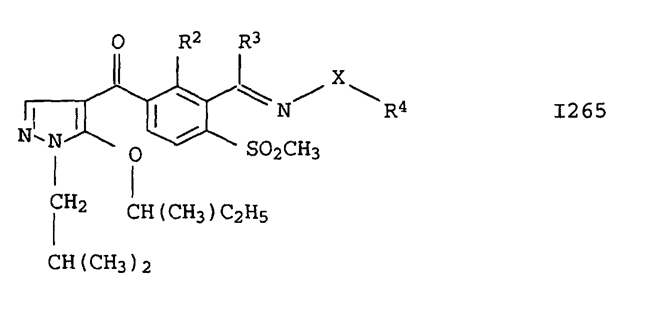
- die Verbindungen I266, insbesondere die Verbindungen I266.001-I266.180, die sich von den entsprechenden Verbindungen I1.001-I1.180 dadurch unterscheiden, daß R¹ für Methylsulfonyl und R¹⁰ für iso-Butyl stehen: 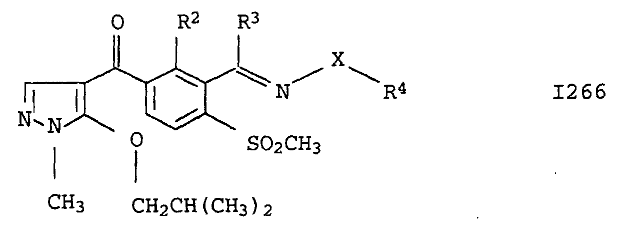
- die Verbindungen I267, insbesondere die Verbindungen I267.001-I267.180, die sich von den entsprechenden Verbindungen I1.001-I1.180 dadurch unterscheiden, daß R¹ für Methylsulfonyl, R⁹ für Ethyl und R¹⁰ für iso-Butyl stehen: 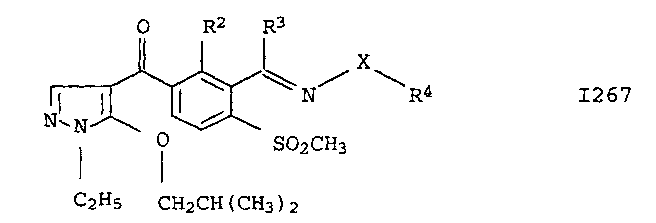
- die Verbindungen I268, insbesondere die Verbindungen I268.001-I268.180, die sich von den entsprechenden Verbindungen I1.001-I1.180 dadurch unterscheiden, daß R¹ für Methylsulfonyl, R⁹ für n-Propyl und R¹⁰ für iso-Butyl stehen: 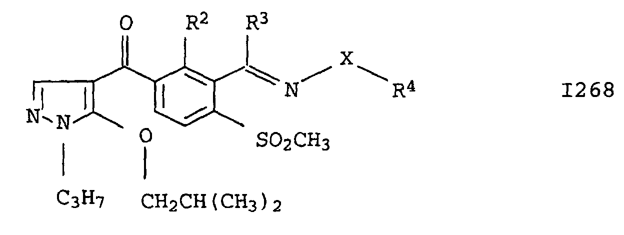
- die Verbindungen I269, insbesondere die Verbindungen I269.001-I269.180, die sich von den entsprechenden Verbindungen I1.001-I1.180 dadurch unterscheiden, daß R¹ für Methylsulfonyl, R⁹ für n-Butyl und R¹⁰ für iso-Butyl stehen: 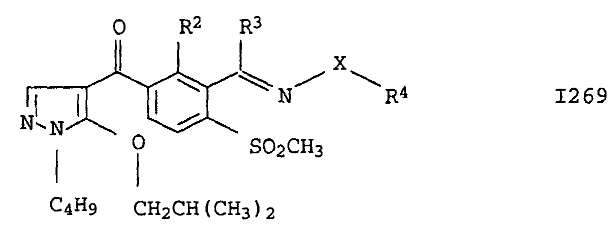
- die Verbindungen I270, insbesondere die Verbindungen I270.001-I270.180, die sich von den entsprechenden Verbindungen I1.001-I1.180 dadurch unterscheiden, daß R¹ für Methylsulfonyl, R⁹ und R¹⁰ für iso-Butyl stehen: 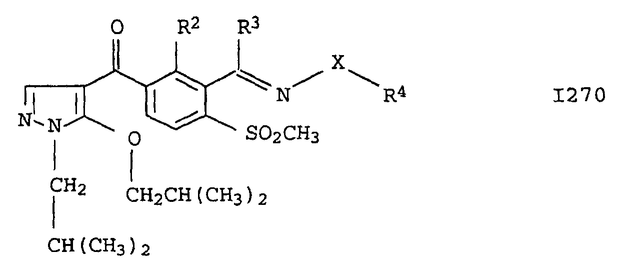
- die Verbindungen I271, insbesondere die Verbindungen I271.001-I271.180, die sich von den entsprechenden Verbindungen I1.001-I1.180 dadurch unterscheiden, daß R¹ für Methylsulfonyl und R¹⁰ für Methylcarbonyl stehen: 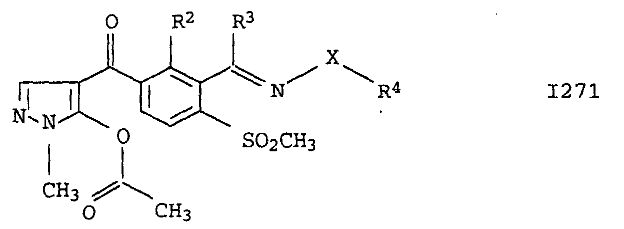
- die Verbindungen I272, insbesondere die Verbindungen I272.001-I272.180, die sich von den entsprechenden Verbindungen I1.001-I1.180 dadurch unterscheiden, daß R¹ für Methylsulfonyl, R⁹ für Ethyl und R¹⁰ für Methylcarbonyl stehen: 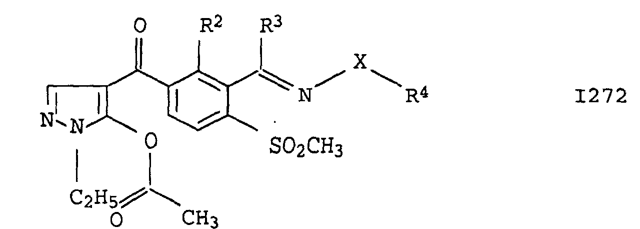
- die Verbindungen I273, insbesondere die Verbindungen I273.001-I273.180, die sich von den entsprechenden Verbindungen I1.001-I1.180 dadurch unterscheiden, daß R¹ für Methylsulfonyl, R⁹ für n-Propyl und R¹⁰ für Methylcarbonyl stehen: 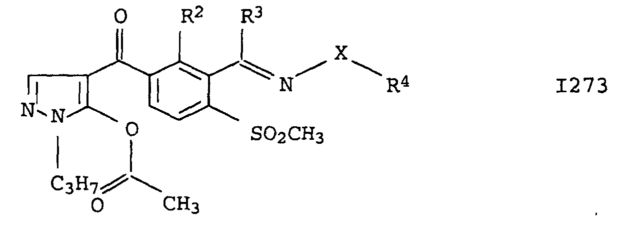
- die Verbindungen I274, insbesondere die Verbindungen I274.001-I274.180, die sich von den entsprechenden Verbindungen I1.001-I1.180 dadurch unterscheiden, daß R¹ für Methylsulfonyl, R⁹ für n-Butyl und R¹⁰ für Methylcarbonyl stehen: 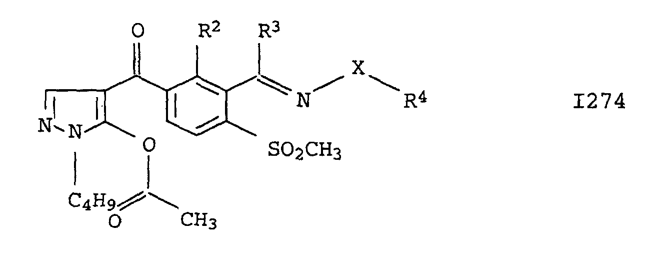
- die Verbindungen I275, insbesondere die Verbindungen I275.001-I275.180, die sich von den entsprechenden Verbindungen I1.001-I1.180 dadurch unterscheiden, daß R¹ für Methylsulfonyl, R⁹ für iso-Butyl und R¹⁰ für Methylcarbonyl stehen: 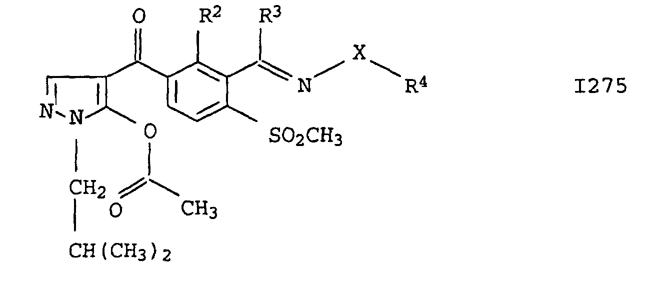
- die Verbindungen I276, insbesondere die Verbindungen I276.001-I276.180, die sich von den entsprechenden Verbindungen I1.001-I1.180 dadurch unterscheiden, daß R¹ für Methylsulfonyl und R¹⁰ für Ethylcarbonyl sthen: 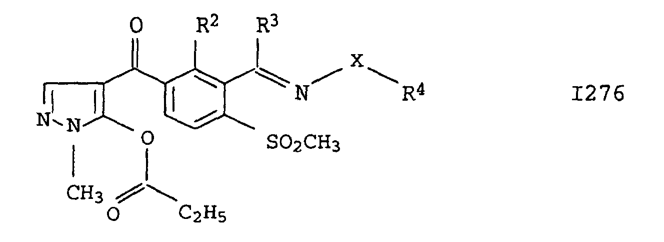
- die Verbindungen I277, insbesondere die Verbindungen I277.001-I277.180, die sich von den entsprechenden Verbindungen I1.001-I1.180 dadurch unterscheiden, daß R¹ für Methylsulfonyl, R⁹ für Ethyl und R¹⁰ für Ethylcarbonyl stehen: 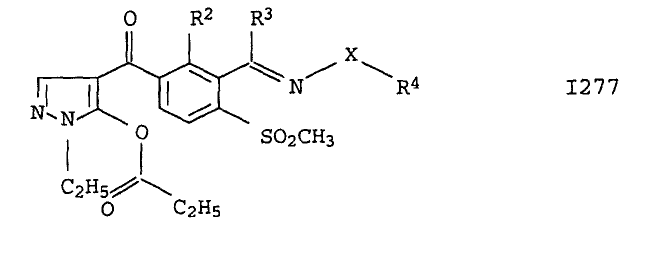
- die Verbindungen I278, insbesondere die Verbindungen I278.001-I278.180, die sich von den entsprechenden Verbindungen I1.001-I1.180 dadurch unterscheiden, daß R¹ für Methylsulfonyl, R⁹ für n-Propyl und R¹⁰ für Ethylcarbonyl stehen: 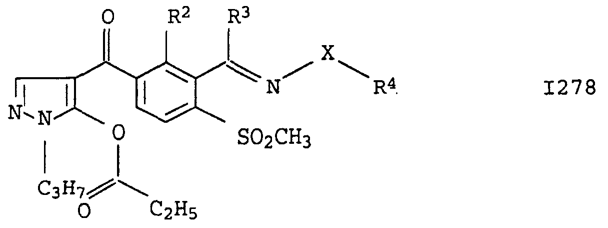
- die Verbindungen I279, insbesondere die Verbindungen I279.001-I279.180, die sich von den entsprechenden Verbindungen I1.001-I1.180 dadurch unterscheiden, daß R¹ für Methylsulfonyl, R⁹ für n-Butyl und R¹⁰ für Ethylcarbonyl stehen: 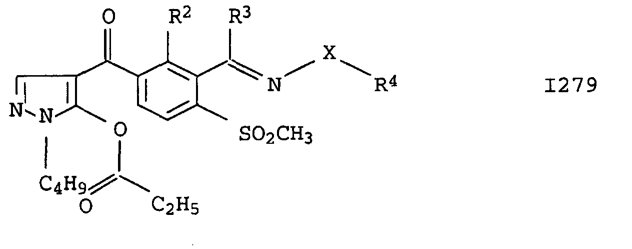
- die Verbindungen I280, insbesondere die Verbindungen I280.001-I280.180, die sich von den entsprechenden Verbindungen I1.001-I1.180 dadurch unterscheiden, daß R¹ für Methylsulfonyl, R⁹ für iso-Butyl und R¹⁰ für Ethylcarbonyl stehen: 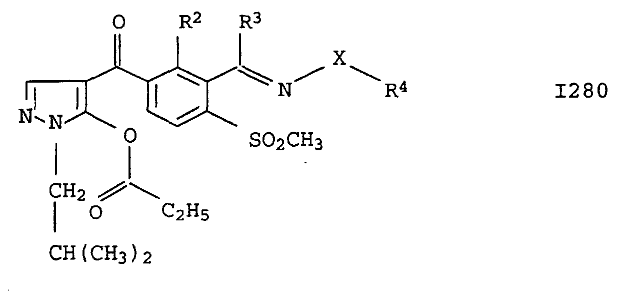
- die Verbindungen I281, insbesondere die Verbindungen I281.001-I281.180, die sich von den entsprechenden Verbindungen I1.001-I1.180 dadurch unterscheiden, daß R¹ für Methylsülfonyl und R¹⁰ für n-Propylcarbonyl stehen: 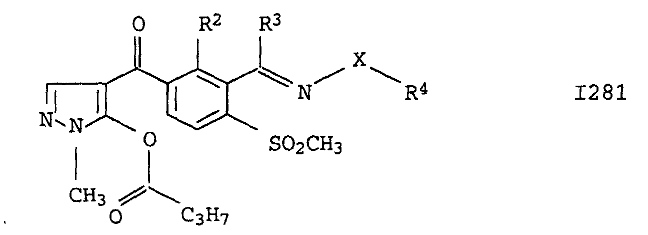
- die Verbindungen I282, insbesondere die Verbindungen I282.001-I282.180, die sich von den entsprechenden Verbindungen I1.001-I1.180 dadurch unterscheiden, daß R¹ für Methylsulfonyl, R⁹ für Ethvl und R¹⁰ für n-Propylcarbonyl stehen: 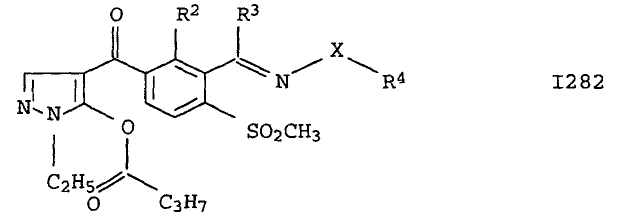
- die Verbindungen I283, insbesondere die Verbindungen I283.001-I283.180, die sich von den entsprechenden Verbindungen I1.001-I1.180 dadurch unterscheiden, daß R¹ für Methylsulfonyl, R⁹ für n-Propyl und R¹⁰ für n-Propylcarbonyl stehen: 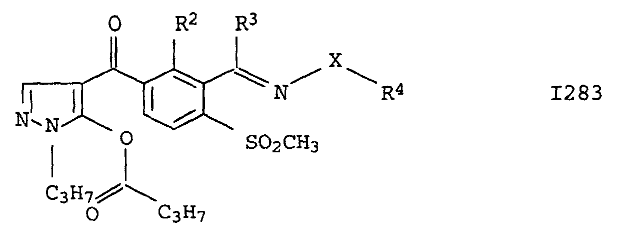
- die Verbindungen I284, insbesondere die Verbindungen I284.001-I284.180, die sich von den entsprechenden Verbindungen I1.001-I1.180 dadurch unterscheiden, daß R¹ für Methylsulfonyl, R⁹ für n-Butyl und R¹⁰ für n-Propylcarbonyl stehen: 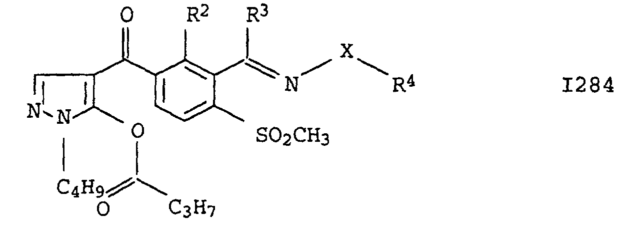
- die Verbindungen I285, insbesondere die Verbindungen I285.001-I285.180, die sich von den entsprechenden Verbindungen I1.001-I1.180 dadurch unterscheiden, daß R¹ für Methylsulfonyl, R⁹ für iso-Butyl und R¹⁰ für n-Propylcarbonyl stehen: 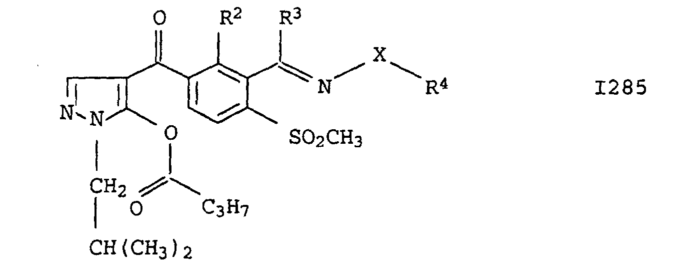
- die Verbindungen I286, insbesondere die Verbindungen I286.001-I286.180, die sich von den entsprechenden Verbindungen I1.001-I1.180 dadurch unterscheiden, daß R¹ für Methylsulfonyl und R⁹ für Trifluormethylcarbonyl stehen: 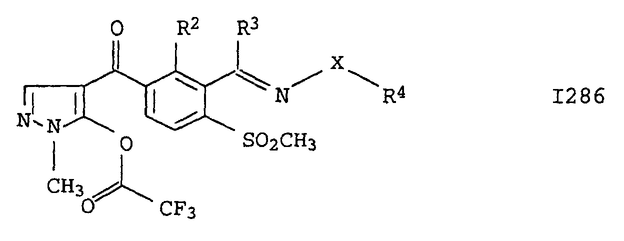
- die Verbindungen I287, insbesondere die Verbindungen I287.001-I287.180, die sich von den entsprechenden Verbindungen I1.001-I1.180 dadurch unterscheiden, daß R¹ für Methylsulfonyl, R⁹ für Ethyl und R¹⁰ für Trifluormethylcarbonyl stehen: 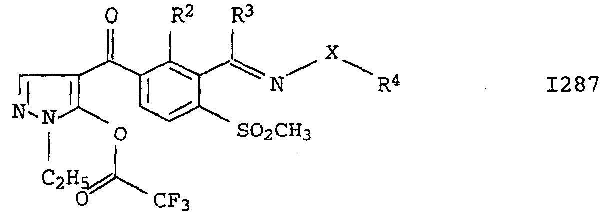
- die Verbindungen I288, insbesondere die Verbindungen I288.001-I288.180, die sich von den entsprechenden Verbindungen I1.001-I1.180 dadurch unterscheiden, daß R¹ für Methylsulfonyl, R⁹ für n-Propyl und R¹⁰ für Trifluormethylcarbonyl stehen: 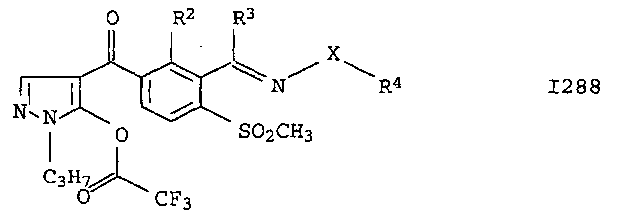
- die Verbindungen I289, insbesondere die Verbindungen I289.001-I289.180, die sich von den entsprechenden Verbindungen I1.001-I1.180 dadurch unterscheiden, daß R¹ für Methylsulfonyl, R⁹ für n-Butyl und R¹⁰ für Trifluormethylcarbonyl stehen: 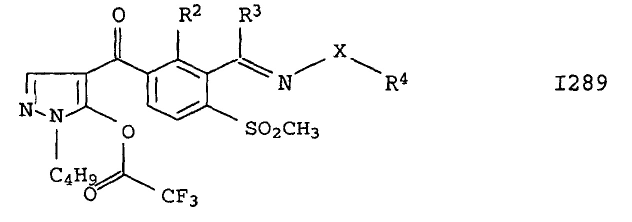
- die Verbindungen I290, insbesondere die Verbindungen I290.001-I290.180, die sich von den entsprechenden Verbindungen I1.001-I1.180 dadurch unterscheiden, daß R¹ für Methylsulfonyl, R⁹ für iso-Butyl und R¹⁰ für Trifluormethylcarbonyl stehen: 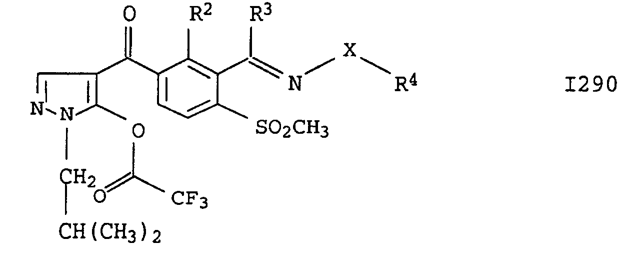
- die Verbindungen I291, insbesondere die Verbindungen I291.001-I291.180, die sich von den entsprechenden Verbindungen I1.001-I1.180 dadurch unterscheiden, daß R¹ und R¹⁰ für Methylsulfonyl stehen: 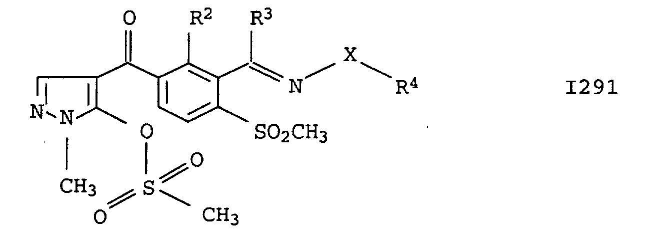
- die Verbindungen I292, insbesondere die Verbindungen I292.001-I292.180, die sich von den entsprechenden Verbindungen I1.001-I1.180 dadurch unterscheiden, daß R¹ und R¹⁰ für Methylsulfonyl und R⁹ für Ethyl stehen: 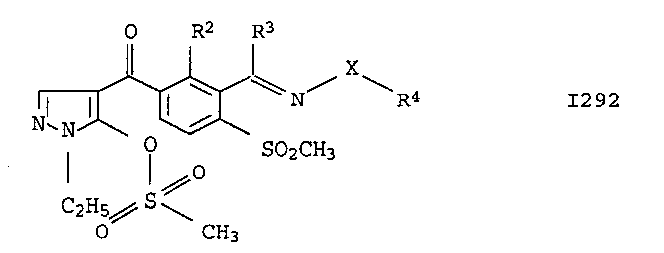
- die Verbindungen I293, insbesondere die Verbindungen I293.001-I293.180, die sich von den entsprechenden Verbindungen I1.001-I1.180 dadurch unterscheiden, daß R¹ und R¹⁰ für Methylsulfonyl und R⁹ für n-Propyl stehen stehen: 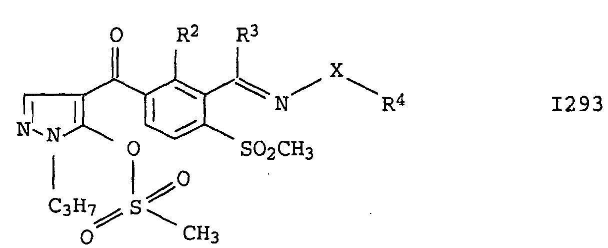
- die Verbindungen I294, insbesondere die Verbindungen I294.001-I294.180, die sich von den entsprechenden Verbindungen I1.001-I1.180 dadurch unterscheiden, daß R¹ und R¹⁰ für Methylsulfonyl und R⁹ für n-Butyl stehen: 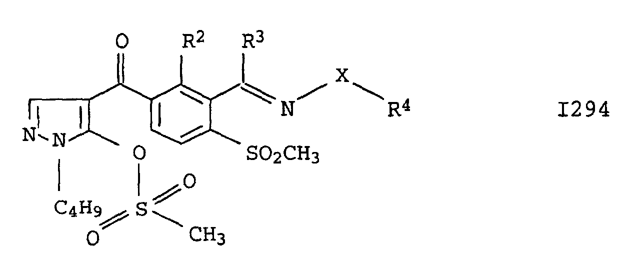
- die Verbindungen I295, insbesondere die Verbindungen I295.001-I295.180, die sich von den entsprechenden Verbindungen I1.001-I1.180 dadurch unterscheiden, daß R¹ und R¹⁰ für Methylsulfonyl und R⁹ für iso-Butyl stehen: 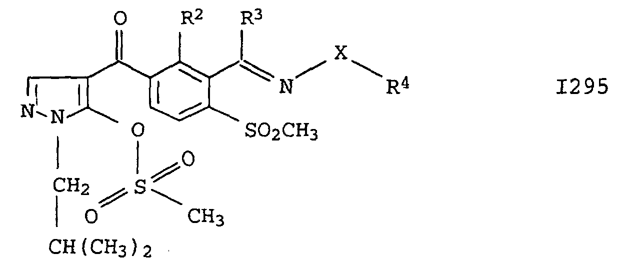
- die Verbindungen I296, insbesondere die Verbindungen I296.001-I296.180, die sich von den entsprechenden Verbindungen I1.001-I1.180 dadurch unterscheiden, daß R¹ für Methylsulfonyl und R¹⁰ für Ethylsulfonyl stehen: 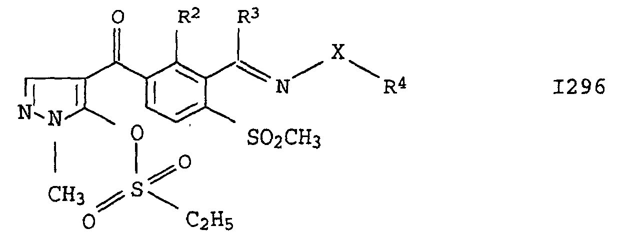
- die Verbindungen I297, insbesondere die Verbindungen I297.001-I297.180, die sich von den entsprechenden Verbindungen I1.001-I1.180 dadurch unterscheiden, daß R¹ für Methylsulfonyl, R⁹ für Ethyl und R¹⁰ für Ethylsulfonyl stehen: 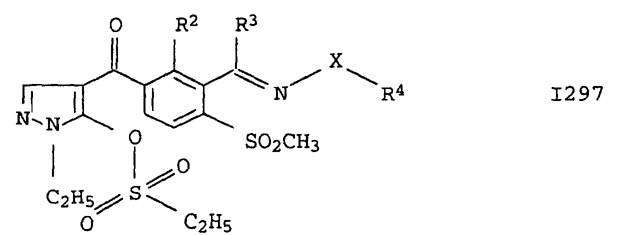
- die Verbindungen I298, insbesondere die Verbindungen I298.001-I298.180, die sich von den entsprechenden Verbindungen I1.001-I1.180 dadurch unterscheiden, daß R¹ für Methylsulfonyl, R⁹ für n-Propyl und R¹⁰ für Ethylsulfonyl stehen: 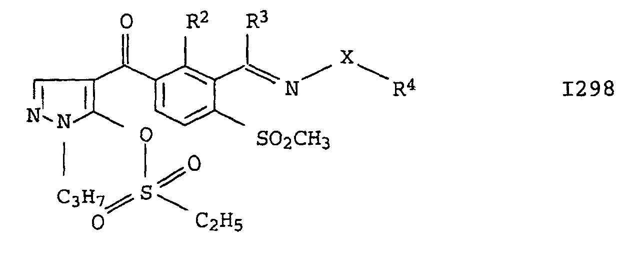
- die Verbindungen I299, insbesondere die Verbindungen I299.001-I299.180, die sich von den entsprechenden Verbindungen I1.001-I1.180 dadurch unterscheiden, daß R¹ für Methylsulfonyl, R⁹ für n-Butyl und R¹⁰ für Ethylsulfonyl stehen: 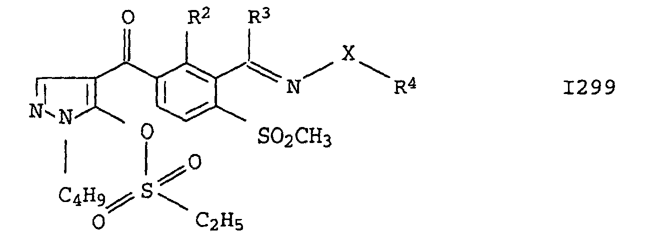
- die Verbindungen I300, insbesondere die Verbindungen I300.001-I300.180, die sich von den entsprechenden Verbindungen I1.001-I1.180 dadurch unterscheiden, daß R¹ für Methylsulfonyl, R⁹ für iso-Butyl und R¹⁰ für Ethylsulfonyl stehen: 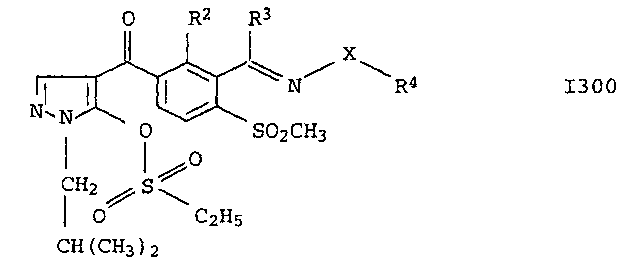
- die Verbindungen I301, insbesondere die Verbindungen I301.001-I301.180, die sich von den entsprechenden Verbindungen I1.001-I1.180 dadurch unterscheiden, daß R¹ für Methylsulfonyl und R¹⁰ für n-Propylsulfonyl stehen: 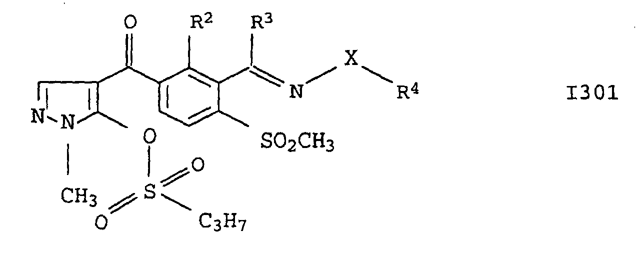
- die Verbindungen I302, insbesondere die Verbindungen I302.001-I302.180, die sich von den entsprechenden Verbindungen I1.001-I1.180 dadurch unterscheiden, daß R¹ für Methylsulfonyl, R⁹ für Ethyl und R¹⁰ für n-Propylsulfonyl stehen: 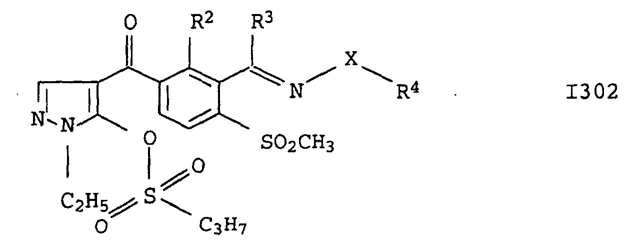
- die Verbindungen I303, insbesondere die Verbindungen I303.001-I303.180, die sich von den entsprechenden Verbindungen I1.001-I1.180 dadurch unterscheiden, daß R¹ für Methylsulfonyl, R⁹ für n-Propyl und R¹⁰ für n-Propylsulfonyl stehen: 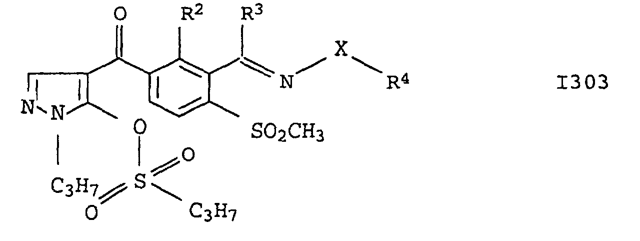
- die Verbindungen I304, insbesondere die Verbindungen I304.001-I304.180, die sich von den entsprechenden Verbindungen I1.001-I1.180 dadurch unterscheiden, daß R¹ für Methylsulfonyl, R⁹ für n-Butyl und R¹⁰ für n-Propylsulfonyl stehen: 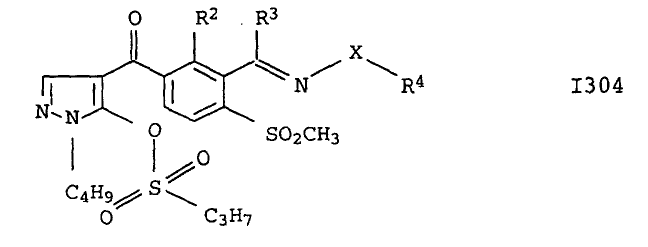
- die Verbindungen I305, insbesondere die Verbindungen I305.001-I305.180, die sich von den entsprechenden Verbindungen I1.001-I1.180 dadurch unterscheiden, daß R¹ für Methylsulfonyl, R⁹ für iso-Butyl und R¹⁰ für n-Propylsulfonyl stehen: 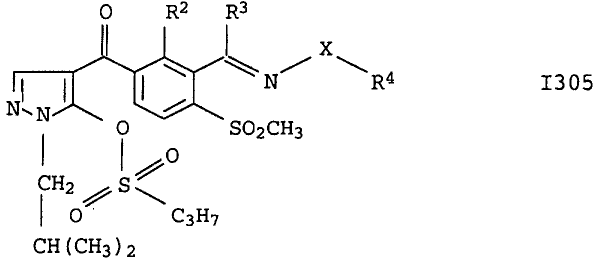
- die Verbindungen I306, insbesondere die Verbindungen I306.001-I306.180, die sich von den entsprechenden Verbindungen I1.001-I1.180 dadurch unterscheiden, daß R¹ für Methylsulfonyl und R¹⁰ für iso-Propylsulfonyl stehen: 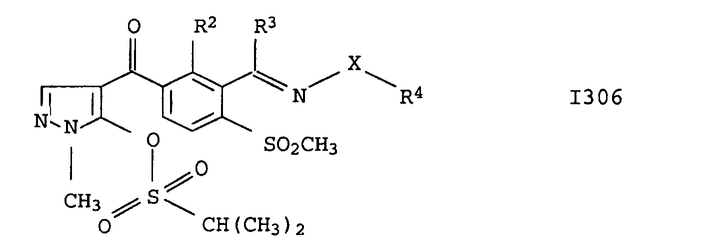
- die Verbindungen I307, insbesondere die Verbindungen I307.001-I307.180, die sich von den entsprechenden Verbindungen I1.001-I1.180 dadurch unterscheiden, daß R¹ für Methylsulfonyl, R⁹ für Ethyl und R¹⁰ für iso-Propylsulfonyl stehen: 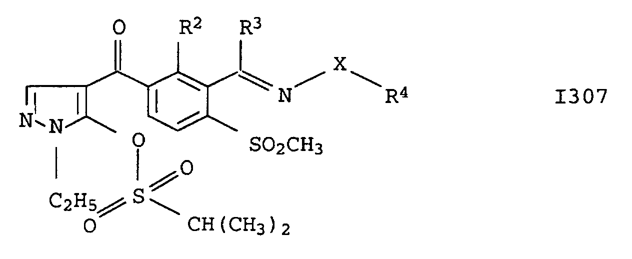
- die Verbindungen I308, insbesondere die Verbindungen I308.001-I308.180, die sich von den entsprechenden Verbindungen I1.001-I1.180 dadurch unterscheiden, daß R¹ für Methylsulfonyl, R⁹ für n-Propyl und R¹⁰ für iso-Propylsulfonyl stehen: 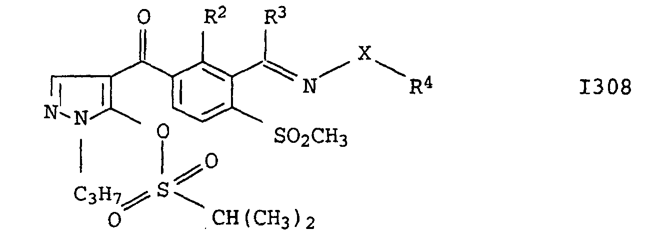
- die Verbindungen I309, insbesondere die Verbindungen I309.001-I309.180, die sich von den entsprechenden Verbindungen I1.001-I1.180 dadurch unterscheiden, daß R¹ für Methylsulfonyl, R⁹ für n-Butyl und R¹⁰ für iso-Propylsulfonyl stehen: 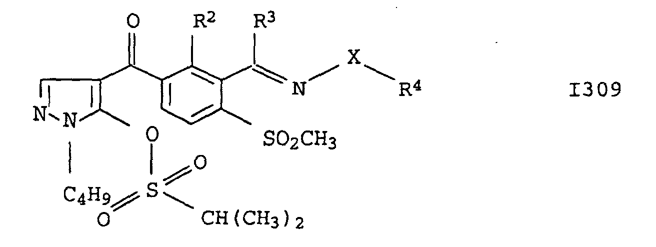
- die Verbindungen I310, insbesondere die Verbindungen I310.001-I310.180, die sich von den entsprechenden Verbindungen I1.001-I1.180 dadurch unterscheiden, daß R¹ für Methylsulfonyl, R⁹ für iso-Butyl und R¹⁰ für iso-Propylsulfonyl stehen: 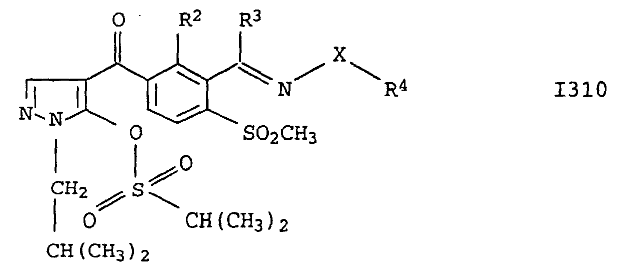
- die Verbindungen I311, insbesondere die Verbindungen I311.001-I311.180, die sich von den entsprechenden Verbindungen I1.001-I1.180 dadurch unterscheiden, daß R¹ für Methylsulfonyl und R¹⁰ für n-Butylsulfonyl stehen: 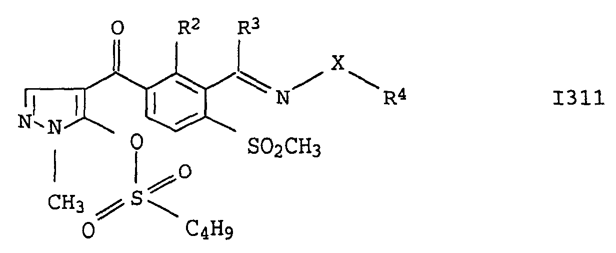
- die Verbindungen I312, insbesondere die Verbindungen I312.001-I312.180, die sich von den entsprechenden Verbindungen I1.001-I1.180 dadurch unterscheiden, daß R¹ für Methylsulfonyl, R⁹ für Ethyl und R¹⁰ für n-Butylsulfonyl stehen: 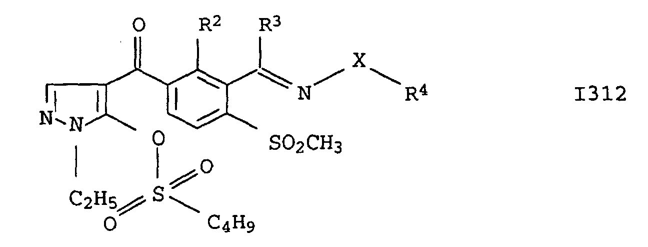
- die Verbindungen I313, insbesondere die Verbindungen I313.001-I313.180, die sich von den entsprechenden Verbindungen I1.001-I1.180 dadurch unterscheiden, daß R¹ für Methylsulfonyl, R⁹ für n-Propyl und R¹⁰ für n-Butylsulfonyl stehen: 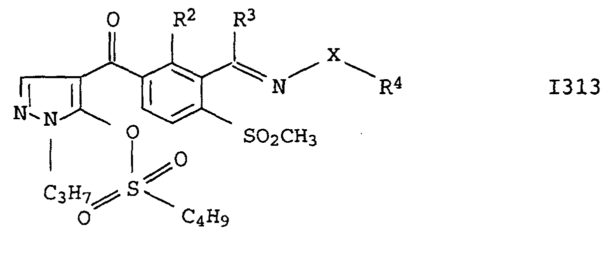
- die Verbindungen I314, insbesondere die Verbindungen I314.001-I314.180, die sich von den entsprechenden Verbindungen I1.001-I1.180 dadurch unterscheiden, daß R¹ für Methylsulfonyl, R⁹ für n-Butyl und R¹⁰ für n-Butylsulfonyl stehen: 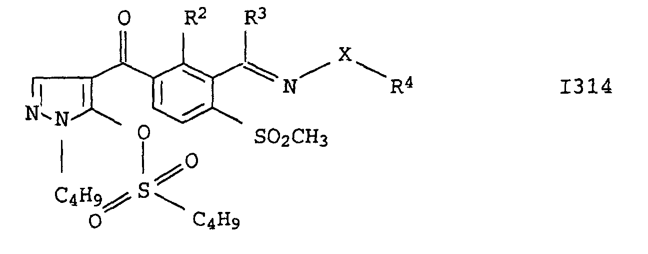
- die Verbindungen I315, insbesondere die Verbindungen I315.001-I315.180, die sich von den entsprechenden Verbindungen I1.001-I1.180 dadurch unterscheiden, daß R¹ für Methylsulfonyl, R⁹ für iso-Butyl und R¹⁰ für n-Butylsulfonyl stehen: 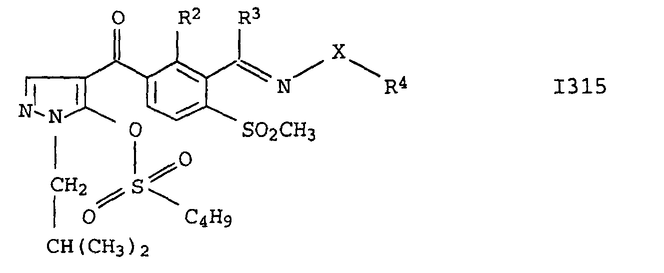
- die Verbindungen I316, insbesondere die Verbindungen I316.001-I316.180, die sich von den entsprechenden Verbindungen I1.001-I1.180 dadurch unterscheiden, daß R¹ für Methylsulfonyl und R¹⁰ für iso-Butylsulfonyl stehen: 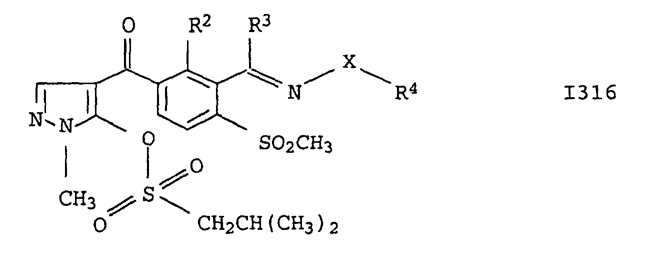
- die Verbindungen I317, insbesondere die Verbindungen I317.001-I317.180, die sich von den entsprechenden Verbindungen I1.001-I1.180 dadurch unterscheiden, daß R¹ für Methylsulfonyl, R⁹ für Ethyl und R¹⁰ für iso-Butylsulfonyl stehen: 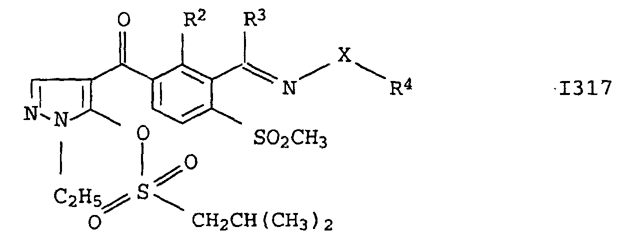
- die Verbindungen I318, insbesondere die Verbindungen I318.001-I318.180, die sich von den entsprechenden Verbindungen I1.001-I1.180 dadurch unterscheiden, daß R¹ für Methylsulfonyl, R⁹ für n-Propyl und R¹⁰ für iso-Butylsulfonyl stehen: 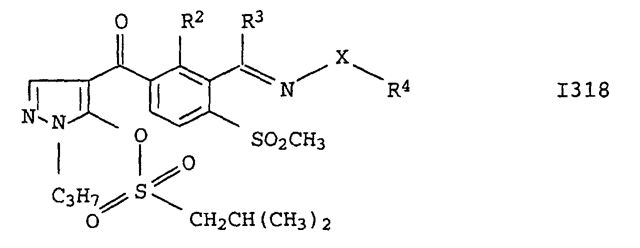
- die Verbindungen I319, insbesondere die Verbindungen I319.001-I319.180, die sich von den entsprechenden Verbindungen I1.001-I1.180 dadurch unterscheiden, daß R¹ für Methylsulfonyl, R⁹ für n-Butyl und R¹⁰ für iso-Butylsulfonyl stehen: 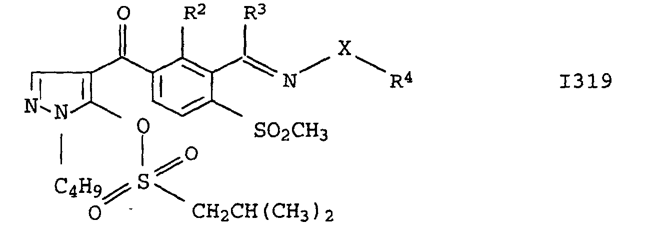
- die Verbindungen I320, insbesondere die Verbindungen I320.001-I320.180, die sich von den entsprechenden Verbindungen I1.001-I1.180 dadurch unterscheiden, daß R¹ für Methylsulfonyl, R⁹ für iso-Butyl und R¹⁰ für iso-Butylsulfonyl stehen: 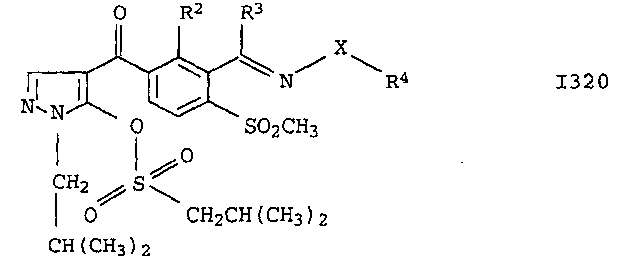
- die Verbindungen I321, insbesondere die Verbindungen I321.001-I321.180, die sich von den entsprechenden Verbindungen I1.001-I1.180 dadurch unterscheiden, daß R¹ für Methylsulfonyl und R¹⁰ für sec-Butylsulfonyl stehen: 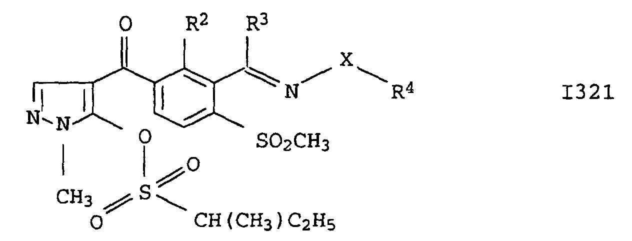
- die Verbindungen I322, insbesondere die Verbindungen I322.001-I322.180, die sich von den entsprechenden Verbindungen I1.001-I1.180 dadurch unterscheiden, daß R¹ für Methylsulfonyl, R⁹ für Ethyl und R¹⁰ für sec-Butylsulfonyl stehen: 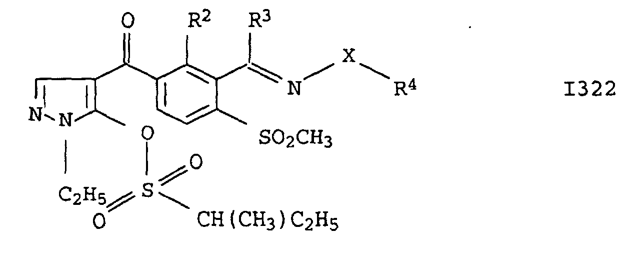
- die Verbindungen I323, insbesondere die Verbindungen I323,001-I323.180, die sich von den entsprechenden Verbindungen I1.001-I1.180 dadurch unterscheiden, daß R¹ für Methylsulfonyl, R⁹ für n-Propyl und R¹⁰ für sec-Butylsulfonyl stehen: 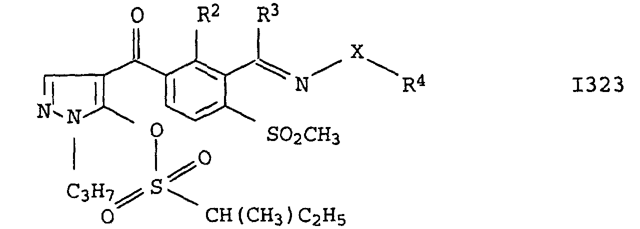
- die Verbindungen I324, insbesondere die Verbindungen I324.001-I324.180, die sich von den entsprechenden Verbindungen I1.001-I1.180 dadurch unterscheiden, daß R¹ für Methylsulfonyl, R⁹ für n-Butyl und R¹⁰ für sec-Butylsulfonyl stehen: 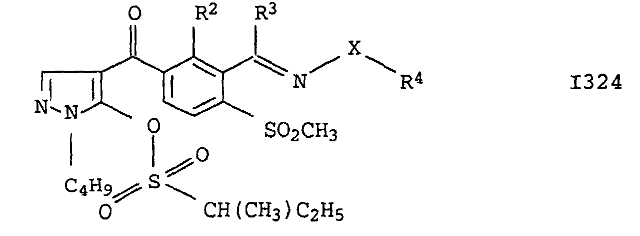
- die Verbindungen I325, insbesondere die Verbindungen I325.001-I325.180, die sich von den entsprechenden Verbindungen I1.001-I1.180 dadurch unterscheiden, daß R¹ für Methylsulfonyl, R⁹ für iso-Butyl und R¹⁰ für sec-Butylsulfonyl stehen: 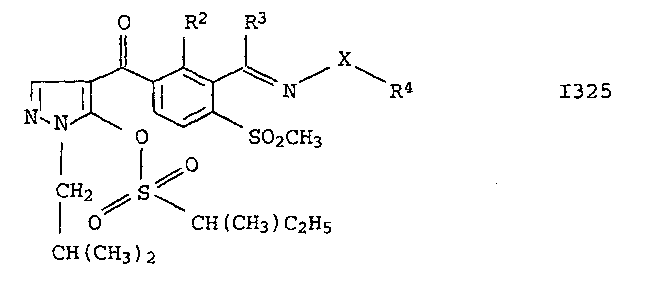
- die Verbindungen I326, insbesondere die Verbindungen I326.001-I326.180, die sich von den entsprechenden Verbindungen I1.001-I1.180 dadurch unterscheiden, daß R¹ für Methylsulfonyl und R¹⁰ für Trifluormethylsulfonyl stehen: 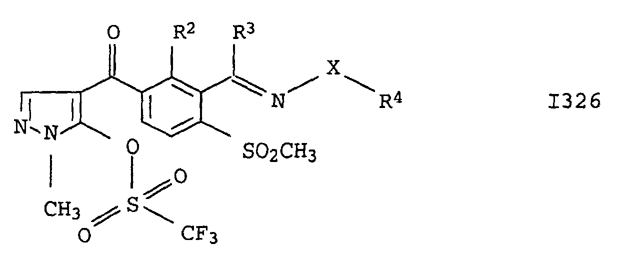
- die Verbindungen I327, insbesondere die Verbindungen I327.001-I327.180, die sich von den entsprechenden Verbindungen I1.001-I1.180 dadurch unterscheiden, daß R¹ für Methylsulfonyl, R⁹ für Ethvl und R¹⁰ für Trifluormethylsulfonyl stehen: 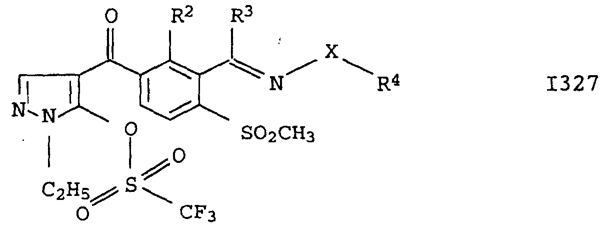
- die Verbindungen I328, insbesondere die Verbindungen I328.001-I328.180, die sich von den entsprechenden Verbindungen I1.001-I1.180 dadurch unterscheiden, daß R¹ für Methylsulfonyl, R⁹ für n-Propyl und R¹⁰ für Trifluormethylsulfonyl stehen: 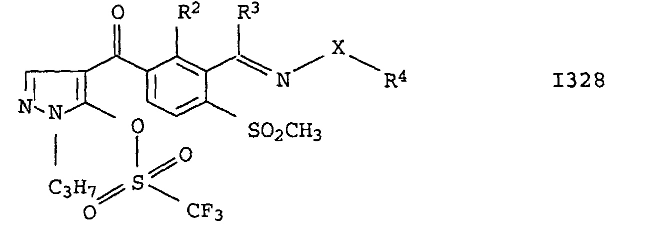
- die Verbindungen I329, insbesondere die Verbindungen I329.001-I329.180, die sich von den entsprechenden Verbindungen I1.001-I1.180 dadurch unterscheiden, daß R¹ für Methylsulfonyl, R⁹ für n-Butyl und R¹⁰ für Trifluormethylsulfonyl stehen: 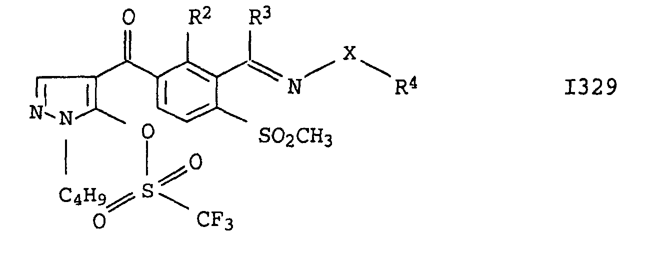
- die Verbindungen I330, insbesondere die Verbindungen I330.001-I330.180, die sich von den entsprechenden Verbindungen I1.001-I1.180 dadurch unterscheiden, daß R¹ für Methylsulfonyl, R⁹ für iso-Butyl und R¹⁰ für Trifluormethylsulfonyl stehen: 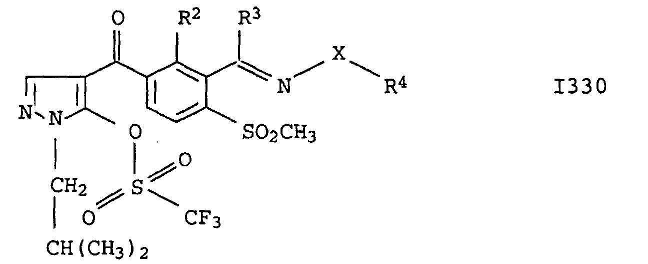
- die Verbindungen I331, insbesondere die Verbindungen I331.001-I331.180, die sich von den entsprechenden Verbindungen I1.001-I1.180 dadurch unterscheiden, daß R¹ für Methylsulfonyl und R¹⁰ für Phenylcarbonylmethyl stehen: 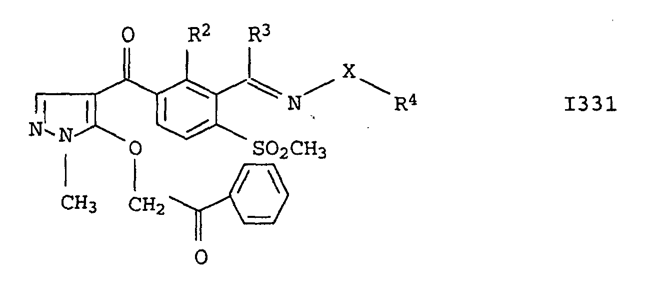
- die Verbindungen I332, insbesondere die Verbindungen I332.001-I332.180, die sich von den entsprechenden Verbindungen I1.001-I1.180 dadurch unterscheiden, daß R¹ für Methylsulfonyl, R⁹ für Ethyl und R¹⁰ für Phenylcarbonylmethyl stehen: 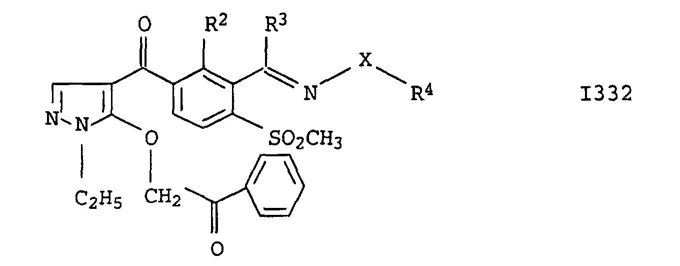
- die Verbindungen I333, insbesondere die Verbindungen I333.001-I333.180, die sich von den entsprechenden Verbindungen I1.001-I1.180 dadurch unterscheiden, daß R¹ für Methylsulfonyl, R⁹ für n-Propyl und R¹⁰ für Phenylcarbonylmethyl stehen: 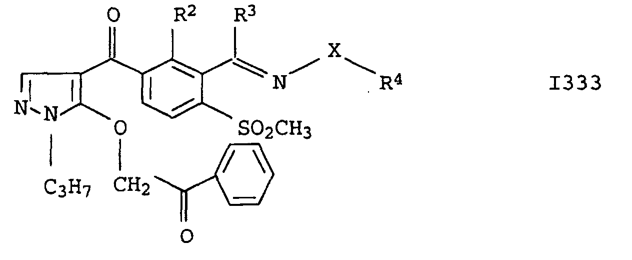
- die Verbindungen I334, insbesondere die Verbindungen I334.001-I334.180, die sich von den entsprechenden Verbindungen I1.001-I1.180 dadurch unterscheiden, daß R¹ für Methylsulfonyl, R⁹ für n-Butyl und R¹⁰ für Phenylcarbonylmethyl stehen: 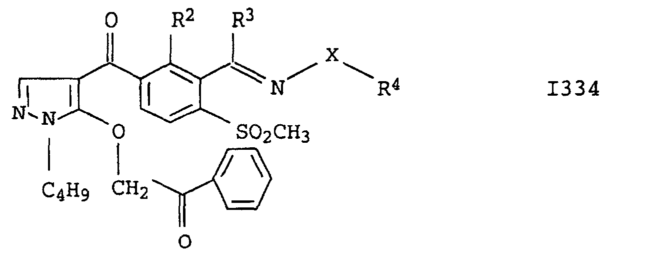
- die Verbindungen I335, insbesondere die Verbindungen I335.001-I335.180, die sich von den entsprechenden Verbindungen I1.001-I1.180 dadurch unterscheiden, daß R¹ für Methylsulfonyl, R⁹ für iso-Butyl und R¹⁰ für Phenylcarbonylmethyl stehen: 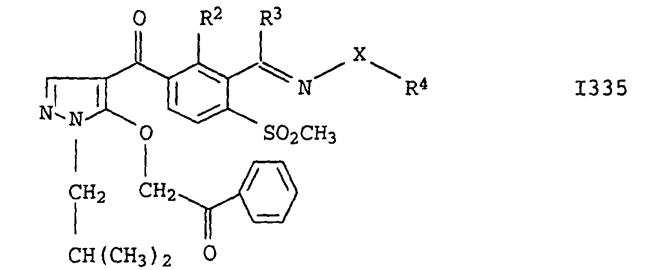
- die Verbindungen I336, insbesondere die Verbindungen I336.001-I336.180, die sich von den entsprechenden Verbindungen I1.001-I1.180 dadurch unterscheiden, daß R¹ für Methylsulfonyl und R¹⁰ für Phenylsulfonyl stehen: 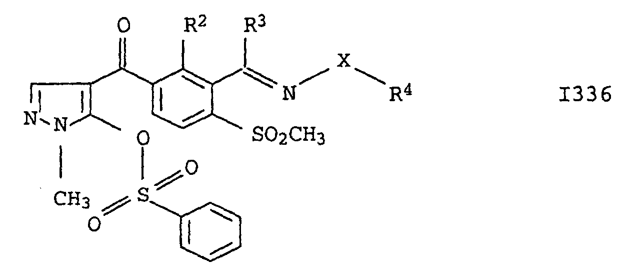
- die Verbindungen I337, insbesondere die Verbindungen I337.001-I337.180, die sich von den entsprechenden Verbindungen I1.001-I1.180 dadurch unterscheiden, daß R¹ für Methylsulfonyl, R⁹ für Ethyl und R¹⁰ für Phenylsulfonyl stehen: 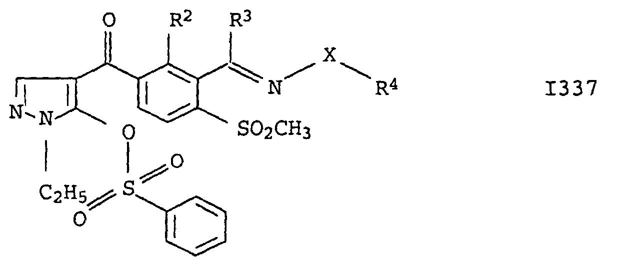
- die Verbindungen I338, insbesondere die Verbindungen I338.001-I338.180, die sich von den entsprechenden Verbindungen I1.001-I1.180 dadurch unterscheiden, daß R¹ für Methylsulfonyl, R⁹ für n-Propyl und R¹⁰ für Phenylsulfonyl stehen: 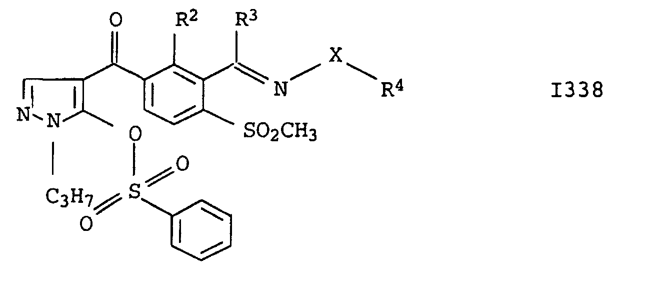
- die Verbindungen I339, insbesondere die Verbindungen I339.001-I339.180, die sich von den entsprechenden Verbindungen I1.001-I1.180 dadurch unterscheiden, daß R¹ für Methylsulfonyl, R⁹ für n-Butyl und R¹⁰ für Phenylsulfonyl stehen: 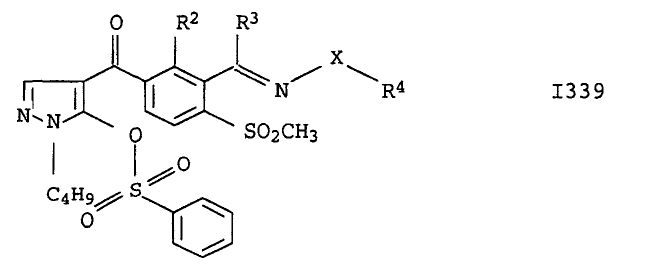
- die Verbindungen I340, insbesondere die Verbindungen I340.001-I340.180, die sich von den entsprechenden Verbindungen I1.001-I1.180 dadurch unterscheiden, daß R¹ für Methylsulfonyl, R⁹ für iso-Butyl und R¹⁰ für Phenylsulfonyl stehen: 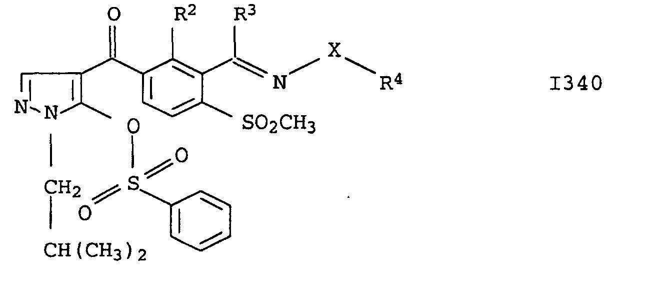
- die Verbindungen I341, insbesondere die Verbindungen I341.001-I341.180, die sich von den entsprechenden Verbindungen I1.001-I1.180 dadurch unterscheiden, daß R¹ für Methylsulfonyl und R¹⁰ für 4-Methylphenylsulfonyl stehen: 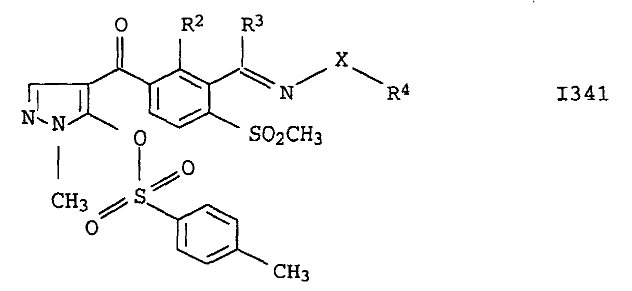
- ddie Verbindungen I342, insbesondere ie Verbindungen I342.001-I342.180, die sich von den entsprechenden Verbindungen I1.001-I1.180 dadurch unterscheiden, daß R¹ für Methylsulfonyl, R⁹ für Ethyl und R¹⁰ für 4-Methylphenylsulfonyl stehen: 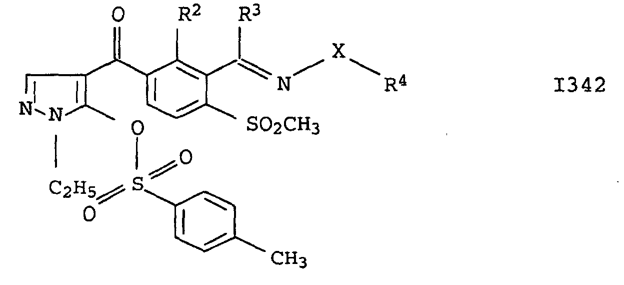
- die Verbindungen I343, insbesondere die Verbindungen I343.001-I343.180, die sich von den entsprechenden Verbindungen. I1.001-I1.180 dadurch unterscheiden, daß R¹ für Methylsulfonyl, R⁹ für n-Propyl und R¹⁰ für 4-Methylphenylsulfonyl stehen: 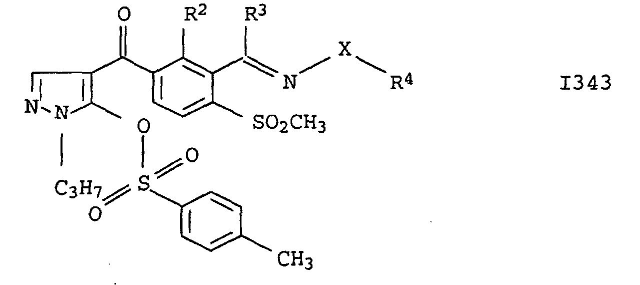
- die Verbindungen I344, insbesondere die Verbindungen I344.001-I344.180, die sich von den entsprechenden Verbindungen I1.001-I1.180 dadurch unterscheiden, daß R¹ für Methylsulfonyl, R⁹ für n-Butyl und R¹⁰ für 4-Methylphenylsulfonyl stehen: 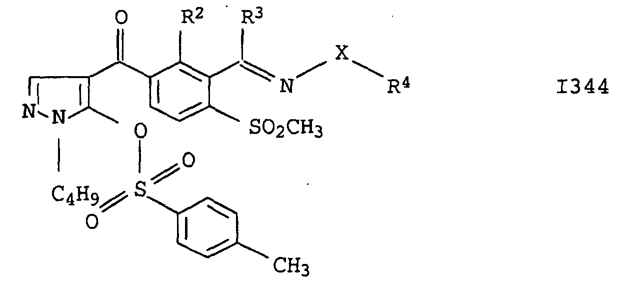
- die Verbindungen I345, insbesondere die Verbindungen I345.001-I345.180, die sich von den entsprechenden Verbindungen I1.001-I1.180 dadurch unterscheiden, daß R¹ für Methylsulfonyl, R⁹ für iso-Butyl und R¹⁰ für 4-Methylphenylsulfonyl stehen: 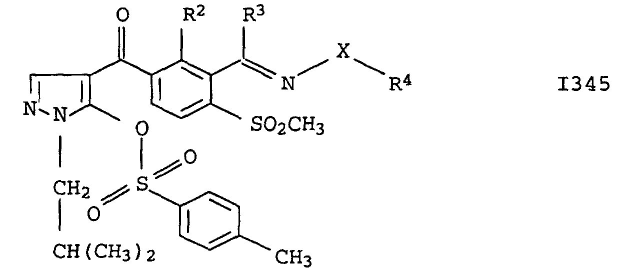
- die Verbindungen I346, insbesondere die Verbindungen I346.001-I346.180, die sich von den entsprechenden Verbindungen I1.001-I1.180 dadurch unterscheiden, daß R¹¹ für Methyl steht: 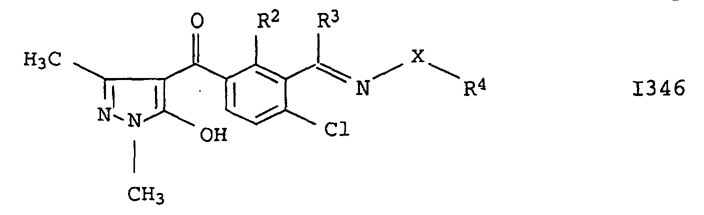
- die Verbindungen I347, insbesondere die Verbindungen I347.001-I347.180, die sich von den entsprechenden Verbindungen I1.001-I1.180 dadurch unterscheiden, daß R⁹ für Ethyl und R¹¹ für Methyl stehen: 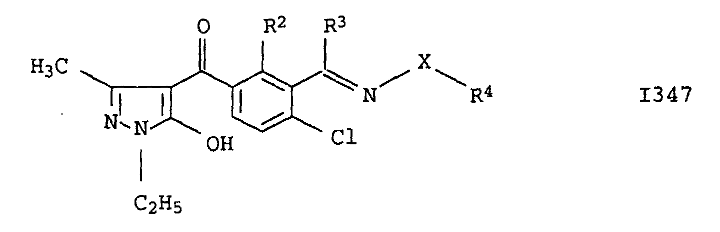
- die Verbindungen I348, insbesondere die Verbindungen I348.001-I348.180, die sich von den entsprechenden Verbindungen I1.001-I1.180 dadurch unterscheiden, daß R⁹ für n-Propyl und R¹¹ für Methyl stehen: 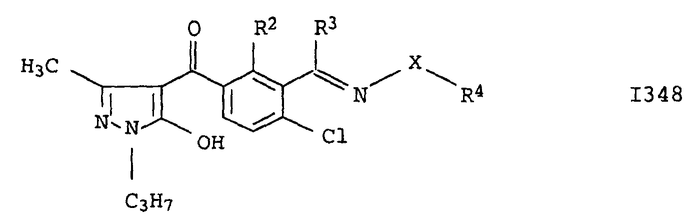
- die Verbindungen I349, insbesondere die Verbindungen I349.001-I349.180, die sich von den entsprechenden Verbindungen I1.001-I1.180 dadurch unterscheiden, daß R⁹ für n-Butyl und R¹¹ für Methyl stehen: 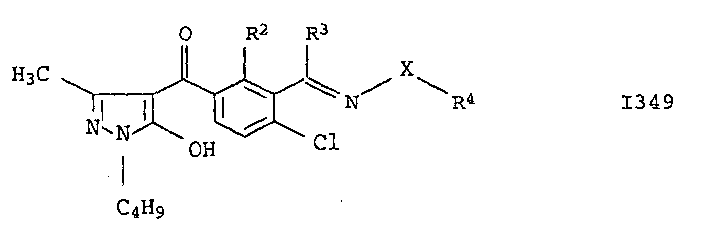
- die Verbindungen I350, insbesondere die Verbindungen I350.001-I350.180, die sich von den entsprechenden Verbindungen I1.001-I1.180 dadurch unterscheiden, daß R⁹ für iso-Butyl und R¹¹ für Methyl stehen: 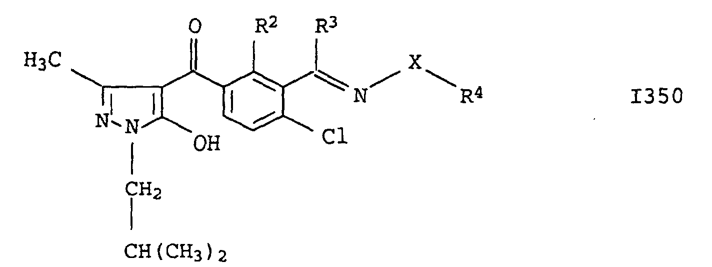
- die Verbindungen I351, insbesondere die Verbindungen I351.001-I351.180, die sich von den entsprechenden Verbindungen I1.001-I1.180 dadurch unterscheiden, daß R¹⁰ und R¹¹ für Methyl stehen: 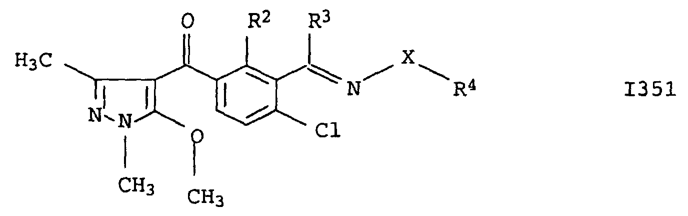
- die Verbindungen I352, insbesondere die Verbindungen I352.001-I352.180, die sich von den entsprechenden Verbindungen I1.001-I1.180 dadurch unterscheiden, daß R⁹ für Ethyl, R¹⁰ und R¹¹ für Methyl stehen: 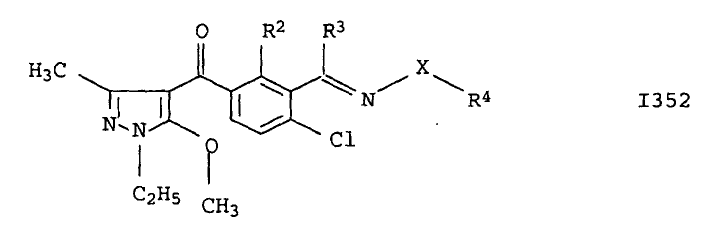
- die Verbindungen I353, insbesondere die Verbindungen I353.001-I353.180, die sich von den entsprechenden Verbindungen I1.001-I1.180 dadurch unterscheiden, daß R⁹ für n-Propyl, R¹⁰ und R¹¹ für Methyl stehen: 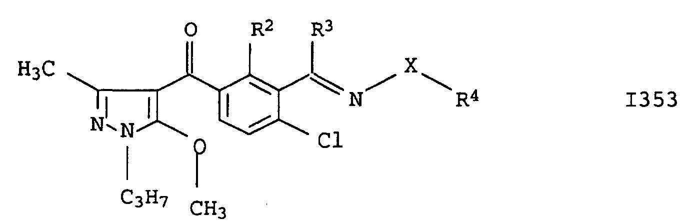
- die Verbindungen I354, insbesondere die Verbindungen I354.001-I354.180, die sich von den entsprechenden Verbindungen I1.001-I1.180 dadurch unterscheiden, daß R⁹ für n-Butyl, R¹⁰ und R¹¹ für Methyl stehen: 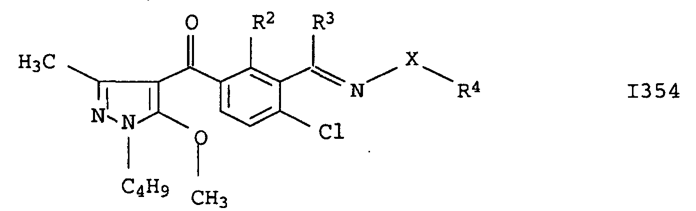
- die Verbindungen I355, insbesondere die Verbindungen I355.001-I355.180, die sich von den entsprechenden Verbindungen I1.001-I1.180 dadurch unterscheiden, daß R⁹ für iso-Butyl, R¹⁰ und R¹¹ für Methyl stehen: 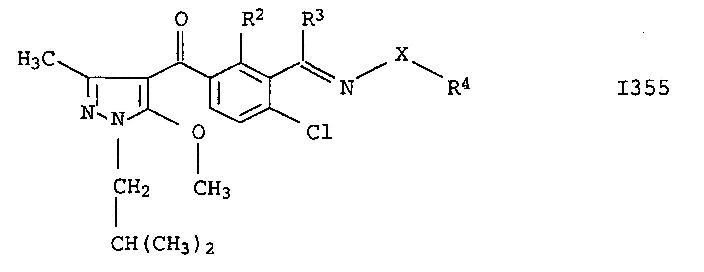
- die Verbindungen I356, insbesondere die Verbindungen I356.001-I356.180, die sich von den entsprechenden Verbindungen I1.001-I1.180 dadurch unterscheiden, daß R¹⁰ für Ethyl und R¹¹ für Methyl stehen: 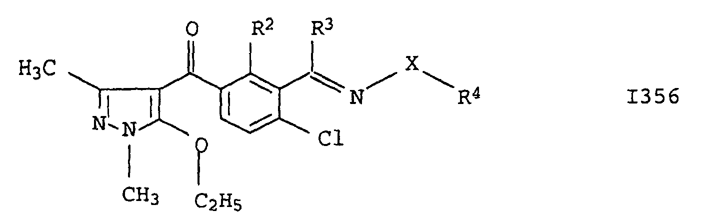
- die Verbindungen I357, insbesondere die Verbindungen I357.001-I357.180, die sich von den entsprechenden Verbindungen I1.001-I1.180 dadurch unterscheiden, daß R⁹ und R¹⁰ für Ethyl und R¹¹ für Methyl stehen: 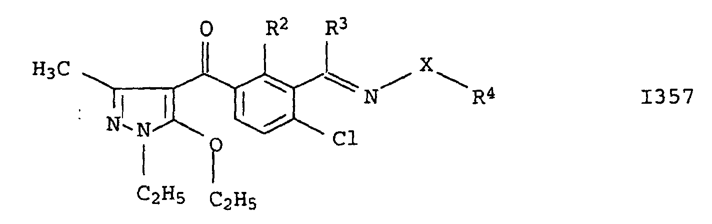
- die Verbindungen I358, insbesondere die Verbindungen I358.001-I358.180, die sich von den entsprechenden Verbindungen I1.001-I1.180 dadurch unterscheiden, daß R⁹ für n-Propyl, R¹⁰ für Ethyl und R¹¹ für Methyl stehen: 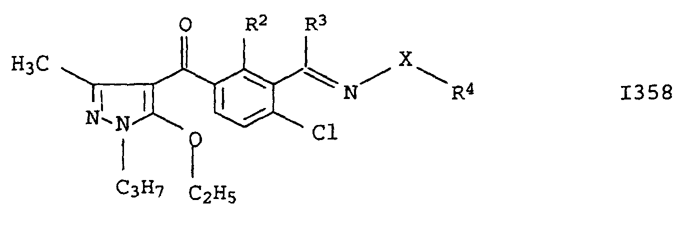
- die Verbindungen I359, insbesondere die Verbindungen I359.001-I359.180, die sich von den entsprechenden Verbindungen I1.001-I1.180 dadurch unterscheiden, daß R⁹ für n-Butyl, R¹⁰ für Ethyl und R¹¹ für Methyl stehen: 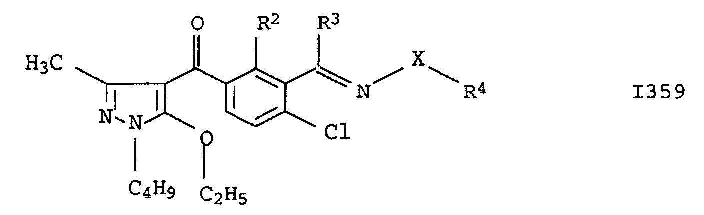
- die Verbindungen I360, insbesondere die Verbindungen I360.001-I360.180, die sich von den entsprechenden Verbindungen I1.001-I1.180 dadurch unterscheiden, daß R⁹ für iso-Butyl, R¹⁰ für Ethyl und R¹¹ für Methyl stehen: 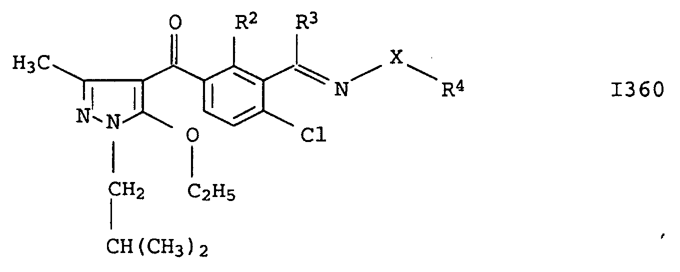
- die Verbindungen I361, insbesondere die Verbindungen I361.001-I361.180, die sich von den entsprechenden Verbindungen I1.001-I1.180 dadurch unterscheiden, daß R⁹ für n-Propyl und R¹¹ für Methyl stehen: 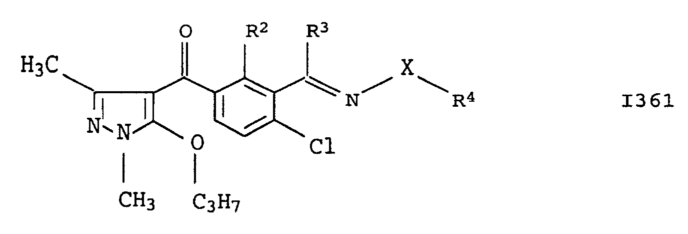
- die Verbindungen I362, insbesondere die Verbindungen I362.001-I362.180, die sich von den entsprechenden Verbindungen I1.001-I1.180 dadurch unterscheiden, daß R⁹ für Ethyl, R¹⁰ für n-Propyl und R¹¹ für Methyl stehen: 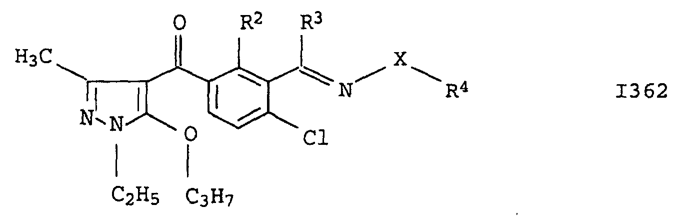
- die Verbindungen I363, insbesondere die Verbindungen I363.001-I363.180, die sich von den entsprechenden Verbindungen I1.001-I1.180 dadurch unterscheiden, daß R⁹ und R¹⁰ für n-Propyl und R¹¹ für Methyl stehen: 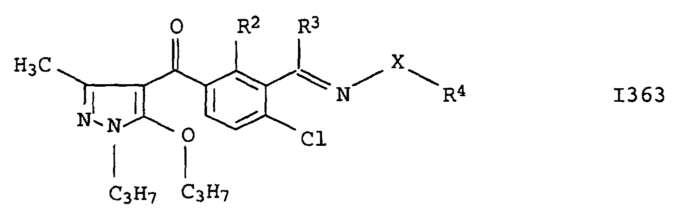
- die Verbindungen I364, insbesondere die Verbindungen I364.001-I364.180, die sich von den entsprechenden Verbindungen I1.001-I1.180 dadurch unterscheiden, daß R⁹ für n-Butyl, R¹⁰ für n-Propyl und R¹¹ für Methyl stehen: 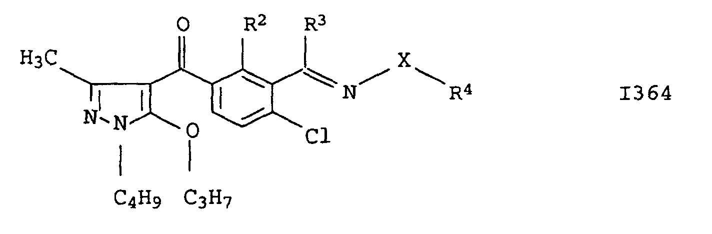
- die Verbindungen I365, insbesondere die Verbindungen I365.001-I365.180, die sich von den entsprechenden Verbindungen I1.001-I1.180 dadurch unterscheiden, daß R⁹ für iso-Butyl, R¹⁰ für n-Propyl und R¹¹ für Methyl stehen: 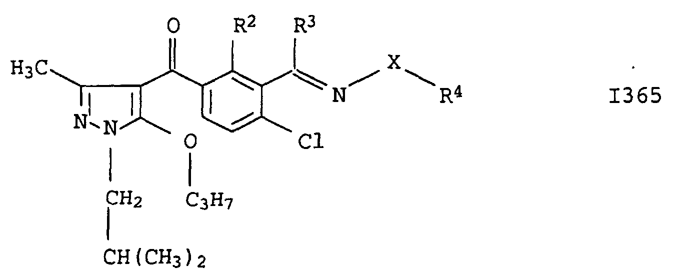
- die Verbindungen I366, insbesondere die Verbindungen I366.001-I366.180, die sich von den entsprechenden Verbindungen I1.001-I1.180 dadurch unterscheiden, daß R¹⁰ für iso-Propyl und R¹¹ für Methyl stehen: 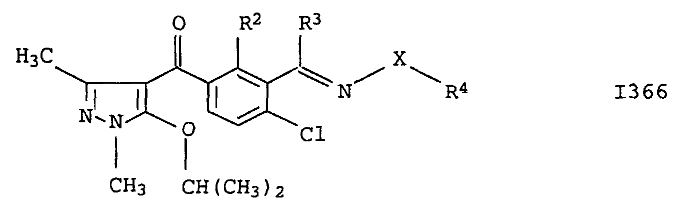
- die Verbindungen I367, insbesondere die Verbindungen I367.001-I367.180, die sich von den entsprechenden Verbindungen I1.001-I1.180 dadurch unterscheiden, daß R⁹ für Ethyl, R¹⁰ für iso-Propyl und R¹¹ für Methyl stehen: 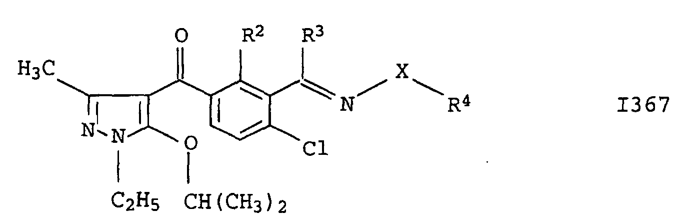
- die Verbindungen I368, insbesondere die Verbindungen I368.001-I368.180, die sich von den entsprechenden Verbindungen I1.001-I1.180 dadurch unterscheiden, daß R⁹ für n-Propyl, R¹⁰ für iso-Propyl und R¹¹ für Methyl stehen: 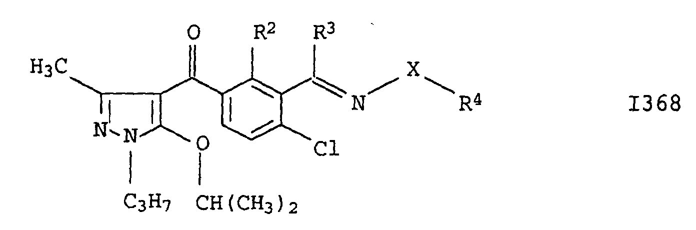
- die Verbindungen I369, insbesondere die Verbindungen I369.001-I369.180, die sich von den entsprechenden Verbindungen I1.001-I1.180 dadurch unterscheiden, daß R⁹ für n-Butyl, R¹⁰ für iso-Propyl und R¹¹ für Methyl stehen: 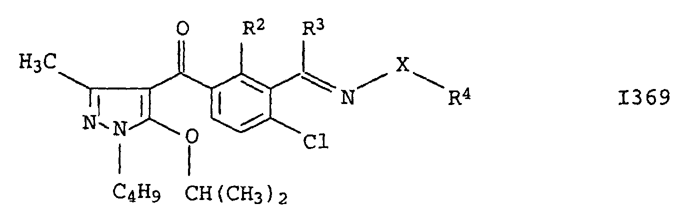
- die Verbindungen I370, insbesondere die Verbindungen I370.001-I370.180, die sich von den entsprechenden Verbindungen I1.001-I1.180 dadurch unterscheiden, daß R⁹ für iso-Butyl, R¹⁰ für iso-Propyl und R¹¹ für Methyl stehen: 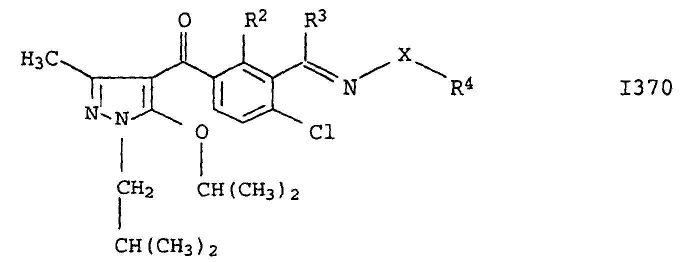
- die Verbindungen I371, insbesondere die Verbindungen I371.001-I371.180, die sich von den entsprechenden Verbindungen I1.001-I1.180 dadurch unterscheiden, daß R¹⁰ für n-Butyl und R¹¹ für Methyl stehen: 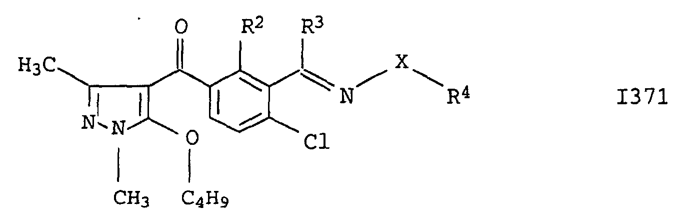
- die Verbindungen I372, insbesondere die Verbindungen I372.001-I372.180, die sich von den entsprechenden Verbindungen I1.001-I1.180 dadurch unterscheiden, daß R⁹ für Ethyl, R¹⁰ für n-Butyl und R¹¹ für Methyl stehen: 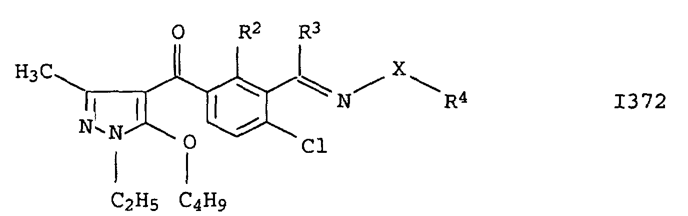
- die Verbindungen I373, insbesondere die Verbindungen I373.001-I373.180, die sich von den entsprechenden Verbindungen I1.001-I1.180 dadurch unterscheiden, daß R⁹ für n-Propyl, R¹⁰ für n-Butyl und R¹¹ für Methyl stehen: 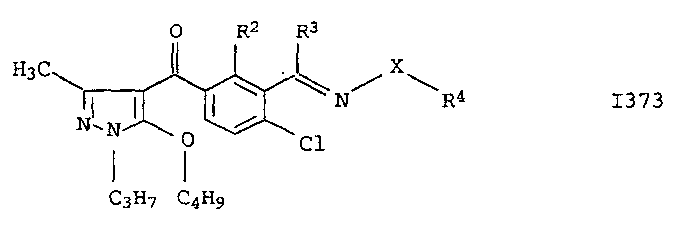
- die Verbindungen I374, insbesondere die Verbindungen I374.001-I374.180, die sich von den entsprechenden Verbindungen I1.001-I1.180 dadurch unterscheiden, daß R⁹ und R¹⁰ für n-Butyl und R¹¹ für Methyl stehen: 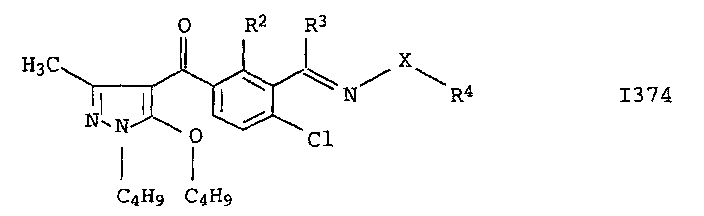
- die Verbindungen I375, insbesondere die Verbindungen I375.001-I375.180, die sich von den entsprechenden Verbindungen I1.001-I1.180 dadurch unterscheiden, daß R⁹ für iso-Butyl, R¹⁰ für n-Butyl und R¹¹ für Methyl stehen: 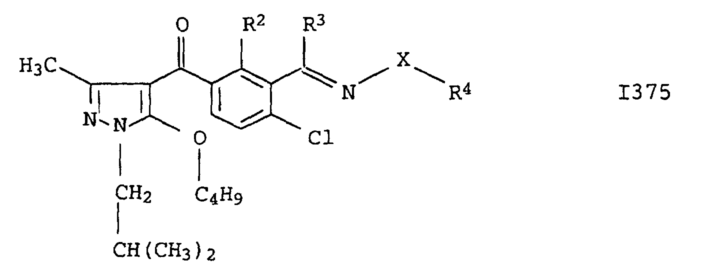
- die Verbindungen I376, insbesondere die Verbindungen I376.001-I376.180, die sich von den entsprechenden Verbindungen I1.001-I1.180 dadurch unterscheiden, daß R¹⁰ für sec-Butyl und R¹¹ für Methyl stehen: 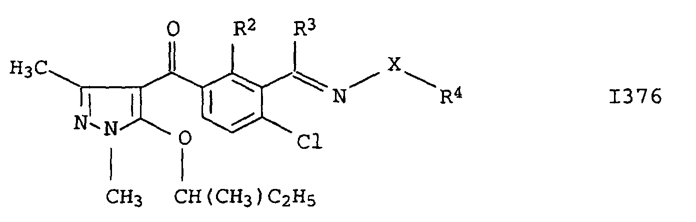
- die Verbindungen I377, insbesondere die Verbindungen I377.001-I377.180, die sich von den entsprechenden Verbindungen I1.001-I1.180 dadurch unterscheiden, daß R⁹ für Ethyl, R¹⁰ für sec-Butyl und R¹¹ für Methyl stehen: 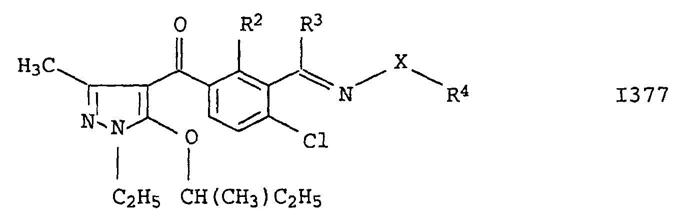
- die Verbindungen I378, insbesondere die Verbindungen I378.001-I378.180, die sich von den entsprechenden Verbindungen I1.001-I1.180 dadurch unterscheiden, daß R⁹ für n-Propyl, R¹⁰ für sec-Butyl und R¹¹ für Methyl stehen: 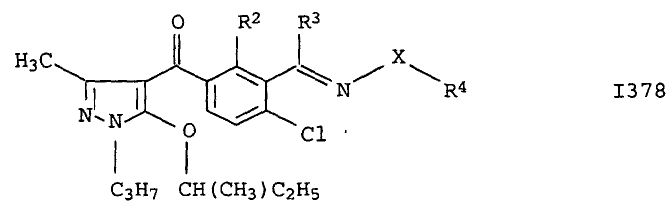
- die Verbindungen I379, insbesondere die Verbindungen I379.001-I379.180, die sich von den entsprechenden Verbindungen I1.001-I1.180 dadurch unterscheiden, daß R⁹ für n-Butyl, R¹⁰ für sec-Butyl und R¹¹ für Methyl stehen: 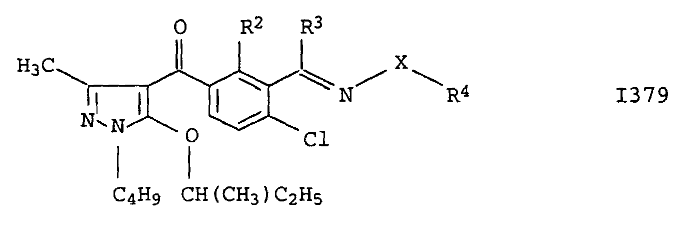
- die Verbindungen I380, insbesondere die Verbindungen I380.001-I380.180, die sich von den entsprechenden Verbindungen I1.001-I1.180 dadurch unterscheiden, daß R⁹ für iso-Butyl, R¹⁰ für sec-Butyl und R¹¹ für Methyl stehen: 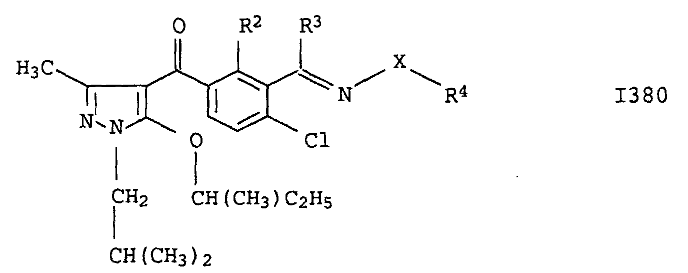
- die Verbindungen I381, insbesondere die Verbindungen I381.001-I381.180, die sich von den entsprechenden Verbindungen I1.001-I1.180 dadurch unterscheiden, daß R¹⁰ für iso-Butyl und R¹¹ für Methyl stehen: 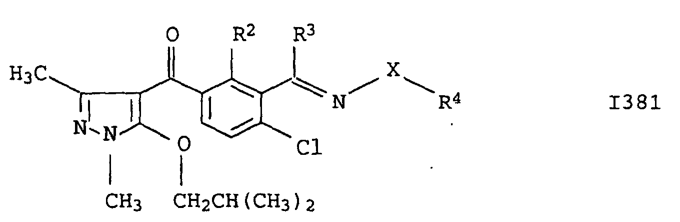
- die Verbindungen I382, insbesondere die Verbindungen I382.001-I382.180, die sich von den entsprechenden Verbindungen I1.001-I1.180 dadurch unterscheiden, daß R⁹ für Ethyl, R¹⁰ für iso-Butyl und R¹¹ für Methyl stehen: 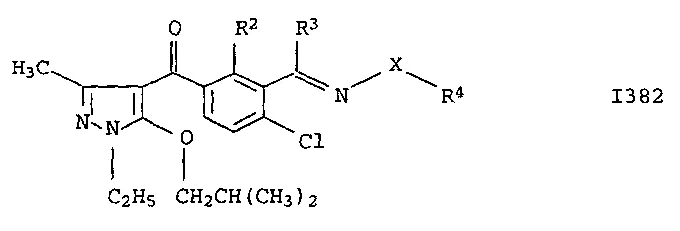
- die Verbindungen I383, insbesondere die Verbindungen I383.001-I383.180, die sich von den entsprechenden Verbindungen I1.001-I1.180 dadurch unterscheiden, daß R⁹ für n-Propyl, R¹⁰ für iso-Butyl und R¹¹ für Methyl stehen: 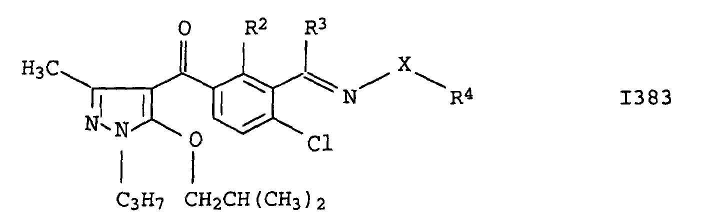
- die Verbindungen I384, insbesondere die Verbindungen I384.001-I384.180, die sich von den entsprechenden Verbindungen I1.001-I1.180 dadurch unterscheiden, daß R⁹ für n-Butyl, R¹⁰ für iso-Butyl und R¹¹ für Methyl stehen: 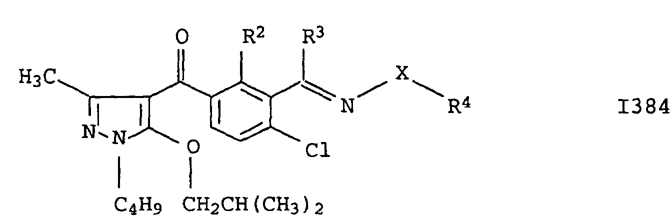
- die Verbindungen I385, insbesondere die Verbindungen I385.001-I385.180, die sich von den entsprechenden Verbindungen I1.001-I1.180 dadurch unterscheiden, daß R⁹ und R¹⁰ für iso-Butyl und R¹¹ für Methyl stehen: 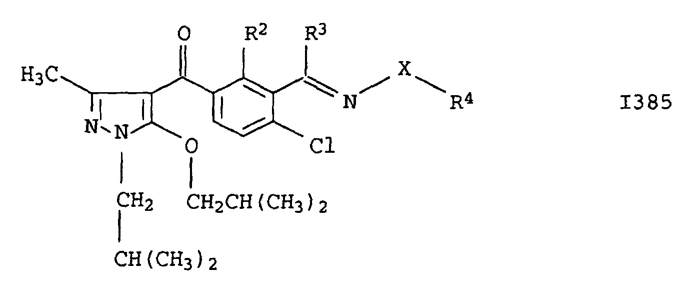
- die Verbindungen I386, insbesondere die Verbindungen I386.001-I386.180, die sich von den entsprechenden Verbindungen I1.001-I1.180 dadurch unterscheiden, daß R¹⁰ für Methylcarbonyl und R¹¹ für Methyl stehen: 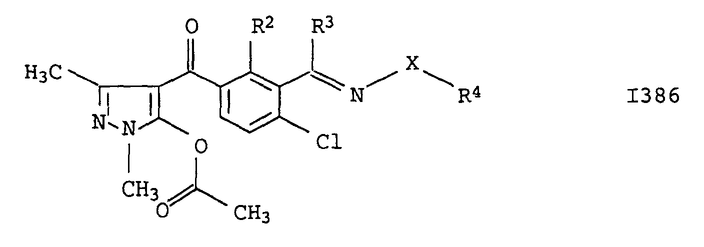
- die Verbindungen I387, insbesondere die Verbindungen I387.001-I387.180, die sich von den entsprechenden Verbindungen I1.001-I1.180 dadurch unterscheiden, daß R⁹ für Ethyl, R¹⁰ für Methylcarbonyl und R¹¹ für Methyl stehen: 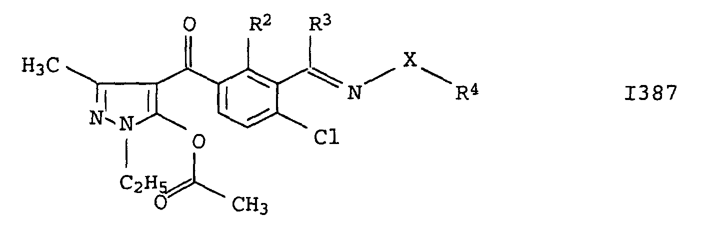
- die Verbindungen I388, insbesondere die Verbindungen I388.001-I388.180, die sich von den entsprechenden Verbindungen I1.001-I1.180 dadurch unterscheiden, daß R⁹ für n-Propyl, R¹⁰ für Methylcarbonyl und R¹¹ für Methyl stehen: 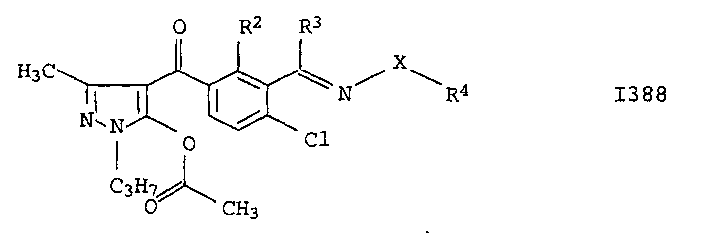
- die Verbindungen I389, insbesondere die Verbindungen I389.001-I389.180, die sich von den entsprechenden Verbindungen I1.001-I1.180 dadurch unterscheiden, daß R⁹ für n-Butyl, R¹⁰ für Methylcarbonyl und R¹¹ für Methyl stehen: 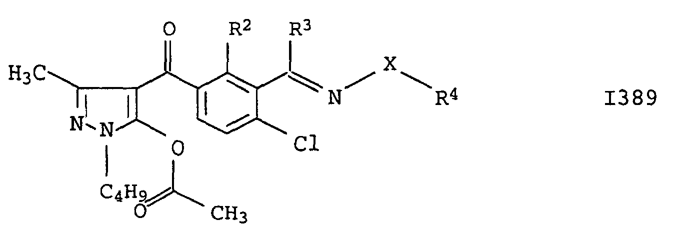
- die Verbindungen I390, insbesondere die Verbindungen I390.001-I390.180, die sich von den entsprechenden Verbindungen I1.001-I1.180 dadurch unterscheiden, daß R⁹ für iso-Butyl, R¹⁰ für Methylcarbonyl und R¹¹ für Methyl stehen: 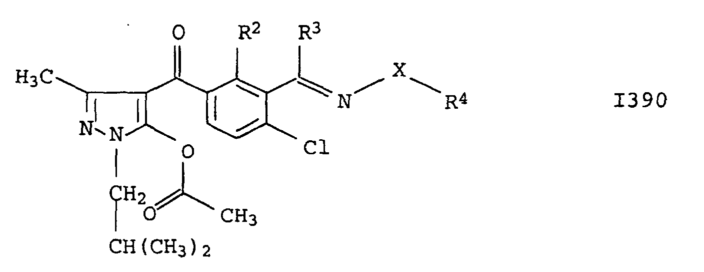
- die Verbindungen I391, insbesondere die Verbindungen I391.001-I391.180, die sich von den entsprechenden Verbindungen I1.001-I1.180 dadurch unterscheiden, daß R⁹ für Ethylcarbonyl und R¹¹ für Methyl stehen: 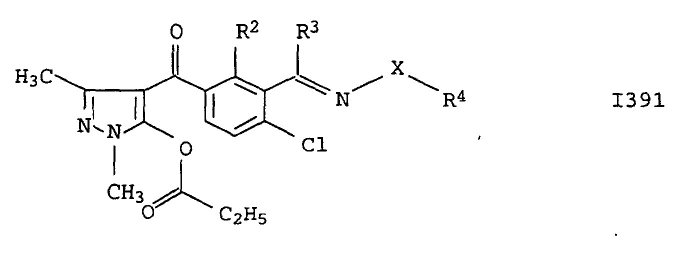
- die Verbindungen I392, insbesondere die Verbindungen I392.001-I392.180, die sich von den entsprechenden Verbindungen I1.001-I1.180 dadurch unterscheiden, daß R⁹ für Ethyl, R¹⁰ für Ethylcarbonyl und R¹¹ für Methyl stehen: 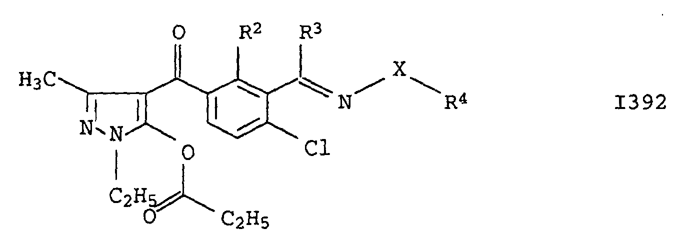
- die Verbindungen I393, insbesondere die Verbindungen I393.001-I393.180, die sich von den entsprechenden Verbindungen I1.001-I1.180 dadurch unterscheiden, daß R⁹ für n-Propyl, R¹⁰ für Ethylcarbonyl und R¹¹ für Methyl stehen: 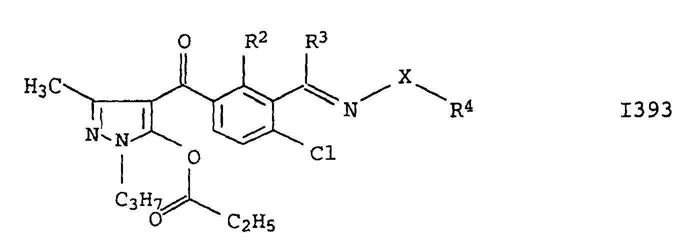
- die Verbindungen I394, insbesondere die Verbindungen I394.001-I394.180, die sich von den entsprechenden Verbindungen I1.001-I1.180 dadurch unterscheiden, daß R⁹ für n-Butyl, R¹⁰ für Ethylcarbonyl und R¹¹ für Methyl stehen: 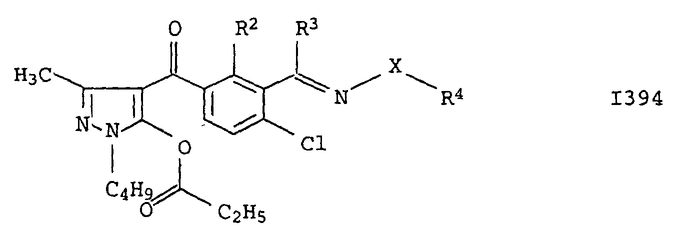
- die Verbindungen I395, insbesondere die Verbindungen I395.001-I395.180, die sich von den entsprechenden Verbindungen I1.001-I1.180 dadurch unterscheiden, daß R⁹ für iso-Butyl, R¹⁰ für Ethylcarbonyl und R¹¹ für Methyl stehen: 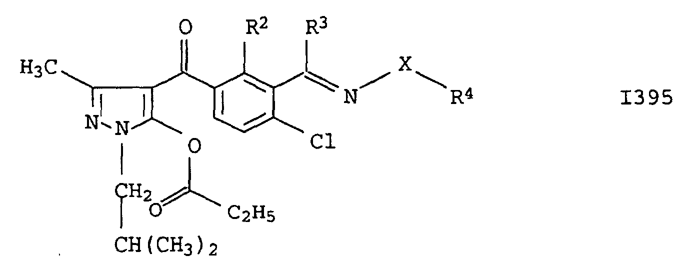
- die Verbindungen I396, insbesondere die Verbindungen I396.001-I396.180, die sich von den entsprechenden Verbindungen I1.001-I1.180 dadurch unterscheiden, daß R¹⁰ für Phenylsulfonyl und R¹¹ für Methyl stehen: 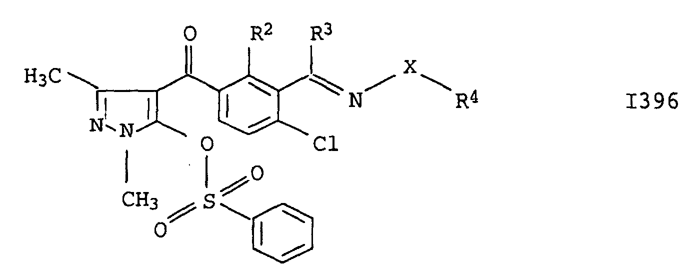
- die Verbindungen I397, insbesondere die Verbindungen I397.001-I397.180, die sich von den entsprechenden Verbindungen I1.001-I1.180 dadurch unterscheiden, daß R⁹ für Ethyl, R¹⁰ für Phenylsulfonyl und R¹¹ für Methyl stehen: 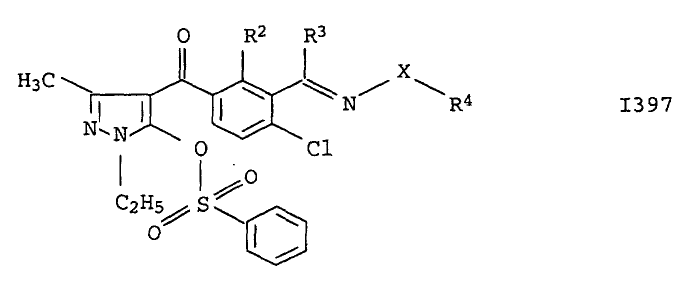
- die Verbindungen I398, insbesondere die Verbindungen I398.001-I398.180, die sich von den entsprechenden Verbindungen I1.001-I1.180 dadurch unterscheiden, daß R⁹ für n-Propyl, R¹⁰ für Phenylsulfonyl und R¹¹ für Methyl stehen: 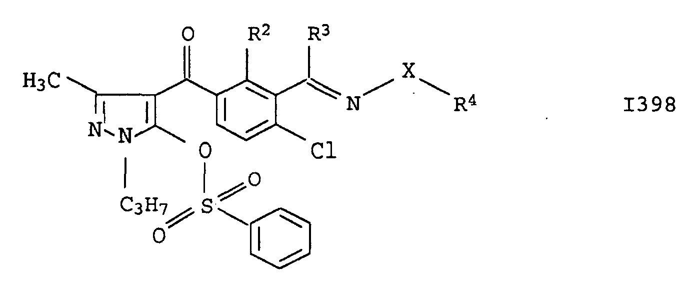
- die Verbindungen I399, insbesondere die Verbindungen I399.001-I399.180, die sich von den entsprechenden Verbindungen I1.001-I1.180 dadurch unterscheiden, daß R⁹ für n-Butyl, R¹⁰ für Phenylsulfonyl und R¹¹ für Methyl stehen: 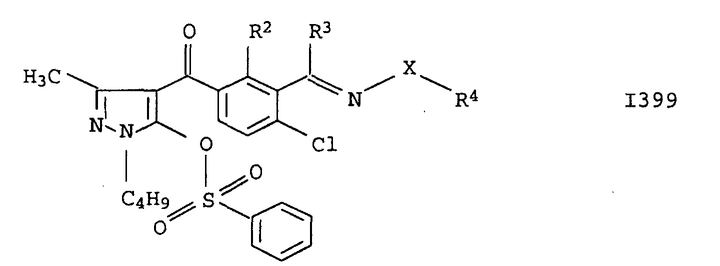
- die Verbindungen I400, insbesondere die Verbindungen I400.001-I400.180, die sich von den entsprechenden Verbindungen I1.001-I1.180 dadurch unterscheiden, daß R⁹ für iso-Butyl, R¹⁰ für Phenylsulfonyl und R¹¹ für Methyl stehen: 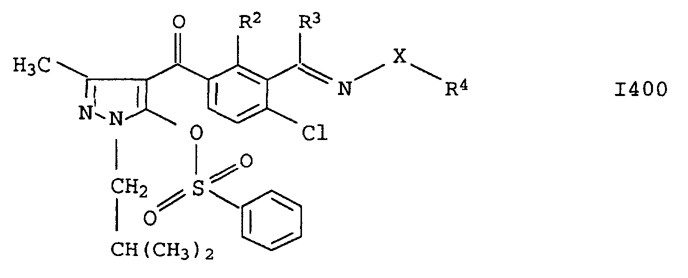
- die Verbindungen I401, insbesondere die Verbindungen I401.001-I402.180, die sich von den entsprechenden Verbindungen I1.001-I1.180 dadurch unterscheiden, daß R¹⁰ für 4-Methylphenylsulfonyl und R¹¹ für Methyl stehen: 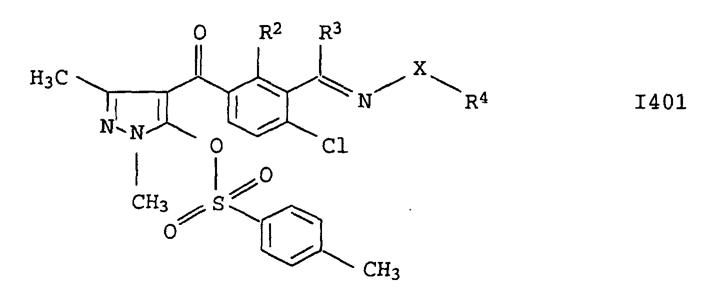
- die Verbindungen I402, insbesondere die Verbindungen I402.001-I402.180, die sich von den entsprechenden Verbindungen I1.001-I1.180 dadurch unterscheiden, daß R⁹ für Ethyl, R¹⁰ für 4-Methylphenylsulfonyl und R¹¹ für Methyl stehen: 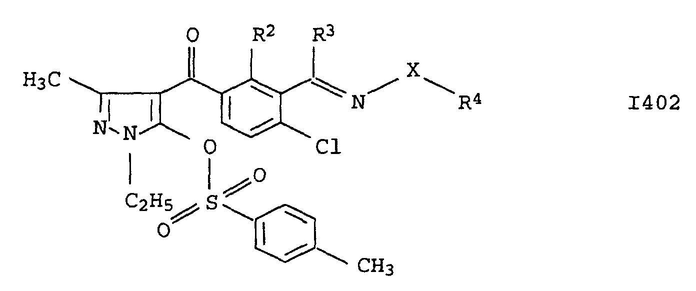
- die Verbindungen I403, insbesondere die Verbindungen I403.001-I403.180, die sich von den entsprechenden Verbindungen I1.001-I1.180 dadurch unterscheiden, daß R⁹ für n-Propyl, R¹⁰ für 4-Methylphenylsulfonyl und R¹¹ für Methyl stehen: 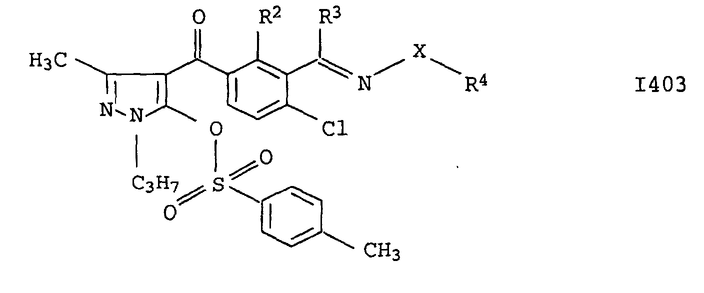
- die Verbindungen I404, insbesondere die Verbindungen I404.001-I404.180, die sich von den entsprechenden Verbindungen I1.001-I1.180 dadurch unterscheiden, daß R⁹ für n-Butyl, R¹⁰ für 4-Methylphenylsulfonyl und R¹¹ für Methyl stehen: 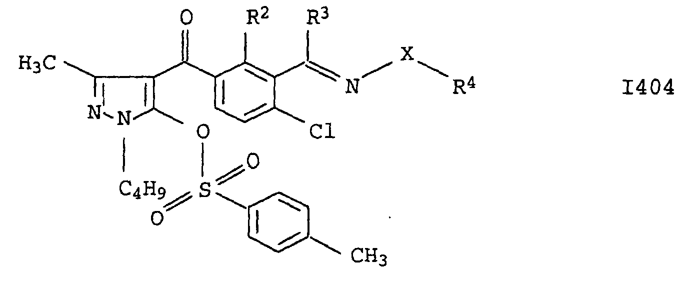
- die Verbindungen I405, insbesondere die Verbindungen I405.001-I405.180, die sich von den entsprechenden Verbindungen I1.001-I1.180 dadurch unterscheiden, daß R⁹ für iso-Butyl, R¹⁰ für 4-Methylphenylsulfonyl und R¹¹ für Methyl stehen: 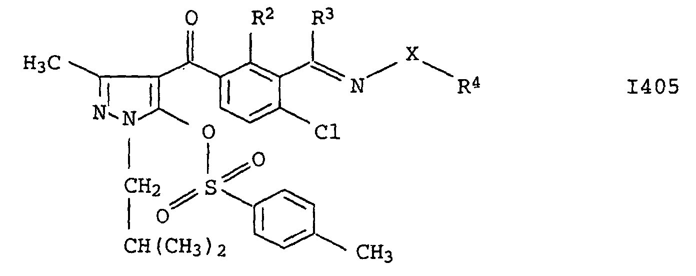
- die Verbindungen I406, insbesondere die Verbindungen I406.001-I406.180, die sich von den entsprechenden Verbindungen I1.001-I1.180 dadurch unterscheiden, daß R¹⁰ für n-Propylcarbonyl und R¹¹ für Methyl stehen: 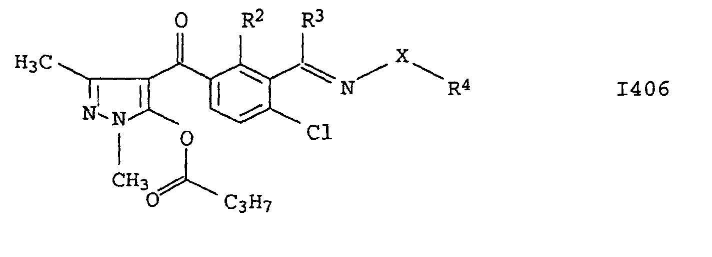
- die Verbindungen I407, insbesondere die Verbindungen I407.001-I407.180, die sich von den entsprechenden Verbindungen I1.001-I1.180 dadurch unterscheiden, daß R⁹ für Ethyl, R¹⁰ für n-Propylcarbonyl und R¹¹ für Methyl stehen: 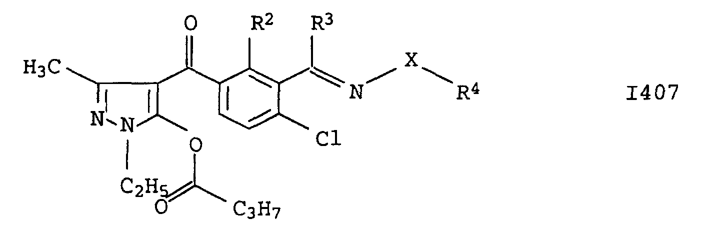
- die Verbindungen I408, insbesondere die Verbindungen I408.001-I408.180, die sich von den entsprechenden Verbindungen I1.001-I1.180 dadurch unterscheiden, daß R⁹ für n-Propyl, R¹⁰ für n-Propylcarbonyl und R¹¹ für Methyl stehen: 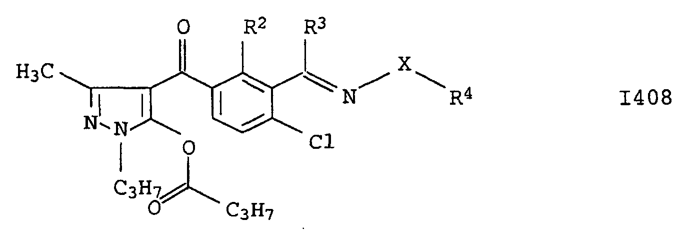
- die Verbindungen I409, insbesondere die Verbindungen I409.001-I409.180, die sich von den entsprechenden Verbindungen I1.001-I1.180 dadurch unterscheiden, daß R⁹ für n-Butyl, R¹⁰ für n-Propylcarbonyl und R¹¹ für Methyl stehen: 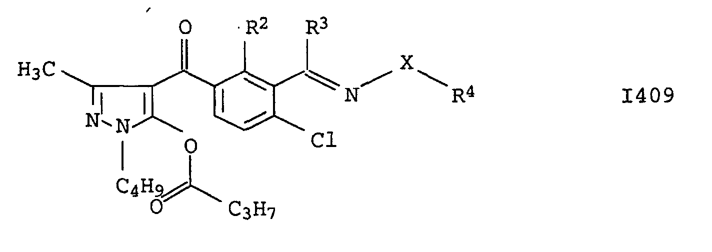
- die Verbindungen I410, insbesondere die Verbindungen I410.001-I410.180, die sich von den entsprechenden Verbindungen I1.001-I1.180 dadurch unterscheiden, daß R⁹ für iso-Butyl, R¹⁰ für n-Propylcarbonyl und R¹¹ für Methyl stehen: 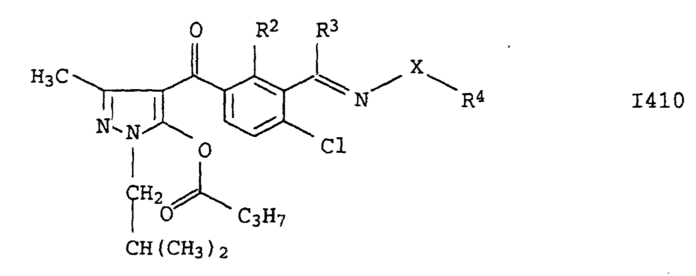
- die Verbindungen I411, insbesondere die Verbindungen I411.001-I411.180, die sich von den entsprechenden Verbindungen I1.001-I1.180 dadurch unterscheiden, daß R¹⁰ für Trifluormethylcarbonyl und R¹¹ für Methyl stehen: 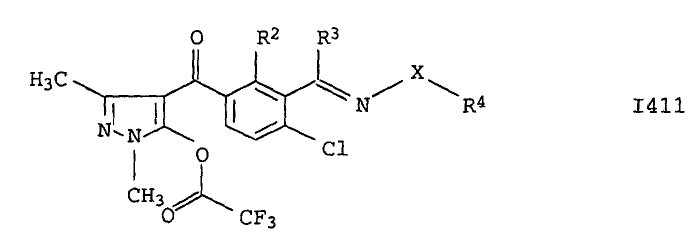
- die Verbindungen I412, insbesondere die Verbindungen I412.001-I412.180, die sich von den entsprechenden Verbindungen I1.001-I1.180 dadurch unterscheiden, daß R⁹ für Ethyl, R¹⁰ für Trifluormethylcarbonyl und R¹¹ für Methyl stehen: 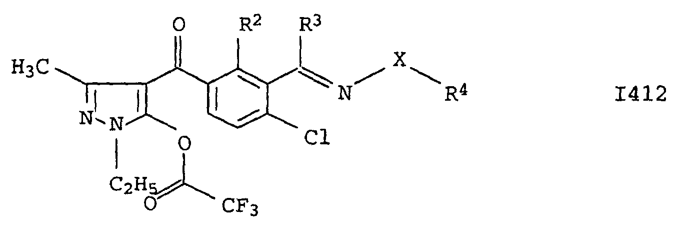
- die Verbindungen I413, insbesondere die Verbindungen I413.001-I413.180, die sich von den entsprechenden Verbindungen I1.001-I1.180 dadurch unterscheiden, daß R⁹ für n-Propyl, R¹⁰ für Trifluormethylcarbonyl und R¹¹ für Methyl stehen: 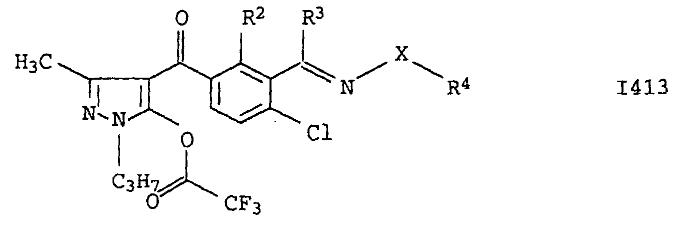
- die Verbindungen I414, insbesondere die Verbindungen I414.001-I414.180, die sich von den entsprechenden Verbindungen I1.001-I1.180 dadurch unterscheiden, daß R⁹ für n-Butyl, R¹⁰ für Trifluormethylcarbonyl und R¹¹ für Methyl stehen: 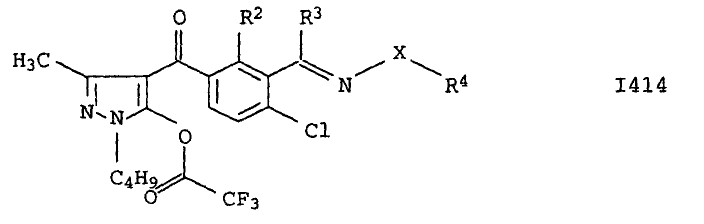
- die Verbindungen I415, insbesondere die Verbindungen I415.001-I415.180, die sich von den entsprechenden Verbindungen I1.001-I1.180 dadurch unterscheiden, daß R⁹ für iso-Butyl, R¹⁰ für Trifluormethylcarbonyl und R¹¹ für Methyl stehen: 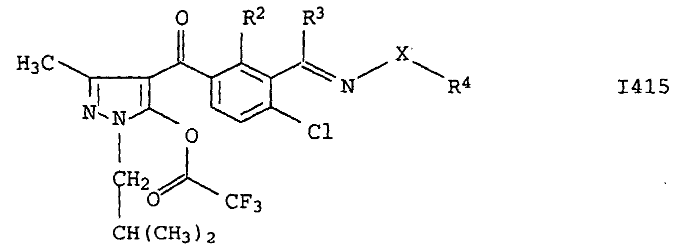
- die Verbindungen I416, insbesondere die Verbindungen I416.001-I416.180, die sich von den entsprechenden Verbindungen I1.001-I1.180 dadurch unterscheiden, daß R¹⁰ für Methylsulfonyl und R¹¹ für Methyl stehen: 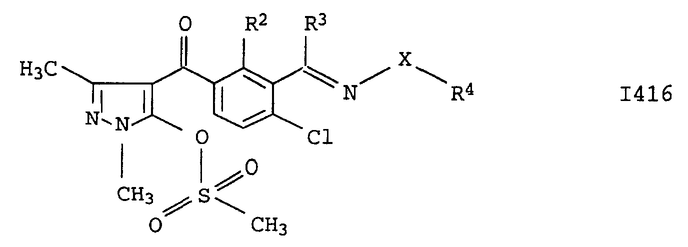
- die Verbindungen I417, insbesondere die Verbindungen I417.001-I417.180, die sich von den entsprechenden Verbindungen I1.001-I1.180 dadurch unterscheiden, daß R⁹ für Ethyl, R¹⁰ für Methylsulfonyl und R¹¹ für Methyl stehen: 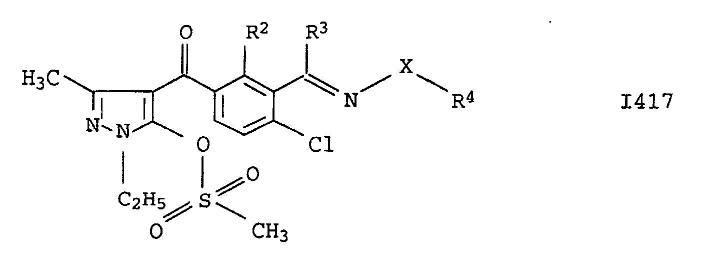
- die Verbindungen I418, insbesondere die Verbindungen I418.001-I418.180, die sich von den entsprechenden Verbindungen I1.001-I1.180 dadurch unterscheiden, daß R⁹ für n-Propyl, R¹⁰ für Methylsulfonyl und R¹¹ für Methyl stehen: 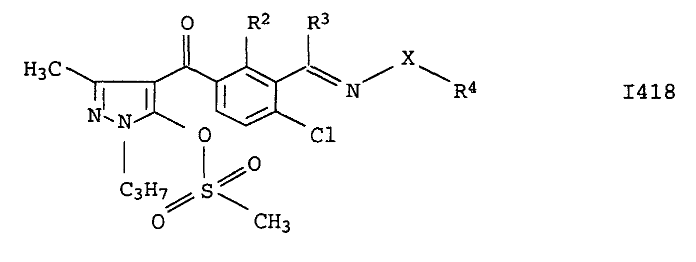
- die Verbindungen I419, insbesondere die Verbindungen I419.001-I419.180, die sich von den entsprechenden Verbindungen I1.001-I1.180 dadurch unterscheiden, daß R⁹ für n-Butyl, R¹⁰ für Methylsulfonyl und R¹¹ für Methyl stehen: 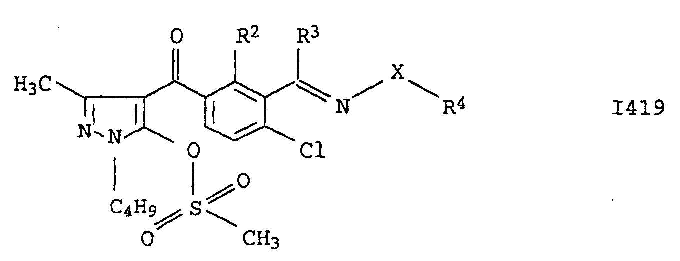
- die Verbindungen I420, insbesondere die Verbindungen I420.001-I420.180, die sich von den entsprechenden Verbindungen I1.001-I1.180 dadurch unterscheiden, daß R⁹ für iso-Butyl, R¹⁰ für Methylsulfonyl und R¹¹ für Methyl stehen: 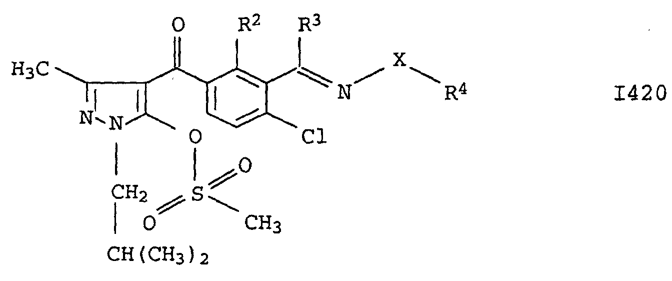
- die Verbindungen I421, insbesondere die Verbindungen I421.001-I421.180, die sich von den entsprechenden Verbindungen I1.001-I1.180 dadurch unterscheiden, daß R¹⁰ für Ethylsulfonyl und R¹¹ für Methyl stehen: 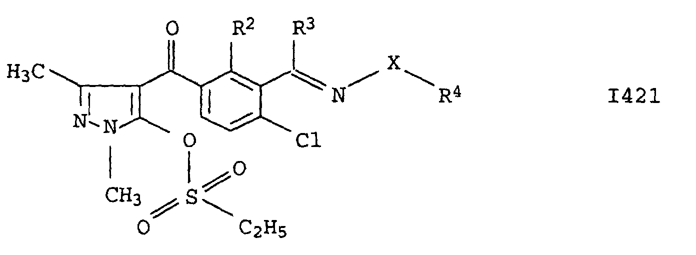
- die Verbindungen I422, insbesondere die Verbindungen I422.001-I422.180, die sich von den entsprechenden Verbindungen I1.001-I1.180 dadurch unterscheiden, daß R⁹ für Ethyl, R¹⁰ für Ethylsulfonyl und R¹¹ für Methyl stehen: 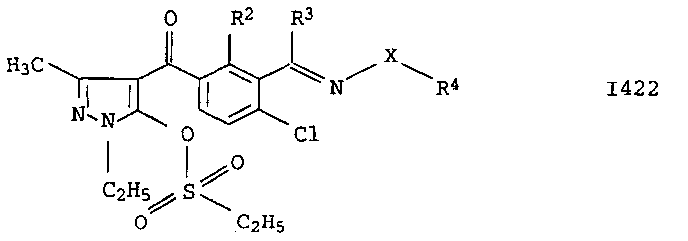
- die Verbindungen I423, insbesondere die Verbindungen I423.001-I423.180, die sich von den entsprechenden Verbindungen I1.001-I1.180 dadurch unterscheiden, daß R⁹ für n-Propyl, R¹⁰ für Ethylsulfonyl und R¹¹ für Methyl stehen: 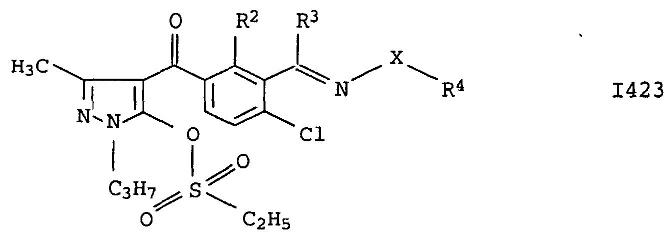
- die Verbindungen I424, insbesondere die Verbindungen I424.001-I424.180, die sich von den entsprechenden Verbindungen I1.001-I1.180 dadurch unterscheiden, daß R⁹ für n-Butyl, R¹⁰ für Ethylsulfonyl und R¹¹ für Methyl stehen: 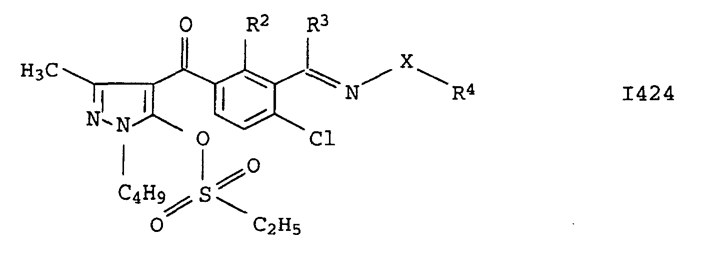
- die Verbindungen I425, insbesondere die Verbindungen I425.001-I425.180, die sich von den entsprechenden Verbindungen I1.001-I1.180 dadurch unterscheiden, daß R⁹ für iso-Butyl, R¹⁰ für Ethylsulfonyl und R¹¹ für Methyl stehen: 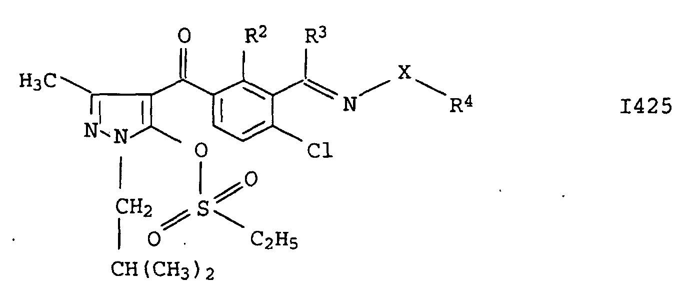
- die Verbindungen I426, insbesondere die Verbindungen I426.001-I426.180, die sich von den entsprechenden Verbindungen I1.001-I1.180 dadurch unterscheiden, daß R¹⁰ für n-Propylsulfonyl und R¹¹ für Methyl stehen: 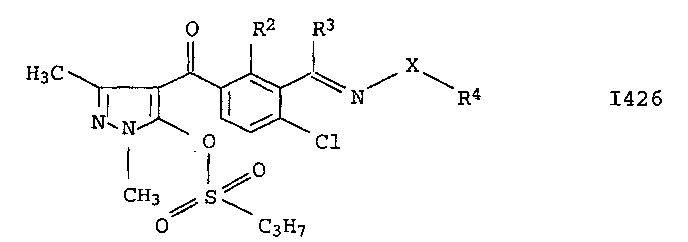
- die Verbindungen I427, insbesondere die Verbindungen I427.001-I427.180, die sich von den entsprechenden Verbindungen I1.001-I1.180 dadurch unterscheiden, daß R⁹ für Ethyl, R¹⁰ für n-Propylsulfonyl und R¹¹ für Methyl stehen: 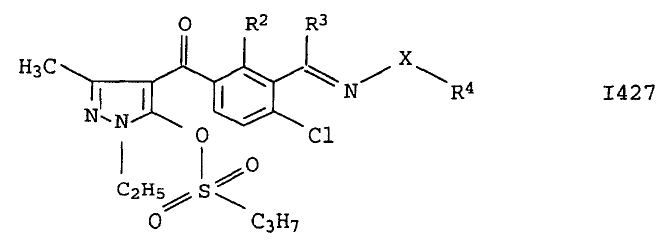
- die Verbindungen I428, insbesondere die Verbindungen I428.001-I428.180, die sich von den entsprechenden Verbindungen I1.001-I1.180 dadurch unterscheiden, daß R⁹ für n-Propyl, R¹⁰ für n-Propylsulfonyl und R¹¹ für Methyl stehen: 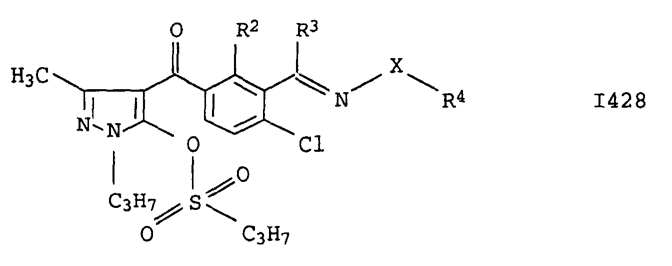
- die Verbindungen I429, insbesondere die Verbindungen I429.001-I429.180, die sich von den entsprechenden Verbindungen I1.001-I1.180 dadurch unterscheiden, daß R⁹ für n-Butyl, R¹⁰ für n-Propylsulfonyl und R¹¹ für Methyl stehen: 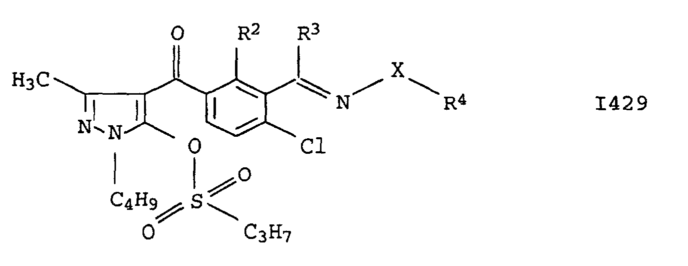
- die Verbindungen I430, insbesondere die Verbindungen I430.001-I430.180, die sich von den entsprechenden Verbindungen I1.001-I1.180 dadurch unterscheiden, daß R⁹ für iso-Butyl, R¹⁰ für Propylsulfonyl und R¹¹ für Methyl stehen: 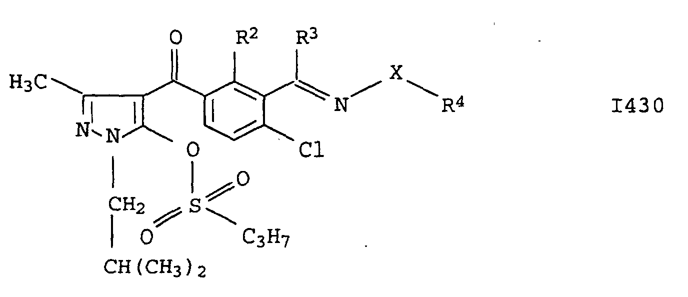
- die Verbindungen I431, insbesondere die Verbindungen I431.001-I431.180, die sich von den entsprechenden Verbindungen I1.001-I1.180 dadurch unterscheiden, daß R¹⁰ für iso-Propylsulfonyl und R¹¹ für Methyl stehen: 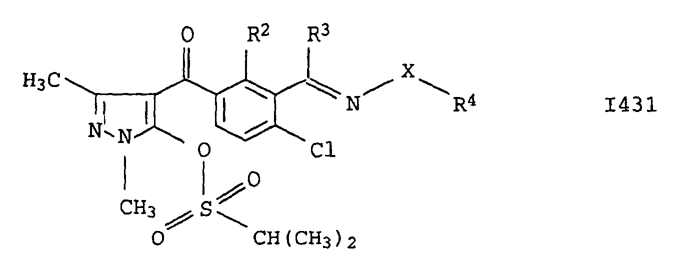
- die Verbindungen I432, insbesondere die Verbindungen I432.001-I432.180, die sich von den entsprechenden Verbindungen I1.001-I1.180 dadurch unterscheiden, daß R⁹ für Ethyl, R¹⁰ für iso-Propylsulfonyl und R¹¹ für Methyl stehen: 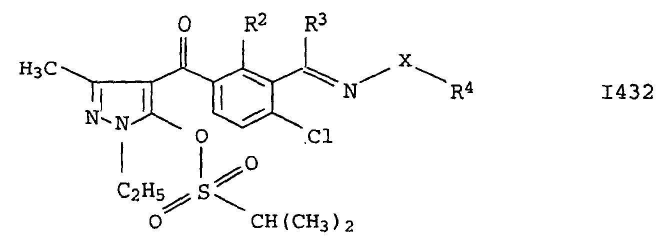
- die Verbindungen I433, insbesondere die Verbindungen I433.001-I433.180, die sich von den entsprechenden Verbindungen I1.001-I1.180 dadurch unterscheiden, daß R⁹ für n-Propyl, R¹⁰ für iso-Propylsulfonyl und R¹¹ für Methyl stehen: 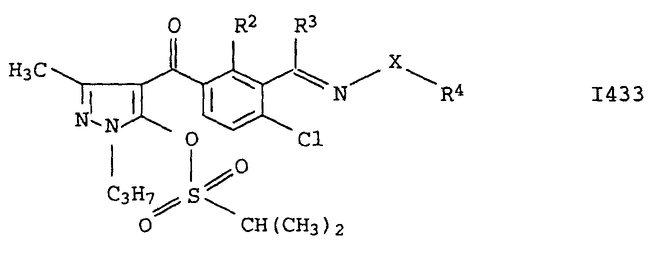
- die Verbindungen I434, insbesondere die Verbindungen I434.001-I434.180, die sich von den entsprechenden Verbindungen I1.001-I1.180 dadurch unterscheiden, daß R⁹ für n-Butyl, R¹⁰ für iso-Propylsulfonyl und R¹¹ für Methyl stehen: 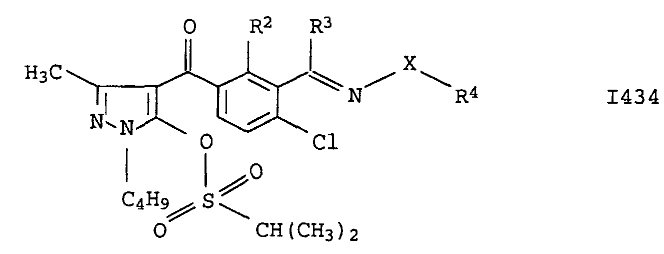
- die Verbindungen I435, insbesondere die Verbindungen I435.001-I435.180, die sich von den entsprechenden Verbindungen I1.001-I1.180 dadurch unterscheiden, daß R⁹ für iso-Butyl, R¹⁰ für iso-Propylsulfonyl und R¹¹ für Methyl stehen: 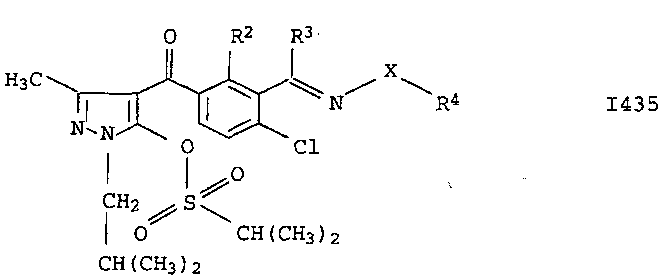
- die Verbindungen I436, insbesondere die Verbindungen I436.001-I436.180, die sich von den entsprechenden Verbindungen I1.001-I1.180 dadurch unterscheiden, daß R¹⁰ für n-Butylsulfonyl und R¹¹ für Methyl stehen: 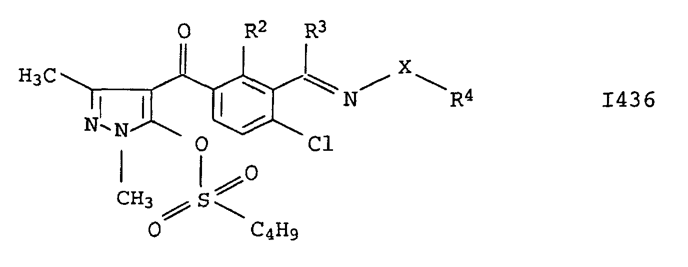
- die Verbindungen I437, insbesondere die Verbindungen I437.001-I437.180, die sich von den entsprechenden Verbindungen I1.001-I1.180 dadurch unterscheiden, daß R⁹ für Ethyl, R¹⁰ für n-Butylsulfonyl und R¹¹ für Methyl stehen: 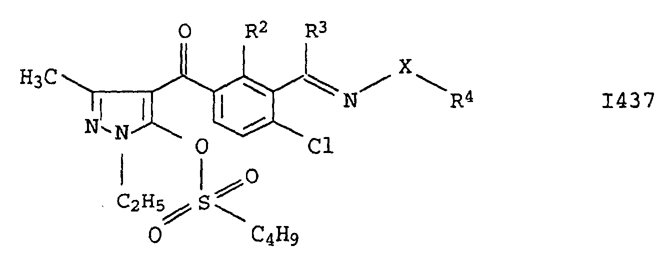
- die Verbindungen I438, insbesondere die Verbindungen I438.001-I438.180, die sich von den entsprechenden Verbindungen I1.001-I1.180 dadurch unterscheiden, daß R⁹ für n-Propyl, R¹⁰ für n-Butylsulfonyl und R¹¹ für Methyl stehen: 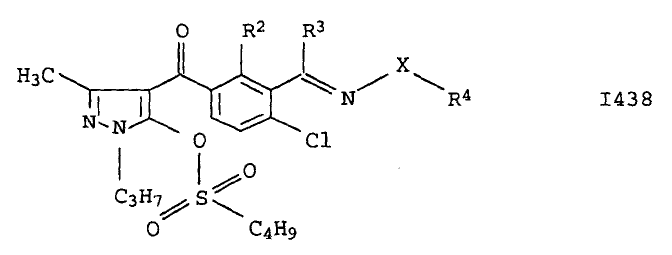
- die Verbindungen I439, insbesondere die Verbindungen I439.001-I439.180, die sich von den entsprechenden Verbindungen I1.001-I1.180 dadurch unterscheiden, daß R⁹ für n-Butyl, R¹⁰ für n-Butylsulfonyl und R¹¹ für Methyl stehen: 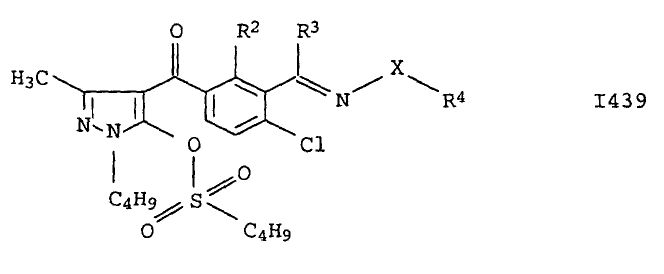
- die Verbindungen I440, insbesondere die Verbindungen I440.001-I440.180, die sich von den entsprechenden Verbindungen I1.001-I1.180 dadurch unterscheiden, daß R⁹ für iso-Butyl, R¹⁰ für n-Butylsulfonyl und R¹¹ für Methyl stehen: 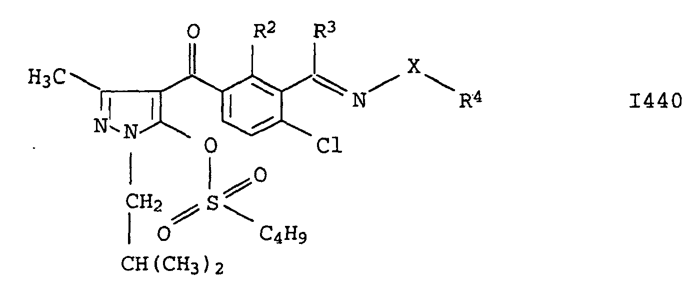
- die Verbindungen I441, insbesondere die Verbindungen I441.001-I441.180, die sich von den entsprechenden Verbindungen I1.001-I1.180 dadurch unterscheiden, daß R¹⁰ für iso-Butylsulfonyl und R¹¹ für Methyl stehen: 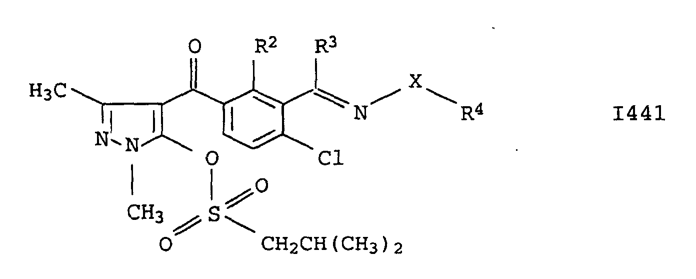
- die Verbindungen I442, insbesondere die Verbindungen I442.001-I442.180, die sich von den entsprechenden Verbindungen I1.001-I1.180 dadurch unterscheiden, daß R⁹ für Ethyl, R¹⁰ für iso-Butylsulfonyl und R¹¹ für Methyl stehen: 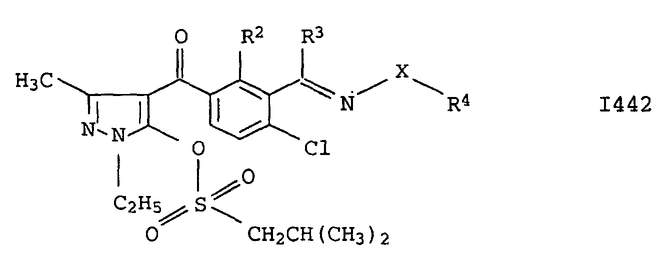
- die Verbindungen I443, insbesondere die Verbindungen I443.001-I443.180, die sich von den entsprechenden Verbindungen I1.001-I1.180 dadurch unterscheiden, daß R⁹ für n-Propyl, R¹⁰ für iso-Butylsulfonyl und R¹¹ für Methyl stehen: 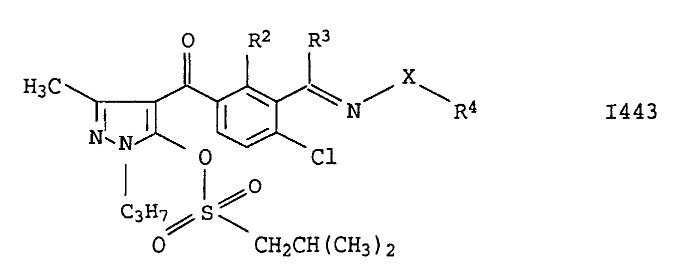
- die Verbindungen I444, insbesondere die Verbindungen I444.001-I444.180, die sich von den entsprechenden Verbindungen I1.001-I1.180 dadurch unterscheiden, daß R⁹ für n-Butyl, R¹⁰ für iso-Butylsulfonyl und R¹¹ für Methyl stehen: 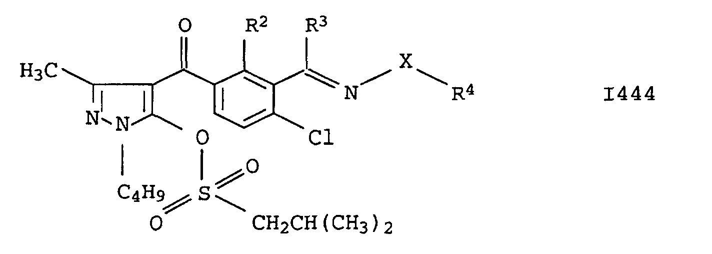
- die Verbindungen I445, insbesondere die Verbindungen I445.001-I445.180, die sich von den entsprechenden Verbindungen I1.001-I1.180 dadurch unterscheiden, daß R⁹ für iso-Butyl, R¹⁰ für iso-Butylsulfonyl und R¹¹ für Methyl stehen: 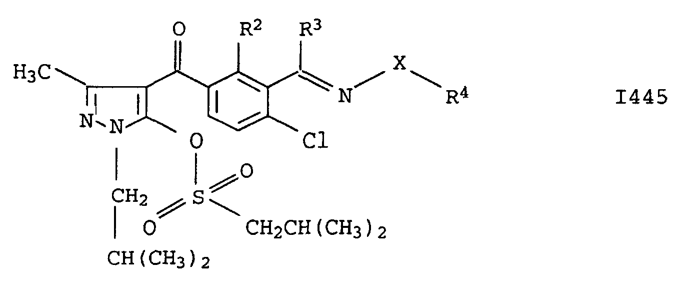
- die Verbindungen I446, insbesondere die Verbindungen I446.001-I446.180, die sich von den entsprechenden Verbindungen I1.001-I1.180 dadurch unterscheiden, daß R¹⁰ für sec-Butylsulfonyl und R¹¹ für Methyl stehen: 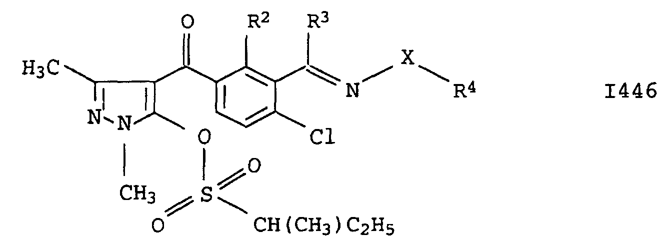
- die Verbindungen I447, insbesondere die Verbindungen I447.001-I447.180, die sich von den entsprechenden Verbindungen I1.001-I1.180 dadurch unterscheiden, daß R⁹ für Ethyl, R¹⁰ für sec-Butylsulfonyl und R¹¹ für Methyl stehen: 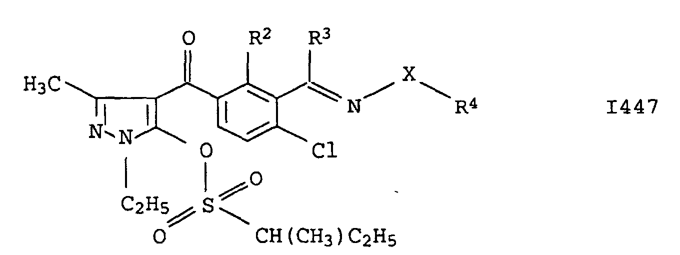
- die Verbindungen I448, insbesondere die Verbindungen I448.001-I448.180, die sich von den entsprechenden Verbindungen I1.001-I1.180 dadurch unterscheiden, daß R⁹ für n-Propyl, R¹⁰ für sec-Butylsulfonyl und R¹¹ für Methyl stehen: 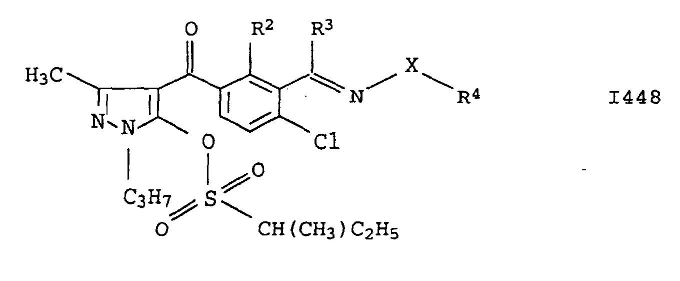
- die Verbindungen I449, insbesondere die Verbindungen I449.001-I449.180, die sich von den entsprechenden Verbindungen I1.001-I1.180 dadurch unterscheiden, daß R⁹ für n-Butyl, R¹⁰ für sec-Butylsulfonyl und R¹¹ für Methyl stehen: 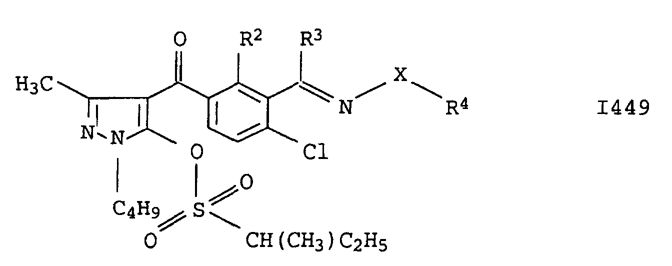
- die Verbindungen I450, insbesondere die Verbindungen I450.001-I450.180, die sich von den entsprechenden Verbindungen I1.001-I1.180 dadurch unterscheiden, daß R⁹ für iso-Butyl, R¹⁰ für sec-Butylsulfonyl und R¹¹ für Methyl stehen: 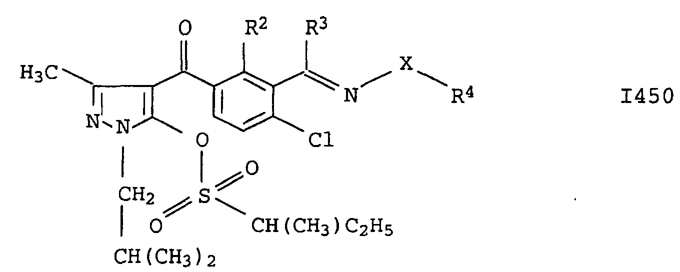
- die Verbindungen I451, insbesondere die Verbindungen I451.001-I451.180, die sich von den entsprechenden Verbindungen I1.001-I1.180 dadurch unterscheiden, daß R¹⁰ für Trifluormethylsulfonyl und R¹¹ für Methyl stehen: 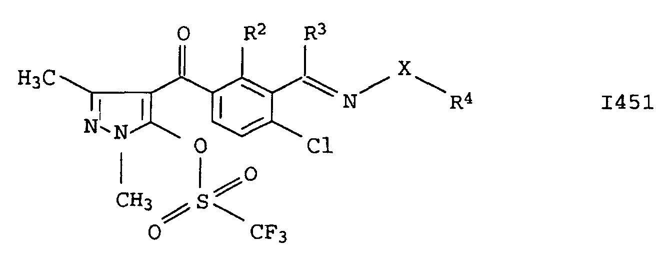
- die Verbindungen I452, insbesondere die Verbindungen I452.001-I452.180, die sich von den entsprechenden Verbindungen I1.001-I1.180 dadurch unterscheiden, daß R⁹ für Ethyl, R¹⁰ für Trifluormethylsulfonyl und R¹¹ für Methyl stehen: 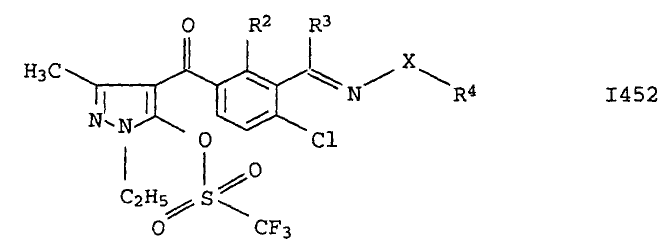
- die Verbindungen I453, insbesondere die Verbindungen I453.001-I453.180, die sich von den entsprechenden Verbindungen I1.001-I1.180 dadurch unterscheiden, daß R⁹ für n-Propyl, R¹⁰ für Trifluormethylsulfonyl und R¹¹ für Methyl stehen: 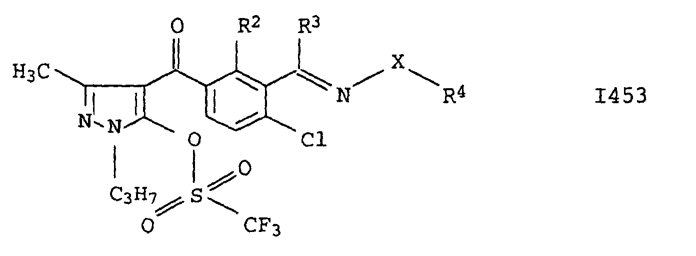
- die Verbindungen I454, insbesondere die Verbindungen I454.001-I454.180, die sich von den entsprechenden Verbindungen I1.001-I1.180 dadurch unterscheiden, daß R⁹ für n-Butyl, R¹⁰ für Trifluormethylsulfonyl und R¹¹ für Methyl stehen: 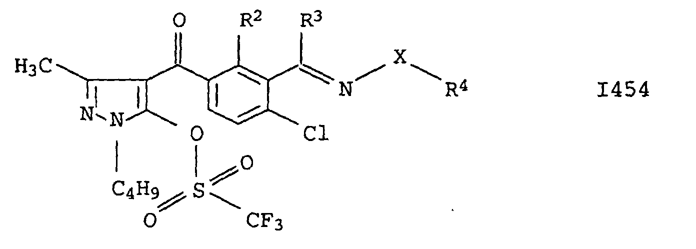
- die Verbindungen I455, insbesondere die Verbindungen I455.001-I455.180, die sich von den entsprechenden Verbindungen I1.001-I1.180 dadurch unterscheiden, daß R⁹ für iso-Butyl, R¹⁰ für Trifluormethylsulfonyl und R¹¹ für Methyl stehen: 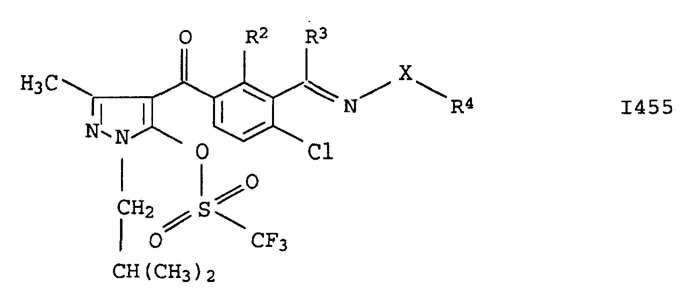
- die Verbindungen I456, insbesondere die Verbindungen I456.001-I456.180, die sich von den entsprechenden Verbindungen I1.001-I1.180 dadurch unterscheiden, daß R⁹ und R¹¹ für Methyl und R¹⁰ für Phenylcarbonylmethyl stehen: 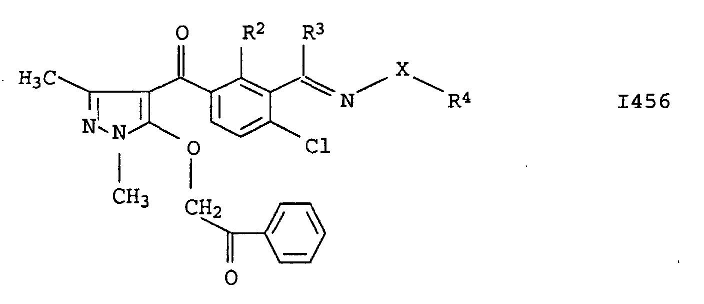
- die Verbindungen I457, insbesondere die Verbindungen I457.001-I457.180, die sich von den entsprechenden Verbindungen I1.001-I1.180 dadurch unterscheiden, daß R⁹ für Ethyl, R¹⁰ für Phenylcarbonylmethyl und R¹¹ für Methyl stehen: 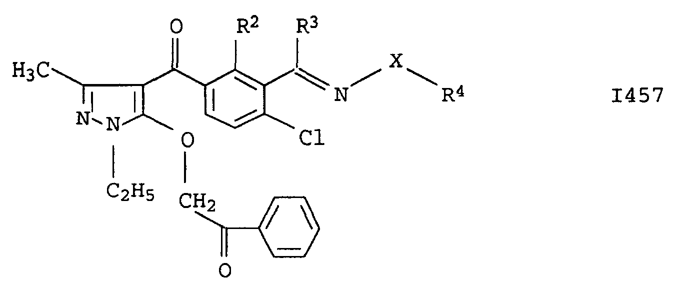
- die Verbindungen I458, insbesondere die Verbindungen I458.001-I458.180, die sich von den entsprechenden Verbindungen I1.001-I1.180 dadurch unterscheiden, daß R⁹ für n-Propyl, R¹⁰ für Phenylcarbonylmethyl und R¹¹ für Methyl stehen: 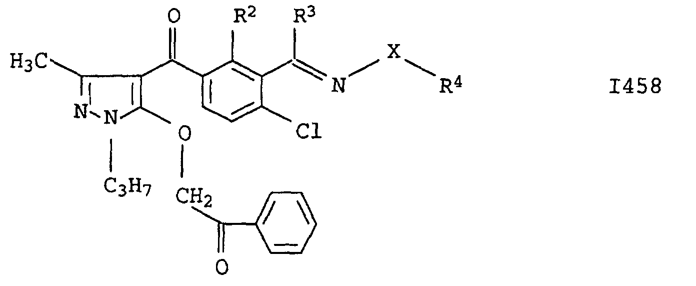
- die Verbindungen I459, insbesondere die Verbindungen I459.001-I459.180, die sich von den entsprechenden Verbindungen I1.001-I1.180 dadurch unterscheiden, daß R⁹ für n-Butyl, R¹⁰ für Phenylcarbonylmethyl und R¹¹ für Methyl stehen: 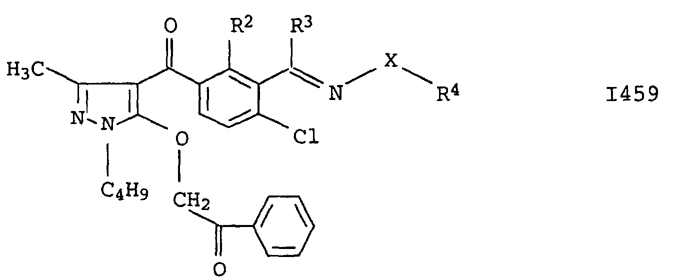
- die Verbindungen I460, insbesondere die Verbindungen I460.001-I460.180, die sich von den entsprechenden Verbindungen I1.001-I1.180 dadurch unterscheiden, daß R⁹ für iso-Butyl, R¹⁰ für Phenylcarbonylmethyl und R¹¹ für Methyl stehen: 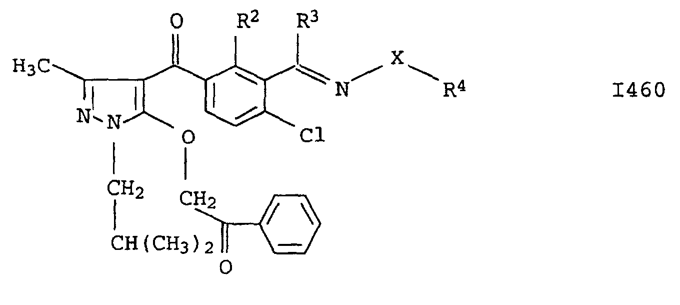
- die Verbindungen I461, insbesondere die Verbindungen I461.001-I461.180, die sich von den entsprechenden Verbindungen I1.001-I1.180 dadurch unterscheiden, daß R¹ für Nitro und R¹¹ für Methyl stehen: 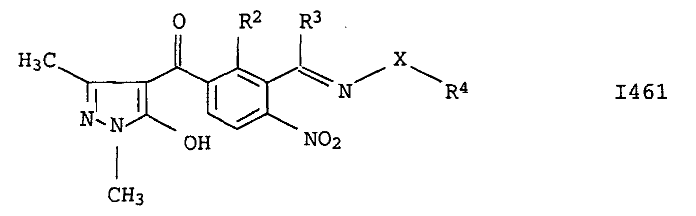
- die Verbindungen I462, insbesondere die Verbindungen I462.001-I462.180, die sich von den entsprechenden Verbindungen I1.001-I1.180 dadurch unterscheiden, daß R¹ für Nitro, R⁹ für Ethyl und R¹¹ für Methyl stehen: 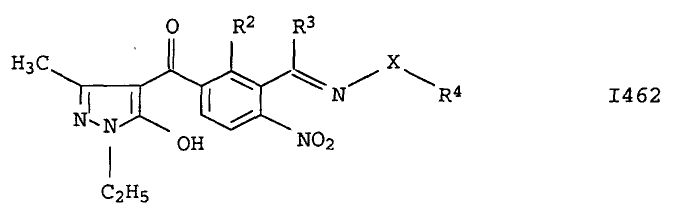
- die Verbindungen I463, insbesondere die Verbindungen I463.001-I463.180, die sich von den entsprechenden Verbindungen I1.001-I1.180 dadurch unterscheiden, daß R¹ für Nitro, R⁹ für n-Propyl und R¹¹ für Methyl stehen: 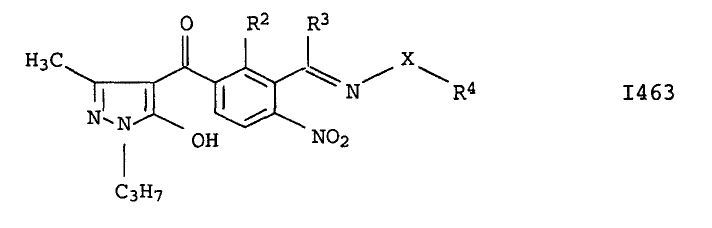
- die Verbindungen I464, insbesondere die Verbindungen I464.001-I464.180, die sich von den entsprechenden Verbindungen I1.001-I1.180 dadurch unterscheiden, daß R¹ für Nitro, R⁹ für n-Butyl und R¹¹ für Methyl stehen: 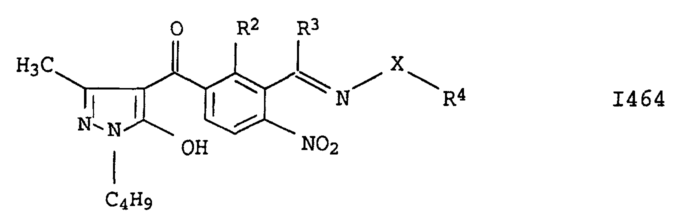
- die Verbindungen I465, insbesondere die Verbindungen I465.001-I465.180, die sich von den entsprechenden Verbindungen I1.001-I1.180 dadurch unterscheiden, daß R¹ für Nitro, R⁹ für iso-Butyl und R¹¹ für Methyl stehen: 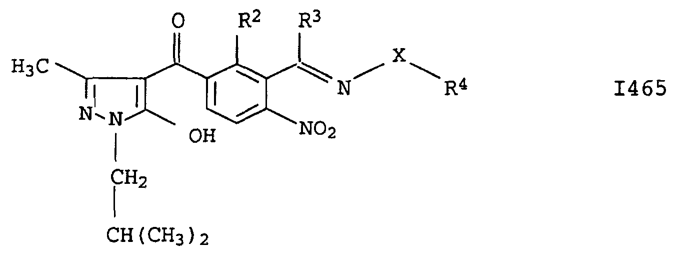
- die Verbindungen I466, insbesondere die Verbindungen I466.001-I466.180, die sich von den entsprechenden Verbindungen I1.001-I1.180 dadurch unterscheiden, daß R¹ für Nitro und R¹⁰ und R¹¹ für Methyl stehen: 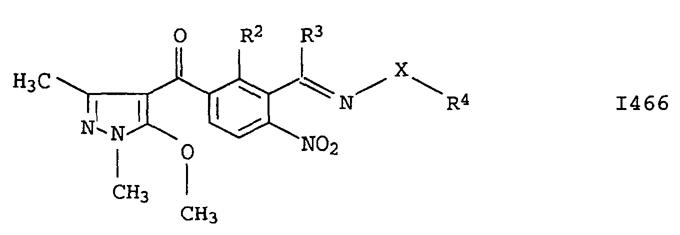
- die Verbindungen I467, insbesondere die Verbindungen I467.001-I467.180, die sich von den entsprechenden Verbindungen I1.001-I1.180 dadurch unterscheiden, daß R¹ für Nitro, R⁹ für Ethyl, R¹⁰ und R¹¹ für Methyl stehen: 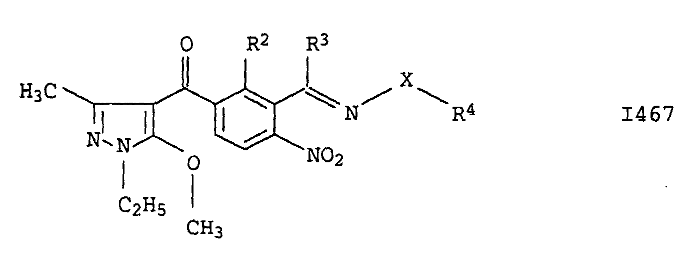
- die Verbindungen I468, insbesondere die Verbindungen I468.001-I468.180, die sich von den entsprechenden Verbindungen I1.001-I1.180 dadurch unterscheiden, daß R¹ für Nitro, R⁹ für n-Propyl, R¹⁰ und R¹¹ für Methyl stehen: 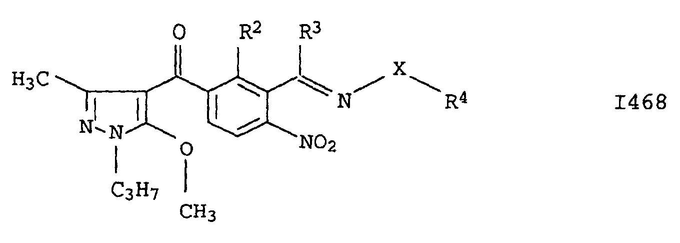
- die Verbindungen I469, insbesondere die Verbindungen I469.001-I469.180, die sich von den entsprechenden Verbindungen I1.001-I1.180 dadurch unterscheiden, daß R¹ für Nitro, R⁹ für n-Butyl, R¹⁰ und R¹¹ für Methyl stehen: 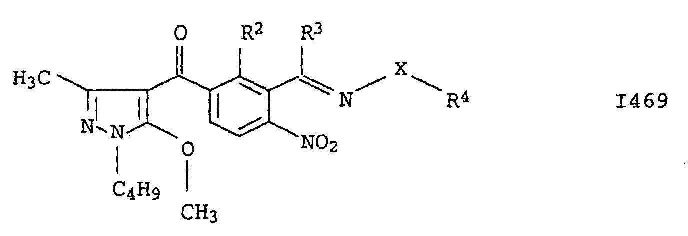
- die Verbindungen I470, insbesondere die Verbindungen I470.001-I470.180, die sich von den entsprechenden Verbindungen I1.001-I1.180 dadurch unterscheiden, daß R¹ für Nitro, R⁹ für iso-Butyl, R¹⁰ und R¹¹ für Methyl stehen: 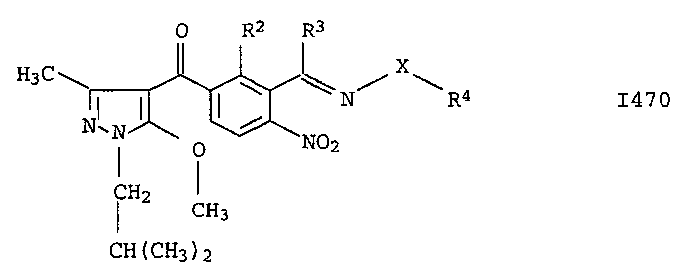
- die Verbindungen I471, insbesondere die Verbindungen I471.001-I471.180, die sich von den entsprechenden Verbindungen I1.001-I1.180 dadurch unterscheiden, daß R¹ für Nitro, R¹⁰ für Ethyl und R¹¹ für Methyl stehen: 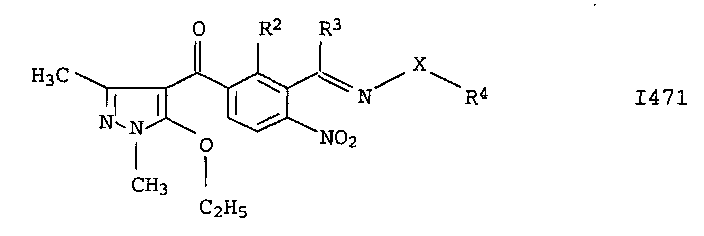
- die Verbindungen I472, insbesondere die Verbindungen I472.001-I472.180, die sich von den entsprechenden Verbindungen I1.001-I1.180 dadurch unterscheiden, daß R¹ für Nitro, R⁹ und R¹⁰ für Ethyl und R¹¹ für Methyl stehen: 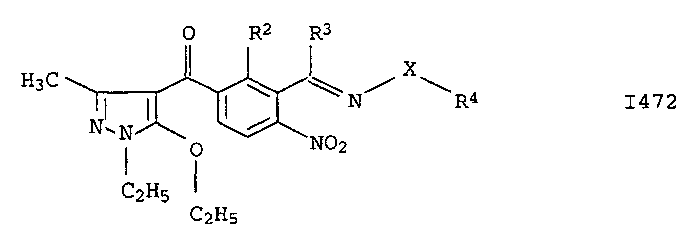
- die Verbindungen I473, insbesondere die Verbindungen I473.001-I473.180, die sich von den entsprechenden Verbindungen I1.001-I1.180 dadurch unterscheiden, daß R¹ für Nitro, R⁹ für n-Propyl, R¹⁰ für Ethyl und R¹¹ für Methyl stehen: 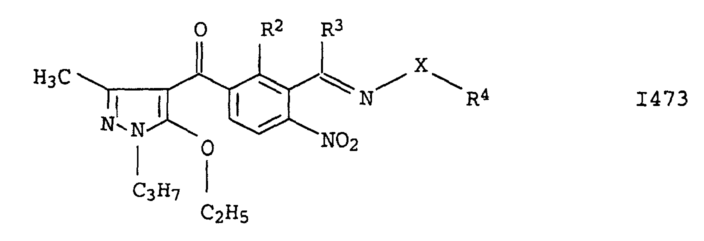
- die Verbindungen I474, insbesondere die Verbindungen I474.001-I474.180, die sich von den entsprechenden Verbindungen I1.001-I1.180 dadurch unterscheiden, daß R¹ für Nitro, R⁹ für n-Butyl, R¹⁰ für Ethyl und R¹¹ für Methyl stehen: 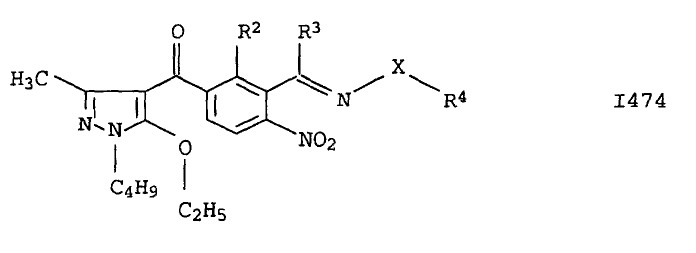
- die Verbindungen I475, insbesondere die Verbindungen I475.001-I475.180, die sich von den entsprechenden Verbindungen I1.001-I1.180 dadurch unterscheiden, daß R¹ für Nitro, R⁹ für iso-Butyl, R¹⁰ für Ethyl und R¹¹ für Methyl stehen: 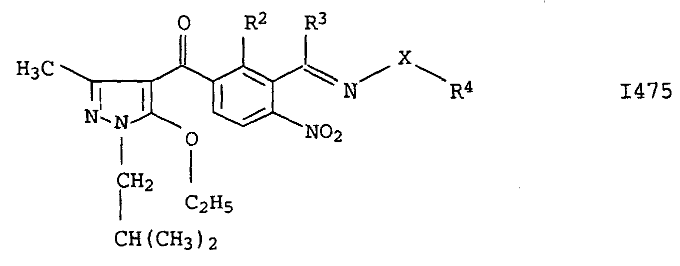
- die Verbindungen I476, insbesondere die Verbindungen I476.001-I476.180, die sich. von den entsprechenden Verbindungen I1.001-I1.180 dadurch unterscheiden, daß R¹ für Nitro, R¹⁰ für n-Propyl und R¹¹ für Methyl stehen: 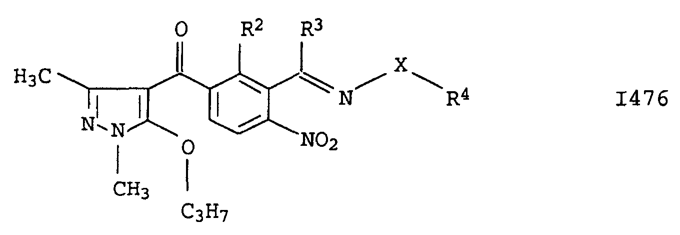
- die Verbindungen I477, insbesondere die Verbindungen I477.001-I477.180, die sich von den entsprechenden Verbindungen I1.001-I1.180 dadurch unterscheiden, daß R¹ für Nitro, R⁹ für Ethyl, R¹⁰ für n-Propyl und R¹¹ für Methyl stehen: 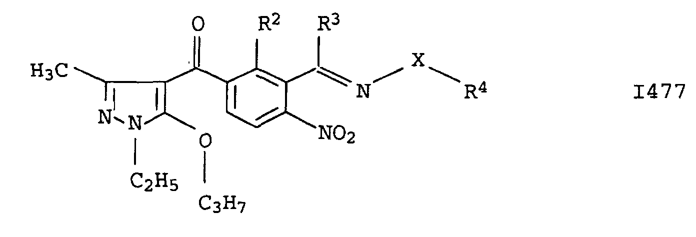
- die Verbindungen I478, insbesondere die Verbindungen I478.001-I478.180, die sich von den entsprechenden Verbindungen I1.001-I1.180 dadurch unterscheiden, daß R¹ für Nitro, R⁹ und R¹⁰ für n-Propyl und R¹¹ für Methyl stehen: 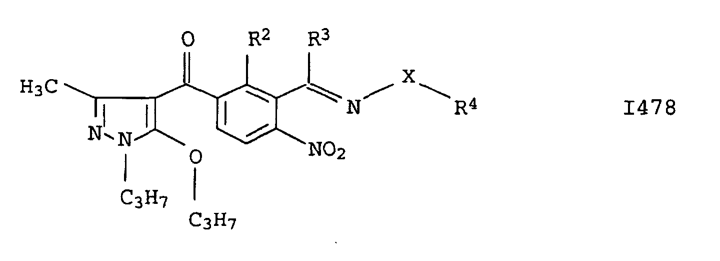
- die Verbindungen I479, insbesondere die Verbindungen I479.001-I479.180, die sich von den entsprechenden Verbindungen I1.001-I1.180 dadurch unterscheiden, daß R¹ für Nitro, R⁹ für n-Butyl, R¹⁰ für n-Propyl und R¹¹ für Methyl stehen: 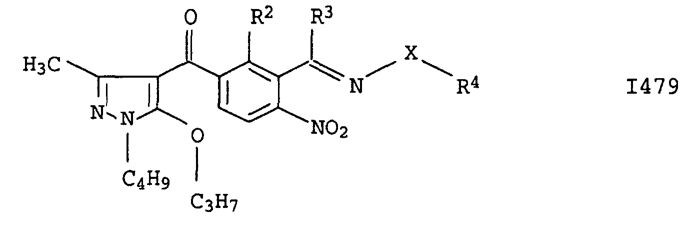
- die Verbindungen I480, insbesondere die Verbindungen I480.001-I480.180, die sich von den entsprechenden Verbindungen I1.001-I1.180 dadurch unterscheiden, daß R¹ für Nitro, R⁹ für iso-Butyl, R¹⁰ für n-Propyl und R¹¹ für Methyl stehen: 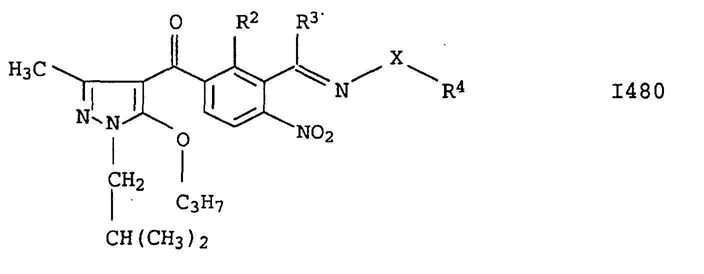
- die Verbindungen I481, insbesondere die Verbindungen I481.001-I481.180, die sich von den entsprechenden Verbindungen I1.001-I1.180 dadurch unterscheiden, daß R¹ für Nitro, R¹⁰ für iso-Propyl und R¹¹ für Methyl stehen: 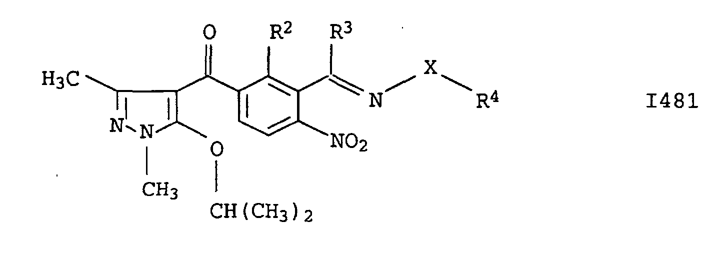
- die Verbindungen I482, insbesondere die Verbindungen I482.001-I482.180, die sich von den entsprechenden Verbindungen I1.001-I1.180 dadurch unterscheiden, daß R¹ für Nitro, R⁹ für Ethyl, R¹⁰ für iso-Propyl und R¹¹ für Methyl stehen: 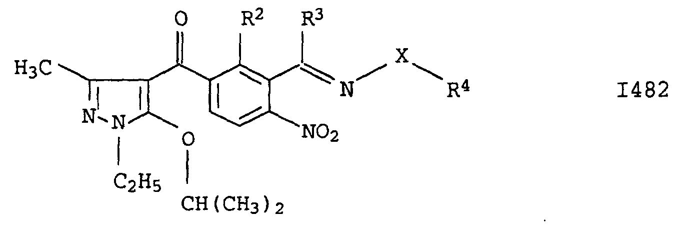
- die Verbindungen I483, insbesondere die Verbindungen I483.001-I483.180, die sich von den entsprechenden Verbindungen I1.001-I1.180 dadurch unterscheiden, daß R¹ für Nitro, R⁹ für n-Propyl, R¹⁰ für iso-Propyl und R¹¹ für Methyl stehen: 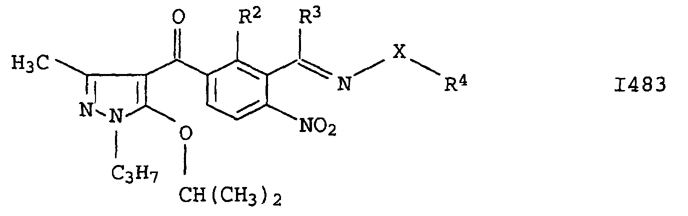
- die Verbindungen I484, insbesondere die Verbindungen I484.001-I484.180, die sich von den entsprechenden Verbindungen I1.001-I1.180 dadurch unterscheiden, daß R¹ für Nitro, R⁹ für n-Butyl, R¹⁰ für iso-Propyl und R¹¹ für Methyl stehen: 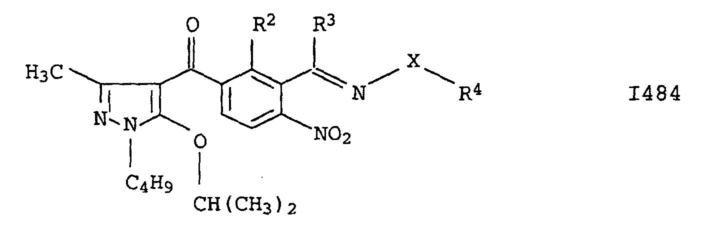
- die Verbindungen I485, insbesondere die Verbindungen I485.001-I485.180, die sich von den entsprechenden Verbindungen I1.001-I1.180 dadurch unterscheiden, daß R¹ für Nitro, R⁹ für iso-Butyl, R¹⁰ für iso-Propyl und R¹¹ für Methyl stehen: 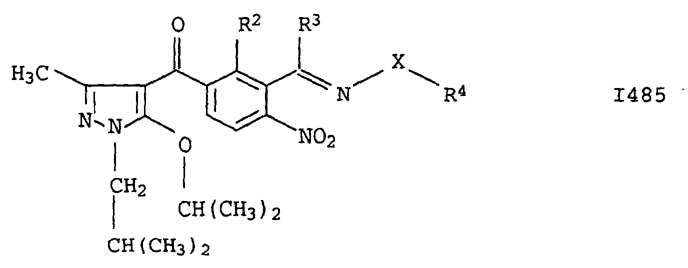
- die Verbindungen I486, insbesondere die Verbindungen I486.001-I486.180, die sich von den entsprechenden Verbindungen I1.001-I1.180 dadurch unterscheiden, daß R¹ für Nitro, R¹⁰ für n-Butyl und R¹¹ für Methyl stehen: 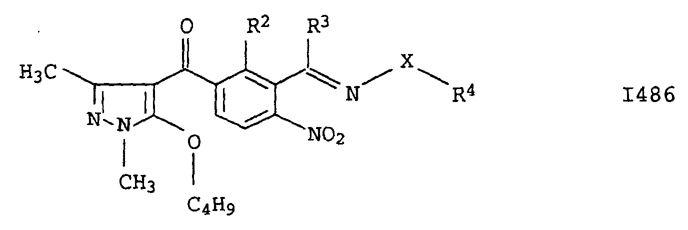
- die Verbindungen I487, insbesondere die Verbindungen I487.001-I487.180, die sich von den entsprechenden Verbindungen I1.001-I1.180 dadurch unterscheiden, daß R¹ für Nitro, R⁹ für Ethyl, R¹⁰ für n-Butyl und R¹¹ für Methyl stehen: 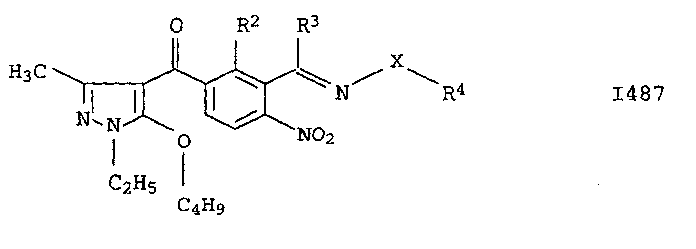
- die Verbindungen I488, insbesondere die Verbindungen I488.001-I488.180, die sich von den entsprechenden Verbindungen I1.001-I1.180 dadurch unterscheiden, daß R¹ für Nitro, R⁹ für n-Propyl, R¹⁰ für n-Butyl und R¹¹ für Methyl stehen: 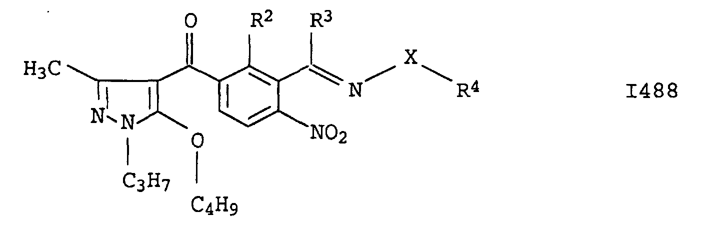
- die Verbindungen I489, insbesondere die Verbindungen I489.001-I489.180, die sich von den entsprechenden Verbindungen I1.001-I1.180 dadurch unterscheiden, daß R¹ für Nitro, R⁹ und R¹⁰ für n-Butyl und R¹¹ für Methyl stehen: 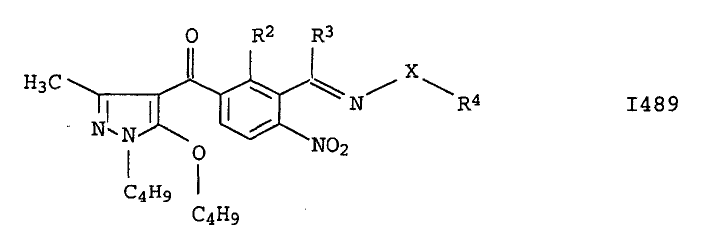
- die Verbindungen I490, insbesondere die Verbindungen I490.001-I490.180, die sich von den entsprechenden Verbindungen I1.001-I1.180 dadurch unterscheiden, daß R¹ für Nitro, R⁹ für iso-Butyl, R¹⁰ für n-Butyl und R¹¹ für Methyl stehen: 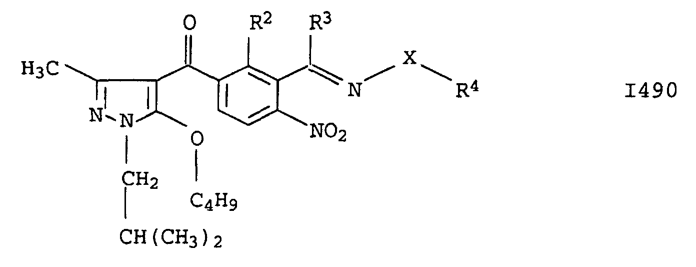
- die Verbindungen I491, insbesondere die Verbindungen I491.001-I491.180, die sich von den entsprechenden Verbindungen I1.001-I1.180 dadurch unterscheiden, daß R¹ für Nitro, R¹⁰ für sec-Butyl und R¹¹ für Methyl stehen: 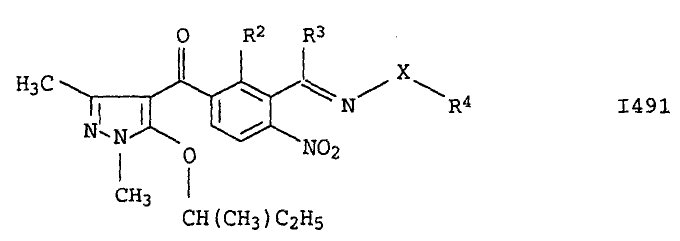
- die Verbindungen I492, insbesondere die Verbindungen I492.001-I492.180, die sich von den entsprechenden Verbindungen I1.001-I1.180 dadurch unterscheiden, daß R¹ für Nitro, R⁹ für Ethyl, R¹⁰ für sec-Butyl und R¹¹ für Methyl stehen: 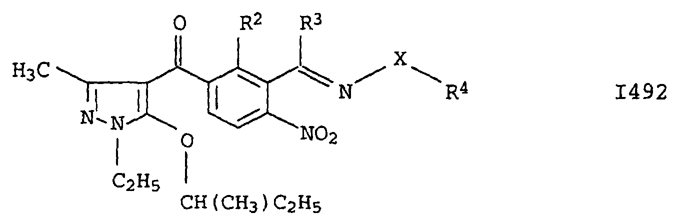
- die Verbindungen I493, insbesondere die Verbindungen I493.001-I493.180, die sich von den entsprechenden Verbindungen I1.001-I1.180 dadurch unterscheiden, daß R¹ für Nitro, R⁹ für n-Propyl, R¹⁰ für sec-Butyl und R¹¹ für Methyl stehen: 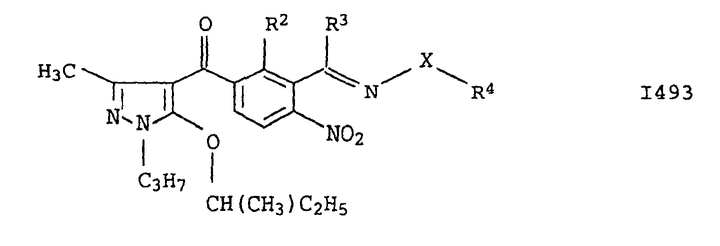
- die Verbindungen I494, insbesondere die Verbindungen I494.001-I494.180, die sich von den entsprechenden Verbindungen I1.001-I1.180 dadurch unterscheiden, daß R¹ für Nitro, R⁹ für n-Butyl, R¹⁰ für sec-Butyl und R¹¹ für Methyl stehen: 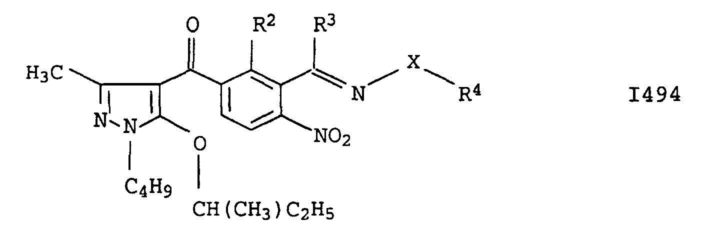
- die Verbindungen I495, insbesondere die Verbindungen I495.001-I495.180, die sich von den entsprechenden Verbindungen I1.001-I1.180 dadurch unterscheiden, daß R¹ für Nitro, R⁹ für iso-Butyl, R¹⁰ für sec-Butyl und R¹¹ für Methyl stehen: 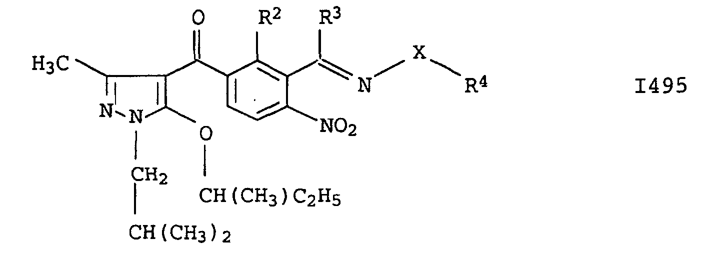
- die Verbindungen I496, insbesondere die Verbindungen I496.001-I496.180, die sich von den entsprechenden Verbindungen I1.001-I1.180 dadurch unterscheiden, daß R¹ für Nitro, R¹⁰ für iso-Butyl und R¹¹ für Methyl stehen: 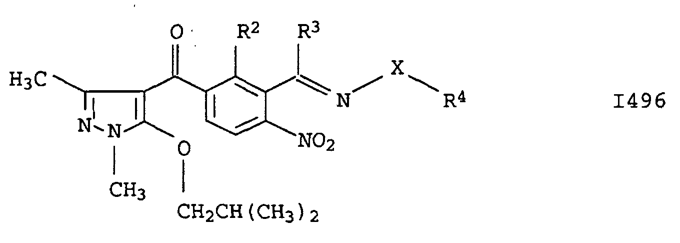
- die Verbindungen I497, insbesondere die Verbindungen I497.001-I497.180, die sich von den entsprechenden Verbindungen I1.001-I1.180 dadurch unterscheiden, daß R¹ für Nitro, R⁹ für Ethyl, R¹⁰ für iso-Butyl und R¹¹ für Methyl stehen: 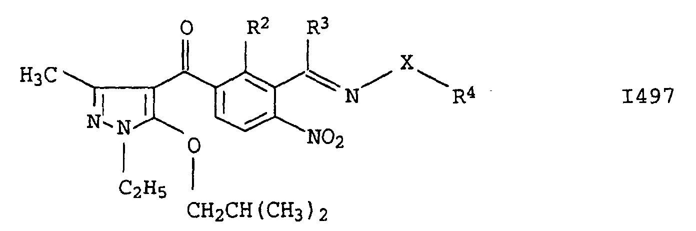
- die Verbindungen I498, insbesondere die Verbindungen I498.001-I498.180, die sich von den entsprechenden Verbindungen I1.001-I1.180 dadurch unterscheiden, daß R¹ für Nitro, R⁹ für n-Propyl, R¹⁰ für iso-Butyl und R¹¹ für Methyl stehen: 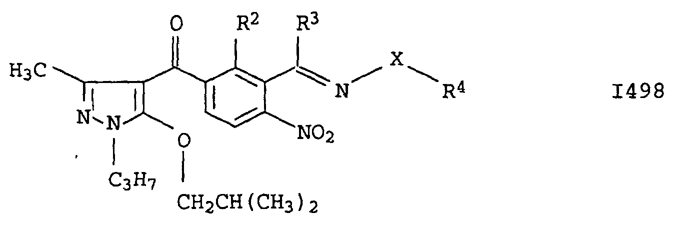
- die Verbindungen I499, insbesondere die Verbindungen I499.001-I499.180, die sich von den entsprechenden Verbindungen I1.001-I1.180 dadurch unterscheiden, daß R¹ für Nitro, R⁹ für n-Butyl, R¹⁰ für iso-Butyl und R¹¹ für Methyl stehen: 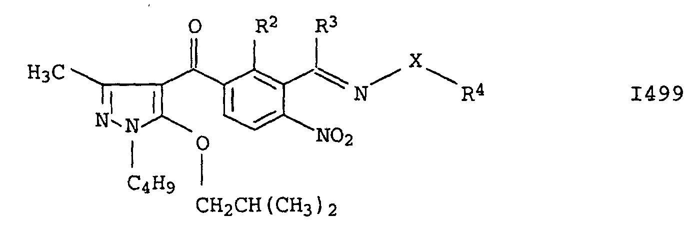
- die Verbindungen I500, insbesondere die Verbindungen I500.001-I500.180, die sich von den entsprechenden Verbindungen I1.001-I1.180 dadurch unterscheiden, daß R¹ für Nitro, R⁹ und R¹⁰ für iso-Butyl und R¹¹ für Methyl stehen: 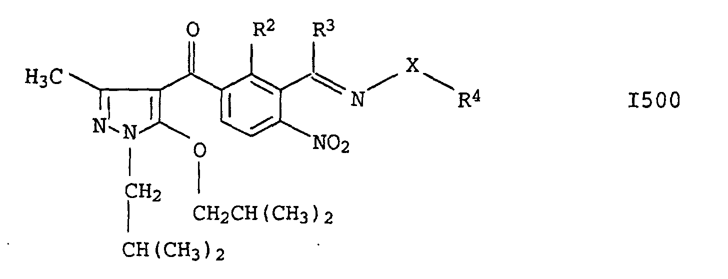
- die Verbindungen I501, insbesondere die Verbindungen I501.001-I501.180, die sich von den entsprechenden Verbindungen I1.001-I1.180 dadurch unterscheiden, daß R¹ für Nitro, R¹⁰ für Methylcarbonyl und R¹¹ für Methyl stehen: 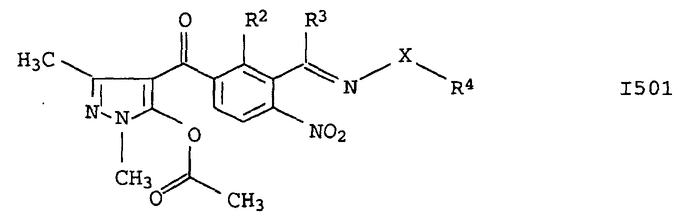
- die Verbindungen I502, insbesondere die Verbindungen I502.001-I502.180, die sich von den entsprechenden Verbindungen I1.001-I1.180 dadurch unterscheiden, daß R¹ für Nitro, R⁹ für Ethyl, R¹⁰ für Methylcarbonyl und R¹¹ für Methyl stehen: 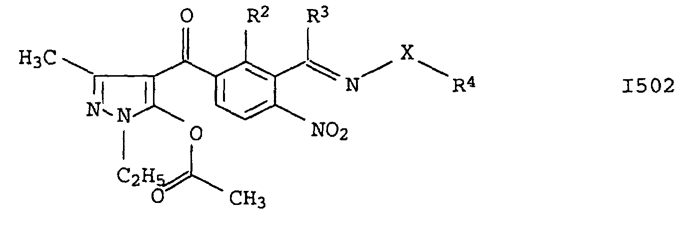
- die Verbindungen I503, insbesondere die Verbindungen I503.001-I503.180, die sich von den entsprechenden Verbindungen I1.001-I1.180 dadurch unterscheiden, daß R¹ für Nitro, R⁹ für n-Propyl, R¹⁰ für Methylcarbonyl und R¹¹ für Methyl stehen: 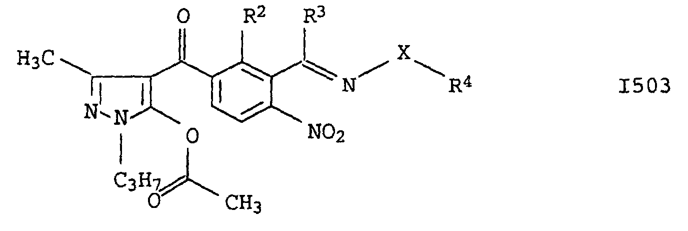
- die Verbindungen I504, insbesondere die Verbindungen I504.001-I504.180, die sich von den entsprechenden Verbindungen I1.001-I1.180 dadurch unterscheiden, daß R¹ für Nitro, R⁹ für n-Butyl, R¹⁰ für Methylcarbonyl und R¹¹ für Methyl stehen: 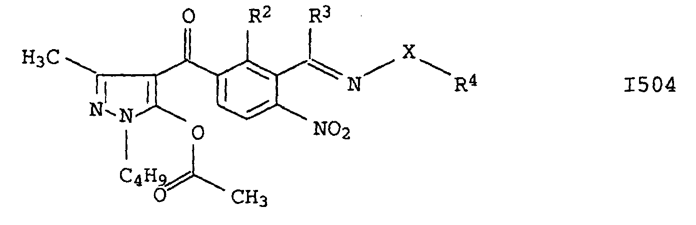
- die Verbindungen I505, insbesondere die Verbindungen I505.001-I505.180, die sich von den entsprechenden Verbindungen I1.001-I1.180 dadurch unterscheiden, daß R¹ für Nitro, R⁹ für iso-Butyl, R¹⁰ für Methylcarbonyl und R¹¹ für Methyl stehen: 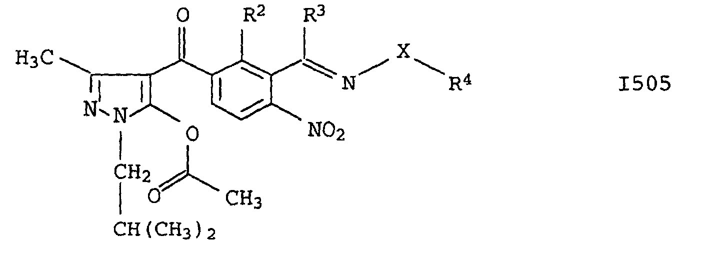
- die Verbindungen I506, insbesondere die Verbindungen I506.001-I506.180, die sich von den entsprechenden Verbindungen I1.001-I1.180 dadurch unterscheiden, daß R¹ für Nitro, R¹⁰ für Ethylcarbonyl und R¹¹ für Methyl stehen: 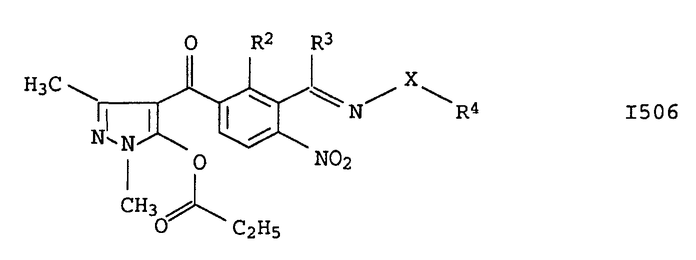
- die Verbindungen I507, insbesondere die Verbindungen I507.001-I507.180, die sich von den entsprechenden Verbindungen I1.001-I1.180 dadurch unterscheiden, daß R¹ für Nitro, R⁹ für Ethyl, R¹⁰ für Ethylcarbonyl und R¹¹ für Methyl stehen: 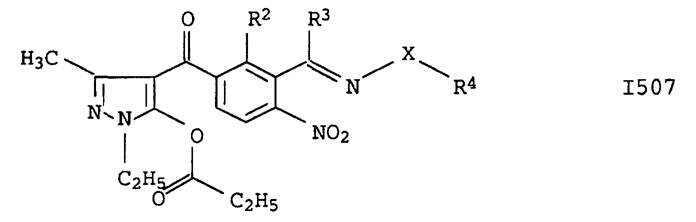
- die Verbindungen I508, insbesondere die Verbindungen I508.001-I508.180, die sich von den entsprechenden Verbindungen I1.001-I1.180 dadurch unterscheiden, daß R¹ für Nitro, R⁹ für n-Propyl, R¹⁰ für Ethylcarbonyl und R¹¹ für Methyl stehen: 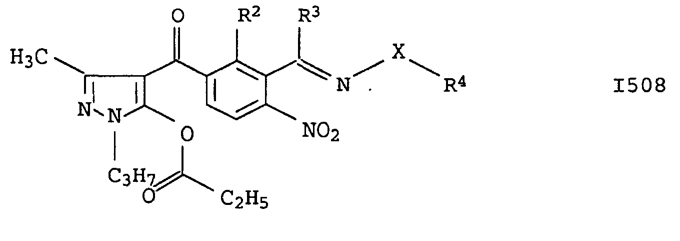
- die Verbindungen I509, insbesondere die Verbindungen I509.001-I509.180, die sich von den entsprechenden Verbindungen I1.001-I1.180 dadurch unterscheiden, daß R¹ für Nitro, R⁹ für n-Butyl, R¹⁰ für Ethylcarbonyl und R¹¹ für Methyl stehen: 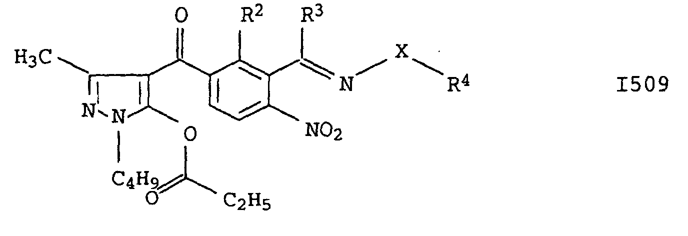
- die Verbindungen I510, insbesondere die Verbindungen I510.001-I510.180, die sich von den entsprechenden Verbindungen I1.001-I1.180 dadurch unterscheiden, daß R¹ für Nitro, R⁹ für iso-Butyl, R¹⁰ für Ethylcarbonyl und R¹¹ für Methyl stehen: 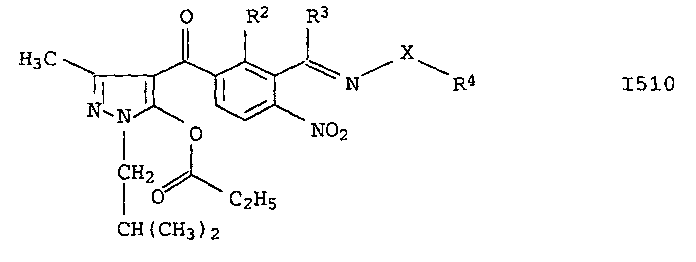
- die Verbindungen I511, insbesondere die Verbindungen I511.001-I511.180, die sich von den entsprechenden Verbindungen I1.001-I1.180 dadurch unterscheiden, daß R¹ für Nitro, R¹⁰ für n-Propylcarbonyl und R¹¹ für Methyl stehen: 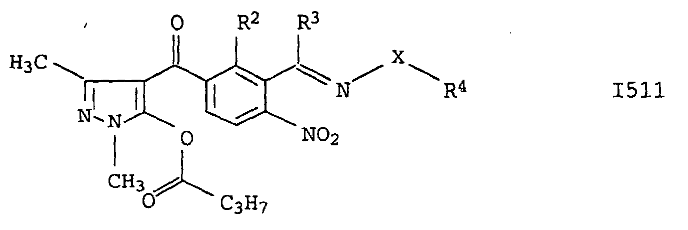
- die Verbindungen I512, insbesondere die Verbindungen I512.001-I512.180, die sich von den entsprechenden Verbindungen I1.001-I1.180 dadurch unterscheiden, daß R¹ für Nitro, R⁹ für Ethyl, R¹⁰ für n-Propylcarbonyl und R¹¹ für Methyl stehen: 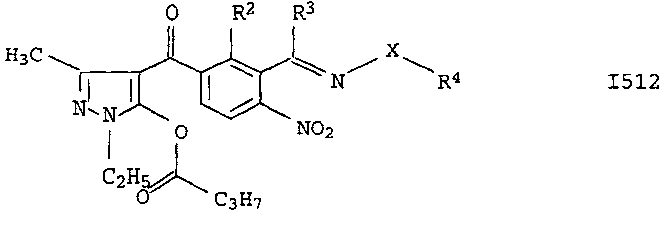
- die Verbindungen I513, insbesondere die Verbindungen I513.001-I513.180, die sich von den entsprechenden Verbindungen I1.001-I1.180 dadurch unterscheiden, daß R¹ für Nitro, R⁹ für n-Propyl, R¹⁰ für n-Propylcarbonyl und R¹¹ für Methyl stehen: 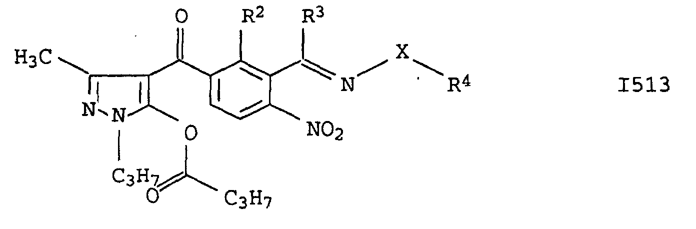
- die Verbindungen I514, insbesondere die Verbindungen I514.001-I514.180, die sich von den entsprechenden Verbindungen I1.001-I1.180 dadurch unterscheiden, daß R¹ für Nitro, R⁹ für n-Butyl, R¹⁰ für n-Propylcarbonyl und R¹¹ für Methyl stehen: 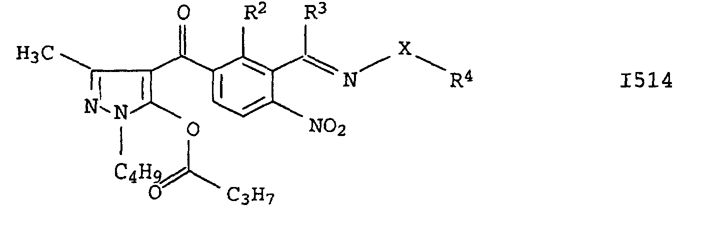
- die Verbindungen I515, insbesondere die Verbindungen I515.001-I515.180, die sich von den entsprechenden Verbindungen I1.001-I1.180 dadurch unterscheiden, daß R¹ für Nitro, R⁹ für iso-Butyl, R¹⁰ für n-Propylcarbonyl und R¹¹ für Methyl stehen: 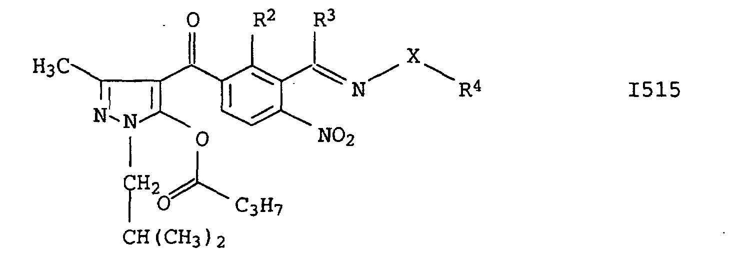
- die Verbindungen I516, insbesondere die Verbindungen I516.001-I516.180, die sich von den entsprechenden Verbindungen I1.001-I1.180 dadurch unterscheiden, daß R¹ für Nitro, R¹⁰ für Trifluormethylcarbonyl und R¹¹ für Methyl stehen: 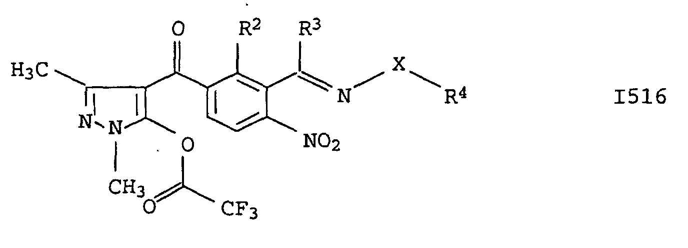
- die Verbindungen I517, insbesondere die Verbindungen I517.001-I517.180, die sich von den entsprechenden Verbindungen I1.001-I1.180 dadurch unterscheiden, daß R¹ für Nitro, R⁹ für Ethyl, R¹⁰ für Trifluormethylcarbonyl und R¹¹ für Methyl stehen: 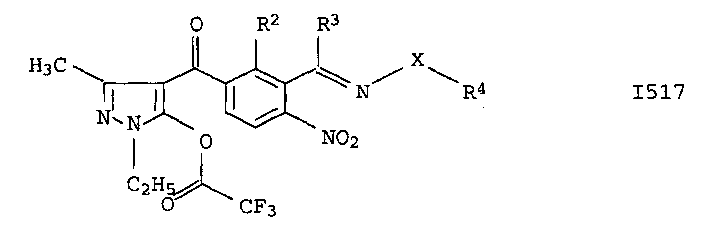
- die Verbindungen I518, insbesondere die Verbindungen I518.001-I518.180, die sich von den entsprechenden Verbindungen I1.001-I1.180 dadurch unterscheiden, daß R¹ für Nitro, R⁹ für n-Propyl, R¹⁰ für Trifluormethylcarbonyl und R¹¹ für Methyl stehen: 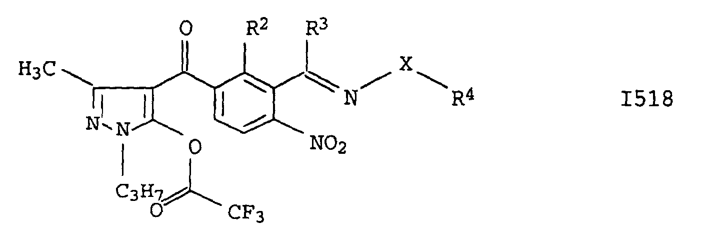
- die Verbindungen I519, insbesondere die Verbindungen I519.001-I519.180, die sich von den entsprechenden Verbindungen I1.001-I1.180 dadurch unterscheiden, daß R¹ für Nitro, R⁹ für n-Butyl, R¹⁰ für Trifluormethylcarbonyl und R¹¹ für Methyl stehen: 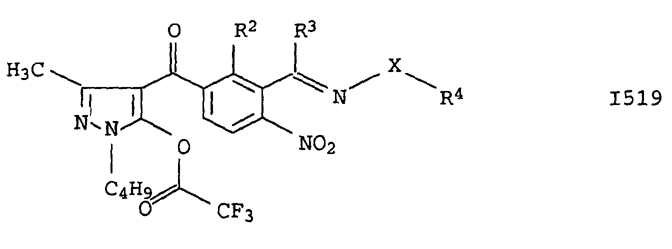
- die Verbindungen I520, insbesondere die Verbindungen I520.001-I520.180, die sich von den entsprechenden Verbindungen I1.001-I1.180 dadurch unterscheiden, daß R¹ für Nitro, R⁹ für iso-Butyl, R¹⁰ für Trifluormethylcarbonyl und R¹¹ für Methyl stehen: 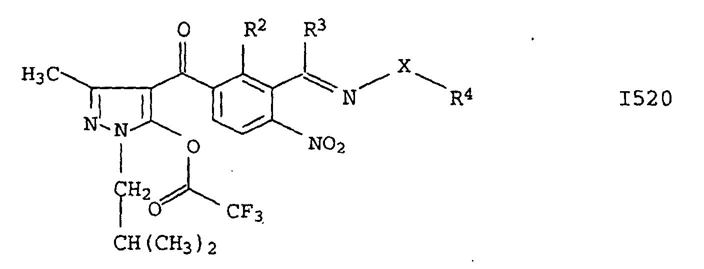
- die Verbindungen I521, insbesondere die Verbindungen I521.001-I521.180, die sich von den entsprechenden Verbindungen I1.001-I1.180 dadurch unterscheiden, daß R¹ für Nitro, R¹⁰ für Methylsulfonyl und R¹¹ für Methyl stehen: 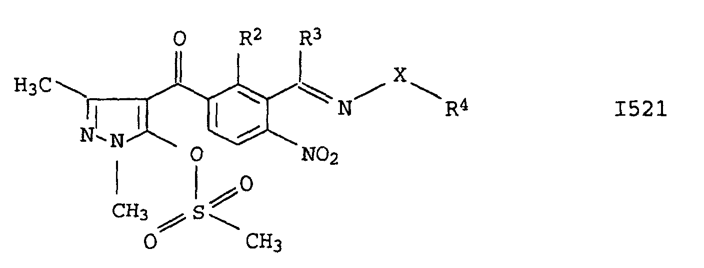
- die Verbindungen I522, insbesondere die Verbindungen I522.001-I522.180, die sich von den entsprechenden Verbindungen I1.001-I1.180 dadurch unterscheiden, daß R¹ für Nitro, R⁹ für Ethyl, R¹⁰ für Methylsulfonyl und R¹¹ für Methyl stehen: 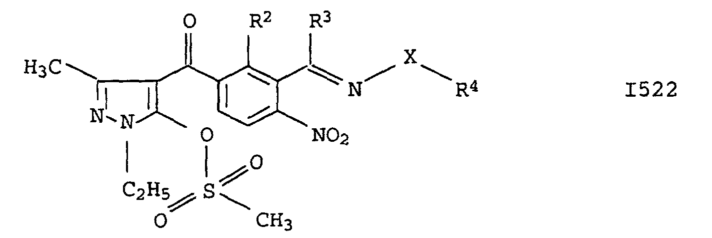
- die Verbindungen I523, insbesondere die Verbindungen I523.001-I523.180, die sich von den entsprechenden Verbindungen I1.001-I1.180 dadurch unterscheiden, daß R¹ für Nitro, R⁹ für n-Propyl, R¹⁰ für Methylsulfonyl und R¹¹ für Methyl stehen: 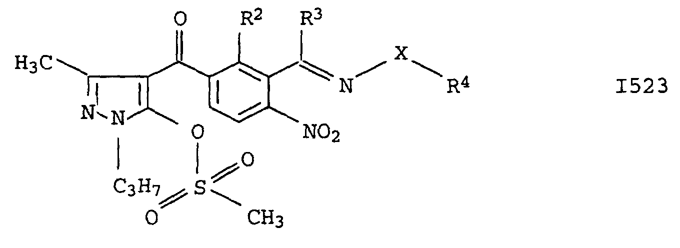
- die Verbindungen I524, insbesondere die Verbindungen I524.001-I524.180, die sich von den entsprechenden Verbindungen I1.001-I1.180 dadurch unterscheiden, daß R¹ für Nitro, R⁹ für n-Butyl, R¹⁰ für Methylsulfonyl und R¹¹ für Methyl stehen: 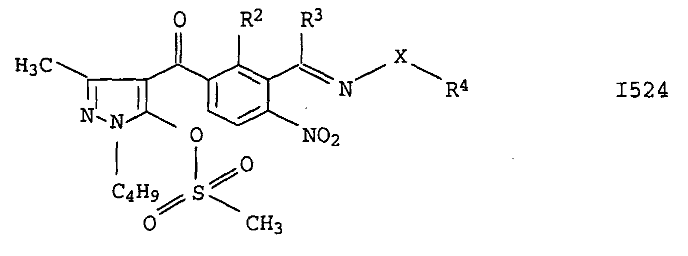
- die Verbindungen I525, insbesondere die Verbindungen I525.001-I525.180, die sich von den entsprechenden Verbindungen I1.001-I1.180 dadurch unterscheiden, daß R¹ für Nitro, R⁹ für iso-Butyl, R¹⁰ für Methylsulfonyl und R¹¹ für Methyl stehen: 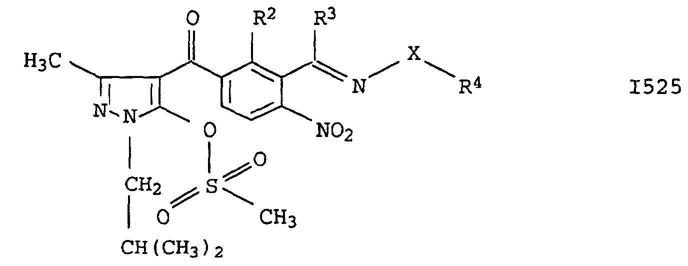
- die Verbindungen I526, insbesondere die Verbindungen I526.001-I526.180, die sich von den entsprechenden Verbindungen I1.001-I1.180 dadurch unterscheiden, daß R¹ für Nitro, R¹⁰ für Ethylsulfonyl und R¹¹ für Methyl stehen: 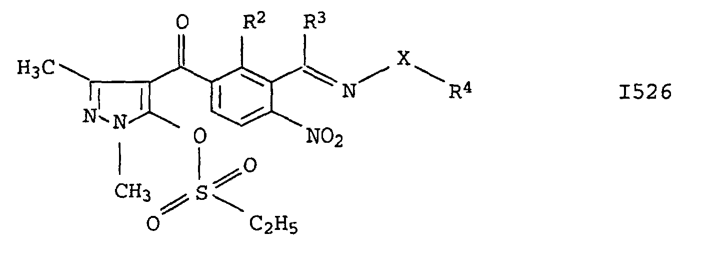
- die Verbindungen I527, insbesondere die Verbindungen I527.001-I527.180, die sich von den entsprechenden Verbindungen I1.001-I1.180 dadurch unterscheiden, daß R¹ für Nitro, R⁹ für Ethyl, R¹⁰ für Ethylsulfonyl und R¹¹ für Methyl stehen: 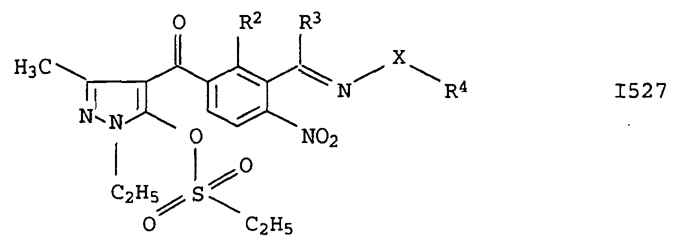
- die Verbindungen I528, insbesondere die Verbindungen I528.001-I528.180, die sich von den entsprechenden Verbindungen I1.001-I1.180 dadurch unterscheiden, daß R¹ für Nitro, R⁹ für n-Propyl, R¹⁰ für Ethylsulfonyl und R¹¹ für Methyl stehen: 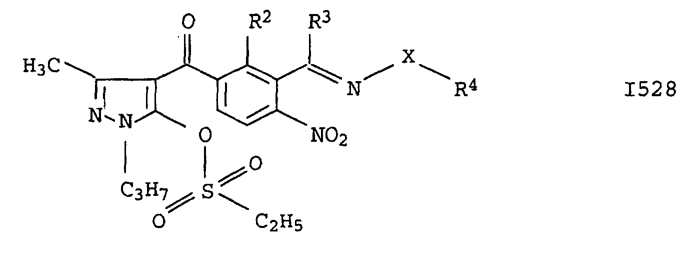
- die Verbindungen I529, insbesondere die Verbindungen I529.001-I529.180, die sich von den entsprechenden Verbindungen I1.001-I1.180 dadurch unterscheiden, daß R¹ für Nitro, R⁹ für n-Butyl, R¹⁰ für Ethylsulfonyl und R¹¹ für Methyl stehen: 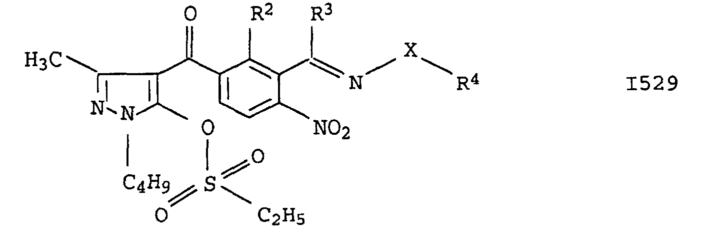
- die Verbindungen I530, insbesondere die Verbindungen I530.001-I530.180, die sich von den entsprechenden Verbindungen I1.001-I1.180 dadurch unterscheiden, daß R¹ für Nitro, R⁹ für iso-Butyl, R¹⁰ für Ethylsulfonyl und R¹¹ für Methyl stehen: 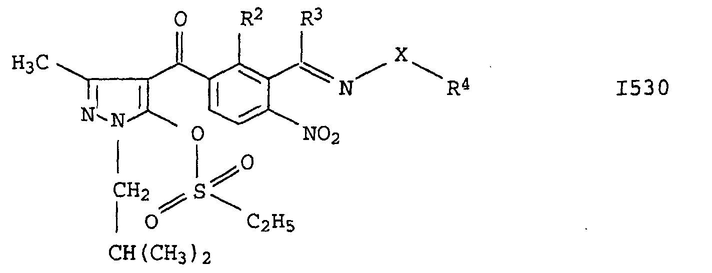
- die Verbindungen I531, insbesondere die Verbindungen I531.001-I531.180, die sich von den entsprechenden Verbindungen I1.001-I1.180 dadurch unterscheiden, daß R¹ für Nitro, R¹⁰ für n-Propylsulfonyl und R¹¹ für Methyl stehen: 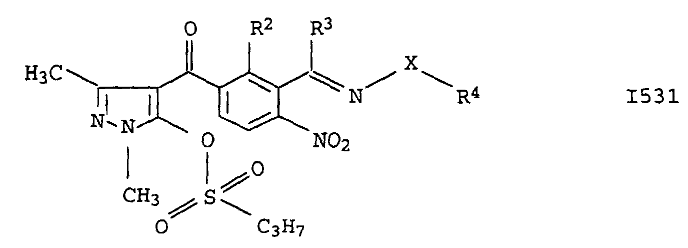
- die Verbindungen I532, insbesondere die Verbindungen I532.001-I532.180, die sich von den entsprechenden Verbindungen I1.001-I1.180 dadurch unterscheiden, daß R¹ für Nitro, R⁹ für Ethyl, R¹⁰ für n-Propylsulfonyl und R¹¹ für Methyl stehen: 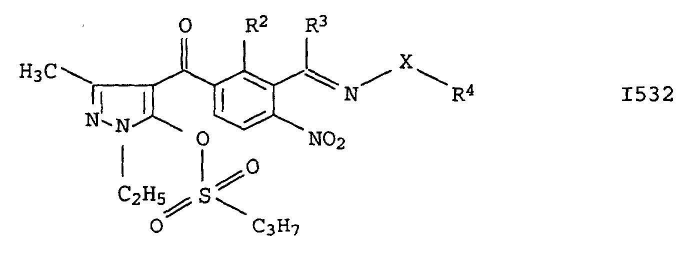
- die Verbindungen I533, insbesondere die Verbindungen I533.001-I533.180, die sich von den entsprechenden Verbindungen I1.001-I1.180 dadurch unterscheiden, daß R¹ für Nitro, R⁹ für n-Propyl, R¹⁰ für n-Propylsulfonyl und R¹¹ für Methyl stehen: 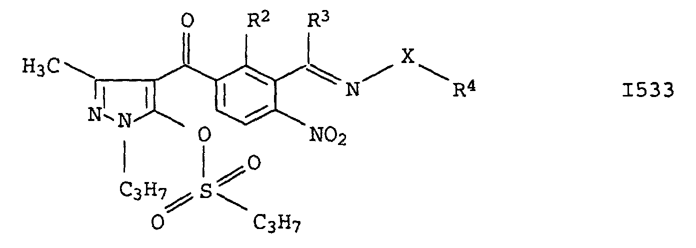
- die Verbindungen I534, insbesondere die Verbindungen I534.001-I534.180, die sich von den entsprechenden Verbindungen I1.001-I1.180 dadurch unterscheiden, daß R¹ für Nitro, R⁹ für n-Butyl, R¹⁰ für n-Propylsulfonyl und R¹¹ für Methyl stehen: 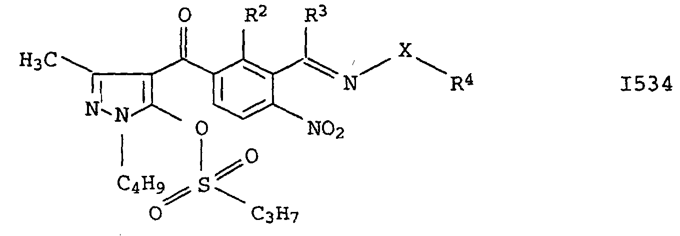
- die Verbindungen I535, insbesondere die Verbindungen I535.001-I535.180, die sich von den entsprechenden Verbindungen I1.001-I1.180 dadurch unterscheiden, daß R¹ für Nitro, R⁹ für iso-Butyl, R¹⁰ für n-Propylsulfonyl und R¹¹ für Methyl stehen: 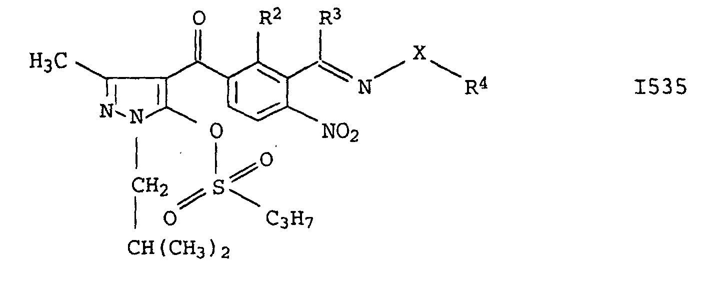
- die Verbindungen I536, insbesondere die Verbindungen I536.001-I536.180, die sich von den entsprechenden Verbindungen I1.001-I1.180 dadurch unterscheiden, daß R¹ für Nitro, R¹⁰ für iso-Propylsulfonyl und R¹¹ für Methyl stehen: 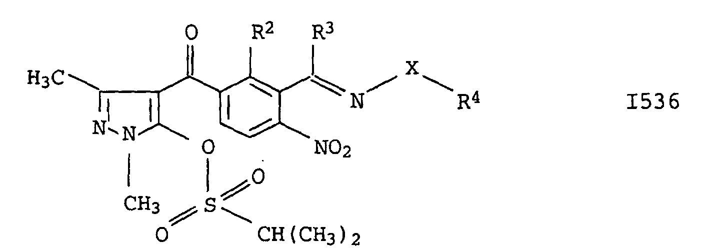
- die Verbindungen I537, insbesondere die Verbindungen I537.001-I537.180, die sich von den entsprechenden Verbindungen I1.001-I1.180 dadurch unterscheiden, daß R¹ für Nitro, R⁹ für Ethyl, R¹⁰ für iso-Propylsulfonyl und R¹¹ für Methyl stehen: 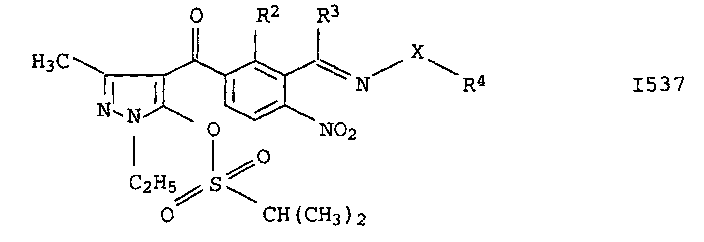
- die Verbindungen I538, insbesondere die Verbindungen I538.001-I538.180, die sich von den entsprechenden Verbindungen I1.001-I1.180 dadurch unterscheiden, daß R¹ für Nitro, R⁹ für n-Propyl, R¹⁰ für iso-Propylsulfonyl und R¹¹ für Methyl stehen: 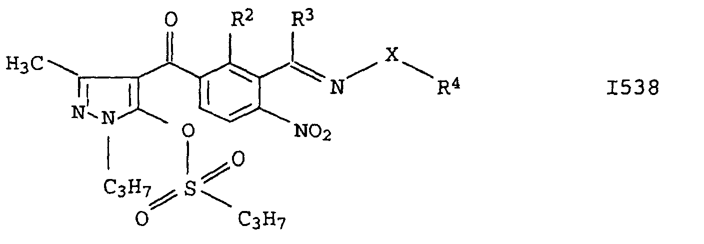
- die Verbindungen I539, insbesondere die Verbindungen I539.001-I539.180, die sich von den entsprechenden Verbindungen I1.001-I1.180 dadurch unterscheiden, daß R¹ für Nitro, R⁹ für n-Butyl, R¹⁰ für iso-Propylsulfonyl und R¹¹ für Methyl stehen: 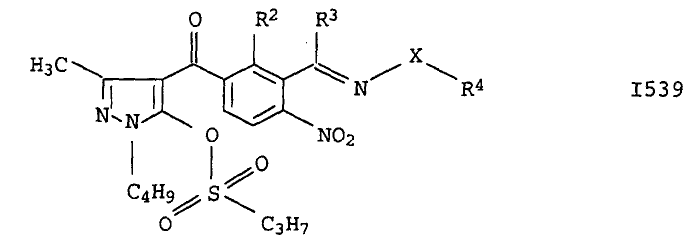
- die Verbindungen I540, insbesondere die Verbindungen I540.001-I540.180, die sich von den entsprechenden Verbindungen I1.001-I1.180 dadurch unterscheiden, daß R¹ für Nitro, R⁹ für iso-Butyl, R¹⁰ für iso-Propylsulfonyl und R¹¹ für Methyl stehen: 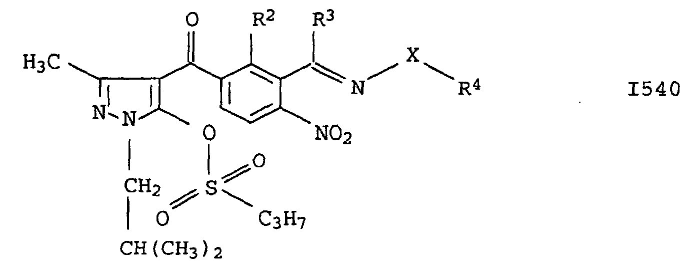
- die Verbindungen I541, insbesondere die Verbindungen I541.001-I541.180, die sich von den entsprechenden Verbindungen I1.001-I1.180 dadurch unterscheiden, daß R¹ für Nitro, R¹⁰ für n-Butylsulfonyl und R¹¹ für Methyl stehen: 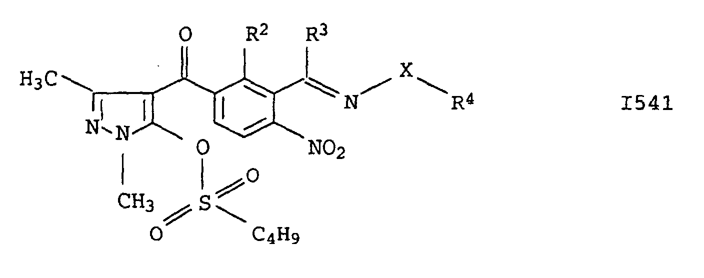
- die Verbindungen I542, insbesondere die Verbindungen I542.001-I542.180, die sich von den entsprechenden Verbindungen I1.001-I1.180 dadurch unterscheiden, daß R¹ für Nitro, R⁹ für Ethyl, R¹⁰ für n-Butylsulfonyl und R¹¹ für Methyl stehen: 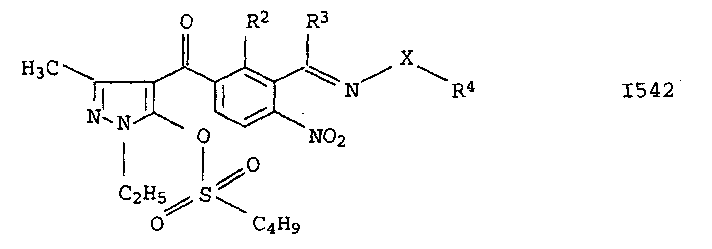
- die Verbindungen I543, insbesondere die Verbindungen I543.001-I543.180, die sich von den entsprechenden Verbindungen I1.001-I1.180 dadurch unterscheiden, daß R¹ für Nitro, R⁹ für n-Propyl, R¹⁰ für n-Butylsulfonyl und R¹¹ für Methyl stehen: 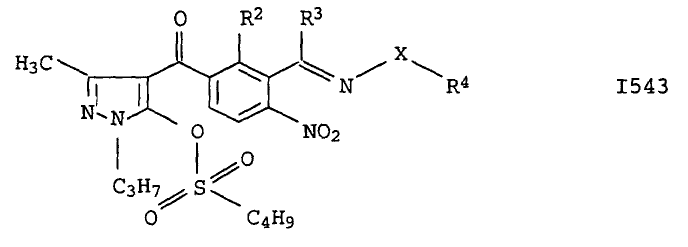
- die Verbindungen I544, insbesondere die Verbindungen I544.001-I544.180, die sich von den entsprechenden Verbindungen I1.001-I1.180 dadurch unterscheiden, daß R¹ für Nitro, R⁹ für n-Butyl, R¹⁰ für n-Butylsulfonyl und R¹¹ für Methyl stehen: 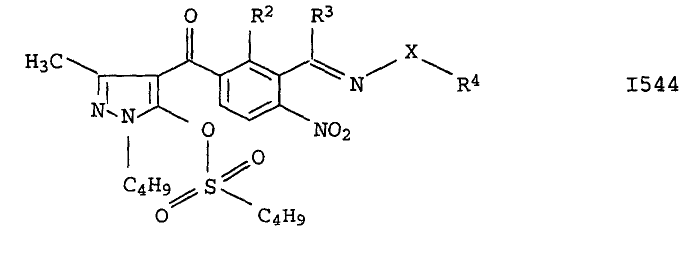
- die Verbindungen I545, insbesondere die Verbindungen I545.001-I545.180, die sich von den entsprechenden Verbindungen I1.001-I1.180 dadurch unterscheiden, daß R¹ für Nitro, R⁹ für iso-Butyl, R¹⁰ für n-Butylsulfonyl und R¹¹ für Methyl stehen: 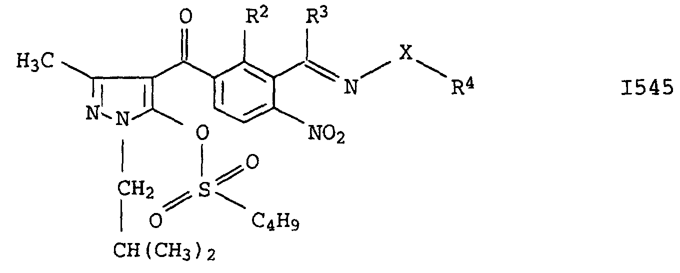
- die Verbindungen I546, insbesondere die Verbindungen I546.001-I546.180, die sich von den entsprechenden Verbindungen I1.001-I1.180 dadurch unterscheiden, daß R¹ für Nitro, R¹⁰ für iso-Butylsulfonyl und R¹¹ für Methyl stehen: 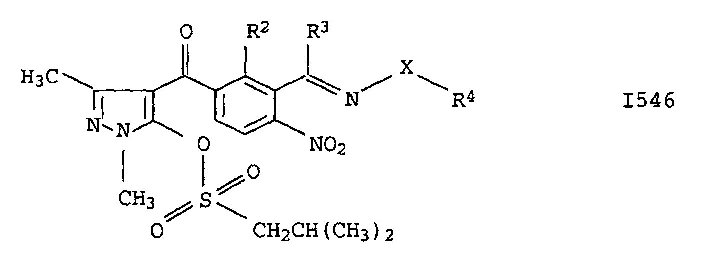
- die Verbindungen I547, insbesondere die Verbindungen I547.001-I547.180, die sich von den entsprechenden Verbindungen I1.001-I1.180 dadurch unterscheiden, daß R¹ für Nitro, R⁹ für Ethyl, R¹⁰ für iso-Butylsulfonyl und R¹¹ für Methyl stehen: 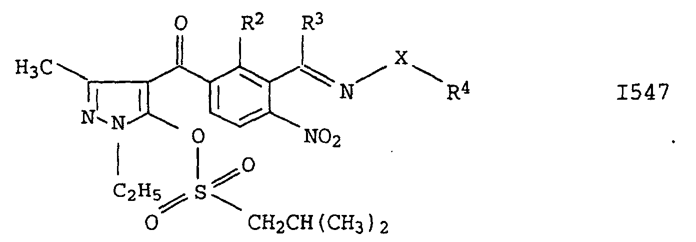
- die Verbindungen I548, insbesondere die Verbindungen I548.001-I548.180, die sich von den entsprechenden Verbindungen I1.001-I1.180 dadurch unterscheiden, daß R¹ für Nitro, R⁹ für n-Propyl, R¹⁰ für iso-Butylsulfonyl und R¹¹ für Methyl stehen: 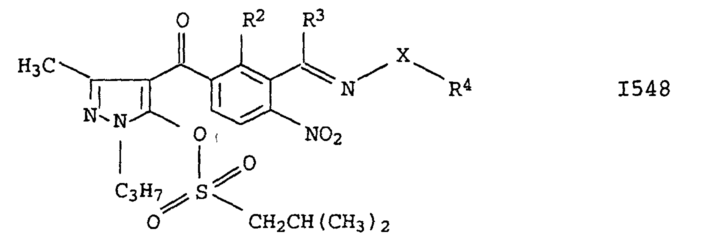
- die Verbindungen I549, insbesondere die Verbindungen I549.001-I549.180, die sich von den entsprechenden Verbindungen I1.001-I1.180 dadurch unterscheiden, daß R¹ für Nitro, R⁹ für n-Butyl, R¹⁰ für iso-Butylsulfonyl und R¹¹ für Methyl stehen: 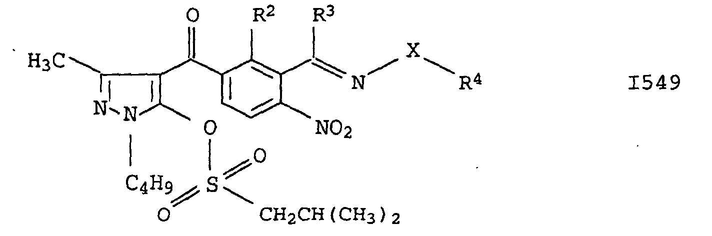
- die Verbindungen I550, insbesondere die Verbindungen I550.001-I550.180, die sich von den entsprechenden Verbindungen I1.001-I1.180 dadurch unterscheiden, daß R¹ für Nitro, R⁹ für iso-Butyl, R¹⁰ für iso-Butylsulfonyl und R¹¹ für Methyl stehen: 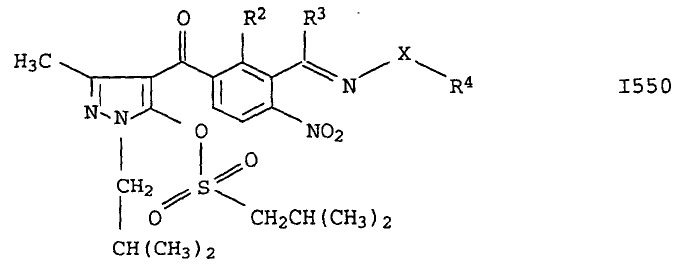
- die Verbindungen I551, insbesondere die Verbindungen I551.001-I551.180, die sich von den entsprechenden Verbindungen I1.001-I1.180 dadurch unterscheiden, daß R¹ für Nitro, R¹⁰ für sec-Butylsulfonyl und R¹¹ für Methyl stehen: 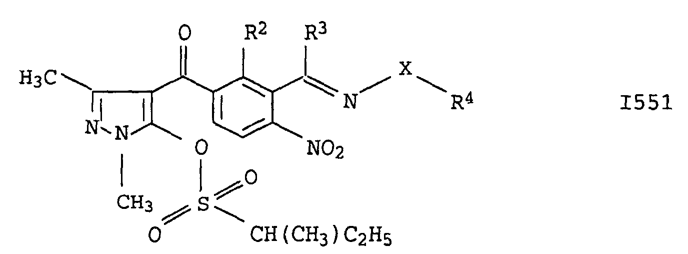
- die Verbindungen I552, insbesondere die Verbindungen I552.001-I552.180, die sich von den entsprechenden Verbindungen I1.001-I1.180 dadurch unterscheiden, daß R¹ für Nitro, R⁹ für Ethyl, R¹⁰ für sec-Butylsulfonyl und R¹¹ für Methyl stehen: 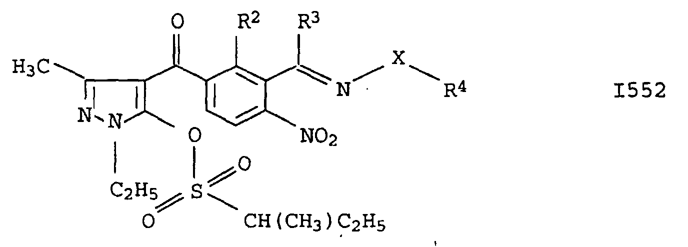
- die Verbindungen I553, insbesondere die Verbindungen I553.001-I553.180, die sich von den entsprechenden Verbindungen I1.001-I1.180 dadurch unterscheiden, daß R¹ für Nitro, R⁹ für n-Propyl, R¹⁰ für sec-Butylsulfonyl und R¹¹ für Methyl stehen: 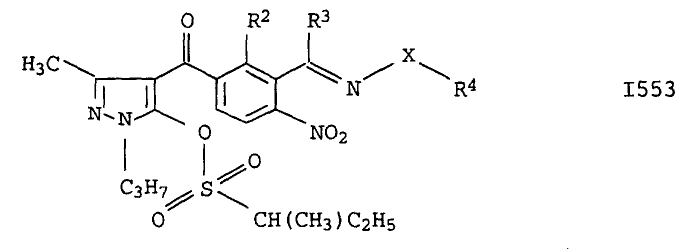
- die Verbindungen I554, insbesondere die Verbindungen I554.001-I554.180, die sich von den entsprechenden Verbindungen I1.001-I1.180 dadurch unterscheiden, daß R¹ für Nitro, R⁹ für n-Butyl, R¹⁰ für sec-Butylsulfonyl und R¹¹ für Methyl stehen: 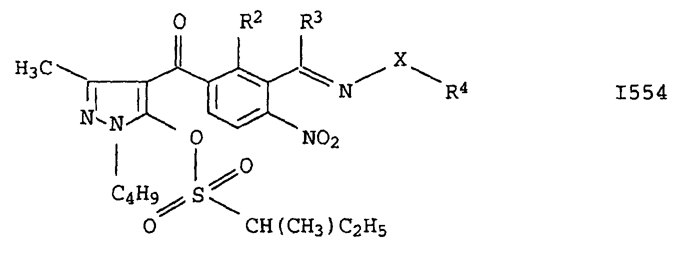
- die Verbindungen I555, insbesondere die Verbindungen I555.001-I555.180, die sich von den entsprechenden Verbindungen I1.001-I1.180 dadurch unterscheiden, daß R¹ für Nitro, R⁹ für iso-Butyl, R¹⁰ für sec-Butylsulfonyl und R¹¹ für Methyl stehen: 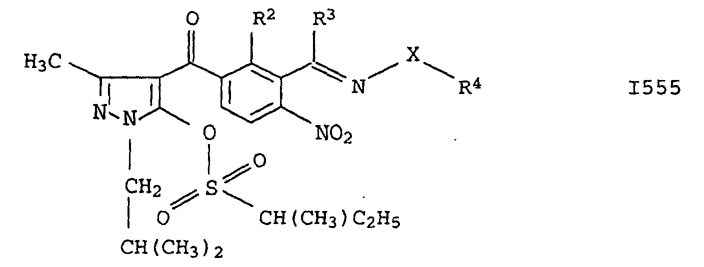
- die Verbindungen I556, insbesondere die Verbindungen I556.001-I556.180, die sich von den entsprechenden Verbindungen I1.001-I1.280 dadurch unterscheiden, daß R¹ für Nitro, R¹⁰ für Trifluormethylsulfonyl und R¹¹ für Methyl stehen: 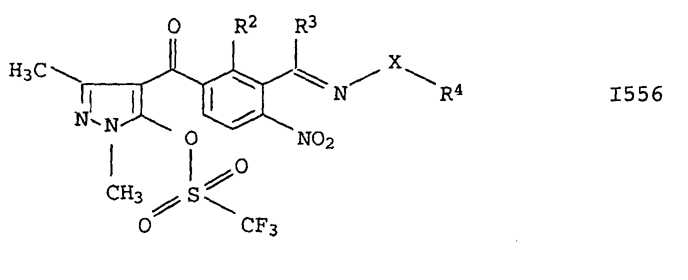
- die Verbindungen I557, insbesondere die Verbindungen I557.001-I557.180, die sich von den entsprechenden Verbindungen I1.001-I1.180 dadurch unterscheiden, daß R¹ für Nitro, R⁹ für Ethyl, R¹⁰ für Trifluormethylsulfonyl und R¹¹ für Methyl stehen: 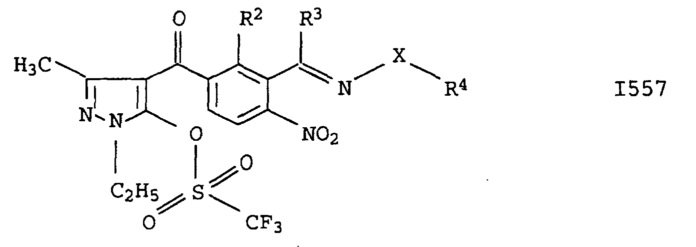
- die Verbindungen I558, insbesondere die Verbindungen I558.001-I558.180, die sich von den entsprechenden Verbindungen I1.001-I1.180 dadurch unterscheiden, daß R¹ für Nitro, R⁹ für n-Propyl, R¹⁰ für Trifluormethylsulfonyl und R¹¹ für Methyl stehen: 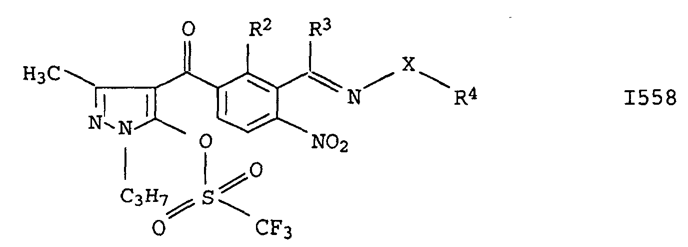
- die Verbindungen I559, insbesondere die Verbindungen I559.001-I559.180, die sich von den entsprechenden Verbindungen I1.001-I1.180 dadurch unterscheiden, daß R¹ für Nitro, R⁹ für n-Butyl, R¹⁰ für Trifluormethylsulfonyl und R¹¹ für Methyl stehen: 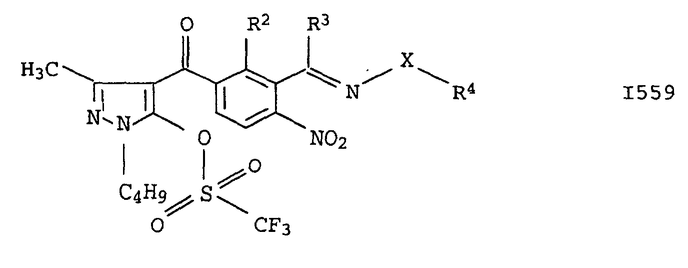
- die Verbindungen I560, insbesondere die Verbindungen I560.001-I560.180, die sich von den entsprechenden Verbindungen I1.001-I1.180 dadurch unterscheiden, daß R¹ für Nitro, R⁹ für iso-Butyl, R¹⁰ für Trifluormethylsulfonyl und R¹¹ für Methyl stehen: 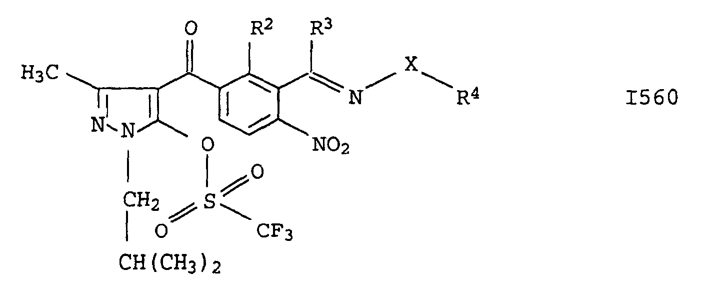
- die Verbindungen I561, insbesondere die Verbindungen I561.001-I561.180, die sich von den entsprechenden Verbindungen I1.001-I1.180 dadurch unterscheiden, daß R¹ für Nitro, R⁹ für Methyl, R¹⁰ für Phenylcarbonylmethyl und R¹¹ für Methyl stehen: 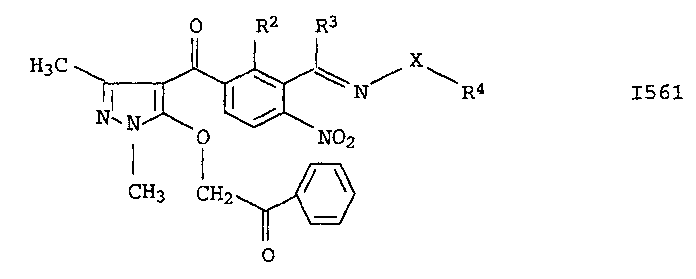
- die Verbindungen I562, insbesondere die Verbindungen I562.001-I562.180, die sich von den entsprechenden Verbindungen I1.001-I1.180 dadurch unterscheiden, daß R¹ für Nitro, R⁹ für Ethyl, R¹⁰ für Phenylcarbonylmethyl und R¹¹ für Methyl stehen: 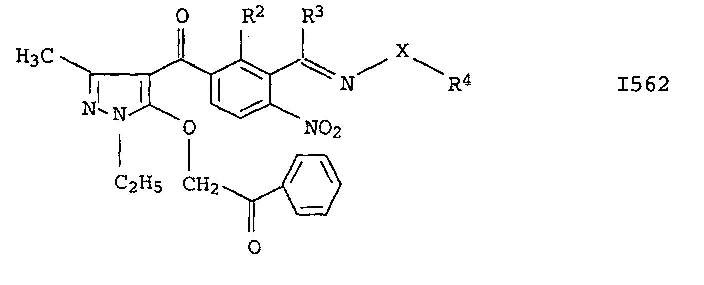
- die Verbindungen I563, insbesondere die Verbindungen I563.001-I563.180, die sich von den entsprechenden Verbindungen I1.001-I1.180 dadurch unterscheiden, daß R¹ für Nitro, R⁹ für n-Propyl, R¹⁰ für Phenylcarbonylmethyl und R¹¹ für Methyl stehen: 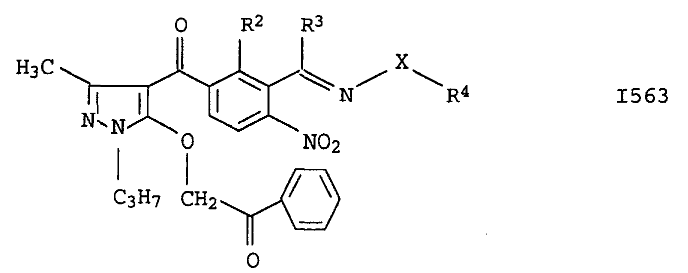
- die Verbindungen I564, insbesondere die Verbindungen I564.001-I564.180, die sich von den entsprechenden Verbindungen I1.001-I1.180 dadurch unterscheiden, daß R¹ für Nitro, R⁹ für n-Butyl, R¹⁰ für Phenylcarbonylmethyl und R¹¹ für Methyl stehen: 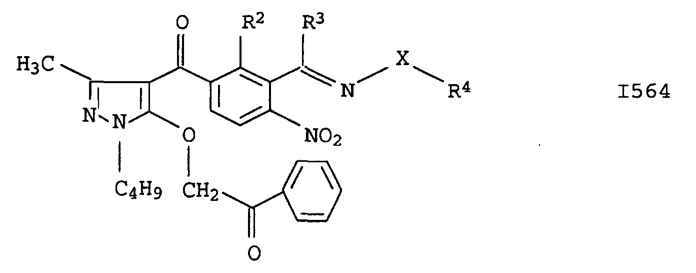
- die Verbindungen I565, insbesondere die Verbindungen I565.001-I565.180, die sich von den entsprechenden Verbindungen I1.001-I1.180 dadurch unterscheiden, daß R¹ für Nitro, R⁹ für iso-Butyl, R¹⁰ für Phenylcarbonylmethyl und R¹¹ für Methyl stehen: 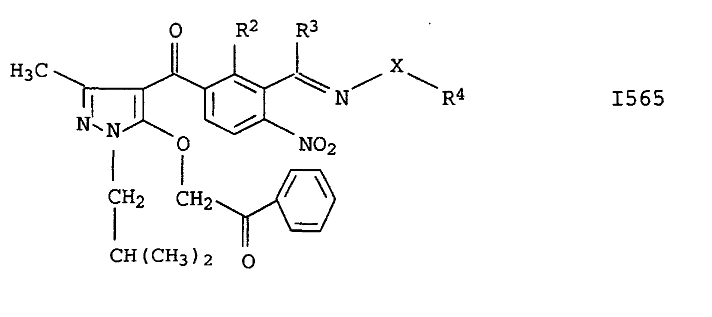
- die Verbindungen I566, insbesondere die Verbindungen I566.001-I566.180, die sich von den entsprechenden Verbindungen I1.001-I1.180 dadurch unterscheiden, daß R¹ für Nitro, R¹⁰ für Phenylsulfonyl und R¹¹ für Methyl stehen: 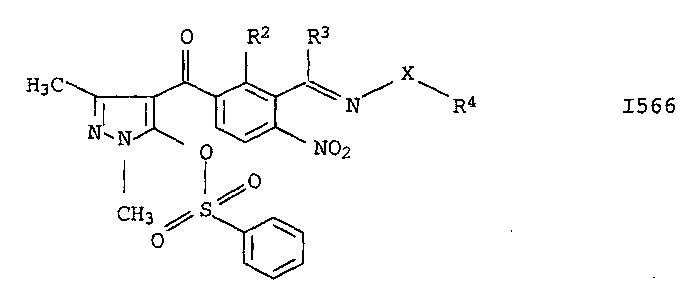
- die Verbindungen I567, insbesondere die Verbindungen I567.001-I567.180, die sich von den entsprechenden Verbindungen I1.001-I1.180 dadurch unterscheiden, daß R¹ für Nitro, R⁹ für Ethyl, R¹⁰ für Phenylsulfonyl und R¹¹ für Methyl stehen: 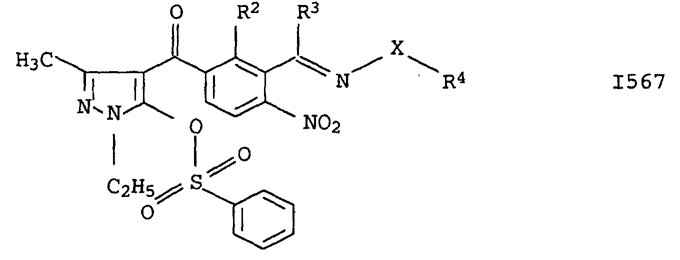
- die Verbindungen I568, insbesondere die Verbindungen I568.001-I568.180, die sich von den entsprechenden Verbindungen I1.001-I1.180 dadurch unterscheiden, daß R¹ für Nitro, R⁹ für Ethyl, R¹⁰ für Phenylsulfonyl und R¹¹ für Methyl stehen: 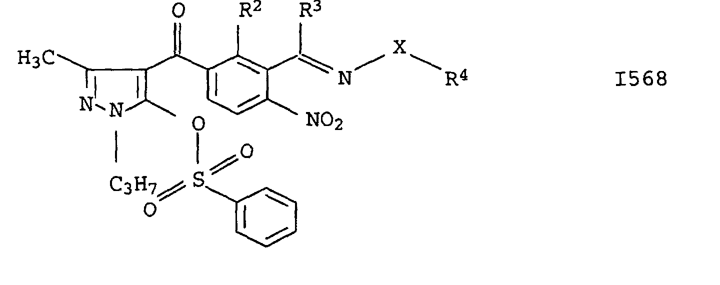
- die Verbindungen I569, insbesondere die Verbindungen I569.001-I569.180, die sich von den entsprechenden Verbindungen I1.001-I1.180 dadurch unterscheiden, daß R¹ für Nitro, R⁹ für n-Butyl, R¹⁰ für Phenylsulfonyl und R¹¹ für Methyl stehen: 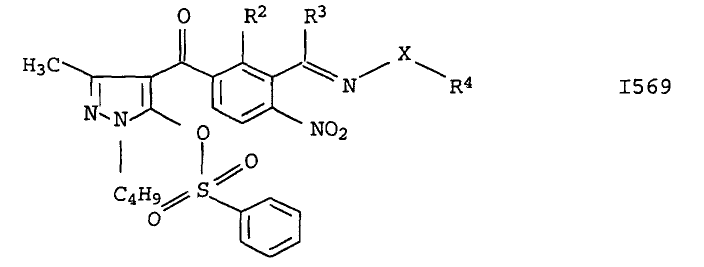
- die Verbindungen I570, insbesondere die Verbindungen I570.001-I570.180, die sich von den entsprechenden Verbindungen I1.001-I1.180 dadurch unterscheiden, daß R¹ für Nitro, R⁹ für iso-Butyl, R¹⁰ für Phenylsulfonyl und R¹¹ für Methyl stehen: 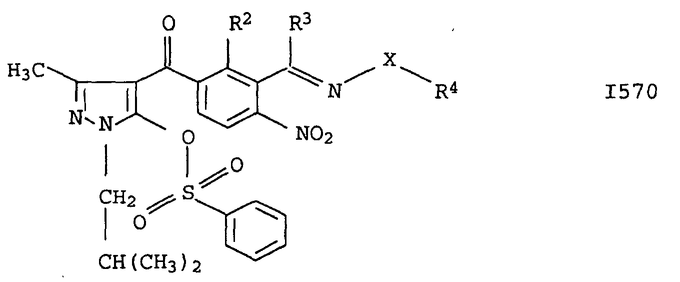
- die Verbindungen I571, insbesondere die Verbindungen I571.001-I571.180, die sich von den entsprechenden Verbindungen I1.001-I1.180 dadurch unterscheiden, daß R¹ für Nitro, R¹⁰ für 4-Methylphenylsulfonyl und R¹¹ für Methyl stehen: 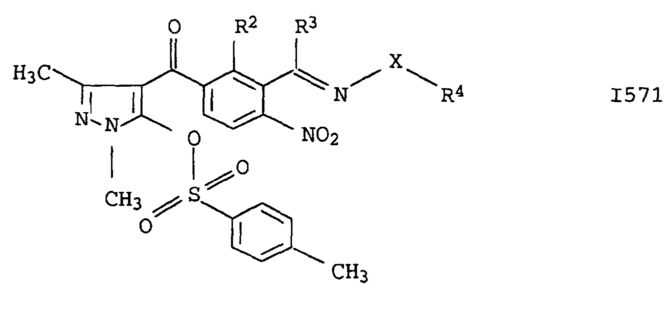
- die Verbindungen I572, insbesondere die Verbindungen I572.001-I572.180, die sich von den entsprechenden Verbindungen I1.001-I1.180 dadurch unterscheiden, daß R¹ für Nitro, R⁹ für Ethyl, R¹⁰ für 4-Methylphenylsulfonyl und R¹¹ für Methyl stehen: 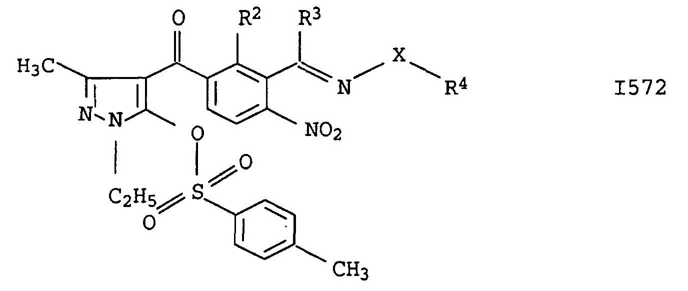
- die Verbindungen I573, insbesondere die Verbindungen I573.001-I573.180, die sich von den entsprechenden Verbindungen I1.001-I1.180 dadurch unterscheiden, daß R¹ für Nitro, R⁹ für n-Propyl, R¹⁰ für 4-Methylphenylsulfonyl und R¹¹ für Methyl stehen: 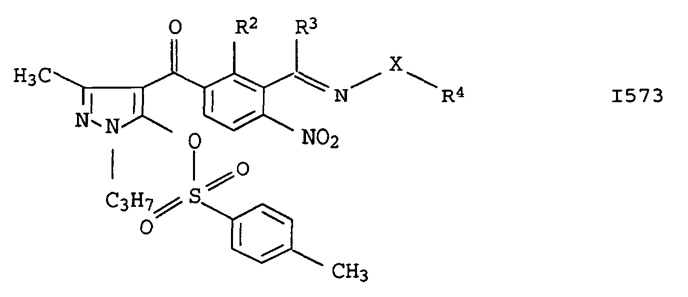
- die Verbindungen I574, insbesondere die Verbindungen I574.001-I574.180, die sich von den entsprechenden Verbindungen I1.001-I1.180 dadurch unterscheiden, daß R¹ für Nitro, R⁹ für n-Butyl, R¹⁰ für 4-Methylphenylsulfonyl und R¹¹ für Methyl stehen: 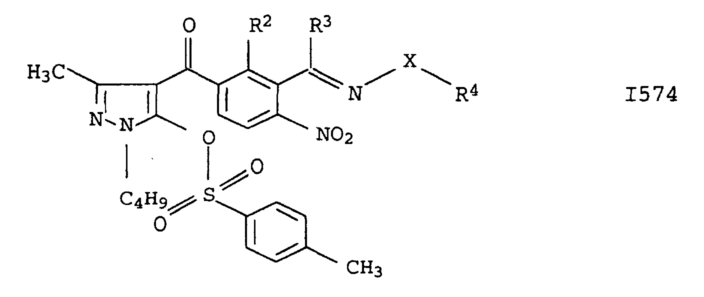
- die Verbindungen I575, insbesondere die Verbindungen I575.001-I575.180, die sich von den entsprechenden Verbindungen I1.001-I1.180 dadurch unterscheiden, daß R¹ für Nitro, R⁹ für iso-Butyl, R¹⁰ für 4-Methylphenylsulfonyl und R¹¹ für Methyl stehen: 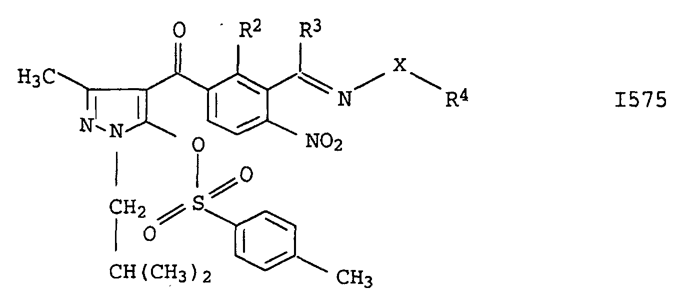
- die Verbindungen I576, insbesondere die Verbindungen I576.001-I576.180, die sich von den entsprechenden Verbindungen I1.001-I1.180 dadurch unterscheiden, daß R¹ für Methylsulfonyl und R¹¹ für Methyl stehen: 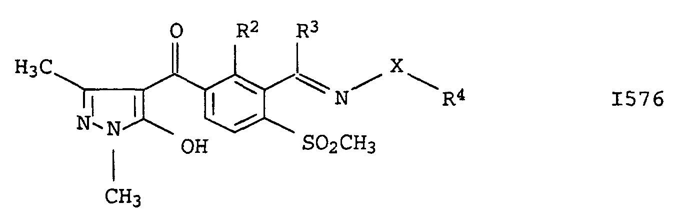
- die Verbindungen I577, insbesondere die Verbindungen I577.001-I577.180, die sich von den entsprechenden Verbindungen I1.001-I1.180 dadurch unterscheiden, daß R¹ für Methylsulfonyl, R⁹ für Ethyl und R¹⁰ für Methyl stehen: 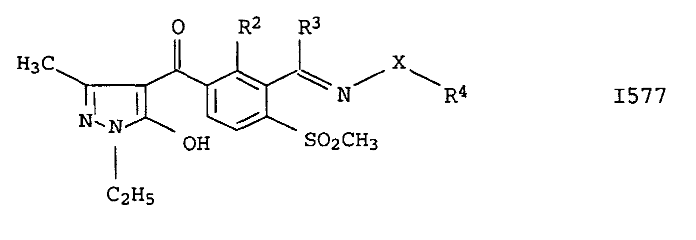
- die Verbindungen I578, insbesondere die Verbindungen I578.001-I578.180, die sich von den entsprechenden Verbindungen I1.001-I1.180 dadurch unterscheiden, daß R¹ für Methylsulfonyl, R⁹ für n-Propyl und R¹¹ für Methyl stehen: 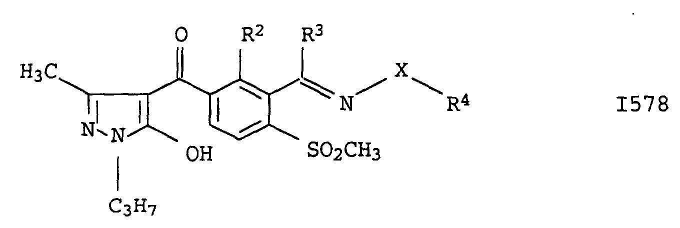
- die Verbindungen I579, insbesondere die Verbindungen I579.001-I579.180, die sich von den entsprechenden Verbindungen I1.001-I1.180 dadurch unterscheiden, daß R¹ für Methylsulfonyl, R⁹ für n-Butyl und R¹¹ für Methyl stehen: 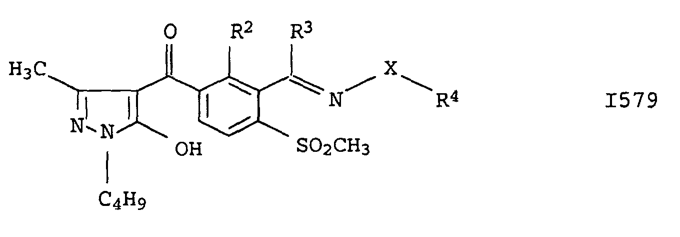
- die Verbindungen I580, insbesondere die Verbindungen I580.001-I580.180, die sich von den entsprechenden Verbindungen I1.001-I1.180 dadurch unterscheiden, daß R¹ für Methylsulfonyl, R⁹ für iso-Butyl und R¹¹ für Methyl stehen: 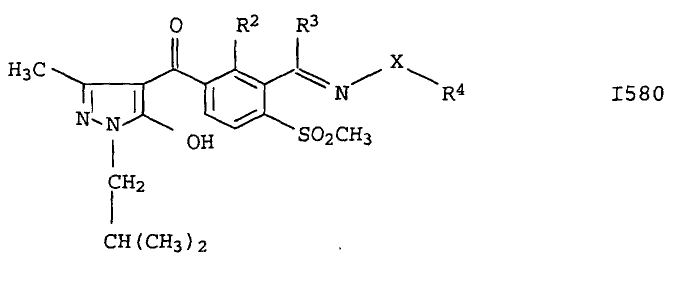
- die Verbindungen I581, insbesondere die Verbindungen I581.001-I581.180, die sich von den entsprechenden Verbindungen I1.001-I1.180 dadurch unterscheiden, daß R¹ für Methylsulfonyl und R¹⁰ und R¹¹ für Methyl stehen: 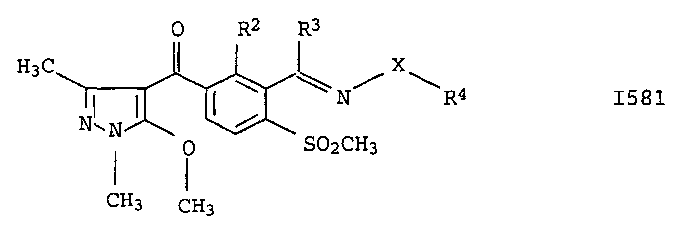
- die Verbindungen I582, insbesondere die Verbindungen I582.001-I582.180, die sich von den entsprechenden Verbindungen I1.001-I1.180 dadurch unterscheiden, daß R¹ für Methylsulfonyl, R⁹ für Ethyl und R¹⁰ und R¹¹ für Methyl stehen: 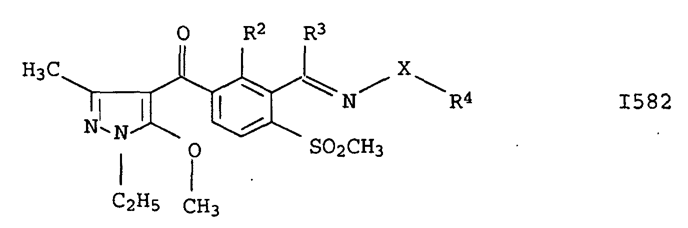
- die Verbindungen I583, insbesondere die Verbindungen I583.001-I583.180, die sich von den entsprechenden Verbindungen I1.001-I1.180 dadurch unterscheiden, daß R¹ für Methylsulfonyl, R⁹ für n-Propyl und R¹⁰ und R¹¹ für Methyl stehen: 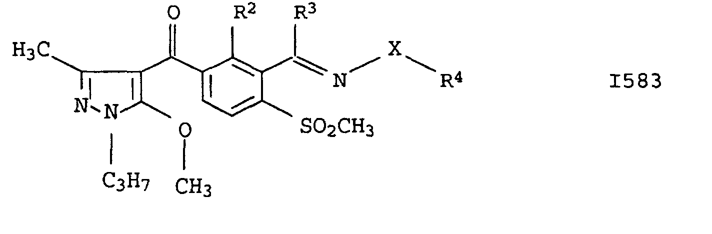
- die Verbindungen I584, insbesondere die Verbindungen I584.001-I584.180, die sich von den entsprechenden Verbindungen I1.001-I1.180 dadurch unterscheiden, daß R¹ für Methylsulfonyl, R⁹ für n-Butyl und R¹⁰ und R¹¹ für Methyl stehen: 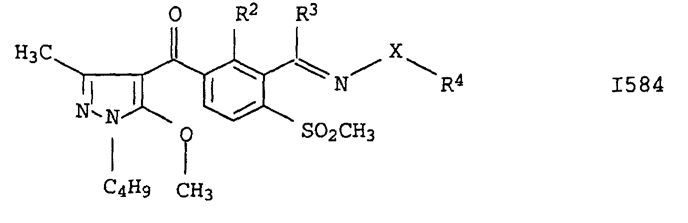
- die Verbindungen I585, insbesondere die Verbindungen I585.001-I585.180, die sich von den entsprechenden Verbindungen I1.001-I1.180 dadurch unterscheiden, daß R¹ für Methylsulfonyl, R⁹ für iso-Butyl und R¹⁰ und R¹¹ für Methyl stehen: 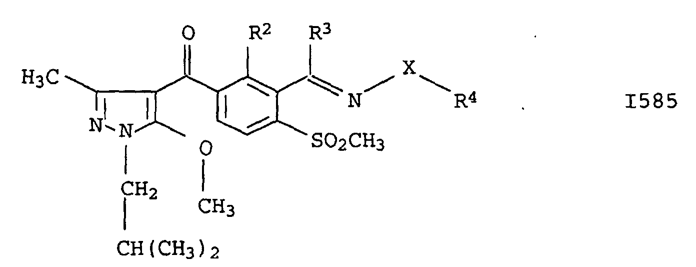
- die Verbindungen I586, insbesondere die Verbindungen I586.001-I586.180, die sich von den entsprechenden Verbindungen I1.001-I1.180 dadurch unterscheiden, daß R¹ für Methylsulfonyl, R¹⁰ für Ethyl und R¹¹ für Methyl stehen: 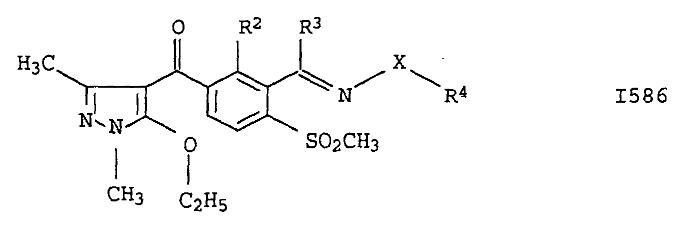
- die Verbindungen I587, insbesondere die Verbindungen I587.001-I587.180, die sich von den entsprechenden Verbindungen I1.001-I1.180 dadurch unterscheiden, daß R¹ für Methylsulfonyl, R⁹ und R¹⁰ für Ethyl und R¹¹ für Methyl stehen: 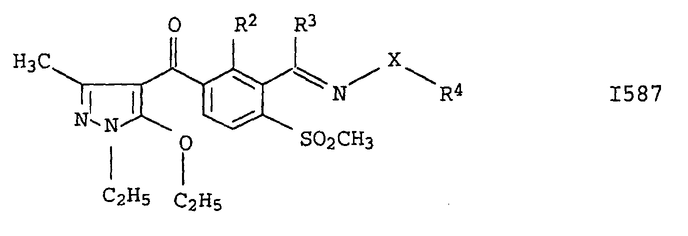
- die Verbindungen I588, insbesondere die Verbindungen I588.001-I588.180, die sich von den entsprechenden Verbindungen I1.001-I1.180 dadurch unterscheiden, daß R¹ für Methylsulfonyl, R⁹ für n-Propyl, R¹⁰ für Ethyl und R¹¹ für Methyl stehen: 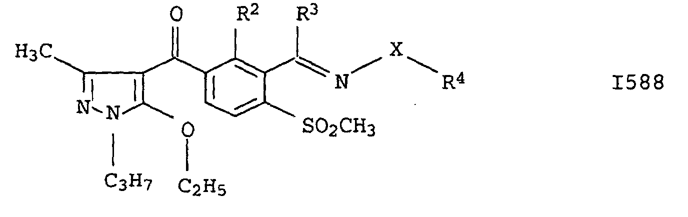
- die Verbindungen I589, insbesondere die Verbindungen I589.001-I589.180, die sich von den entsprechenden Verbindungen I1.001-I1.180 dadurch unterscheiden, daß R¹ für Methylsulfonyl, R⁹ für n-Butyl, R¹⁰ für Ethyl und R¹¹ für Methyl stehen: 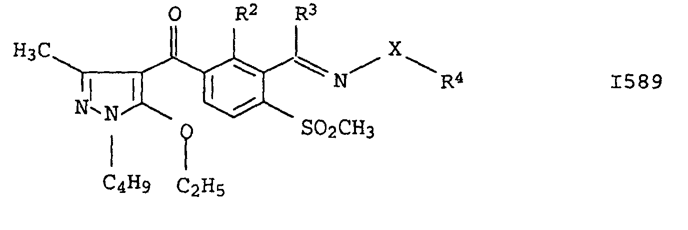
- die Verbindungen I590, insbesondere die Verbindungen I590.001-I590.180, die sich von den entsprechenden Verbindungen I1.001-I1.180 dadurch unterscheiden, daß R¹ für Methylsulfonyl, R⁹ für iso-Butyl, R¹⁰ für Ethyl und R¹¹ für Methyl stehen: 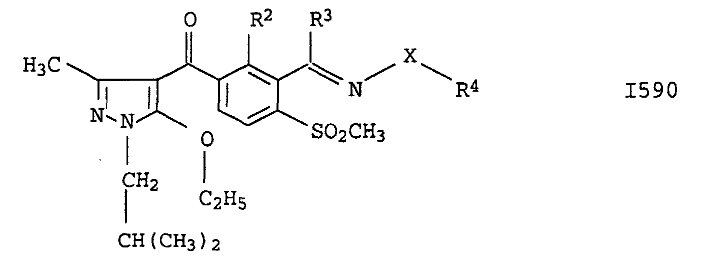
- die Verbindungen I591, insbesondere die Verbindungen I591.001-I591.180, die sich von den entsprechenden Verbindungen I1.001-I1.180 dadurch unterscheiden, daß R¹ für Methylsulfonyl, R¹⁰ für n-Propyl und R¹¹ für Methyl stehen: 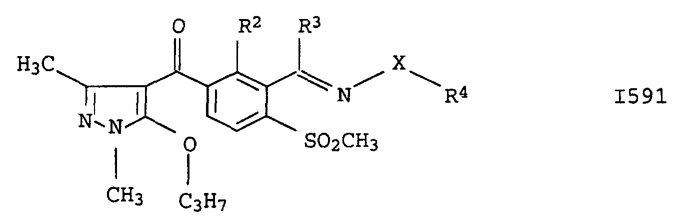
- die Verbindungen I592, insbesondere die Verbindungen I592.001-I592.180, die sich von den entsprechenden Verbindungen I1.001-I1.180 dadurch unterscheiden, daß R¹ für Methylsulfonyl, R⁹ für Ethyl, R¹⁰ für n-Propyl und R¹¹ für Methyl stehen: 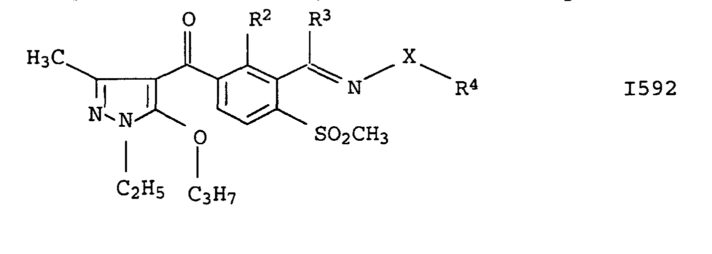
- die Verbindungen I593, insbesondere die Verbindungen I593.001-I593.180, die sich von den entsprechenden Verbindungen I1.001-I1.180 dadurch unterscheiden, daß R¹ für Methylsulfonyl, R⁹ und R¹⁰ für n-Propyl und R¹¹ für Methyl stehen: 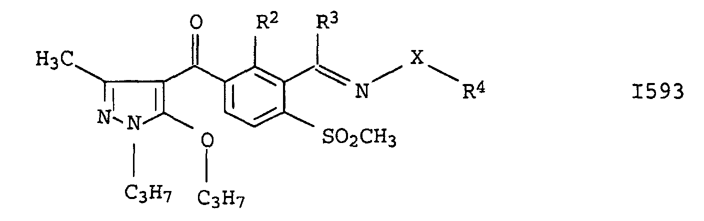
- die Verbindungen I594, insbesondere die Verbindungen I594.001-I594.180, die sich von den entsprechenden Verbindungen I1.001-I1.180 dadurch unterscheiden, daß R¹ für Methylsulfonyl, R⁹ für n-Butyl, R¹⁰ für n-Propyl und R¹¹ für Methyl stehen: 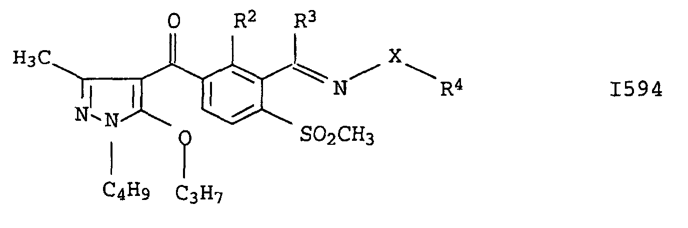
- die Verbindungen I595, insbesondere die Verbindungen I595.001-I595.180, die sich von den entsprechenden Verbindungen I1.001-I1.180 dadurch unterscheiden, daß R¹ für Methylsulfonyl, R⁹ für iso-Butyl, R¹⁰ für n-Propyl und R¹¹ für Methyl stehen: 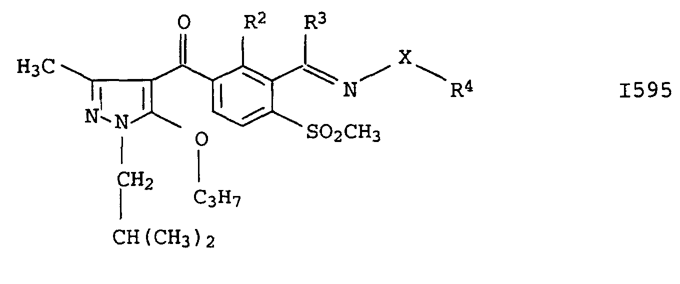
- die Verbindungen I596, insbesondere die Verbindungen I596.001-I596.180, die sich von den entsprechenden Verbindungen I1.001-I1.180 dadurch unterscheiden, daß R¹ für Methylsulfonyl, R¹⁰ für iso-Propyl und R¹¹ für Methyl stehen: 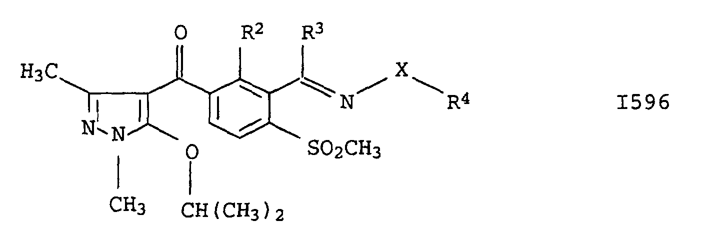
- die Verbindungen I597, insbesondere die Verbindungen I597.001-I597.180, die sich von den entsprechenden Verbindungen I1.001-I1.180 dadurch unterscheiden, daß R¹ für Methylsulfonyl, R⁹ für Ethyl, R¹⁰ für iso-Propyl und R¹¹ für Methyl stehen: 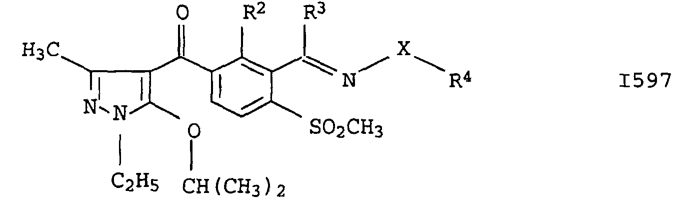
- die Verbindungen I598, insbesondere die Verbindungen I598.001-I598.180, die sich von den entsprechenden Verbindungen I1.001-I1.180 dadurch unterscheiden, daß R¹ für Methylsulfonyl, R⁹ für n-Propyl, R¹⁰ für iso-Propyl und R¹¹ für Methyl stehen: 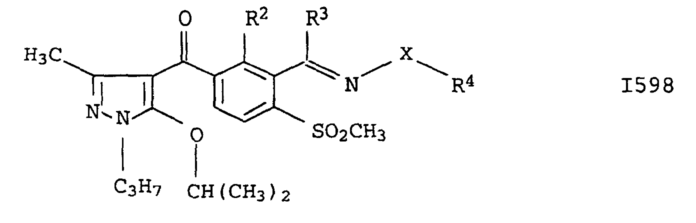
- die Verbindungen I599, insbesondere die Verbindungen I599.001-I599.180, die sich von den entsprechenden Verbindungen I1.001-I1.180 dadurch unterscheiden, daß R¹ für Methylsulfonyl, R⁹ für n-Butyl, R¹⁰ für iso-Propyl und R¹¹ für Methyl stehen: 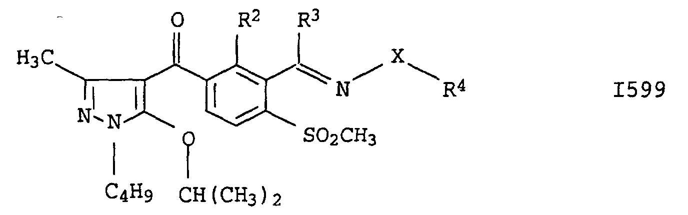
- die Verbindungen I600, insbesondere die Verbindungen I600.001-I600.180, die sich von den entsprechenden Verbindungen I1.001-I1.180 dadurch unterscheiden, daß R¹ für Methylsulfonyl, R⁹ für iso-Butyl, R¹⁰ für iso-Propyl und R¹¹ für Methyl stehen: 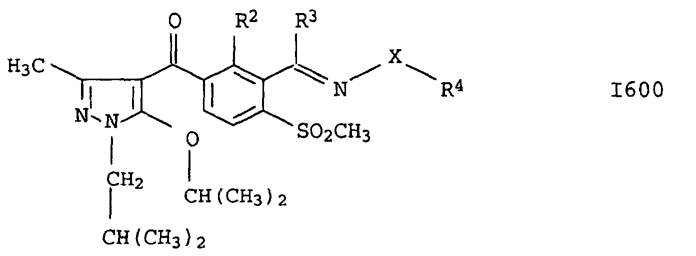
- die Verbindungen I601, insbesondere die Verbindungen I601.001-I601.180, die sich von den entsprechenden Verbindungen I1.001-I1.180 dadurch unterscheiden, daß R¹ für Methylsulfonyl, R¹⁰ für n-Butyl und R¹¹ für Methyl stehen: 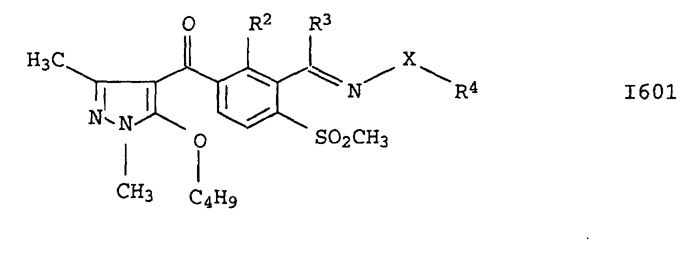
- die Verbindungen I602, insbesondere die Verbindungen I602.001-I602.180, die sich von den entsprechenden Verbindungen I1.001-I1.180 dadurch unterscheiden, daß R¹ für Methylsulfonyl, R⁹ für Ethyl, R¹⁰ für n-Butyl und R¹¹ für Methyl stehen: 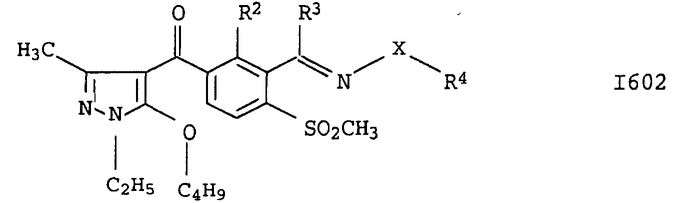
- die Verbindungen I603, insbesondere die Verbindungen I603.001-I603.180, die sich von den entsprechenden Verbindungen I1.001-I1.180 dadurch unterscheiden, daß R¹ für Methylsulfonyl, R⁹ für n-Propyl, R¹⁰ für n-Butyl und R¹¹ für Methyl stehen: 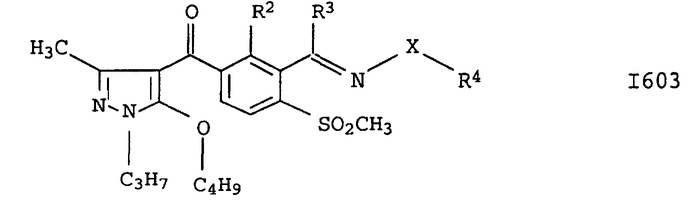
- die Verbindungen I604, insbesondere die Verbindungen I604.001-I604.180, die sich von den entsprechenden Verbindungen I1.001-I1.180 dadurch unterscheiden, daß R¹ für Methylsulfonyl, R⁹ und R¹⁰ für n-Butyl und R¹¹ für Methyl stehen: 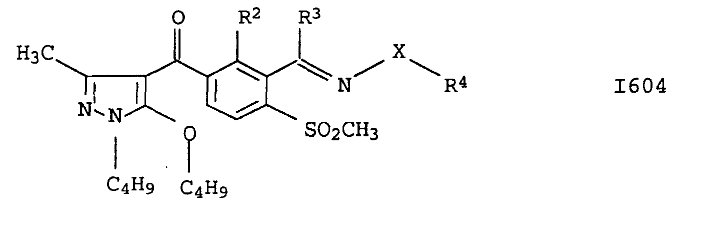
- die Verbindungen I605, insbesondere die Verbindungen I605.001-I605.180, die sich von den entsprechenden Verbindungen I1.001-I1.180 dadurch unterscheiden, daß R¹ für Methylsulfonyl, R⁹ für iso-Butyl, R¹⁰ für n-Butyl und R¹¹ für Methyl stehen: 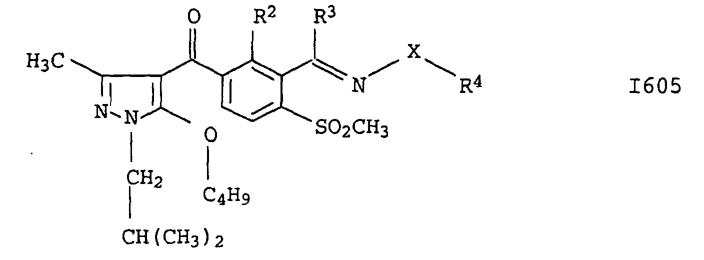
- die Verbindungen I606, insbesondere die Verbindungen I606.001-I606.180, die sich von den entsprechenden Verbindungen I1.001-I1.180 dadurch unterscheiden, daß R¹ für Methylsulfonyl, R¹⁰ für sec-Butyl und R¹¹ für Methyl stehen: 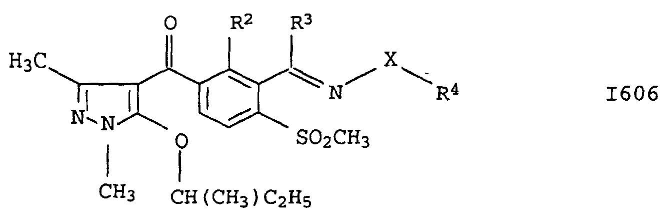
- die Verbindungen I607, insbesondere die Verbindungen I607.001-I607.180, die sich von den entsprechenden Verbindungen I1.001-I1.180 dadurch unterscheiden, daß R¹ für Methylsulfonyl, R⁹ für Ethyl, R¹⁰ für sec-Butyl und R¹¹ für Methyl stehen: 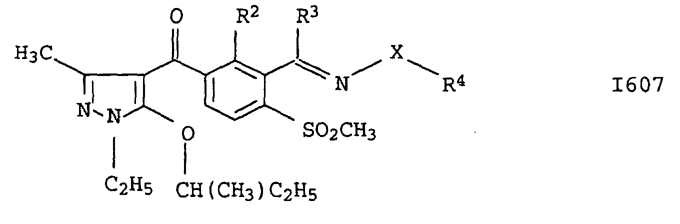
- die Verbindungen I608, insbesondere die Verbindungen I608.001-I608.180, die sich von den entsprechenden Verbindungen I1.001-I1.180 dadurch unterscheiden, daß R¹ für Methylsulfonyl, R9 für n-Propyl, R¹⁰ für sec-Butyl und R¹¹ für Methyl stehen: 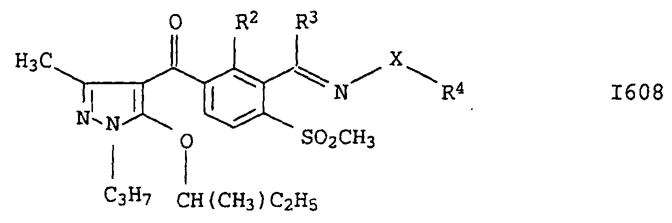
- die Verbindungen I609, insbesondere die Verbindungen I609.001-I609.180, die sich von den entsprechenden Verbindungen I1.001-I1.180 dadurch unterscheiden, daß R¹ für Methylsulfonyl, R⁹ für n-Butyl, R¹⁰ für sec-Butyl und R¹¹ für Methyl stehen: 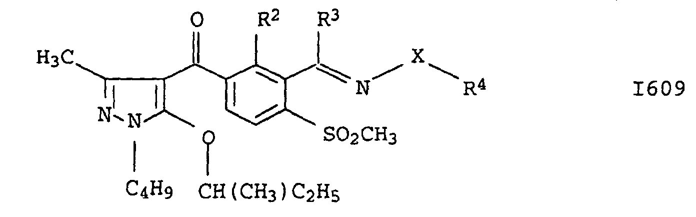
- die Verbindungen I610, insbesondere die Verbindungen I610.001-I610.180, die sich von den entsprechenden Verbindungen I1.001-I1.180 dadurch unterscheiden, daß R¹ für Methylsulfonyl, R⁹ für iso-Butyl, R¹⁰ für sec-Butyl und R¹¹ für Methyl stehen: 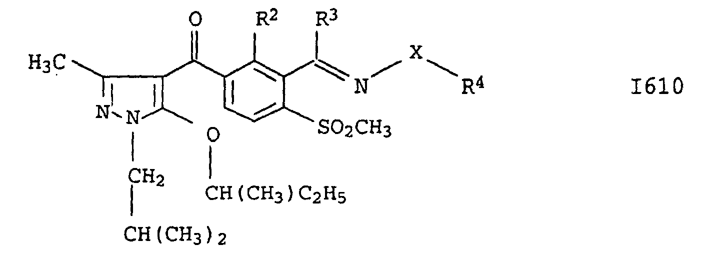
- die Verbindungen I611, insbesondere die Verbindungen I611.001-I611.180, die sich von den entsprechenden Verbindungen I1.001-I1.180 dadurch unterscheiden, daß R¹ für Methylsulfonyl, R¹⁰ für iso-Butyl und R¹¹ für Methyl stehen: 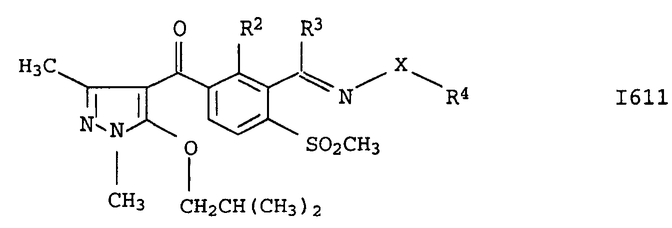
- die Verbindungen I612, insbesondere die Verbindungen I612.001-I612.180, die sich von den entsprechenden Verbindungen I1.001-I1.180 dadurch unterscheiden, daß R¹ für Methylsulfonyl, R⁹ für Ethyl, R¹⁰ für iso-Butyl und R¹¹ für Methyl stehen: 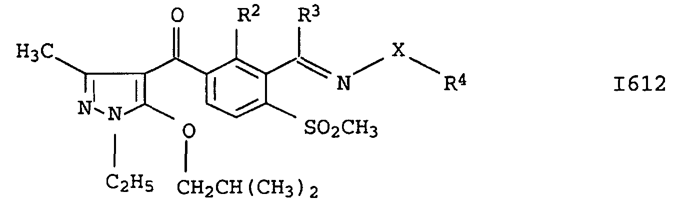
- die Verbindungen I613, insbesondere die Verbindungen I613.001-I613.180, die sich von den entsprechenden Verbindungen I1.001-I1.180 dadurch unterscheiden, daß R¹ für Methylsulfonyl, R⁹ für n-Propyl, R¹⁰ für iso-Butyl und R¹¹ für Methyl stehen: 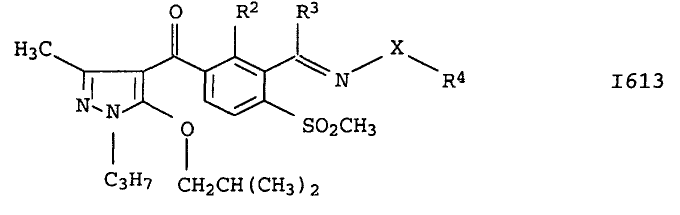
- die Verbindungen I614, insbesondere die Verbindungen I614.001-I614.180, die sich von den entsprechenden Verbindungen I1.001-I1.180 dadurch unterscheiden, daß R¹ für Methylsulfonyl, R⁹ für n-Butyl, R¹⁰ für iso-Butyl und R¹¹ für Methyl stehen: 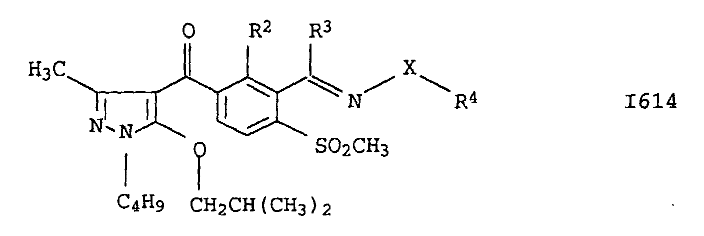
- die Verbindungen I615, insbesondere die Verbindungen I615.001-I615.180, die sich von den entsprechenden Verbindungen I1.001-I1.180 dadurch unterscheiden, daß R¹ für Methylsulfonyl, R⁹ und R¹⁰ für iso-Butyl und R¹¹ für Methyl stehen: 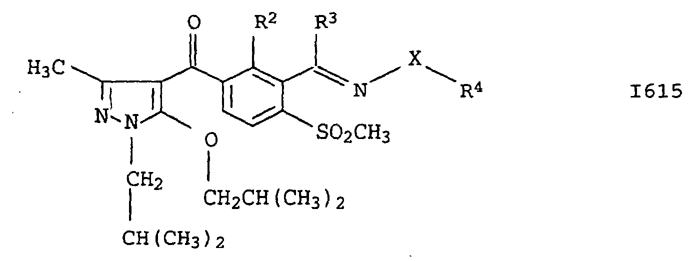
- die Verbindungen I616, insbesondere die Verbindungen I616.001-I616.180 die sich von den entsprechenden Verbindungen I1.001-I1.180 dadurch unterscheiden, daß R¹ für Methylsulfonyl, R¹⁰ für Methylcarbonyl und R¹¹ für Methyl stehen: 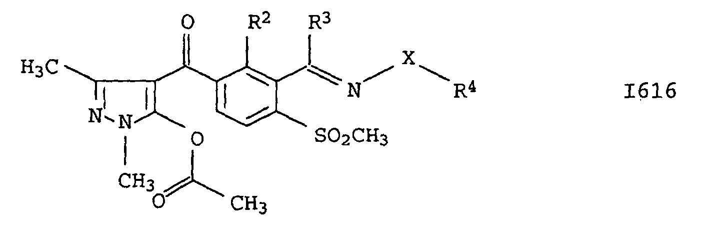
- die Verbindungen I617, insbesondere die Verbindungen I617.001-I617.180, die sich von den entsprechenden Verbindungen I1.001-I1.180 dadurch unterscheiden, daß R¹ für Methylsulfonyl, R⁹ für Ethyl, R¹⁰ für Methylcarbonyl und R¹¹ für Methyl stehen: 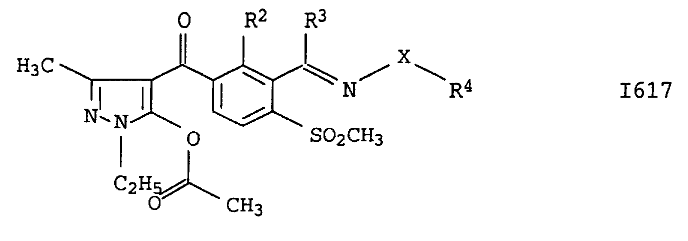
- die Verbindungen I618, insbesondere die Verbindungen I618.001-I618.180, die sich von den entsprechenden Verbindungen I1.001-I1.180 dadurch unterscheiden, daß R¹ für Methylsulfonyl, R⁹ für n-Propyl, R¹⁰ für Methylcarbonyl und R¹¹ für Methyl stehen: 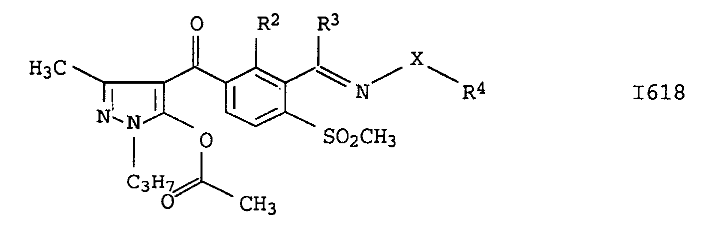
- die Verbindungen I619, insbesondere die Verbindungen I619.001-I619.180, die sich von den entsprechenden Verbindungen I1.001-I1.180 dadurch unterscheiden, daß R¹ für Methylsulfonyl, R⁹ für n-Butyl, R¹⁰ für Methylcarbonyl und R¹¹ für Methyl stehen: 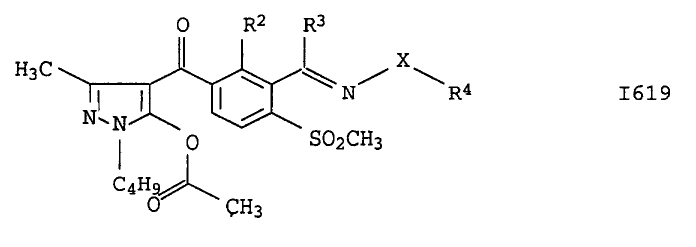
- die Verbindungen I620, insbesondere die Verbindungen I620.001-I620.180, die sich von den entsprechenden Verbindungen I1.001-I1.180 dadurch unterscheiden, daß R¹ für Methylsulfonyl, R⁹ für iso-Butyl, R¹⁰ für Methylcarbonyl und R¹¹ für Methyl stehen: 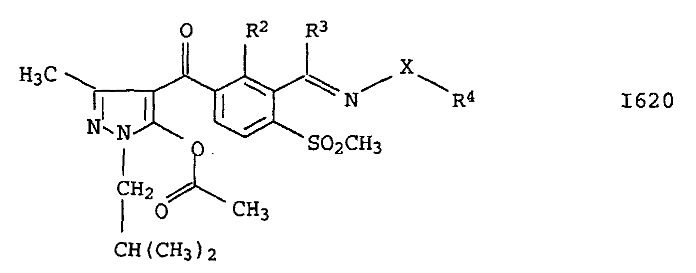
- die Verbindungen I621, insbesondere die Verbindungen I621.001-I621.180, die sich von den entsprechenden Verbindungen I1.001-I1.180 dadurch unterscheiden, daß R¹ für Methylsulfonyl, R¹⁰ für Ethylcarbonyl und R¹¹ für Methyl stehen: 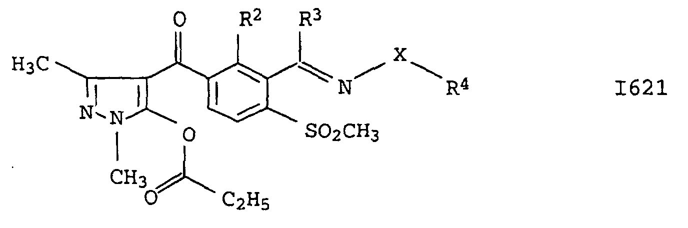
- die Verbindungen I622, insbesondere die Verbindungen I622.001-I622.180, die sich von den entsprechenden Verbindungen I1.001-I1.180 dadurch unterscheiden, daß R¹ für Methylsulfonyl, R⁹ für Ethyl, R¹⁰ für Ethylcarbonyl und R¹¹ für Methyl stehen: 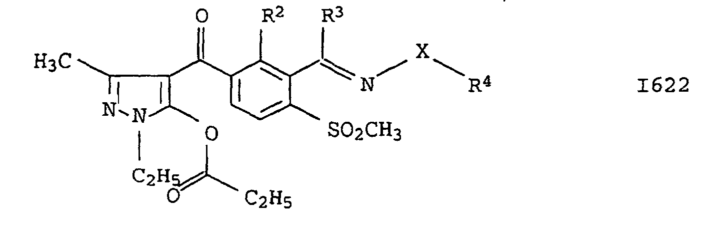
- die Verbindungen I623, insbesondere die Verbindungen I623.001-I623.180, die sich von den entsprechenden Verbindungen I1.001-I1.180 dadurch unterscheiden, daß R¹ für Methylsulfonyl, R⁹ für n-Propyl, R¹⁰ für Ethylcarbonyl und R¹¹ für Methyl stehen: 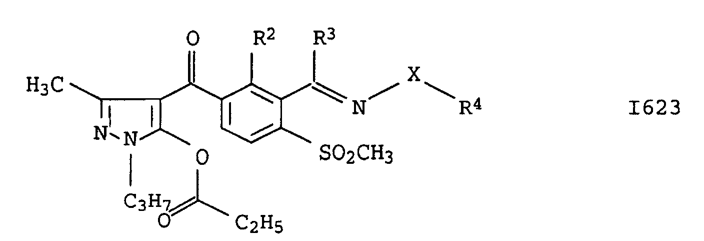
- die Verbindungen I624, insbesondere die Verbindungen I624.001-I624.180, die sich von den entsprechenden Verbindungen I1.001-I1.180 dadurch unterscheiden, daß R¹ für Methylsulfonyl, R⁹ für n-Butyl, R¹⁰ für Ethylcarbonyl und R¹¹ für Methyl stehen: 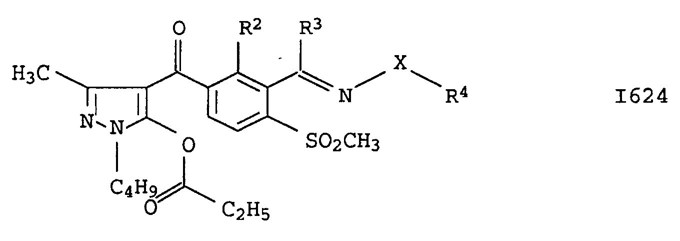
- die Verbindungen I625, insbesondere die Verbindungen I625.001-I625.180, die sich von den entsprechenden Verbindungen I1.001-I1.180 dadurch unterscheiden, daß R¹ für Methylsulfonyl, R9 für iso-Butyl, R¹⁰ für Ethylcarbonyl und R¹¹ für Methyl stehen: 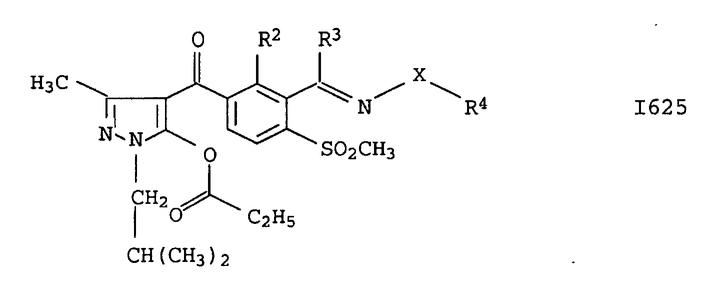
- die Verbindungen I626, insbesondere die Verbindungen I626.001-I626.180, die sich von den entsprechenden Verbindungen I1.001-I1.180 dadurch unterscheiden, daß R¹ für Methylsulfonyl, R¹⁰ für n-Propylcarbonyl und R¹¹ für Methyl stehen: 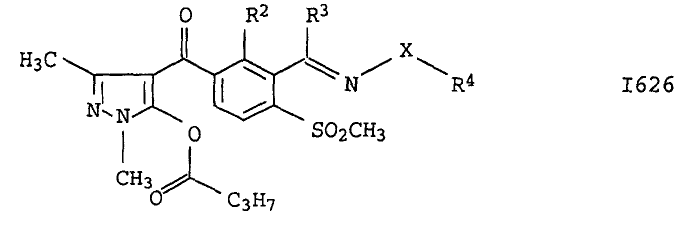
- die Verbindungen I627, insbesondere die Verbindungen I627.001-I627.180, die sich von den entsprechenden Verbindungen I1.001-I1.180 dadurch unterscheiden, daß R¹ für Methylsulfonyl, R⁹ für Ethyl, R¹⁰ für n-Propylcarbonyl und R¹¹ für Methyl stehen: 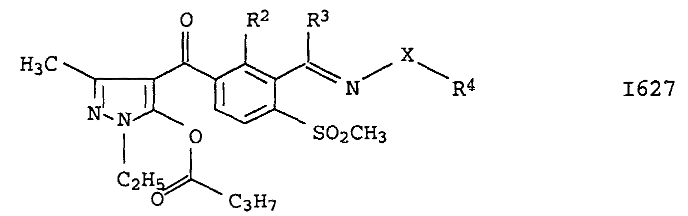
- die Verbindungen I628, insbesondere die Verbindungen I628.001-I628.180, die sich von den entsprechenden Verbindungen I1.001-I1.180 dadurch unterscheiden, daß R¹ für Methylsulfonyl, R⁹ für n-Propyl, R¹⁰ für n-Propylcarbonyl und R¹¹ für Methyl stehen: 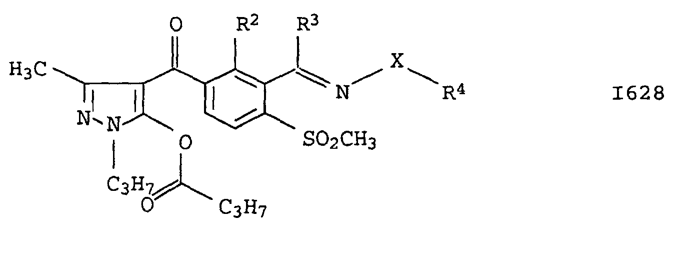
- die Verbindungen I629, insbesondere die Verbindungen I629.001-I629.180, die sich von den entsprechenden Verbindungen I1.001-I1.180 dadurch unterscheiden, daß R¹ für Methylsulfonyl, R⁹ für n-Butyl, R¹⁰ für n-Propylcarbonyl und R¹¹ für Methyl stehen: 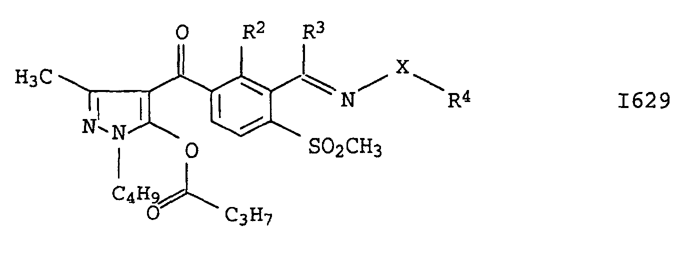
- die Verbindungen I630, insbesondere die Verbindungen I630.001-I630.180, die sich von den entsprechenden Verbindungen I1.001-I1.180 dadurch unterscheiden, daß R¹ für Methylsulfonyl, R⁹ für iso-Butyl, R¹⁰ für n-Propylcarbonyl und R¹¹ für Methyl stehen: 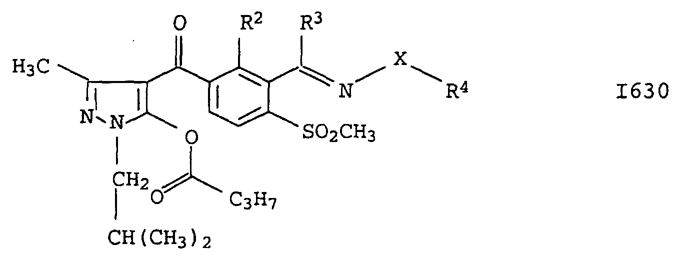
- die Verbindungen I631, insbesondere die Verbindungen I631.001-I631.180, die sich von den entsprechenden Verbindungen I1.001-I1.180 dadurch unterscheiden, daß R¹ für Methylsulfonyl, R¹⁰ für Trifluormethylcarbonyl und R¹¹ für Methyl stehen: 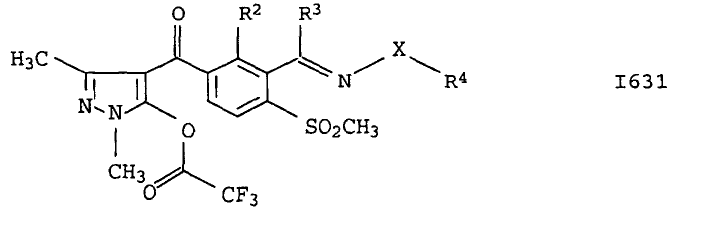
- die Verbindungen I632, insbesondere die Verbindungen I632.001-I632.180, die sich von den entsprechenden Verbindungen I1.001-I1.180 dadurch unterscheiden, daß R¹ für Methylsulfonyl, R⁹ für Ethyl, R¹⁰ für Trifluormethylcarbonyl und R¹¹ für Methyl stehen: 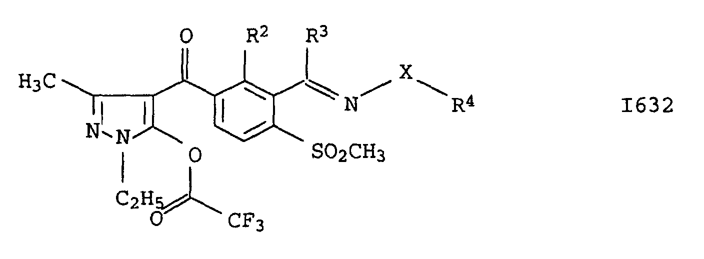
- die Verbindungen I633, insbesondere die Verbindungen I633.001-I633.180, die sich von den entsprechenden Verbindungen I1.001-I1.180 dadurch unterscheiden, daß R¹ für Methylsulfonyl, R⁹ für n-Propyl, R¹⁰ für Trifluormethylcarbonyl und R¹¹ für Methyl stehen: 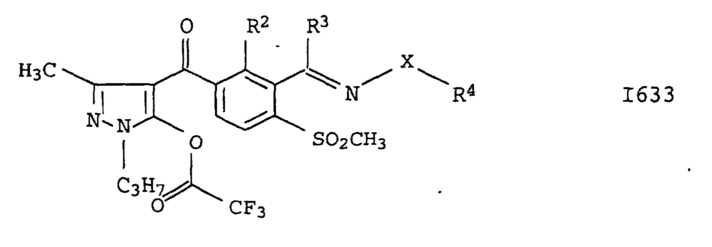
- die Verbindungen I634, insbesondere die Verbindungen I634.001-I634.180, die sich von den entsprechenden Verbindungen I1.001-I1.180 dadurch unterscheiden, daß R¹ für Methylsulfonyl, R⁹ für n-Butyl, R¹⁰ für Trifluormethylcarbonyl und R¹¹ für Methyl stehen: 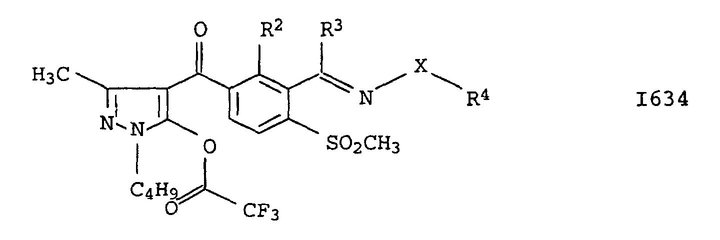
- die Verbindungen I635, insbesondere die Verbindungen I635.001-I635.180, die sich von den entsprechenden Verbindungen I1.001-I1.180 dadurch unterscheiden, daß R¹ für Methylsulfonyl, R⁹ für iso-Butyl, R¹⁰ für Trifluormethylcarbonyl und R¹¹ für Methyl stehen: 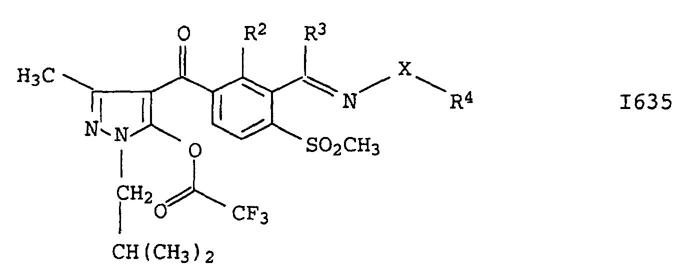
- die Verbindungen I636, insbesondere die Verbindungen I636.001-I636.180, die sich von den entsprechenden Verbindungen I1.001-I1.180 dadurch unterscheiden, daß R¹ und R¹⁰ für Methylsulfonyl und R¹¹ für Methyl stehen: 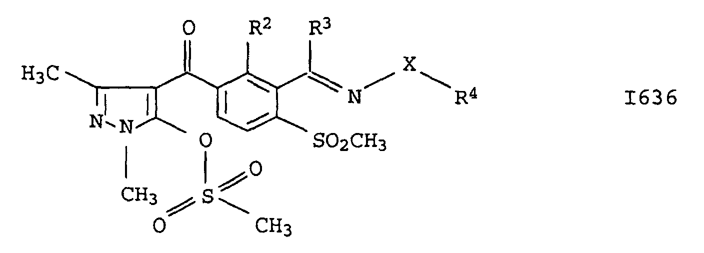
- die Verbindungen I637, insbesondere die Verbindungen I637.001-I637.180, die sich von den entsprechenden Verbindungen I1.001-I1.180 dadurch unterscheiden, daß R¹ und R¹⁰ für Methylsulfonyl. R⁹ für Ethyl und R¹¹ für Methyl stehen: 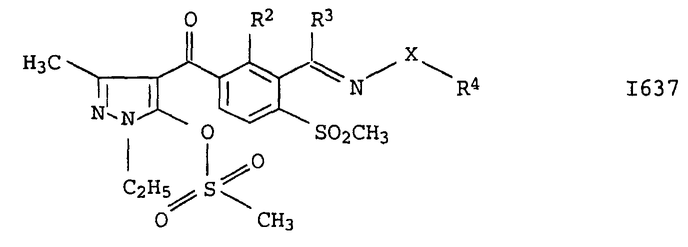
- die Verbindungen I638, insbesondere die Verbindungen I638.001-I638.180, die sich von den entsprechenden Verbindungen I1.001-I1.180 dadurch unterscheiden, daß R¹ und R¹⁰ für Methylsulfonyl, R⁹ für n-Propyl und R¹¹ für Methyl stehen: 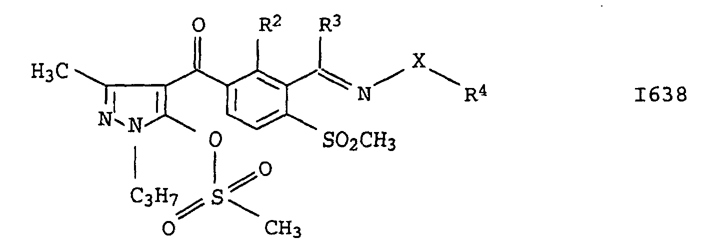
- die Verbindungen I639, insbesondere die Verbindungen I639.001-I639.180, die sich von den entsprechenden Verbindungen I1.001-I1.180 dadurch unterscheiden, daß R¹ und R¹⁰ für Methylsulfonyl, R⁹ für n-Butyl und R¹¹ für Methyl stehen: 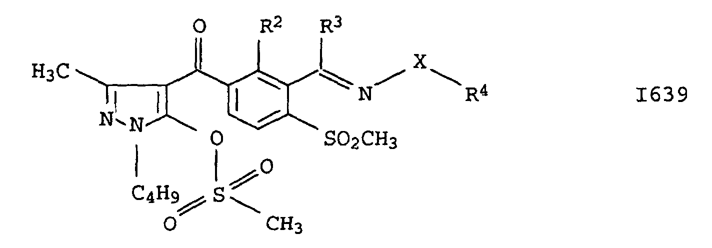
- die Verbindungen I640, insbesondere die Verbindungen I640.001-I640.180, die sich von den entsprechenden Verbindungen I1.001-I1.180 dadurch unterscheiden, daß R¹ und R¹⁰ für Methylsulfonyl, R⁹ für iso-Butyl und R¹¹ für Methyl stehen: 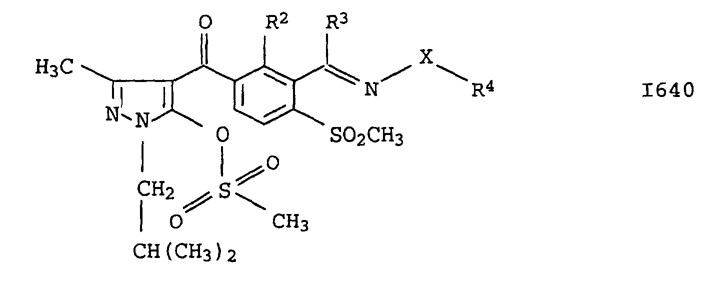
- die Verbindungen I641, insbesondere die Verbindungen I641.001-I641.180, die sich von den entsprechenden Verbindungen I1.001-I1.180 dadurch unterscheiden, daß R¹ für Methylsulfonyl, R¹⁰ für Ethylsulfonyl und R¹¹ für Methyl stehen: 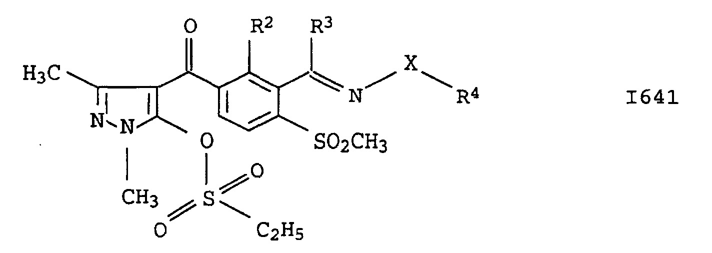
- die Verbindungen I642, insbesondere die Verbindungen I642.001-I642.180, die sich von den entsprechenden Verbindungen I1.001-I1.180 dadurch unterscheiden, daß R¹ für Methylsulfonyl, R⁹ für Ethyl, R¹⁰ für Ethylsulfonyl und R¹¹ für Methyl stehen: 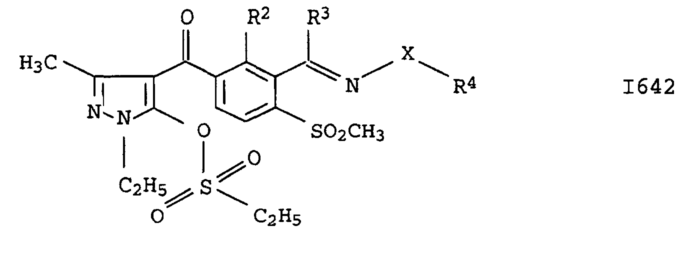
- die Verbindungen I643, insbesondere die Verbindungen I643.001-I643.180, die sich von den entsprechenden Verbindungen I1.001-I1.180 dadurch unterscheiden, daß R¹ für Methylsulfonyl, R⁹ für n-Propyl, R¹⁰ für Ethylsulfonyl und R¹¹ für Methyl stehen: 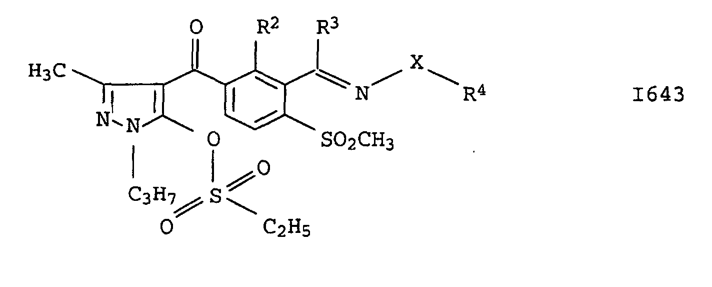
- die Verbindungen I644, insbesondere die Verbindungen I644.001-I644.180, die sich von den entsprechenden Verbindungen I1.001-I1.180 dadurch unterscheiden, daß R¹ für Methylsulfonyl, R⁹ für n-Butyl, R¹⁰ für Ethylsulfonyl und R¹¹ für Methyl stehen: 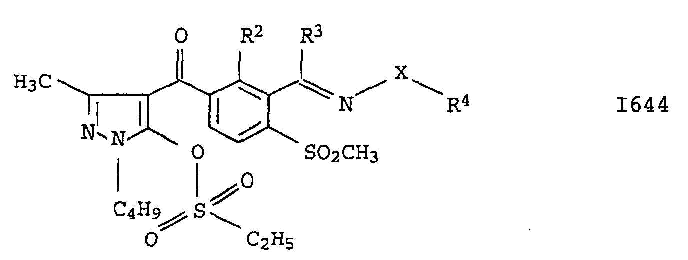
- die Verbindungen I645, insbesondere die Verbindungen I645.001-I645.180, die sich von den entsprechenden Verbindungen I1.001-I1.180 dadurch unterscheiden, daß R¹ für Methylsulfonyl, R⁹ für iso-Butyl, R¹⁰ für Ethylsulfonyl und R¹¹ für Methyl stehen: 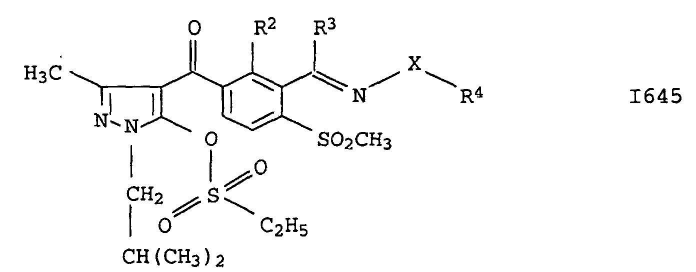
- die Verbindungen I646, insbesondere die Verbindungen I646.001-I646.180, die sich von den entsprechenden Verbindungen I1.001-I1.180 dadurch unterscheiden, daß R¹ für Methylsulfonyl, R¹⁰ für n-Propylsulfonyl und R¹¹ für Methyl stehen: 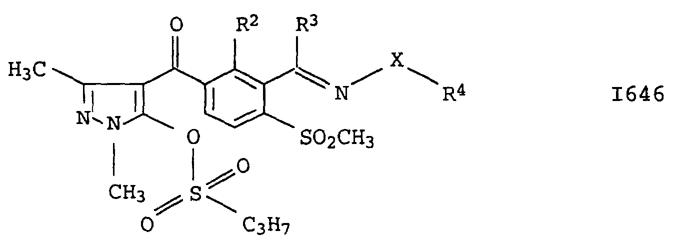
- die Verbindungen I647, insbesondere die Verbindungen I647.001-I647.180, die sich von den entsprechenden Verbindungen I1.001-I1.180 dadurch unterscheiden, daß R¹ für Methylsulfonyl, R⁹ für Ethyl, R¹⁰ für n-Propylsulfonyl und R¹¹ für Methyl stehen: 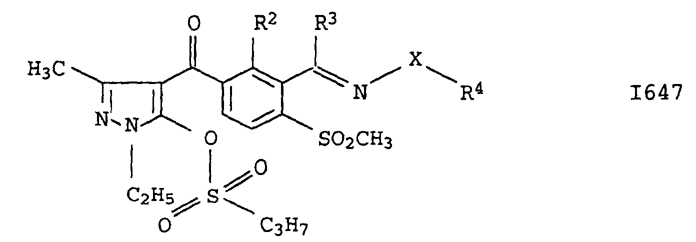
- die Verbindungen I648, insbesondere die Verbindungen I648.001-I648.180, die sich von den entsprechenden Verbindungen I1.001-I1.180 dadurch unterscheiden, daß R¹ für Methylsulfonyl, R⁹ für n-Propyl, R¹⁰ für n-Propylsulfonyl und R¹¹ für Methyl stehen: 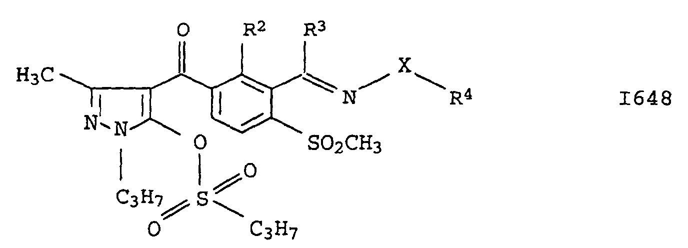
- die Verbindungen I649, insbesondere die Verbindungen I649.001-I649.180, die sich von den entsprechenden Verbindungen I1.001-I1.180 dadurch unterscheiden, daß R¹ für Methylsulfonyl, R⁹ für n-Butyl, R¹⁰ für n-Propylsulfonyl und R¹¹ für Methyl stehen: 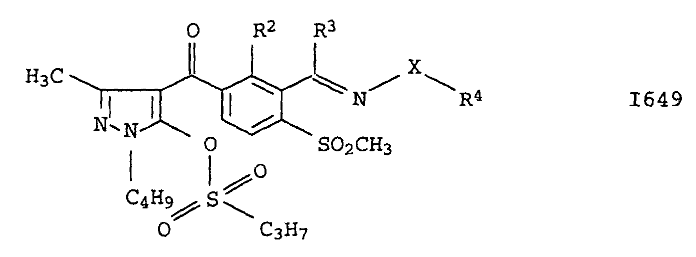
- die Verbindungen I650, insbesondere die Verbindungen I650.001-I650.180, die sich von den entsprechenden Verbindungen I1.001-I1.180 dadurch unterscheiden, daß R¹ für Methylsulfonyl, R⁹ für iso-Butyl, R¹⁰ für n-Propylsulfonyl und R¹¹ für Methyl stehen: 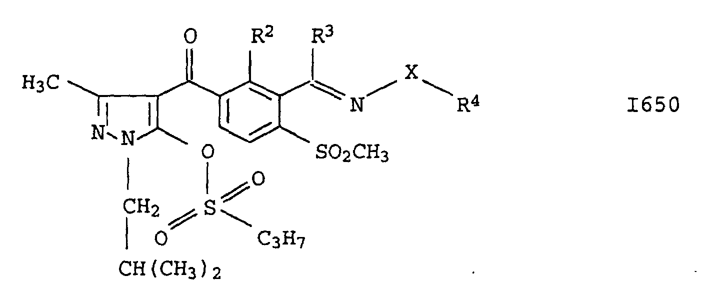
- die Verbindungen I651, insbesondere die Verbindungen I651.001-I651.180, die sich von den entsprechenden Verbindungen I1.001-I1.180 dadurch unterscheiden, daß R¹ für Methylsulfonyl, R¹⁰ für iso-Propylsulfonyl und R¹¹ für Methyl stehen: 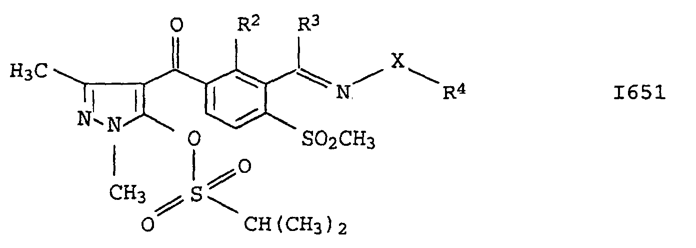
- die Verbindungen I652, insbesondere die Verbindungen I652.001-I652.180, die sich von den entsprechenden Verbindungen I1.001-I1.180 dadurch unterscheiden, daß R¹ für Methylsulfonyl, R⁹ für Ethyl, R¹⁰ für iso-Propylsulfonyl und R¹¹ für Methyl stehen: 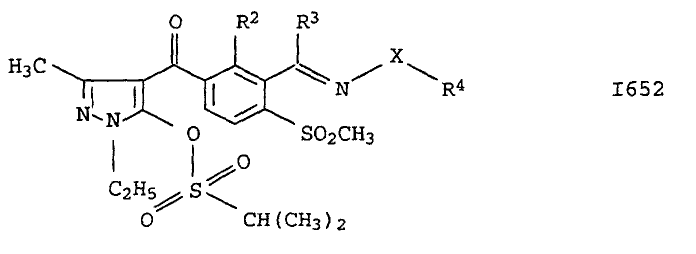
- die Verbindungen I653, insbesondere die Verbindungen I653.001-I653.180, die sich von den entsprechenden Verbindungen I1.001-I1.180 dadurch unterscheiden, daß R¹ für Methylsulfonyl, R⁹ für n-Propyl, R¹⁰ für iso-Propylsulfonyl und R¹¹ für Methyl stehen: 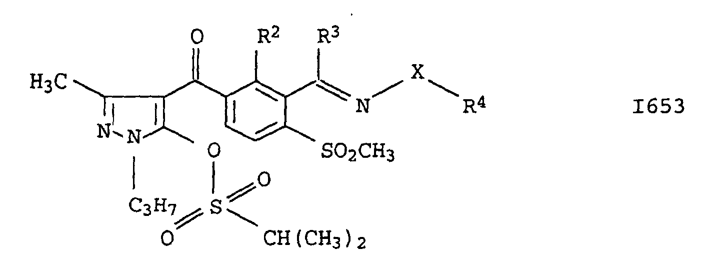
- die Verbindungen I654, insbesondere die Verbindungen I654.001-I654.180, die sich von den entsprechenden Verbindungen I1.001-I1.180 dadurch unterscheiden, daß R¹ für Methylsulfonyl, R⁹ für n-Butyl, R¹⁰ für iso-Propylsulfonyl und R¹¹ für Methyl stehen: 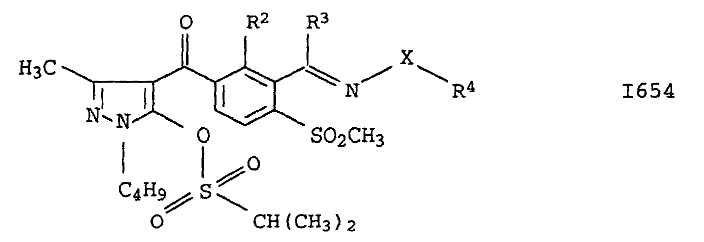
- die Verbindungen I655, insbesondere die Verbindungen I655.001-I655.180, die sich von den entsprechenden Verbindungen I1.001-I1.180 dadurch unterscheiden, daß R¹ für Methylsulfonyl, R⁹ für iso-Butyl, R¹⁰ für iso-Propylsulfonyl und R¹¹ für Methyl stehen: 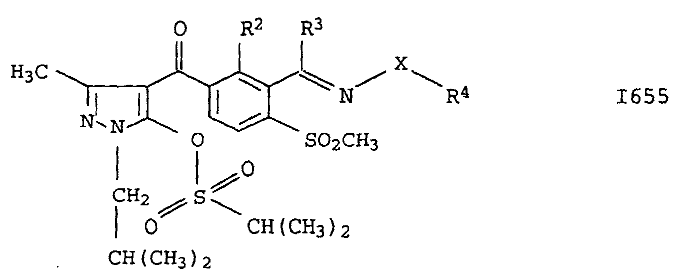
- die Verbindungen I656, insbesondere die Verbindungen I656.001-I656.180, die sich von den entsprechenden Verbindungen I1.001-I1.180 dadurch unterscheiden, daß R¹ für Methylsulfonyl, R¹⁰ für n-Butylsulfonyl und R¹¹ für Methyl stehen: 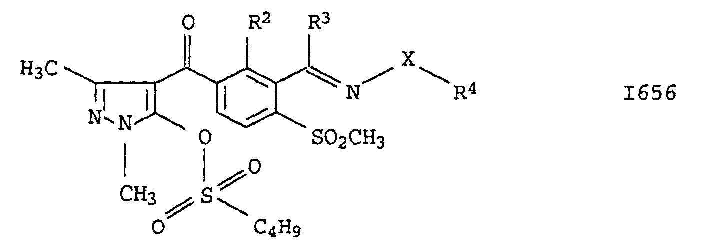
- die Verbindungen I657, insbesondere die Verbindungen I657.001-I657.180, die sich von den entsprechenden Verbindungen I1.001-I1.180 dadurch unterscheiden, daß R¹ für Methylsulfonyl, R⁹ für Ethyl, R¹⁰ für n-Butylsulfonyl und R¹¹ für Methyl stehen: 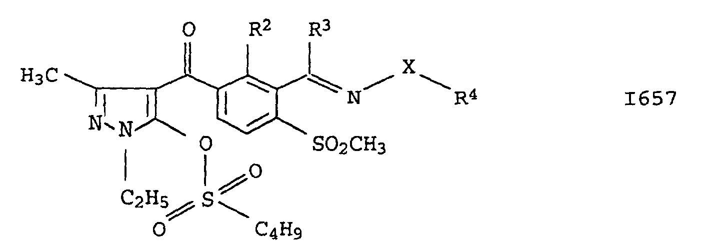
- die Verbindungen I658, insbesondere die Verbindungen I658.001-I658.180, die sich von den entsprechenden Verbindungen I1.001-I1.180 dadurch unterscheiden, daß R¹ für Methylsulfonyl, R⁹ für n-Propyl, R¹⁰ für n-Butylsulfonyl und R¹¹ für Methyl stehen: 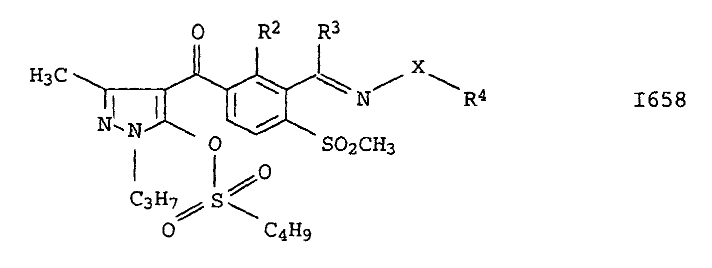
- die Verbindungen I659, insbesondere die Verbindungen I659.001-I659.180, die sich von den entsprechenden Verbindungen I1.001-I1.180 dadurch unterscheiden, daß R¹ für Methylsulfonyl, R⁹ für n-Butyl, R¹⁰ für n-Butylsulfonyl und R¹¹ für Methyl stehen: 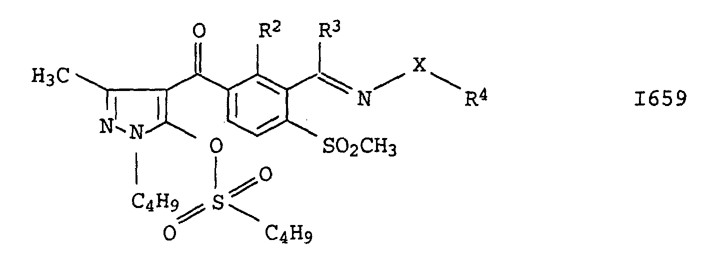
- die Verbindungen I660, insbesondere die Verbindungen I660.001-I660.180, die sich von den entsprechenden Verbindungen I1.001-I1.180 dadurch unterscheiden, daß R¹ für Methylsulfonyl, R⁹ für iso-Butyl, R¹⁰ für n-Butylsulfonyl und R¹¹ für Methyl stehen: 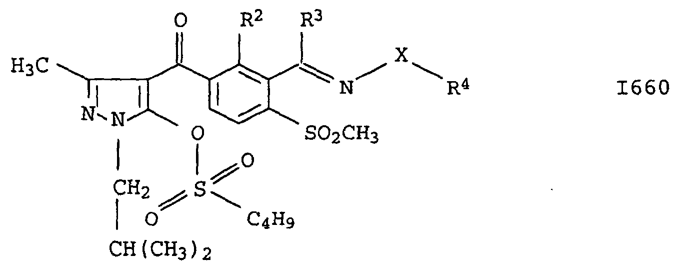
- die Verbindungen I661, insbesondere die Verbindungen I661.001-I661.180, die sich von den entsprechenden Verbindungen I1.001-I1.180 dadurch unterscheiden, daß R¹ für Methylsulfonyl, R¹⁰ für iso-Butylsulfonyl und R¹¹ für Methyl stehen: 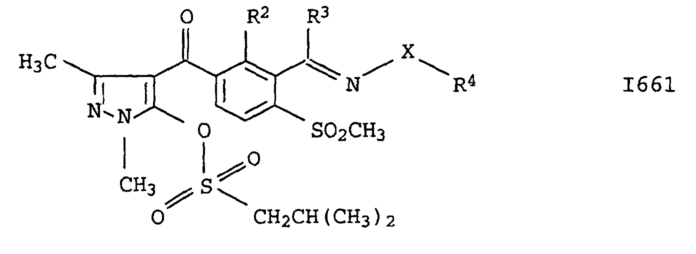
- die Verbindungen I662, insbesondere die Verbindungen I662.001-I662.180, die sich von den entsprechenden Verbindungen I1.001-I1.180 dadurch unterscheiden, daß R¹ für Methylsulfonyl, R⁹ für Ethyl, R¹⁰ für iso-Butylsulfonyl und R¹¹ für Methyl stehen: 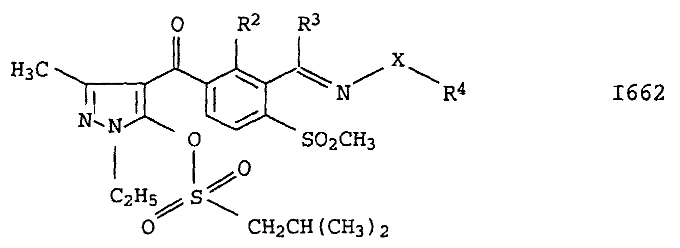
- die Verbindungen I663, insbesondere die Verbindungen I663.001-I663.180, die sich von den entsprechenden Verbindungen I1.001-I1.180 dadurch unterscheiden, daß R¹ für Methylsulfonyl, R⁹ für n-Propyl, R¹⁰ für iso-Butylsulfonyl und R¹¹ für Methyl stehen: 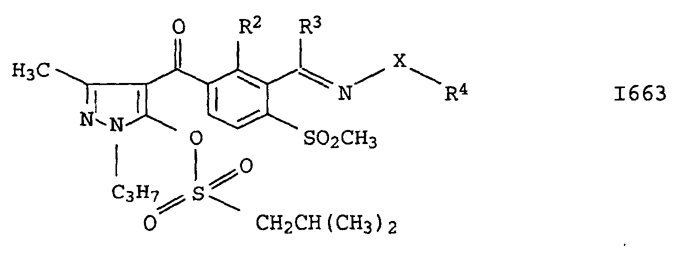
- die Verbindungen I664, insbesondere die Verbindungen I664.001-I664.180, die sich von den entsprechenden Verbindungen I1.001-I1.180 dadurch unterscheiden, daß R¹ für Methylsulfonyl, R⁹ für n-Butyl, R¹⁰ für iso-Butylsulfonyl und R¹¹ für Methyl stehen: 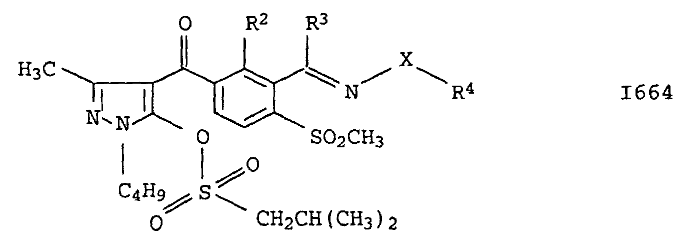
- die Verbindungen I665, insbesondere die Verbindungen I665.001-I665.180, die sich von den entsprechenden Verbindungen I1.001-I1.180 dadurch unterscheiden, daß R¹ für Methylsulfonyl, R⁹ für iso-Butyl, R¹⁰ für iso-Butylsulfonyl und R¹¹ für Methyl stehen: 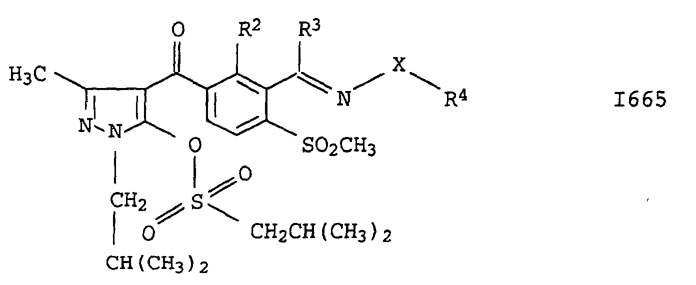
- die Verbindungen I666, insbesondere die Verbindungen I666.001-I666.180, die sich von den entsprechenden Verbindungen I1.001-I1.180 dadurch unterscheiden, daß R¹ für Methylsulfonyl, R¹⁰ für sec-Butylsulfonyl und R¹¹ für Methyl stehen: 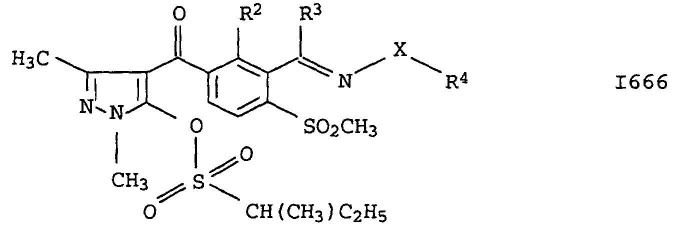
- die Verbindungen I667, insbesondere die Verbindungen I667.001-I667.180, die sich von den entsprechenden Verbindungen I1.001-I1.180 dadurch unterscheiden, daß R¹ für Methylsulfonyl, R⁹ für Ethyl, R¹⁰ für sec-Butylsulfonyl und R¹¹ für Methyl stehen: 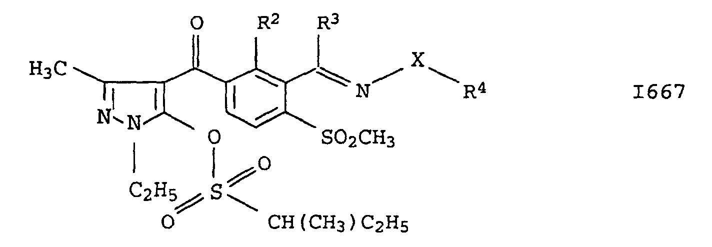
- die Verbindungen I668, insbesondere die Verbindungen I668.001-I668.180, die sich von den entsprechenden Verbindungen I1.001-I1.180 dadurch unterscheiden, daß R¹ für Methylsulfonyl, R⁹ für n-Propyl, R¹⁰ für sec-Butylsulfonyl und R¹¹ für Methyl stehen: 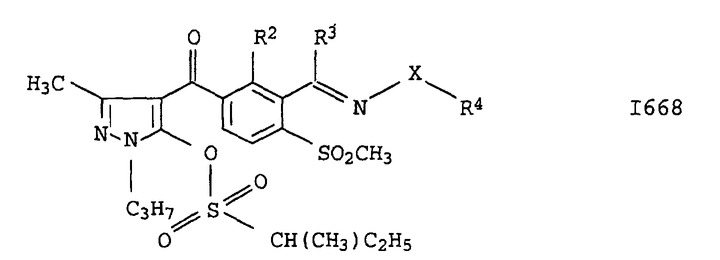
- die Verbindungen I669, insbesondere die Verbindungen I669.001-I669.180, die sich von den entsprechenden Verbindungen I1.001-I1.180 dadurch unterscheiden, daß R¹ für Methylsulfonyl, R⁹ für n-Butyl, R¹⁰ für sec-Butylsulfonyl und R¹¹ für Methyl stehen: 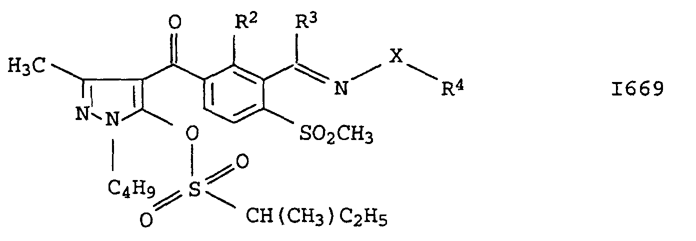
- die Verbindungen I670, insbesondere die Verbindungen I670.001-I670.180, die sich von den entsprechenden Verbindungen I1.001-I1.180 dadurch unterscheiden, daß R¹ für Methylsulfonyl, R⁹ für iso-Butyl, R¹⁰ für sec-Butylsulfonyl und R¹¹ für Methyl stehen: 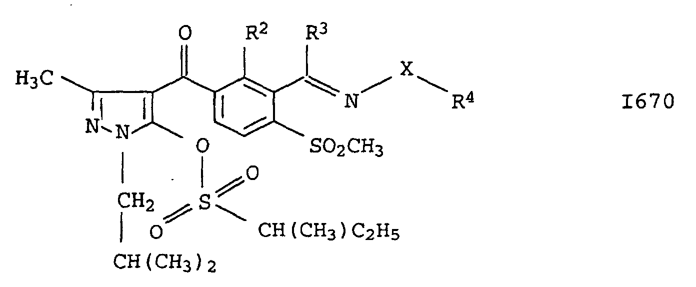
- die Verbindungen I671, insbesondere die Verbindungen I671.001-I671.180, die sich von den entsprechenden Verbindungen I1.001-I1.180 dadurch unterscheiden, daß R¹ für Methylsulfonyl, R¹⁰ für Trifluormethylsulfonyl und R¹¹ für Methyl stehen: 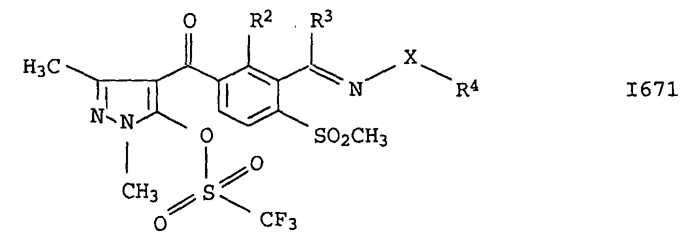
- die Verbindungen I672, insbesondere die Verbindungen I672.001-I672.180, die sich von den entsprechenden Verbindungen I1.001-I1.180 dadurch unterscheiden, daß R¹ für Methylsulfonyl, R⁹ für Ethyl, R¹⁰ für Trifluormethylsulfonyl und R¹¹ für Methyl stehen: 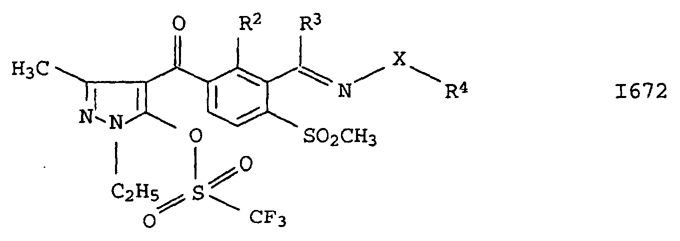
- die Verbindungen I673, insbesondere die Verbindungen I673.001-I673.180, die sich von den entsprechenden Verbindungen I1.001-I1.180 dadurch unterscheiden, daß R¹ für Methylsulfonyl, R⁹ für n-Propyl, R¹⁰ für Trifluormethylsulfonyl und R¹¹ für Methyl stehen: 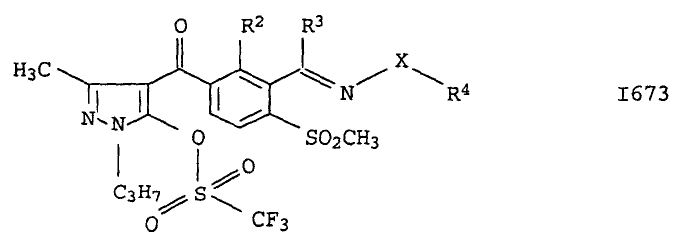
- die Verbindungen I674, insbesondere die Verbindungen I674.001-I674.180, die sich von den entsprechenden Verbindungen I1.001-I1.180 dadurch unterscheiden, daß R¹ für Methylsulfonyl, R⁹ für n-Butyl, R¹⁰ für Trifluormethylsulfonyl und R¹¹ für Methyl stehen: 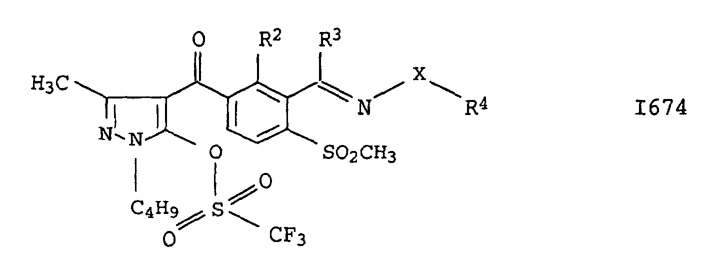
- die Verbindungen I675, insbesondere die Verbindungen I675.001-I675.180, die sich von den entsprechenden Verbindungen I1.001-I1.180 dadurch unterscheiden, daß R¹ für Methylsulfonyl, R⁹ für iso-Butyl, R¹⁰ für Trifluormethylsulfonyl und R¹¹ für Methyl stehen: 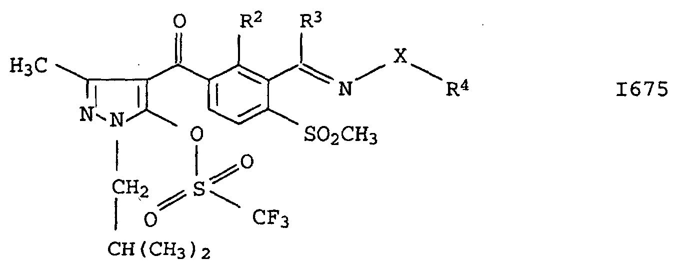
- die Verbindungen I676, insbesondere die Verbindungen I676.001-I676.180, die sich von den entsprechenden Verbindungen I1.001-I1.180 dadurch unterscheiden, daß R¹ für Methylsulfonyl, R⁹ für Methyl, R¹⁰ für Phenylcarbonylmethyl und R¹¹ für Methyl stehen: 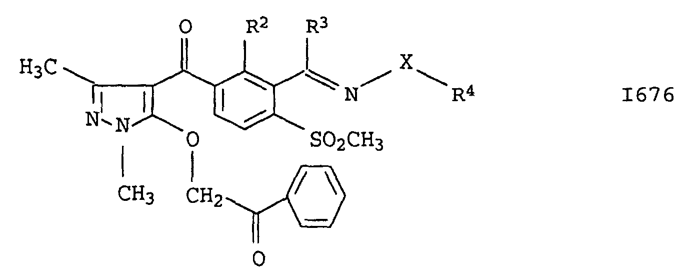
- die Verbindungen I677, insbesondere die Verbindungen I677.001-I677.180, die sich von den entsprechenden Verbindungen I1.001-I1.180 dadurch unterscheiden, daß R¹ für Methylsulfonyl, R⁹ für Ethyl, R¹⁰ für Phenylcarbonylmethyl und R¹¹ für Methyl stehen: 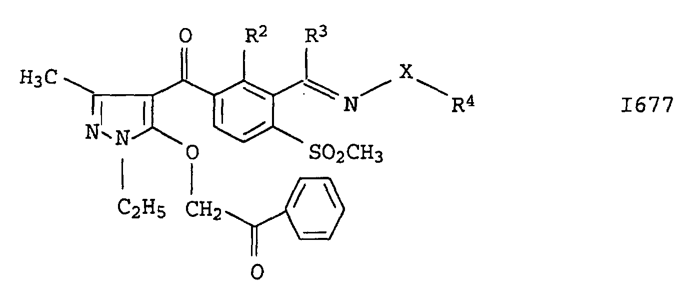
- die Verbindungen I678, insbesondere die Verbindungen I678.001-I678.180, die sich von den entsprechenden Verbindungen I1.001-I1.180 dadurch unterscheiden, daß R¹ für Methylsulfonyl, R⁹ für n-Propyl, R¹⁰ für Phenylcarbonylmethyl und R¹¹ für Methyl stehen: 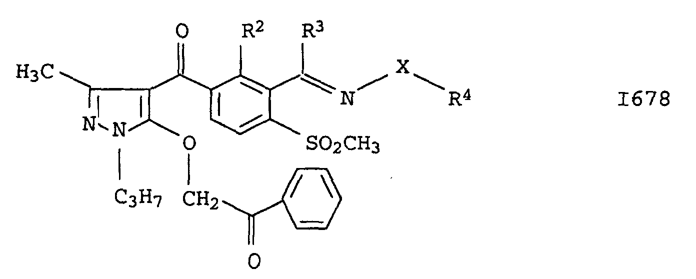
- die Verbindungen I679, insbesondere die Verbindungen I679.001-I679.180, die sich von den entsprechenden Verbindungen I1.001-I1.180 dadurch unterscheiden, daß R¹ für Methylsulfonyl, R⁹ für n-Butyl, R¹⁰ für Phenylcarbonylmethyl und R¹¹ für Methyl stehen: 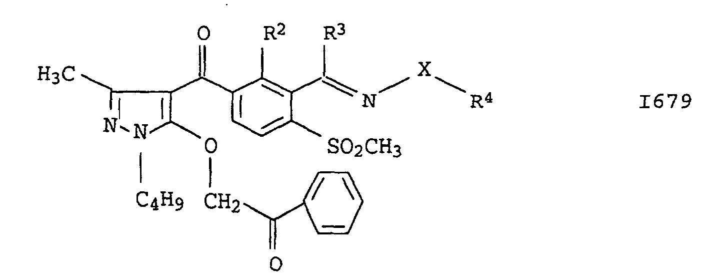
- die Verbindungen I680, insbesondere die Verbindungen I680.001-I680.180, die sich von den entsprechenden Verbindungen I1.001-I1.180 dadurch unterscheiden, daß R¹ für Methylsulfonyl, R⁹ für iso-Butyl, R¹⁰ für Phenylcarbonylmethyl und R¹¹ für Methyl stehen: 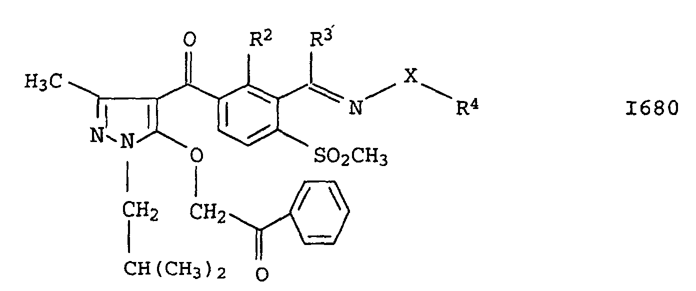
- die Verbindungen I681, insbesondere die Verbindungen I681.001-I681.180, die sich von den entsprechenden Verbindungen I1.001-I1.180 dadurch unterscheiden, daß R¹ für Methylsulfonyl, R¹⁰ für Phenylsulfonyl und R¹¹ für Methyl stehen: 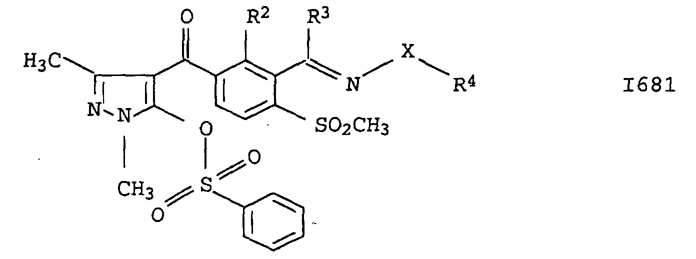
- die Verbindungen I682, insbesondere die Verbindungen I682.001-I682.180, die sich von den entsprechenden Verbindungen I1.001-I1.180 dadurch unterscheiden, daß R¹ für Methylsulfonyl, R⁹ für Ethyl, R¹⁰ für Phenylsulfonyl und R¹¹ für Methyl stehen: 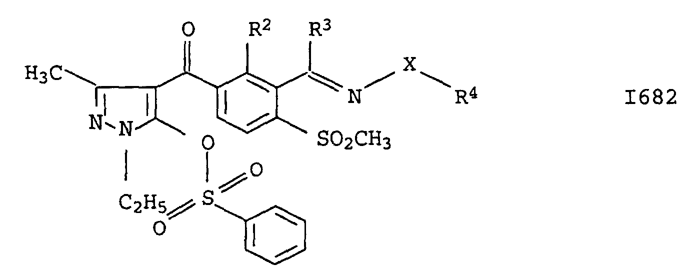
- die Verbindungen I683, insbesondere die Verbindungen I683.001-I683.180, die sich von den entsprechenden Verbindungen I1.001-I1.180 dadurch unterscheiden, daß R¹ für Methylsulfonyl, R⁹ für n-Propyl, R¹⁰ für Phenylsulfonyl und R¹¹ für Methyl stehen: 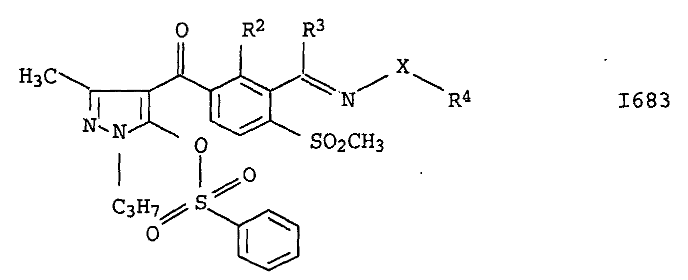
- die Verbindungen I684, insbesondere die Verbindungen I684.001-I684.180, die sich von den entsprechenden Verbindungen I1.001-I1.180 dadurch unterscheiden, daß R¹ für Methylsulfonyl, R⁹ für n-Butyl, R¹⁰ für Phenylsulfonyl und R¹¹ für Methyl stehen: 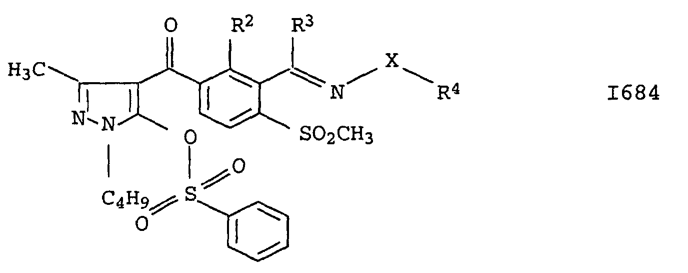
- die Verbindungen I685, insbesondere die Verbindungen I685.001-I685.180, die sich von den entsprechenden Verbindungen I1.001-I1.180 dadurch unterscheiden, daß R¹ für Methylsulfonyl, R⁹ für iso-Butyl, R¹⁰ für Phenylsulfonyl und R¹¹ für Methyl stehen: 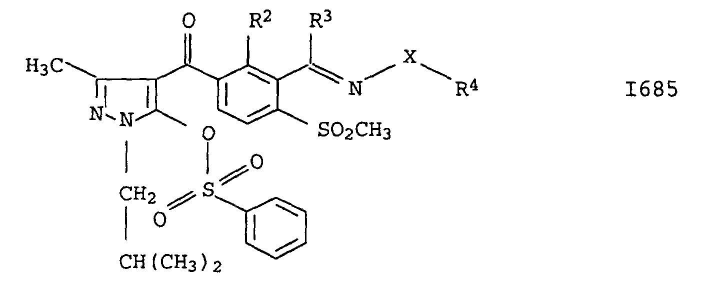
- die Verbindungen I686, insbesondere die Verbindungen I686.001-I686.180, die sich von den entsprechenden Verbindungen I1.001-I1.180 dadurch unterscheiden, daß R¹ für Methylsulfonyl, R¹⁰ für 4-Methylphenylsulfonyl und R¹¹ für Methyl stehen: 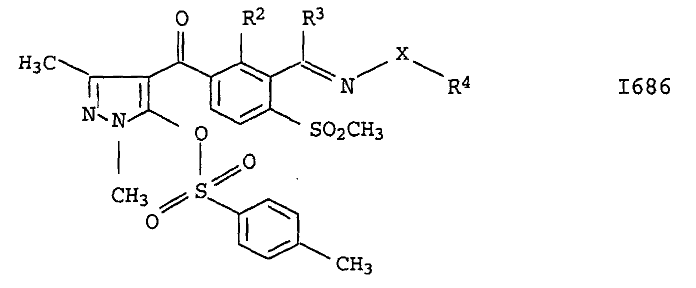
- die Verbindungen I687, insbesondere die Verbindungen I687.001-I687.180, die sich von den entsprechenden Verbindungen I1.001-I1.180 dadurch unterscheiden, daß R¹ für Methylsulfonyl, R⁹ für Ethyl, R¹⁰ für 4-Methylphenylsulfonyl und R¹¹ für Methyl stehen: 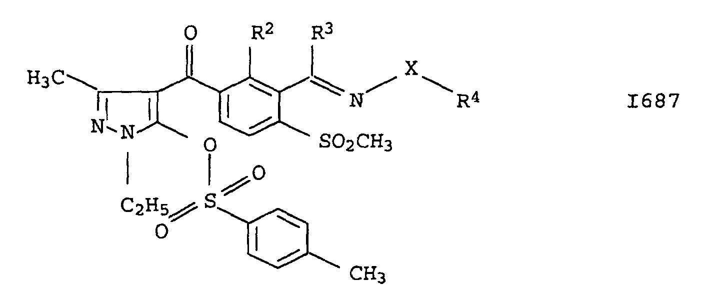
- die Verbindungen I688, insbesondere die Verbindungen I688.001-I688.180, die sich von den entsprechenden Verbindungen I1.001-I1.180 dadurch unterscheiden, daß R¹ für Methylsulfonyl, R⁹ für n-Propyl, R¹⁰ für 4-Methylphenylsulfonyl und R¹¹ für Methyl stehen: 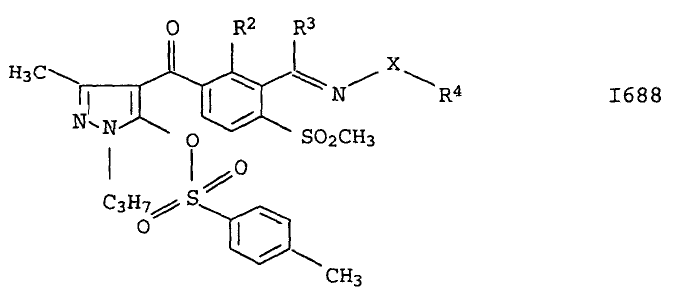
- die Verbindungen I689, insbesondere die Verbindungen I689.001-I689.180, die sich von den entsprechenden Verbindungen I1.001-I1.180 dadurch unterscheiden, daß R¹ für Methylsulfonyl, R⁹ für n-Butyl, R¹⁰ für 4-Methylphenylsulfonyl und R¹¹ für Methyl stehen: 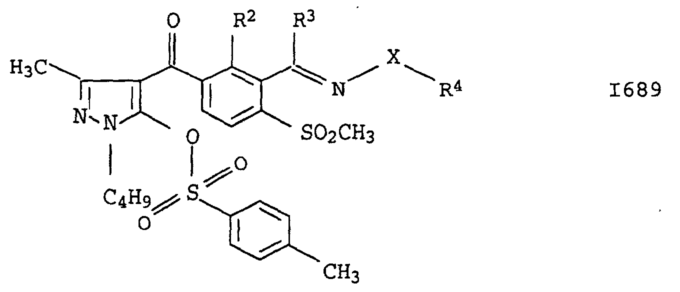
- die Verbindungen I690, insbesondere die Verbindungen I690.001-I690.180, die sich von den entsprechenden Verbindungen I1.001-I1.180 dadurch unterscheiden, daß R¹ für Methylsulfonyl, R⁹ für iso-Butyl, R¹⁰ für 4-Methylphenylsulfonyl und R¹¹ für Methyl stehen:

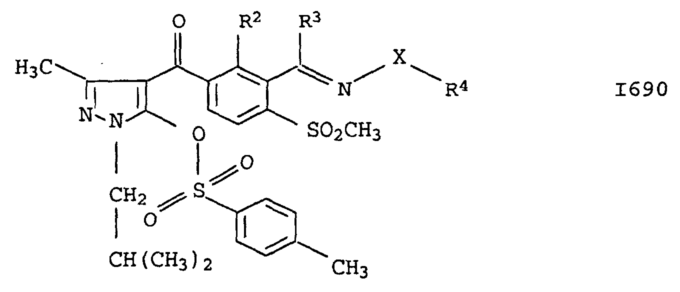

Ganz insbesonderst außerordentlich bevorzugt sind die Verbindungen der Formel I' 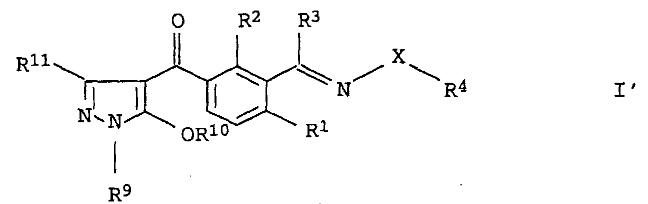 wobei die Variablen folgende Bedeutung haben:
- R¹: Halogen oder C₁-C₄-Alkylsulfonyl;
- R²: Halogen oder C₁-C₄-Alkyl, insbesondere Halogen;
- R³: Wasserstoff oder C₁-C₄-Alkyl, insbesondere Wasserstoff;
- R⁴: C₁-C₆-Alkyl, C₃-C₆-Alkinyl, wobei diese zwei Substituenten partiell oder vollständig halogeniert sein können und/oder eine bis drei der folgenden Gruppen tragen können: Phenyl oder Hetaryl, wobei diese ihrerseits partiell oder vollständig halogeniert sein können;
- X: Sauerstoff;
- R⁹: C₁-C₆-Alkyl;
- R¹⁰: Wasserstoff;
- R¹¹: Wasserstoff.

Die 4-Benzoyl-pyrazole der Formel I sind auf verschiedene Art und Weise erhältlich, beispielsweise nach folgenden Verfahren:

### Verfahren A:

Umsetzung von Pyrazolen der Formel II mit R¹⁰ = H mit einer aktivierten Carbonsäure III oder einer Carbonsäure IIIβ, die vorzugsweise in situ aktiviert wird, zu dem Acylierungsprodukt IV und anschließende Umlagerung.

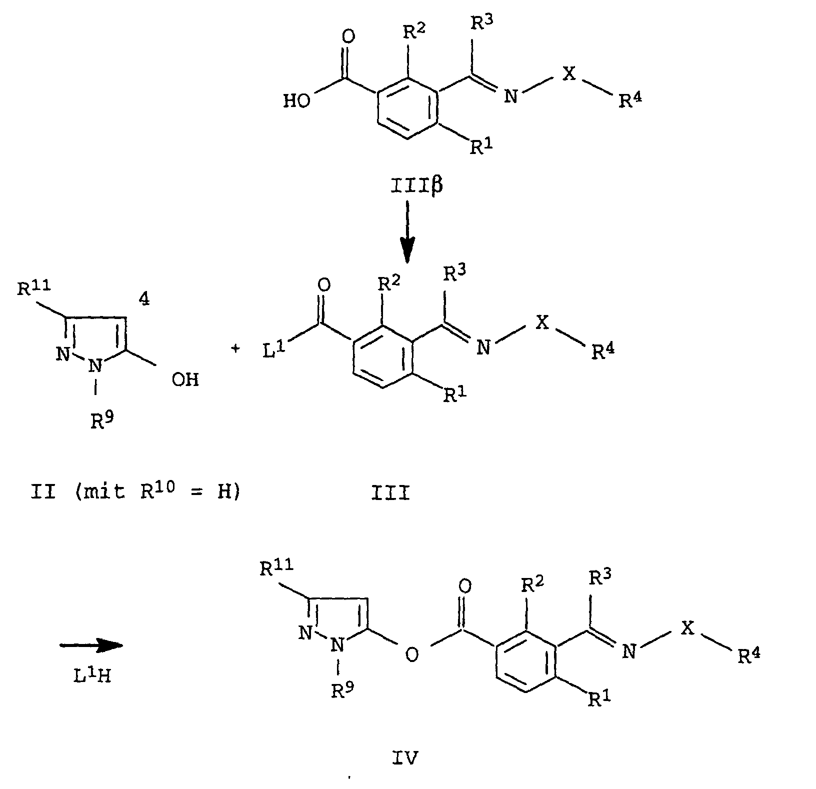

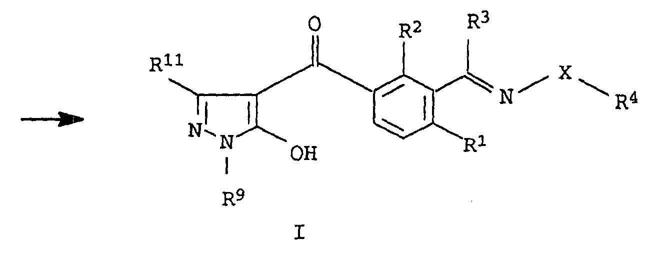

L¹ steht für eine nucleophil verdrängbare Abgangsgruppe, wie Halogen z.B. Brom, Chlor, Hetaryl, z.B. Imidazolyl, Pyridyl, Carboxylat, z.B. Acetat, Trifluoracetat etc.

Die aktivierte Carbonsäure kann direkt eingesetzt werden, wie im Fall der Carbonsäurehalogenide oder in situ erzeugt werden, z.B. mit Dicyclocarbodiimid, Triphenylphosphin/Azodicarbonsäureester, 2-Pyridindisulfit/Triphenylphosphin, Carbonyldiimidazol etc.

Gegebenenfalls kann es von Vorteil sein, die Acylierungsreaktion in Gegenwart einer Base auszuführen. Die Reaktanden und die Hilfsbase werden dabei zweckmäßigerweise in äquimolaren Mengen eingesetzt. Ein geringer Überschuß der Hilfsbase z.B. 1,2 bis 1,5 Moläquivalente, bezogen auf II, kann unter Umständen vorteilhaft sein.

Als Hilfsbasen eignen sich tertiäre Alkylamine, Pyridin oder Alkalimetallcarbonate. Als Lösungsmittel können z.B. chlorierte Kohlenwasserstoffe, wie Methylenchlorid, 1,2-Dichlorethan, aromatische Kohlenwasserstoffe, wie Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester wie Essigsäureethylester oder Gemische hiervon verwendet werden.

Werden Carbonsäurehalogenide als aktivierte Carbonsäurekomponente eingesetzt, so kann es zweckmäßig sein, bei Zugabe dieses Reaktionspartners die Reaktionsmischung auf 0-10°C abzukühlen. Anschließend rührt man bei 20 - 100°C, vorzugsweise bei 25 - 50°C, bis die Umsetzung vollständig ist. Die Aufarbeitung erfolgt in üblicher Weise, z.B. wird das Reaktionsgemisch auf Wasser gegossen, das Wertprodukt extrahiert. Als Lösungsmittel eignen sich hierfür besonders Methylenchlorid, Diethylether und Essigsäureethylester. Nach Trocknen der organischen Phase und Entfernen des Lösungsmittels wird der rohe Enolester der Formel IV vorzugsweise durch Chromatographie gereinigt. Es ist aber auch möglich, den rohen Enolester der Formel IV ohne weitere Reinigung zur Umlagerung einzusetzen.

Die Umlagerung der Enolster der Formel IV zu den Verbindungen der Formel I erfolgt zweckmäßigerweise bei Temperaturen von 20 bis 40°C in einem Lösungsmittel und in Gegenwart einer Base sowie gegebenenfalls in Gegenwart einer Cyanoverbindung.

Als Lösungsmittel können z.B. Acetonitril, Methylenchlorid, 1,2-Dichlorethan, Dioxan, Essigsäureethylester, Toluol oder Gemische hiervon verwendet werden. Bevorzugte Lösungsmittel sind Acetonitril und Dioxan.

Geeignete Basen sind tertiäre Amine wie Triethylamin, Pyridin oder Alkalicarbonate, wie Natriumcarbonat, Kaliumcarbonat, die vorzugsweise in äquimolarer Menge oder bis zu einem vierfachen Überschuß, bezogen auf den Ester, eingesetzt werden. Bevorzugt werden Triethylamin oder Alkalicarbonate verwendet.

Als Cyanoverbindungen kommen anorganische Cyanide, wie Natriumcyanid, Kaliumcyanid und organische Cyanoverbindungen, wie Acetoncyanhydrin, Trimethylsilycyanid in Betracht. Sie werden in einer Menge von 1 bis 50 Molprozent, bezogen auf den Ester, eingesetzt. Vorzugsweise werden Acetoncyanhydrin oder Trimethylsilylcyanid, z.B. in einer Menge von 5 bis 15, vorzugsweise 10 Molprozent, bezogen auf den Ester, eingesetzt.

Besonders bevorzugt werden Alkalicarbonate, wie Kaliumcarbonat, in Acetonitril oder Dioxan eingesetzt.

Die Aufarbeitung kann in an sich bekannter Weise erfolgen. Das Reaktionsgemisch wird z.B. mit verdünnter Mineralsäure, wie 5 %ige Salzsäure oder Schwefelsäure, angesäuert, mit einem organischen Lösungsmittel, z.B. Methylenchlorid, Essigsäureethylester extrahiert. Der organische Extrakt kann mit 5-10%iger Alkalicarbonatlösung, z.B. Natriumcarbonat-, Kaliumcarbonatlösung extrahiert werden. Die wäßrige Phase wird angesäuert und der sich bildende Niederschlag abgesaugt und/oder mit Methylenchlorid oder Essigsäureethylester extrahiert, getrocknet und eingeengt.
(Beispiele für die Darstellung von Estern von Hydroxypyrazolen und für die Umlagerung der Ester sind z.B. in EP-A 282 944 oder US 4 643 757 genannt).

### Verfahren B:

Umsetzung von 4-Benzoyl-pyrazolen der Formel I mit R¹⁰ = H mit einer Verbindung der Formel V (mit R¹⁰ *≠* H) :

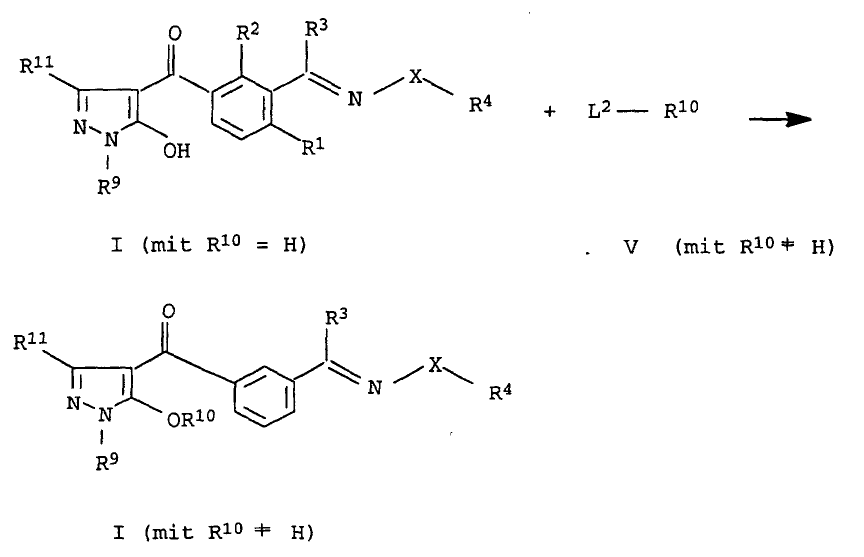

L² steht für eine nucleophil verdrängbare Abgangsgruppe, wie Halogen, z.B. Brom, Chlor, Hetaryl, z.B. Imidazolyl, Pyridyl, Carboxylat, z.B. Acetat, Trifluoracetat, Sulfonat, z.B. Mesylat, Triflat etc.

Die Verbindungen der Formel V können direkt eingesetzt werden, wie z.B. im Fall der Alkylhalogenide, Carbonsäurehalogenide, Sulfonsäurehalogenide, Carbonsäureanhydride und Sulfonsäureanhydride oder in situ erzeugt werden, z.B. aktivierte Carbonsäuren (mittels Carbonsäure und Dicyclohexylcarbodiimid, Carbonyldiimidazol etc.).

Die Ausgangsverbindungen werden in der Regel im äquimolaren Verhältnis eingesetzt. Es kann aber auch von Vorteil sein, die eine oder andere Komponente im Überschuß einzusetzen.

Gegebenenfalls kann es von Vorteil sein, die Umsetzung in Gegenwart einer Base durchzuführen. Die Reaktanden und die Hilfsbase werden dabei zweckmäßigerweise in äquimolaren Mengen eingesetzt. Ein Überschuß der Hilfsbase z.B. 1,5 bis 3 Moläquivalente, bezogen auf II, kann unter Umständen vorteilhaft sein.

Als Hilfsbasen eignen sich tertiäre Alkylamine, wie Triethylamin, Pyridin, Alkalimetallcarbonate, z.B. Natriumcarbonat, Kaliumcarbonat und Alkalimetallhydride, z.B. Natriumhydrid. Bevorzugt verwendet werden Triethylamin, Pyridin und Kaliumcarbonat.

Als Lösungsmittel kommen z.B. chlorierte Kohlenwasserstoffe, wie Methylenchlorid, 1,2-Dichlorethan, aromatische Kohlenwasserstoffe, z.B. Toluol, Xylol, Chlorbenzol, Ether, wie Diethylether, Methyl-tert.-butylether, Tetrahydrofuran, Dioxan, polare aprotische Lösungsmittel, wie Acetonitril, Dimethylformamid, Dimethylsulfoxid oder Ester, wie Essigsäureethylester, oder Gemische hiervon in Betracht.

In der Regel liegt die Reaktionstemperatur im Bereich von 0°C bis zur Höhe des Siedepunktes des Reaktionsgemisches.

Die Aufarbeitung kann in an sich bekannter Weise zum Produkt hin erfolgen.

Die als Ausgangsmaterialien verwendeten Pyrazole der Formel II (mit R¹⁰ = H) sind bekannt oder können nach an sich bekannten Verfahren hergestellt werden (z.B. EP-A 240 001, J. Prakt. Chem. 315, 383 (1973)).

Die Benzoesäurederivate der Formel III sind neu, 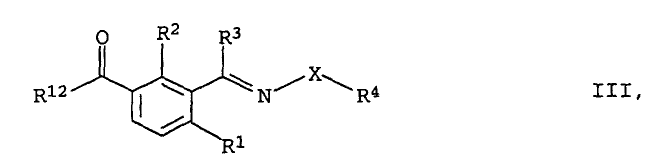 wobei die Variablen folgende Bedeutung haben:
- R¹, R²: Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁶;
- R³: Wasserstoff, Cyano, C₁-C₆-Alkyl, C₁-C₆-Halogenalkyl, -OR⁶, -SR⁶ oder -NR⁶R⁸;
- R⁴: Wasserstoff, C₁-C₆-Alkyl, C₃-C₆-Cycloalkyl, C₃-C₆-Alkenyl oder C₃-C₆-Alkinyl, wobei die vier letztgenannten Substituenten
partiell oder vollständig halogeniert sein können und/oder eine bis drei der folgenden Gruppen tragen können: Hydroxy, Mercapto, Amino, Cyano, R⁸, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy und Hetaryloxy, wobei die acht letztgenannten Reste ihrerseits partiell oder vollständig halogeniert sein können und/oder einen bis drei Reste aus folgender Gruppe tragen können: Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
und wobei der voranstehende Heterocyclyl- bzw. Heterocyclyloxy-Rest ein bis drei Heteroatome ausgewählt aus der Gruppe Sauerstoff, Stickstoff oder Schwefel enthält, 3-7 gliedrig, mono- oder polycyclisch ist und gesättigt oder partiell ungesättigt ist;
und wobei der voranstehend genannte Hetaryl- bzw. Hetaryloxy-Rest neben Kohlenstoffringgliedern zusätzlich ein bis vier Stickstoffatome oder ein- bis drei Stickstoffatome und ein Sauerstoff- oder ein Schwefelatom, oder ein Sauerstoff- oder ein Schwefelatom enthält und aromatisch ist;
- X: Sauerstoff oder NR⁷;
- n: 0, 1 oder 2;
- R⁵: Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁶: C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
- R⁷: Wasserstoff oder C₁-C₆-Alkyl;
- R⁸: C₁-C₆-Alkyl, C₁-C₆-Halogenalkyl, C₃-C₆-Alkenyl oder C₃-C₆-Alkinyl;
- R¹²: Hydroxy oder ein abhydrolysierbarer Rest.

Beispiele für abhydrolysierbare Reste sind Alkoxy, Phenoxy-, Alkylthio-, Phenylthio-Reste, die substituiert sein können, Halogenide, Hetarylreste, die über Stickstoff gebunden sind, Amino-, Imino-Reste, die substituiert sein können etc.

Bevorzugt sind Benzoesäurehalogenide III mit L = Halogen (≙ III mit R¹² = Halogen), 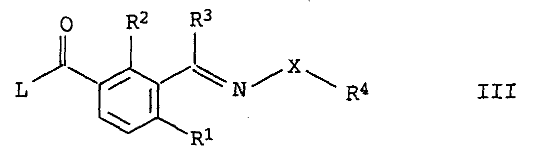 wobei die Variablen R¹ bis R⁴ und X die unter Formel III genannte Bedeutung haben und
- L: Halogen, insbesondere Chlor oder Brom, bedeuten.

Ebenso bevorzugt sind Benzoesäuren der Formel IIIβ (≙ III mit R¹² = Hydroxy), 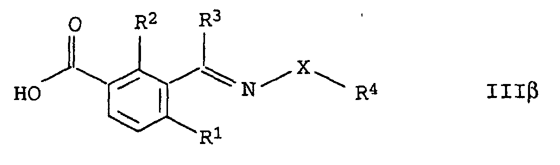 wobei die Variablen R¹ bis R⁴ und X die unter Formel III genannte Bedeutung haben.

Ebenso bevorzugt sind Benzoesäureester der Formel IIIγ (≙ III mit R¹² = C₁-C₆-Alkoxy), 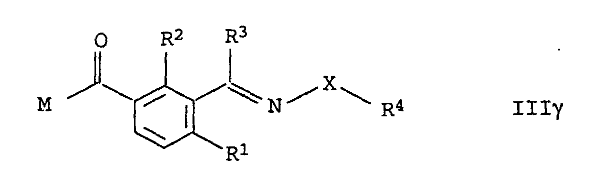 wobei die Variablen R¹ bis R⁴ und X die unter Formel III genannte Bedeutung haben und
- M: C₁-C₆-Alkoxy;
bedeutet.

Im Hinblick auf die bevorzugten Verbindungen der Formel III gelten für die Reste R¹ bis R⁴ und X die unter den Verbindungen der Formel I gemachten Ausführungen.

Die Verbindungen der Formel III (mit L¹ = Halogen) können in Analogie zu literaturbekannten Methoden (vgl. L.G. Fieser, M. Fieser "Reagents for Organic Synthesis", Bd. I, S. 767-769 (1967)) durch Umsetzung von Benzoesäuren der Formel IIIβ mit Halogenierungsreagentien wie Thionylchlorid, Thionylbromid, Phosgen, Diphosgen, Triphosgen, Oxalylchlorid, Oxalylbromid dargestellt werden.

Die Benzoesäuren der Formel IIIβ können u.a. durch Verseifung der Benzoesäureester der Formel IIIγ (mit M = C₁-C₆-Alkoxy) erhalten werden.

Die Benzoesäureester der Formel IIIγ sind auf verschiedene Art und Weise erhältlich, beispielsweise nach folgenden Verfahren:

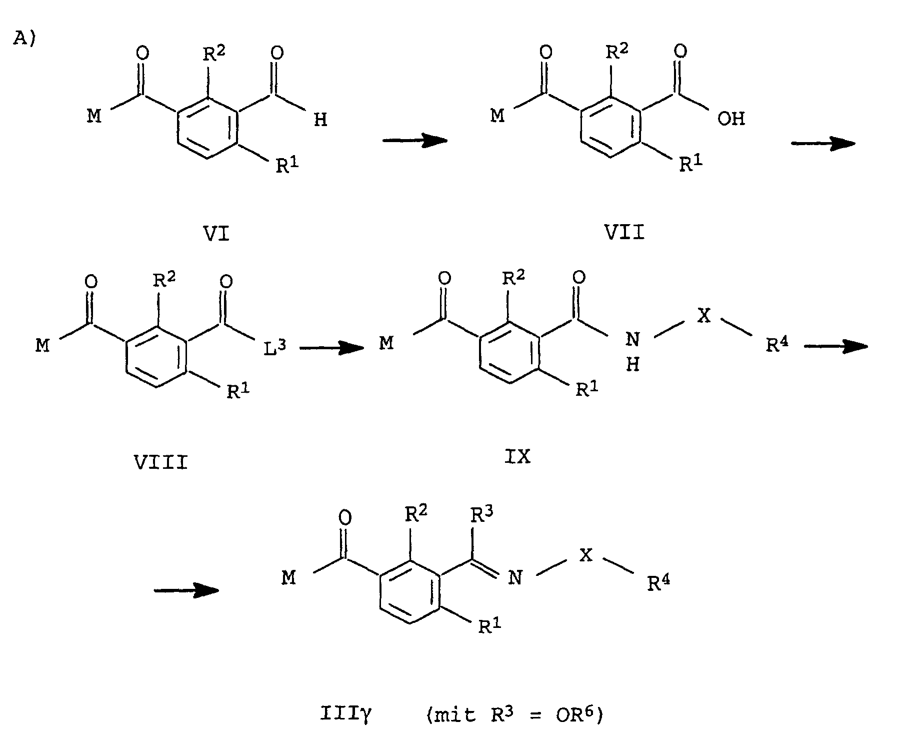

Durch Oxidation von Aldehyden der Formel VI können auf an sich bekannte Art und Weise Isophthalsäurederivate der Formel VII erhalten werden (J. March, "Advanced Organic Chemistry", 3. Auflage, S. 629 ff, Wiley-Interscience Publication, 1985).

In Analogie zu literaturbekannten Verfahren können die Verbindungen der Formel VII zunächst in die entsprechenden aktivierten Carbonsäuren VIII, wobei L³ für eine nucleophil verdrängbare Abgangsgruppe, wie Halogen, z.B. Brom, Chlor, Hetaroyl, z.B. Imidazolyl, Pyridyl, Carboxylat, z.B. Acetat, Trifluoracetat etc. steht und anschließend in die entsprechenden Hydroxamsäure- bzw. Carbonsäurehydrazidderivate der Formel IX übergeführt werden (Australian J. Chem. (1969), 22, 1731-1735; ibid (1969), 22, 161-173; J. Org. Chem. (1974), 27, 1341-1349).

Die Alkylierung von Verbindungen der Formel IX führt zu Verbindungen der Formel IIIγ (mit R³ = OR⁶) auf an sich bekannte Art und Weise (EP-A 463 989; Synthesis (1983), 220-222; US 4 931 088; J. Org. Chem. (1971), 31, 284-294; J. Chem. Soc. Perk. II (1977), 1080-1084).

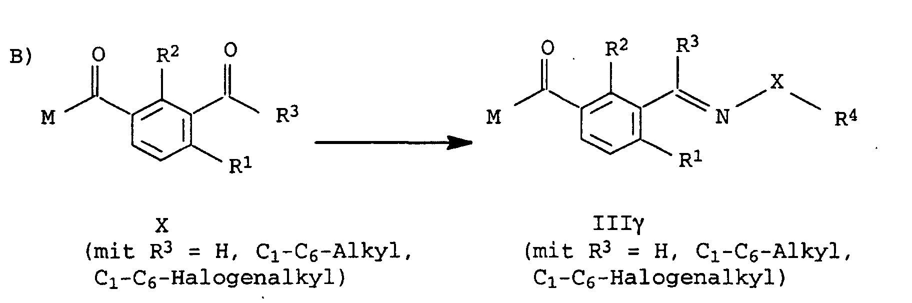

Durch Umsetzung von Aldehyden/Ketonen der Formel X mit "Alkoxaminen bzw. Alkylhydrazinen" erhält man Verbindungen der Formel IIIγ auf an sich bekannte Weise. In Analogie zu literaturbekannten Verfahren ist es möglich, Aldehyde/Ketone der Formel X mit Hydroxylamin bzw. Hydrazin umzusetzen und anschließend zu alkylieren (J. March, "Advanced Organic Chemistry", 3. Auflage, S. 359, S. 805-806, Wiley-Interscience Publication, 1985).

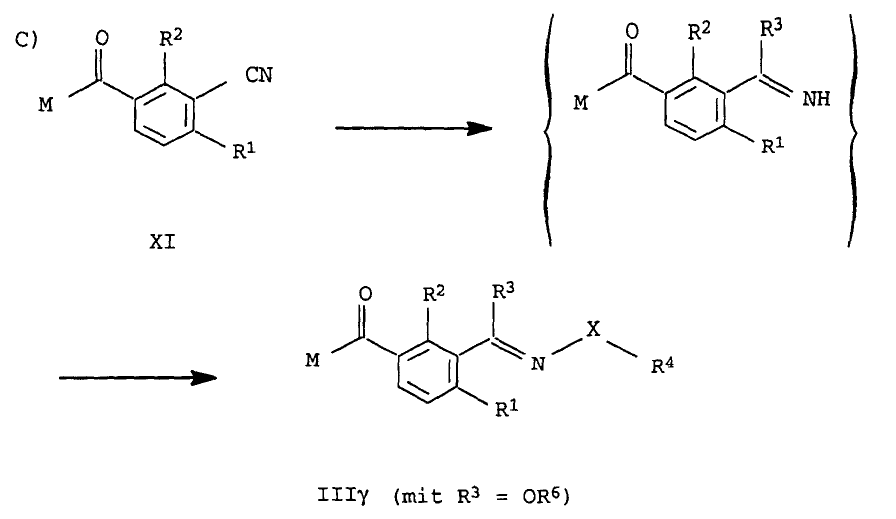

Auf an sich bekannte Weise können Nitrile der Formel XI durch Alkoholyse (R⁶OH) in Iminoester übergeführt werden, die in einem weiteren Schritt mit Hydroxylaminen bzw. Hydrazinen zu Verbindungen der Formel IIIγ umgesetzt werden können (J. March, "Advanced Organic Chemistry", 3. Auflage, S. 792-793, Wiley-Interscience Publication, 1985; US 4 965 390).

Die Nitrile der Formel XI können in Analogie zu literaturbekannten Verfahren aus den entsprechenden Aldehyden VI dargestellt werden (J. March, "Advanced Organic Chemistry", 3. Auflage, S. 806-807, Wiley-Interscience Publication, 1985). Ebenso ist es möglich, Nitrile der Formel XI aus Anilinen der Formel XII mittels Sandmeyer-Reaktion zu erhalten oder durch Rosemund/von Braun-Reaktion aus Arylhalogeniden der Formel XIII mit Metallcyaniden, insbesondere CuCN (J. March, "Advanced Organic Chemistry". 3. Auflage, S. 594, S. 648, Wiley-Interscience Publication, 1985).

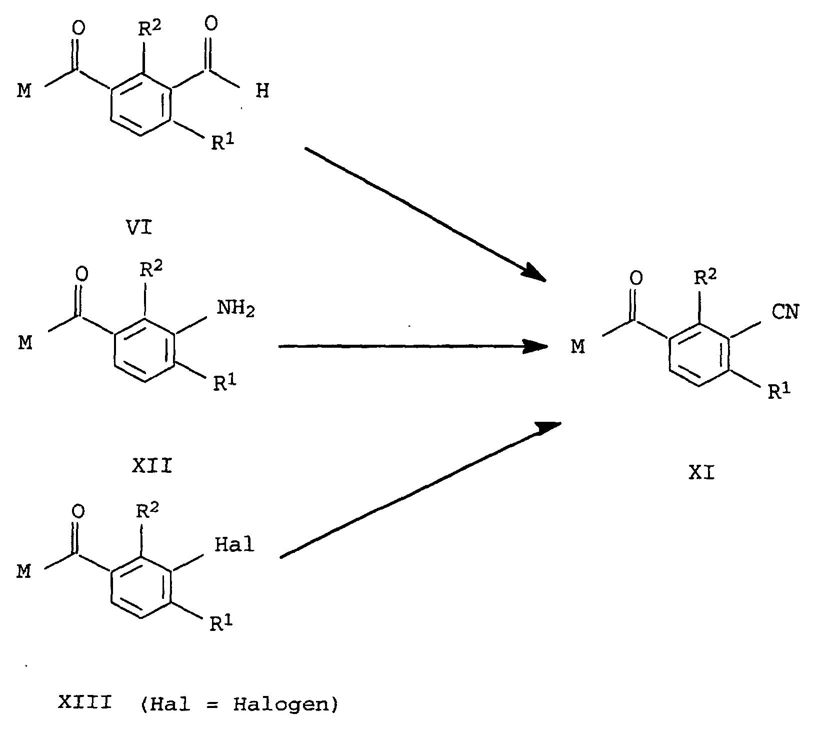

Die Aldehyde der Formel VI können in Analogie zu literaturbekannten Verfahren aus entsprechenden Toluolen der Formel XIV dargestellt werden, indem man sie in das ω-Halogentoluol XV überführt und anschließend oxidiert (vgl. Synth. Commun. 22, 1967-1971 (1992)).

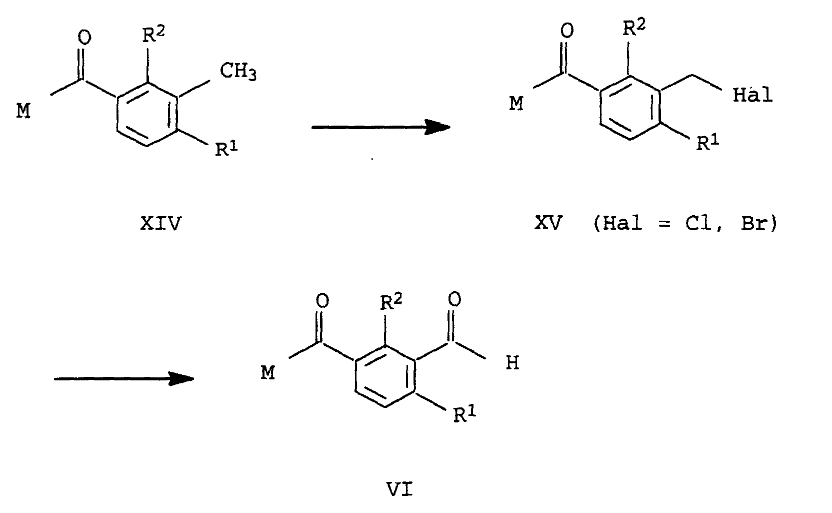

### Herstellungsbeispiele

### 4-(2,4-Dichlor-3-ethoxyiminomethyl-benzoyl)-2-ethyl-3-hyroxypyrazol (Verbindung 2.03)

Eine Lösung von 1,50 g (0,006 mol) 2,4-Dichlor-3-ethoxyiminomethyl-benzoesäure, 0,61 g (0,006 mol) 2-Ethyl-3-hydroxypyrazol und 1,13 g (0,006 mol) Dicyclohexylcarbodiimid in 50 ml trockenem Acetonitril wurde 12 h bei Raumtemperatur gerührt. Anschließend wurde der Niederschlag abgesaugt und das Filtrat in Wasser aufgenommen. Diese wäßrige Lösung wurde mit Essigsäureethylester extrahiert. Die vereinigten organischen Phasen wurden getrocknet und am Vakuum eingeengt. Der Rückstand wurde in 50 ml trockenem Acetonitril aufgenommen, mit 1,20 g (0,0087 mol) fein gepulvertem Kaliumcarbonat versetzt und 3,5 h unter Rückfluß erhitzt. Nach Abkühlen wurde das Lösungsmittel am Vakuum entfernt und der Rückstand in Wasser aufgenommen. Die wäßrige Phase wurde mit 10 %iger Salzsäurelösung auf pH = 1-2 eingestellt und mit Essigsäureethylester extrahiert. Die vereinigten organischen Phasen wurden anschließend mit Wasser neutral gewaschen, getrocknet und das Lösungsmittel am Vakuum abdestilliert. Man erhielt 0,70 g 4-(2,4-Dichlor-3-ethoxyiminomethyl-benzoyl)-2-ethyl-3-hydroxypyrazol, der zur Reinigung aus Essigsäureethylester mit n-Hexan ausgefällt wurde. (Fp.: 97-98°C)

In der nachfolgenden Tabelle 2 sind neben den voranstehend beschriebenen Benzoylderivaten der Formel I noch weitere aufgeführt, die in analoger Weise hergestellt wurden oder herstellbar sind:

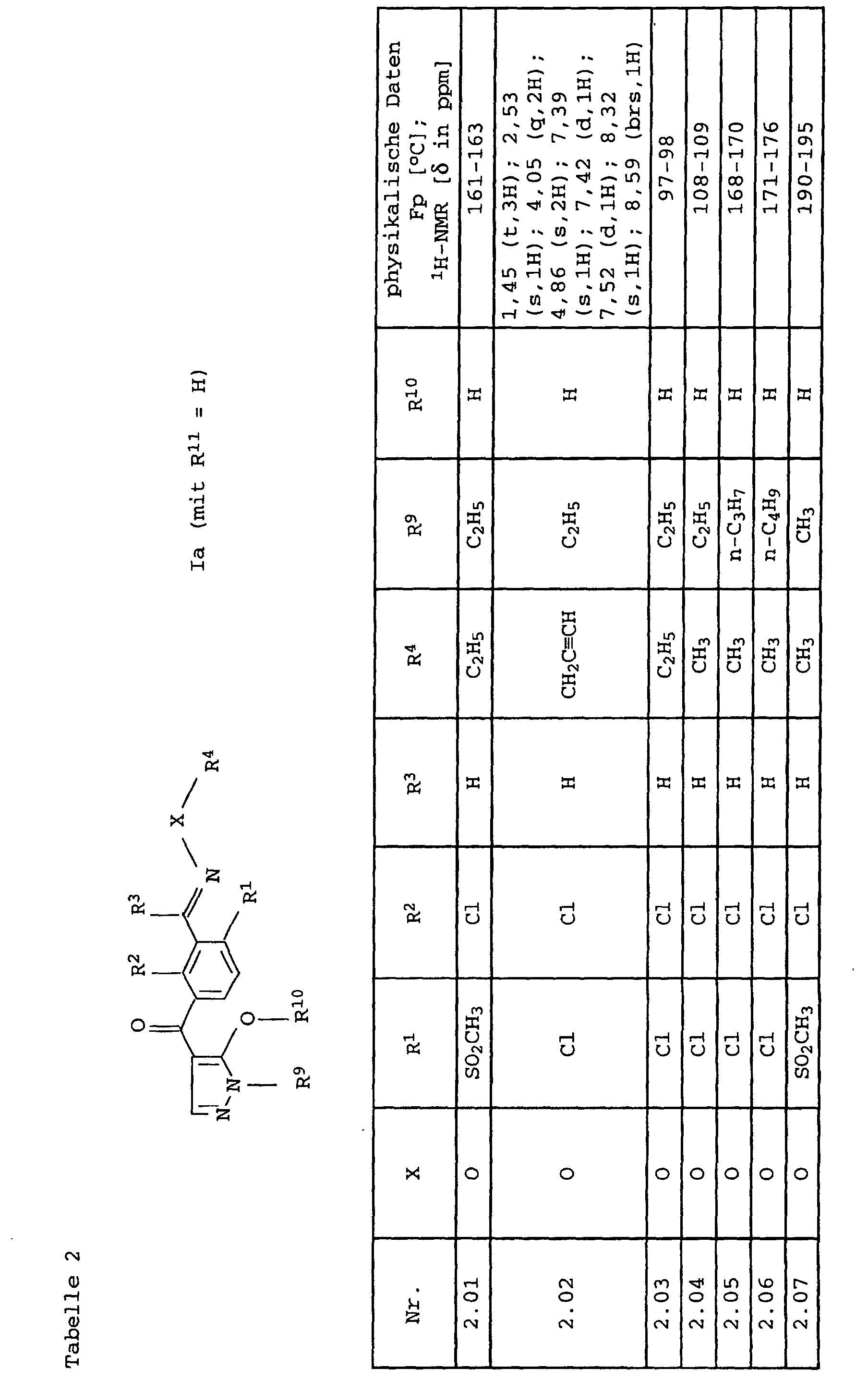

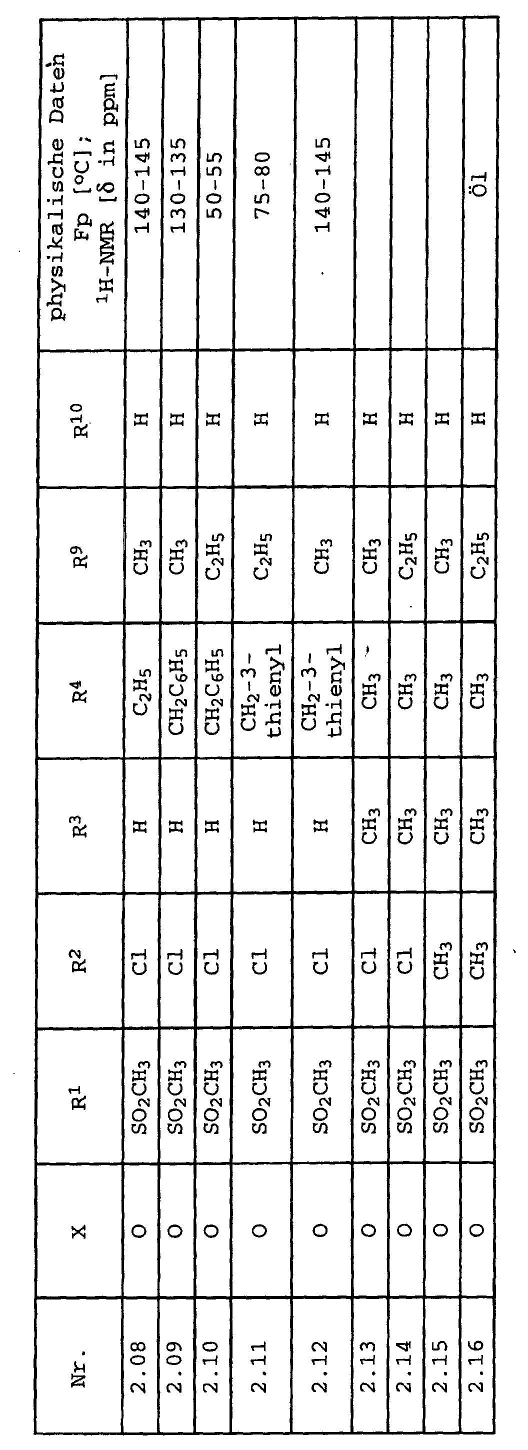

Nachfolgend sind die Synthesen einiger Ausgangsstoffe aufgeführt:
2-Chlor-3-ethoxyiminomethyl-4-methylsulfonyl-benzoesäure (Verbindung 3.04)
   Stufe a) 2-Chlor-3-methyl-4-methylthio-acetophenon
      Zu einer Suspension von 286 g (2,14 mol) Aluminiumtrichlorid in 420 ml 1,2-Dichlorethan wurde bei 15-20°C eine Lösung von 157 g (2 mol) Acetylchlorid in 420 ml 1,2-Dichlorethan getropft. Anschließend wurde eine Lösung von 346 g (2 mol) 2-Chlor-6-methylthio-toluol in 1 l 1,2-Dichlorethan zugetropft. Nach 12 Stunden Rühren wurde das Reaktionsgemisch in eine Mischung aus 3 l Eis und 1 l konz. HCl gegossen. Es wurde mit Methylenchlorid extrahiert, die organische Phase mit Wasser gewaschen, mit Natriumsulfat getrocknet und eingeengt. Der Rückstand wurde im Vakuum destilliert. Man erhielt 256 g (60 % d.Th.) 2-Chlor-3-methyl-4-methylthio-acetophenon.
      (Fp.: 46°C)
   Stufe b) 2-Chlor-3-methyl-4-methylsulfonyl-acetophenon
      163,0 g (0,76 mol) 2-Chlor-3-methyl-4-methylthio-acetophenon wurden in 1,5 l Eisessig gelöst, mit 18,6 g Natriumwolframat versetzt und unter Kühlung 173,3 g 30 %ige Wasserstoffperoxidlösung zugetropft. Es wurde 2 Tage nachgerührt und anschließend mit Wasser verdünnt. Der ausgefallene Feststoff wurde abgesaugt, mit Wasser gewaschen und getrocknet. Man erhielt 164,0 g (88 % d.Th.) 2-Chlor-3-methyl-4-methylsulfonyl-acetophenon.
      (Fp.: 110-111°C)
   Stufe c) 2-Chlor-3-methyl-4-methylsulfonyl-benzoesäure
      82 g (0,33 mol) 2-Chlor-3-methyl-4-methylsulfonyl-acetophenon wurden in 700 ml Dioxan gelöst und bei Raumtemperatur mit 1 l einer 12,5 %igen Natriumhypochloritlösung versetzt. Anschließend wurde 1 Stunde bei 80°C nachgerührt. Nach dem Abkühlen bildeten sich zwei Phasen, von denen die untere mit Wasser verdünnt und schwach angesäuert wurde. Der ausgefallene Feststoff wurde mit Wasser nachgewaschen und getrocknet. Man erhielt 60 g (73 % d.Th) 2-Chlor-3-methyl-4-methylsulfonyl-benzoesäure.
      (Fp.: 230-231°C)
   Stufe d) 2-Chlor-3-methyl-4-methylsulfonyl-benzoesäuremethylester
      100 g (0,4 mol) 2-Chlor-3-methyl-4-methylsulfonyl-benzoesäure wurden in 1 l Methanol gelöst und bei Rückflußtemperatur 5 Stunden mit Chlorwasserstoff begast.
      Anschließend wurde eingeengt. Man erhielt 88,5 g (84 % d.Th.) 2-Chlor-3-methyl-4-methylsulfonyl-benzoesäuremethylester.
      (Fp.: 107-108°C)
   Stufe e) 3-Brommethyl-2-chlor-4-methylsulfonyl-benzoesäuremethylester
      82 g (0,31 mol) 2-Chlor-3-methyl-4-methylsulfonyl-benzoesäuremethylester werden in 2 l Tetrachlormethan gelöst und unter Belichtung portionsweise mit 56 g (0,31 mol) N-Bromsuccinimid versetzt. Das Reaktionsgemisch wurde filtriert, das Filtrat eingeengt und der Rückstand in 200 ml Methyl-tert.-butylether aufgenommen. Die Lösung wurde mit Petrolether versetzt, der ausgefallene Feststoff abgesaugt und getrocknet. Man erhielt 74,5 g (70 % d.Th) 3-Brommethyl-2-chlor-4-methylsulfonyl-benzoesäuremethylester.
      (Fp.: 74-75°C)
   Stufe f) 2-Chlor-3-formyl-4-methylsulfonyl-benzoesäuremethylester
      Eine Lösung von 41,0 g (0,12 mol) 3-Brommethyl-2-chlor-4-methylsulfonyl-benzoesäuremethylester in 250 ml Acetonitril wurde mit 42,1 g (0,36 mol) N-Methylmorpholin-N-oxid versetzt. Der Ansatz wurde 12 Stunden bei Raumtemperatur gerührt, anschließend eingeengt und der Rückstand in Essigsäureethylester aufgenommen. Die Lösung wurde mit Wasser extrahiert, mit Natriumsulfat getrocknet und eingeengt. Man erhielt 31,2 g (94 % d.Th.)
      2-Chlor-3-formyl-4-methylsulfonyl-benzoesäuremethylester
      (Fp.: 98-105°C)
   Stufe g) 2-Chlor-3-formyl-4-methylsulfonyl-benzoesäure
      Zu einer Lösung von 9,60 g (0,072 mol) Lithiumiodid und 70 ml trockenem Pyridin wurde bei Rückflußtemperatur langsam eine Lösung von 5,00 g 2-Chlor-3-formyl-4-methylsulfonyl-benzoesäuremethylester getropft. Nach 2 Stunden Rühren unter Rückfluß, kühlte man das Reaktionsgemisch ab und entfernte das Lösungsmittel am Vakuum. Der Rückstand wurde anschließend in Wasser aufgenommen und mit verdünnter Salzsäure auf pH 1-2 eingestellt. Nach Extraktion der wäßrigen Phase mit Essigsäureethylester wurden die gesammelten organischen Phasen mit Wasser neutralgewaschen, getrocknet und eingeengt. Man erhielt 4,00 g 2-Chlor-3-formyl-4-methylsulfonyl-benzoesäure (85 % Ausbeute).
      (¹H-NMR (d⁶-DMSO, δ in ppm): 3,41 (s, 3H); 8,05 (d, 1H); 8,11 (d, 1H); 10,49 (s, 1H); 14,21 (s, br., 1H).)
   Stufe h) 2-Chlor-3-ethoxyiminomethyl-4-methylsulfonyl-benzoesäure
      1,63 g (0,017 mol) Ethoxyaminhydrochlorid und 1,15 g (0,0085 mol) fein gepulvertes Kaliumcarbonat wurden in 60 ml trockenem Methanol 1 Stunde gerührt. Anschließend gab man 4,00 g (0,015 mol) 2-Chlor-3-formyl-4-methylsulfonyl-benzoesäure in 40 ml Methanol zu. Nach 12 Stunden Rühren bei Raumtemperatur wurde das Lösungsmittel entfernt, der Rückstand in Essigsäureethylester aufgenommen und die organische Phase viermal mit Wasser gewaschen. Nach Trocknen und Abdestillieren des Lösungsmittels erhielt man 3,60 g 2-Chlor-3-ethoxyiminomethyl-4-methylsulfonyl-benzoesäure (78 % Ausbeute).
      (Fp.: 155-160°C)
alternativ:
   Stufe g') 2-Chlor-3-ethoxyiminomethyl-4-methylsulfonyl-benzoesäuremethylester (Verbindung 3.01)
      1,90 g (0,0195 mol) Ethoxyaminhydrochlorid und 1,35 g (0,0097 mol) fein gepulvertes Kaliumcarbonat wurden in 60 ml trockenem Methanol 1 Stunde bei Raumtemperatur gerührt und anschließend 4,90 g (0,0177 mol) 2-Chlor-3-formyl-4-methylsulfonyl-benzoesäuremethylester hinzugegeben. Nach 8 Stunden Rühren bei Raumtemperatur wurde das Lösungsmittel entfernt, der Rückstand in Essigsäureethylester aufgenommen, die organische Phase mit Wasser neutralgewaschen, getrocknet und am Vakuum eingeengt. Man erhielt 5,00 g 2-Chlor-3-ethoxyiminomethyl-4-methylsulfonyl-benzoesäuremethylester. (Ausbeute 88 %).
      (¹H-NMR (CDCl₃, δ in ppm): 1,34 (t, 3H); 3,29 (s, 3H); 3,98 (s, 3H); 4,26 (q, 2H); 7,91 (d, 1H); 8,10 (d, 1H); 8,38 (s, 1H).)
   Stufe h') 2-Chlor-3-ethoxyiminomethyl-4-methylsulfonyl-benzoesäure
      Zu 7,29 g (0,055 mol) Lithiumiodid in 50 ml trockenem Pyridin wurde langsam eine Lösung von 4,37 g (0,0137 mol) 2-Chlor-3-ethoxyiminomethyl-4-methylsulfonyl-benzoesäuremethylester getropft. Nach 2 Stunden Rühren unter Rückfluß, kühlte man ab und entfernte das Lösungsmittel am Vakuum. Der Rückstand wurde in Wasser aufgenommen und mit verdünnter Salzsäure auf pH = 1-2 gestellt. Nach Extraktion der wäßrigen Phase mit Essigsäureethylester wurden die vereinigten organischen Phasen mit Wasser gewaschen, getrocknet und am Vakuum eingeengt. Man erhielt 3,70 g 2-Chlor-3-ethoxyiminomethyl-4-methylsulfonyl-benzoesäure. (Ausbeute 89 %).
      (Fp.: 155-160°C)
2-Chlor-3-chlorcarbonyl-4-methylsulfonyl-benzoesäuremethylester
   Stufe a) 2-Chlor-3-hydroxycarbonyl-4-methylsulfonyl-benzoesäuremethylester
      Zu einer Lösung von 115,3 g (0,42 mol) 2-Chlor-3-formyl-4-methylsulfonyl-benzoesäuremethylester und 2000 ml Acetonitril wurden bei 5°C nacheinander 13,8 g (0,11 mol) Natriumhydrogenphosphatmonohydrat in 170 ml Wasser, 49,3 g (0,43 mol) 30 %ige Wasserstoffperoxidlösung und 66,2 g (0,59 mol) 80 %ige wäßrige Natriumchloritlösung gegeben. Die Reaktionslösung wurde anschließend 1 Stunde bei 5°C und 12 Stunden bei Raumtemperatur gerührt. Dann wurde mit 10 %iger Salzsäure auf pH = 1 eingestellt und 1500 ml wäßrige 40 %ige Natriumhydrogensulfit-Lösung zugegeben. Nach 1 Stunde Rühren bei Raumtemperatur wurde die wäßrige Phase dreimal mit Essigsäureethylester extrahiert. Die vereinigten organischen Phasen wurden mit Natriumhydrogensulfit-Lösung gewaschen und getrocknet. Nach Abdestillation des Lösungsmittels erhielt man 102,0 g 2-Chlor-3-hydroxycarbonyl-4-methylsulfonyl-benzoesäuremethylester.
      (¹H-NMR (d⁶-DMSO, δ in ppm): 3,34 (s, 3H) ; 3,93 (s, 3H); 8,08 (s, 2H); 14,50 (s, br., 1H).)
   Stufe b) 2-Chlor-3-chlorcarbonyl-4-methylsulfonyl-benzoesäuremethylester
      Zu einer Lösung von 6,0 g (0,021 mol) 2-Chlor-3-hydroxycarbonyl-4-methylsulfonyl-benzoesäuremethylester und 50 ml trockenem Toluol wurden 2 Tropfen Dimethylformamid und 11,9 g (0,1 mol) Thionylchlorid gegeben. Die Lösung wurde 4 Stunden unter Rückfluß erhitzt. Nach Entfernen des Lösungsmittels am Vakuum erhielt man 6,2 g 2-Chlor-3-chlorcarbonyl-4-methylsulfonyl-benzoesäuremethylester.
      (¹HNMR (CDCl₃; δ in ppm): 3,21 (s, 3H); 4,02 (s, 3H); 8,02 (d, 1H); 8,07 (d, 1H).)
2,4-Dichlor-3-(1'-methoxyimino-1'-(methoxy)methyl)-benzoylchlorid (Verbindung 3.14)
   Stufe a) 2,4-Dichlor-3-methyl-acetophenon
      Zu einer Lösung von 502,0 g (3,12 mol) 2,6-Dichlortoluol und 408,0 g (3,06 mol) Aluminiumtrichlorid wurde bei 100°C unter Rühren 235,0 g (3,0 mol) Acetylchlorid über einen Zeitraum von 2 Stunden zugetropft. Nach 2 Stunden Rühren bei 100-105°C, kühlte man ab und goß das Reaktionsgemisch auf 3 l Eis und 1 l Wasser. Der dabei ausgefallene Feststoff wurde abgesaugt und mit 800 ml Wasser neutral gewaschen. Nach dem Trocknen bei 40°C erhielt man 500,0 g 2,4-Dichlor-3-methyl-acetophenon als Rohprodukt, das anschließend im Hochvakuum destilliert wurde.
      (Sdp.: 121-128°C (4 mbar))
   Stufe b) 2,4-Dichlor-3-methyl-benzoesäure
      In eine Lösung von 520,0 g (13 mol) Natriumhydroxid in 2600 ml Wasser wurden bei 0-10°C zunächst 655,2 g (4,1 mol) Brom und anschließend 203,0 g (1,0 mol) 2,4-Dichlor-3-methyl-acetophenon in 1300 ml 1,4-Dioxan zugetropft. Nach 12 Stunden Rühren, trennte man die organische Phase ab, versetzte die wäßrige Phase mit einer 30 %igen Lösung, dargestellt aus Natriumpyrosulfit und Wasser, und stellte mit Salzsäure einen pH-Wert von 1 ein. Der ausgefallene Niederschlag wurde abgesaugt, mit Wasser gewaschen und am Vakuum bei 60°C getrocknet. Man erhielt 197,0 g 2,4-Dichlor-3-methyl-benzoesäure.
      (Fp.: 173-175°C)
   Stufe c) 2,4-Dichlor-3-methyl-benzoesäuremethylester
      In einer Lösung von 424,0 g (2 mol) 2,4-Dichlor-3-methylbenzoesäure und 1500 ml Methanol wurden 60 ml konz. Schwefelsäure getropft. Nach 5 Stunden Erhitzen unter Rückfluß, wurde das Reaktionsgemisch abgekühlt, am Vakuum eingeengt und anschließend in 1000 ml Methylenchlorid aufgenommen. Die organische Phase wurde mit Wasser, anschließend mit 5 %iger Natriumhydrogencarbonat-Lösung und dann wiederum mit Wasser gewaschen, getrocknet und am Vakuum eingeengt. Man erhielt 401,0 g 2,4-Dichlor-3-methylbenzoesäuremethylester.
      (Sdp: 103-107°C (1-1,5 mbar))
   Stufe d) 3-Brommethyl-2,4-dichlor-benzoesäuremethylester
      Zu einer Lösung von 84,0 g (0,38 mol) 2,4-Dichlor-3-methyl-benzoesäuremethylester und 67,6 g (0,38 mol) N-Bromsuccinimid in 380 ml Tetrachlorkohlenstoff wurde 1,0 g Azobisisobutyronitril gegeben. Nach 3,5 Stunden Erhitzen unter Rückfluß wurde das Reaktionsgemisch abgekühlt und der gebildete Niederschlag abgesaugt. Das Filtrat wurde am Vakuum eingeengt und der resultierende Rückstand aus Methyl-tert.butylether ausgerührt. Man erhielt 108,0 g 3-Brommethyl-2,4-dichlor-benzoesäuremethylester.
      (Fp.: 51-54°C)
   Stufe e) 2,4-Dichlor-3-formyl-benzoesäuremethylester
      Zu einer Lösung von 312,0 g (0,99 mol) 3-Brommethyl-2,4-dichlor-benzoesäuremethylester in 2 l Acetonitril wurden unter Rückfluß 696,2 g (2,97 mol) wäßrige 50 %ige N-Methylmorpholin-N-oxid-Lösung getropft. Nach 48 Stunden Rühren bei Raumtemperatur wurde die Reaktionslösung in 6 l Wasser eingerührt. Der ausgefallene Niederschlag wurde abgesaugt, mit Wasser gewaschen und am Vakuum getrocknet. Man erhielt 141,3 g 2,4-Dichlor-3-formylbenzoesäuremethylester.
      (¹H-NMR (CDCl₃, δ in ppm): 3,98 (s, 3H); 7,47 (d, 1H); 7,84 (d, 1H); 10,48 (s, 1H).)
   Stufe f) 2,4-Dichlor-3-hydroxycarbonyl-benzoesäuremethylester
      Zu einer Lösung von 40,0 g (0,172 mol) 2,4-Dichlor-3-formyl-benzoesäuremethylester und 500 ml Acetonitril wurden bei 5°C nacheinander 5,9 g (0,043 mol) Natriumdihydrogenphosphatmonohydrat in 70 ml Wasser, 20,5 g (0,181 mol) 30 %ige Wasserstoffperoxid-Lösung und 27,3 g (0,241 mol) 80 %ige Natriumchloritlösung gegeben. Die Reaktionslösung wurde 1 Stunde bei 5°C und 12 Stunden bei Raumtemperatur gerührt. Anschließend wurde mit 10 %iger Salzsäure ein pH-Wert von 1 eingestellt und 500 ml 40 %ige Natriumhydrogensulfit-Lösung zugegeben. Nach 1 Stunde Rühren bei Raumtemperatur wurde die wäßrige Phase dreimal mit Essigsäureethylester extrahiert, die vereinigten organischen Phasen mit 1,0 l 10 %iger Natriumhydrogensulfit-Lösung gewaschen und anschließend getrocknet. Nach Abdestillieren des Lösungsmittels erhielt man 40,0 g 2,4-Dichlor- 3-hydroxycarbonyl-benzoesäuremethylester.
      (¹H-NMR (d⁶-DMSO, δ in ppm): 3,90 (s, 3H); 7,69 (d, 1H); 7,89 (d, 1H).)
   Stufe g) 3-Chlorcarbonyl-2,4-dichlor-benzoesäuremethylester
      Zu einer Lösung von 5,00 g (0,02 mol) 2,4-Dichlor-3-hydroxycarbonyl-benzoesäuremethylester und 50 ml trockenen Toluol wurden 2 Tropfen Dimethylformamid und 11,90 g (0,1 mol) Thionylchlorid gegeben. Die Lösung wurde 4 Stunden unter Rückfluß erhitzt. Nach Abdestillieren des Lösungsmittels erhielt man 5,35 g 3-Chlorcarbonyl-2,4-dichlor-benzoesäuremethylester.
   Stufe h) 2,4-Dichlor-3-methoxyaminocarbonyl-benzoesäuremethylester
      Zu einer Lösung von 5,35 g (0,02 mol) 3-Chlorcarbonyl-2,4-dichlor-benzoesäuremethylester und 100 ml Dichlormethan wurden 4,60 g (0,045 mol) Triethylamin und 3,75 g (0,045 mol) Methoxyaminhydrochlorid gegeben. Nach 12 Stunden Rühren bei Raumtemperatur wurde die Reaktionslösung mit verdünnter Phosphorsäure gewaschen, getrocknet und eingeengt. Der erhaltene Rückstand wurde mit Diethylether ausgerührt. Man erhielt 4,80 g 2,4-Dichlor-3-methoxyaminocarbonyl-benzoesäuremethylester.
      (Fp.: 162-164°C)
   Stufe i) 2,4-Dichlor-3-(1'-methoxyimino-1'-(methoxy)methyl)-benzoesäuremethylester (Verbindung 3.09)
      Ein Gemisch von 16,0 g (0,058 mol) 2,4-Dichlor-3-methoxyaminocarbonyl-benzoesäuremethylester und 10,1 g (0,073 mol) Kaliumcarbonat in 300 ml Dimethylformamid wurde 30 Minuten bei Raumtemperatur gerührt. Anschließend wurden 11,0 g (0,087 mol) Dimethylsulfat zugetropft, 12 Stunden bei Raumtemperatur gerührt und erneut 11,0 g Dimethylsulfat zugegeben. Nach 6 Stunden Erhitzen auf 60°C wurde abgekühlt und in 2 l Eiswasser eingerührt. Nun wurde die wäßrige Phase mit Essigsäureethylester extrahiert, die vereinigten organischen Phasen getrocknet und das Lösungsmittel am Vakuum abdestilliert. Nach Chromatographie des Rückstands an Kieselgel (Eluent: Toluol/Essigsäureethylester = 9/1) erhielt man 2,0 g 2,4-Dichlor-3-(1'-methoxyimino-1'-(methoxy)methyl)-benzoesäuremethylester.
      ⁽¹H-NMR (CDCl₃, δ in ppm) : 3,43 (s, 3H); 3,58 (s, 3H); 3,92 (s, 3H); 7,35 (d, 1H); 7,82 (d, 1H).)
   Stufe j) 2,4-Dichlor-3-(1'-methoxyimino-1'-(methoxy)methyl)benzoesäure (Verbindung 3.10)
      Eine Lösung von 2,20 g (0,008 mol) 2,4-Dichlor-3-(1'methoxyimino-1'-(methoxy)methyl)benzoesäuremethylester und 3,00 g (0,075 mol) Natriumhydroxid in 50 ml Wasser wurde 2 Stunden bei 80°C gerührt. Nach Abkühlen wurde das Reaktionsgemisch in 200 ml Eiswasser eingerührt und mit konzentrierter Salzsäure auf pH = 1 gestellt. Die wäßrige Phase wurde mit Essigsäureethylester extrahiert, die vereinigten organischen Phasen getrocknet und am Vakuum eingeengt. Man erhielt 2,10 g 2,4-Dichlor-3-(1'-methoxyimino-1'-(methoxy)methyl)benzoesäure.
      ¹H-NMR (d⁶-DMSO, δ in ppm): 3,53 (s, 3H); 3,72 (s, 3H); 7,74 (d, 1H); 7,95 (d, 1H).)
   Stufe k) 2,4-Dichlor-3-(1'-methoxyimino-1'-(methoxy)methyl)-benzoylchlorid (Verbindung 3.14)
      Eine Lösung von 2,10 g (0,0076 mol) 2,4-Dichlor-3-(1'-(methoxy)imino-1'-methoxymethyl)benzoesäure und 20,00 g Thionylchlorid in 50 ml trockenem Toluol wurde 2 Stunden bei 80°C gerührt. Nach dem Entfernen des Lösungsmittels am Vakuum erhielt man 2,25 g 2,4-Dichlor-3-(1'-methoxyimino-1'-(methoxy)methyl)benzoylchlorid.
2,4-Dichlor-3-propoxyaminocarbonyl-benzoesäuremethylester
   Zu einer Lösung von 4,50 g (0,04 mol) Propoxyaminhydrochlorid und 4.05 g (0,04 mol) Triethylamin in 200 ml Methylenchlorid wurden langsam bei 30°C 10,7 g (0,04 mol) 3-Chlorcarbonyl-2,4-dichlorbenzoesäuremethylester in 100 ml Methylenchlorid getropft. Nach 2 Stunden Rühren bei Raumtemperatur wurde das Reaktionsgemisch mit verdünnter Phosphorsäure gewaschen, getrocknet und eingeengt. Der erhaltene Rückstand wurde am Kieselgel chromatographiert (Eluent: Toluol/Essigsäureethylester = 9/1). Man erhielt 11,50 g 2,4-Dichlor-3-propoxyaminocarbonyl-benzoesäuremethylester.
   (Fp.: 80-81°C)
3-(4-Chlorbenzyloxyaminocarbonyl)-2,4-dichlor-benzoesäuremethylester
   Zu einer Lösung von 7,76 g (0,04 mol) 4-Chlorbenzyloxyaminhydrochlorid und 4,05 g (0,04 mol) Triethylamin in 200 ml Methylenchlorid wurden langsam bei ca. 30°C 10,70 g (0,04 mol) 3-Chlorcarbonyl-2,4-dichlor-benzoesäuremethylester in 50 ml Methylenchlorid getropft. Nach 12 Stunden Rühren bei Raumtemperatur wurde das Reaktionsgemisch mit verdünnter Phosphorsäure gewaschen, getrocknet und eingeengt. Nach Ausrühren des Rückstandes mit Diethylether erhielt man 19,00 g 3-(4-Chlorbenzyloxyaminocarbonyl) -2,4-dichlor-benzoesäuremethylester.
   (Fp.: 120-121°C)
3-(1'-Methoxyiminoeth-1'-yl)-2-methyl-4-methylsulfonyl-benzoesäure (Verbindung 3.22)
   Stufe a) 3-(1'-Methoxyiminoeth-l'-yl)-2-methyl-anilin
      50,0 g (0,335 mol) 3-Amino-2-methyl-acetophenon, 66,3 g (0,838 mol) Pyridin und 42,0 g (0,503 mol) O-Methylhydroxylamin-Hydrochlorid wurden in 400 ml Ethanol bei Raumtemperatur gerührt. Nach Entfernen des Lösungsmittels wurde der Rückstand in Methylenchlorid aufgenommen und mit Wasser gewaschen, getrocknet und eingeengt. Man erhielt 54,0 g (91 % d.Th.) 3-(1'-Methoxyiminoeth-1'-yl)-2-methyl-anilin.
   Stufe b) 3-(1'-Methoxyiminoeth-1'-yl)-2-methyl-4-rhodano-anilin
      Zu 54,0 g (0,303 mol) 3-(1'-Methoxyiminoeth-1'-yl)-2-methyl-anilin, 49,3 g (0,479 mol) Natriumbromid und 77,5 g (0,956 mol) Natriumrhodanid in 300 ml Methanol wurden bei -20 bis -15°C 50,9 g (0,319 mol) Brom zugetropft. Nach 30 Minuten Rühren bei dieser Temperatur wurden die unlöslichen Bestandteile abgesaugt, das Filtrat mit Essigsäureethylester versetzt und mit wäßriger Natriumhydrogencarbonat-Lösung ein pH-Wert von 8 eingestellt. Die organische Phase wurde abgetrennt und die verbleibende wäßrige Phase mehrmals mit Essigsäureethylester extrahiert. Die vereinigten organischen Phasen wurden dann mit Wasser gewaschen, getrocknet und eingeengt. Man erhielt 67,3 g (95 % d.Th.) 3-(1'-Methoxyiminoeth-1'-yl)-2-methyl-4-rhodanoanilin.
   Stufe c) 3-(1'-Methoxyiminoeth-1'-yl)-2-methyl-4-methylthioanilin
      Zu 40,4 g (0,315 mol) Natriumsulfid in 200 ml Wasser wurden bei 20 bis 30°C 67,3 g (0,286 mol) 3-(1'-Methoxyiminoeth-1'-yl)-2-methyl-4-rhodano-anilin in 600 ml Methanol getropft. Nach 3 Stunden Rühren bei Raumtemperatur wurden 45,1 g (0,318 mol) Methyliodid in 200 ml Methanol, ebenfalls bei 20 bis 30°C zugegeben. Anschließend wurde 12 Stunden bei Raumtemperatur gerührt, das Lösungsmittel entfernt, der Rückstand in Wasser aufgenommen und mehrmals mit Essigsäureethylester extrahiert. Die vereinigten organischen Phasen wurden dann mit Wasser gewaschen, getrocknet, eingeengt und der so erhaltene Rückstand mit n-Hexan/Methyltert.-butylether digeriert. Man erhielt 43,2 g (67 % d.Th.) 3-(1'-Methoxyiminoeth-1'-yl)-2-methyl-4-methylthio-anilin.
      (Fp.: 83-89°C)
   Stufe d) 6-Brom-2-(1'-methoxyiminoeth-1'-yl)-3-methylthio-toluol
      Zu 3,00 g (13,4 mmol) 3-(1'-Methoxyiminoeth-1'-yl)-2-methyl-4-methylthio-anilin in 13,40 g Eisessig wurden bei Raumtemperatur 9,23 g 47 %ige Bromwasserstoffsäure zugetropft. Anschließend wurden 9,23 g Wasser zugegeben, 10 Minuten bei Raumtemperatur gerührt und bei -5 bis 0°C 0,92 g (13,4 mmol) Natriumnitrit in 1,9 ml Wasser zugegeben. Das resultierende Reaktionsgemisch wurde dann bei 0°C zu 1,92 g (13,4 mmol) Kupfer-(I)-bromid in 6 ml 47 %iger Bromwasserstoffsäure getropft. Nach 12 Stunden Rühren bei Raumtemperatur wurde auf Eiswasser gegossen und mit Methylenchlorid extrahiert. Die organische Phase wurde dann mit Natriumsulfit-Lösung und Wasser gewaschen, getrocknet und das Lösungsmittel entfernt. Man erhielt 2,50 g (65 % d.Th.) 6-Brom-2-(1'-methoxyiminoeth-1'-yl)-3-methylthiotoluol.
   Stufe e) 6-Brom-2-(1'-methoxyiminoeth-1'-yl)-3-methylsulfonyltoluol
      Zu 2,5 g (8,71 mmol) 6-Brom-2-(1'-methoxyimino-1'yl)-3-methylthio-toluol in 50 ml Methylenchlorid wurden innerhalb von 96 Stunden in Portionen insgesamt 7,0 g (34,80 mmol) m-Chlorperbenzoesäure gegeben. Nach Entfernen des Lösungsmittels wurde in organischem Lösungsmittel aufgenommen, mit Natriumcarbonatlösung, Natriumsulfitlösung und Wasser gewaschen, getrocknet und eingeengt. Der Rückstand wurde dann an Kieselgel (Eluent: Toluol/Essigsäureethylester) chromatographiert. Man erhielt 0,8 g (29 % d.Th.) 6-Brom-2-(1'-methoxyiminoeth-1'-yl)-3-methylsulfonyl-toluol.
   Stufe f) 3-(1'-Methoxyiminoeth-1'-yl)-2-methyl-4-methylsulfonylbenzoesäure
      0,77 g (2,41 mmol) 6-Brom-2-(1'-methoxyiminoeth-1'-yl)-3-methylsulfonyl-toluol, 0,03 g (0,1 mmol) Palladiumacetat, 0,14 g (0,49 mmol) Tricyclohexylphosphin, 0,10 g (2,4 mmol) Lithiumchlorid und 0,49 g (4,81 mmol) Triethylamin wurden in 37,5 ml Toluol und 17,5 ml Wasser suspendiert und bei 140°C, bei einem Druck von 20 bar 36 Stunden begast. Anschließend wurden nach Abkühlen die unlöslichen Bestandteile abgetrennt, die organische Phase mit Wasser (das mit 1 ml Triethylamin versetzt wurde) extrahiert, die resultierende wäßrige Phase mit Salzsäure auf pH = 1 gestellt und mit Methylenchlorid extrahiert. Diese organische Phase wurde getrocknet und eingeengt. Man erhielt 0,62 g (90 % d.Th.) 3-(1'-Methoxyiminoeth-1'-yl)-2-methyl-4-methylsulfonyl-benzoesäure.

In nachfolgender Tabelle 3 sind neben den voranstehend beschriebenen Verbindungen weitere Benzoesäurederivate der Formel III aufgeführt, die in analoger Weise hergestellt wurden oder herstellbar sind.

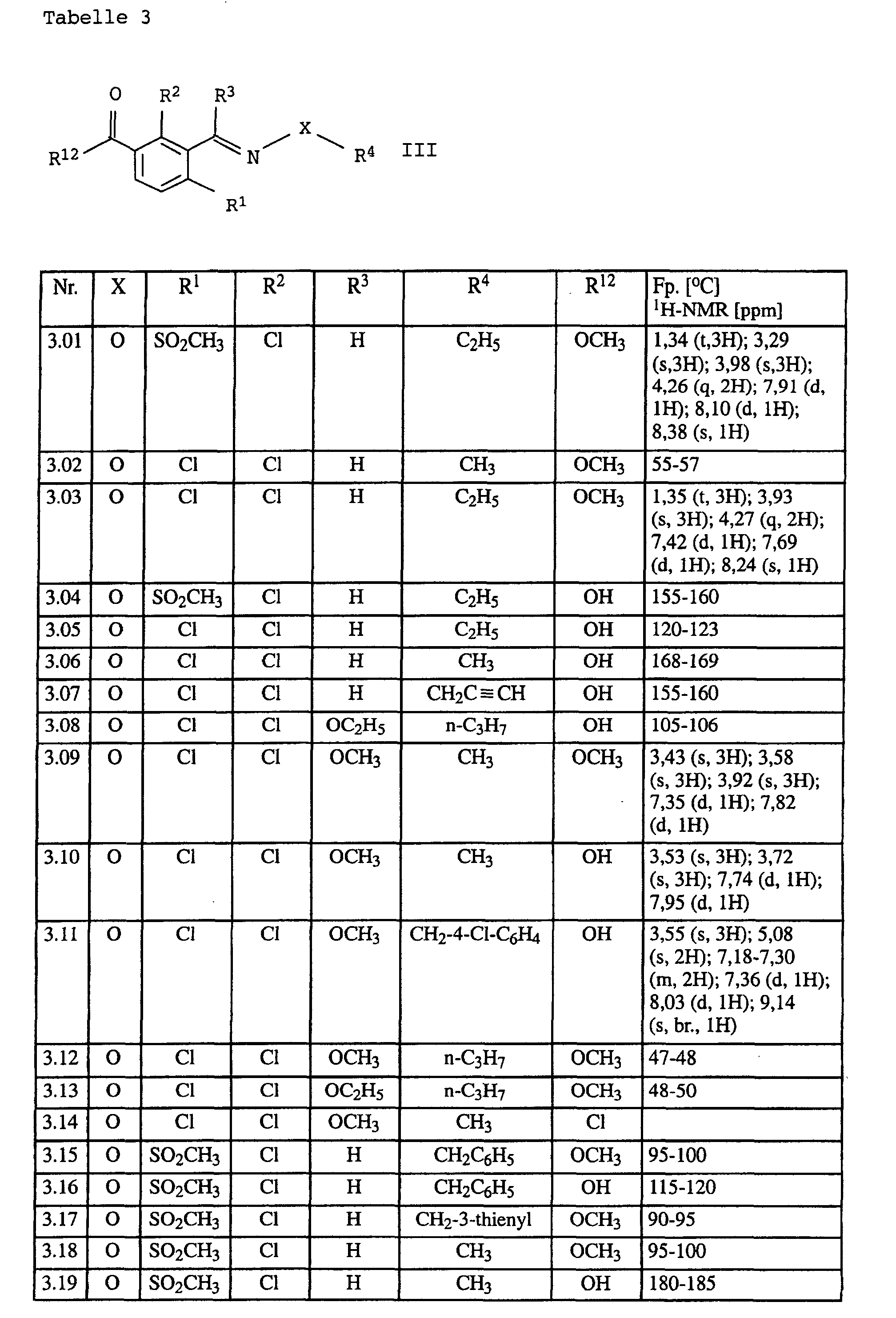

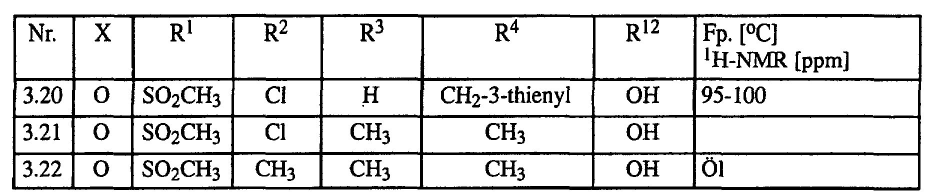

Die 4-Benzoyl-pyrazole der Formel I und deren landwirtschaftlich brauchbaren Salze eignen sich - sowohl als Stereoisomerengemische als auch in Form der reinen Stereoisomeren - als Herbizide. Die Verbindungen der Formel I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die 4-Benzoyl-pyrazole der Formel I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen der Formel I auch in Kulturen, die durch Züchtung einschließlich gentechnischer Methoden gegen die Wirkung von Herbiziden tolerant sind, verwandt werden.

Die Verbindungen der Formel I bzw. die sie enthaltenden herbiziden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel I oder eines landwirtschaftlichen brauchbaren Salzes von I und für die Formulierung von Pflanzenschutzmitteln üblichen Hilfsmittel.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z.B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Verbindungen der Formel I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile der Verbindung Nr. 2.01 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.-% des Wirkstoffs enthält.
II. 20 Gewichtsteile der Verbindung Nr. 2.03 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
III. 20 Gewichtsteile des Wirkstoffs Nr. 2.05 werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.-% des Wirkstoffs enthält.
IV. 20 Gewichtsteile des Wirkstoffs Nr. 2.06 werden mit 3 Gewichtsteilen des Natriumsalzes der Diisobutylnaphthalin-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile des Wirkstoffs Nr. 2.01 werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile des Wirkstoffs Nr. 2.04 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil der Verbindung 2.03 wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil der Verbindung 2.05 wird in einer Mischung gelöst, die aus 80 Gewichtsteilen Cyclohexanon und 20 Gewichtsteilen Wettol® EM 31 (nicht ionischer Emulgator auf der Basis von ethoxyliertem Ricinusöl). Man erhält ein stabiles Emulsionskonzentrat.

Die Applikation der Wirkstoffe der Formel I bzw. der herbiziden Mittel bzw. kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Zur Verbreiterung des Wirkungsspektrums und zur Erzielung synergistischer Effekte können die Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF₃-phenylderivate, Carbamate, Chinolincarbonsäure und deren Derivate, Chloracetanilide, Cyclohexenon-oximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen der Formel I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1,0 kg/ha aktive Substanz (a. S.)

### Anwendungsbeispiele

Die herbizide Wirkung der 4-Benzoyl-pyrazole der Formel I ließ sich durch Gewächshausversuche zeigen:

Als Kulturgefäße dienten Plastiktöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,125 bzw. 0,0625 kg/ha a. S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Englischer Name | Deutscher Name |
|---|---|---|
| Chenopodium album | lambsquarters (goosefoot) | Weißer Gänsefuß |
| Echinochloa crus galli | barnyardgrass | Hühnerhirse |
| Sinapis alba | white mustard | Weißer Senf |
| Setaria faberii | giant foxtail | Borstenhirse |
| Triticum aestivum | summer wheat | Sommerweizen |

Bei Aufwendungen von 0,125 bzw. 0,0625 kg/ha (a. S.) zeigte die Verbindung 2.01 (Tabelle 2) im Nachauflauf eine sehr gute Wirkung gegen oben genannte mono- und dikotyle Schadpflanzen und gute Verträglichkeit in Sommerweizen.

## Patentansprüche

1. 4-Benzoyl-pyrazole der Formel I 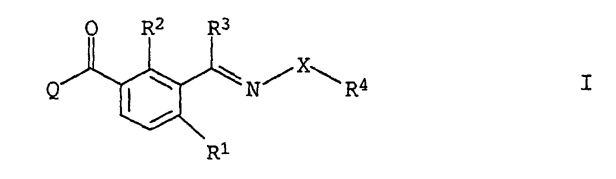 in der die Variablen folgende Bedeutung haben:
R¹, R² Nitro, Halogen, Cyano, Rhodano, C₁-C₆-Alkyl, C₁-C₆-Halogenalkyl, C₁-C₆-Alkoxy-C₁-C₆-alkyl, C₂-C₆-Alkenyl, C₂-C₆-Alkinyl, -OR⁵ oder -S(O)ₙR⁶;
R³ Wasserstoff, Cyano, C₁-C₆-Alkyl, C₁-C₆-Halogenalkyl, -OR⁶, -SR⁶ oder -NR⁶R⁸;
R⁴ Wasserstoff, C₁-C₆-Alkyl, C₃-C₆-Cycloalkyl, C₃-C₆-Alkenyl oder C₃-C₆-Alkinyl, wobei die vier letzgenannten Substituenten partiell oder vollständig halogeniert sein können und/oder eine bis drei der folgenden Gruppen tragen können:
Hydroxy, Mercapto, Amino, Cyano, R⁸, -OR⁸, =NOR⁸, -OCOR⁸, -CO₂R⁸, -COSR⁸, -CONR⁷R⁸, C₁-C₄-Alkyliminooxy, C₁-C₄-Alkylcarbonyl, C₁-C₄-Alkoxy-C₂-C₆-alkoxycarbonyl, Heterocyclyl, Heterocyclyloxy, Phenyl, Benzyl, Hetaryl, Phenoxy, Benzyloxy und Hetaryloxy, wobei die acht letztgenannten Reste ihrerseits partiell oder vollständig halogeniert sein können und/oder einen bis drei Reste aus folgender Gruppe tragen können:
Nitro, Cyano, C₁-C₄-Alkyl, C₁-C₄-Halogenalkyl, C₁-C₄-Alkoxy, C₁-C₄-Halogenalkoxy, C₁-C₄-Alkoxycarbonyl;
und wobei der voranstehend genannte Heterocyclyl-bzw. Heterocycloxy-Rest ein bis drei Heteroatome ausgewählt aus der Gruppe Sauerstoff, Stickstoff und Schwefel enthält, 3-7 gliedrig, mono- oder polycyclisch ist und gesättigt oder partiell ungesättigt ist;
und wobei der voranstehend genannte Hetaryl- bzw. Hetaryloxy-Rest neben Kohlenstoffringgliedern zusätzlich ein bis vier Stickstoffatome oder ein bis drei Stickstoffatome und ein Sauerstoff- oder ein Schwefelatom, oder ein Sauerstoff-, oder ein Schwefelatom enthält und aromatisch ist;
X Sauerstoff oder NR⁷;
n 0, 1 oder 2;
R⁵ Wasserstoff, C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁶ C₁-C₆-Alkyl, C₁-C₆-Halogenalkyl, C₁-C₆-Alkoxy-C₂-C₆-alkyl, C₃-C₆-Alkenyl oder C₃-C₆-Alkinyl;
R⁷ Wasserstoff oder C₁-C₆-Alkyl;
R⁸ C₁-C₆-Alkyl, C₁-C₆-Halogenalkyl, C₃-C₆-Alkenyl oder C₃-C₆-Alkinyl;
Q ein in 4-Stellung verknüpftes Pyrazol der Formel II 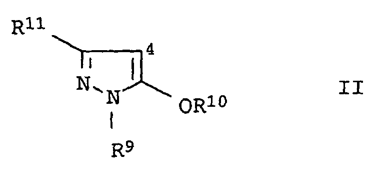 wobei
R⁹ für C₁-C₆-Alkyl, C₁-C₆-Halogenalkyl, Phenyl oder Phenyl das partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹⁰ für Wasserstoff, C₁-C₆-Alkyl, C₁-C₆-Halogenalkyl, C₁-C₆-Alkylcarbonyl, C₁-C₆-Halogenalkylcarbonyl, C₁-C₆-Alkoxycarbonyl, C₁-C₆-Alkylsulfonyl, C₁-C₆-Halogenalkylsulfonyl, Phenylcarbonyl, Phenylcarbonylmethyl, Phenoxycarbonyl oder Phenylsulfonyl, wobei die vier letztgenannten Substituenten unsubstituiert sind oder der Phenylring jeweils partiell oder vollständig halogeniert ist und/oder einen bis drei der folgenden Reste trägt:
Nitro, Cyano, C₁-C₄-Alkyl, C₁-C₄-Halogenalkyl, C₁-C₄-Alkoxy, C₁-C₄-Halogenalkoxy;
R¹¹ für Wasserstoff, C₁-C₆-Alkyl oder C₁-C₆-Halogenalkyl;
stehen;
sowie deren landwirtschaftlich brauchbaren Salze.

2. Verfahren zur Herstellung von 4-Benzoyl-pyrazolen der Formel I gemäß Anspruch 1, **dadurch gekennzeichnet, daß** man ein Pyrazol der Formel II (mit R¹⁰ = H), in der die Variablen R⁹ und R¹¹ die unter Anspruch 1 genannte Bedeutung haben, 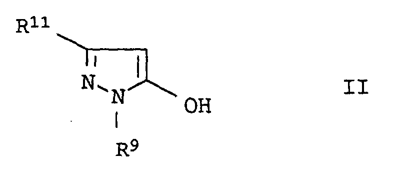 mit einer aktivierten Carbonsäure IIIα oder mit einer Carbonsäure IIIβ, 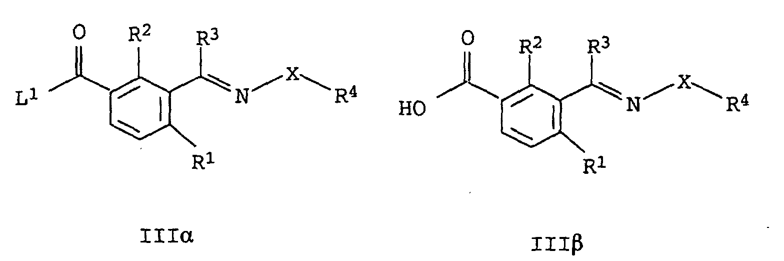 wobei die Variablen R¹ bis R⁴ und X die unter Anspruch 1 genannten Bedeutungen haben und L¹ für eine nucleophil verdrängbare Abgangsgruppe steht, acyliert und das Acylierungsprodukt gegebenenfalls in Gegenwart eines Katalysators zu den Verbindungen I (mit R¹⁰ = H) umlagert und gewünschtenfalls zur Herstellung von 4-Benzoyl-pyrazolen der Formel I mit R¹⁰ ≠ H mit einer Verbindung der Formel V,
L² ― R¹⁰ V
(mit R¹⁰ ≠ H)
in der R¹⁰ abgesehen von Wasserstoff die unter Anspruch 1 genannte Bedeutung hat und L² für eine nucleophil verdrängbare Abgangsgruppe steht, umsetzt.

3. Mittel, enthaltend eine herbizid wirksame Menge mindestens eines 4-Benzoyl-pyrazols der Formel I oder eines landwirtschaftlich brauchbaren Salzes von I gemäß Anspruch 1 und für die Formulierung von Pflanzenschutzmitteln übliche Hilfsmittel.

4. Verfahren zur Herstellung von herbizid wirksamen Mitteln gemäß Anspruch 3, **dadurch gekennzeichnet, daß** man eine herbizid wirksame Menge mindestens eines 4-Benzoyl-pyrazols der Formel I oder eines landwirtschaftlich brauchbaren Salzes von I gemäß Anspruch 1 und für die Formulierung von Pflanzenschutzmitteln übliche Hilfsmittel mischt.

5. Verfahren zur Bekämpfung von unerwünschtem Pflanzenwuchs, **dadurch gekennzeichnet, daß** man eine herbizid wirksame Menge mindestens eines 4-Benzoyl-pyrazols der Formel I oder eines landwirtschaftlich brauchbaren Salzes von I gemäß Anspruch 1 auf Pflanzen, deren Lebensraum und/oder auf Samen einwirken läßt.

6. Verwendung der 4-Benzoyl-pyrazole der Formel I und deren landwirtschaftlich brauchbaren Salze gemäß Anspruch 1 als Herbizide.

## Claims

1. A 4-benzoylpyrazole of the formula I 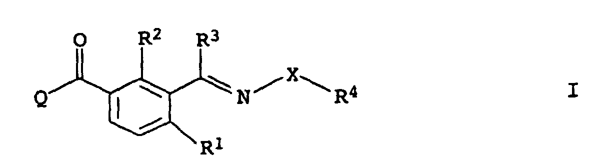 where the variables have the following meanings:
R¹, R² are nitro, halogen, cyano, thiocyanato, C₁-C₆-alkyl, C₁-C₆-haloalkyl, C₁-C₆-alkoxy-C₁-C₆-alkyl, C₂-C₆-alkenyl, C₂-C₆-alkynyl, -OR⁵ or -S(O)ₙR⁶;
R³ is hydrogen, cyano, C₁-C₆-alkyl, C₁-C₆-haloalkyl, -OR⁶, -SR⁶ or -NR⁶R⁸;
R⁴ is hydrogen, C₁-C₆-alkyl, C₃-C₆-cycloalkyl, C₃-C₆-alkenyl or C₃-C₆-alkynyl, it being possible for the four last-mentioned substituents to be partially or fully halogenated and/or to have attached to them one to three of the following groups:
hydroxyl, mercapto, amino, cyano, R⁸, -OR⁸, =NOR⁸, -OCOR⁸, -CO₂R⁸, -COSR⁸, -CONR⁷R⁸, C₁-C₄-alkyliminooxy, C₁-C₄-alkylcarbonyl, C₁-C₄-alkoxy-C₂-C₆-alkoxycarbonyl, heterocyclyl, heterocyclyloxy, phenyl, benzyl, hetaryl, phenoxy, benzyloxy and hetaryloxy, it being possible for the eight last-mentioned radicals, in turn, to be partially or fully halogenated and/or to have attached to them one to three radicals of the following group:
nitro, cyano, C₁-C₄-alkyl, C₁-C₄-haloalkyl, C₁-C₄-alkoxy, C₁-C₄-haloalkoxy, C₁-C₄-alkoxycarbonyl; and the abovementioned heterocyclyl, or heterocycloxy, radical containing one to three hetero atoms selected from the group oxygen, nitrogen and sulfur, being 3-7-membered, mono- or polycyclic and saturated or partially unsaturated;
and the abovementioned hetaryl, or hetaryloxy, radical, besides carbon ring members, additionally containing one to four nitrogen atoms or one to three nitrogen atoms and one oxygen or one sulfur atom, or one oxygen atom or one sulfur atom and being aromatic;
X is oxygen or NR⁷;
n is 0, 1 or 2;
R⁵ is hydrogen, C₁-C₆-alkyl, C₁-C₆-haloalkyl, C₁-C₆-alkoxy-C₂-C₆-alkyl, C₃-C₆-alkenyl or C₃-C₆-alkynyl;
R⁶ is C₁-C₆-alkyl, C₁-C₆-haloalkyl, C₁-C₆-alkoxy-C₂-C₆-alkyl, C₃-C₆-alkenyl or C₃-C₆-alkynyl;
R⁷ is hydrogen or C₁-C₆-alkyl;
R⁸ is C₁-C₆-alkyl, C₁-C₆-haloalkyl, C₃-C₆-alkenyl or C₃-C₆-alkynyl;
Q is a pyrazole of the formula II 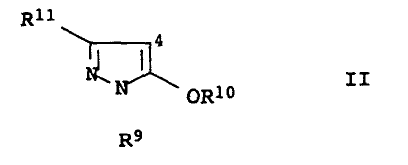 which is linked in the 4-position and where
R⁹ is C₁-C₆-alkyl, C₁-C₆-haloalkyl, phenyl or phenyl which is partially or fully halogenated and/or has attached to it one to three of the following radicals:
nitro, cyano, C₁-C₄-alkyl, C₁-C₄-haloalkyl, C₁-C₄-alkoxy, C₁-C₄-haloalkoxy;
R¹⁰ is hydrogen, C₁-C₆-alkyl, C₁-C₆-haloalkyl, C₁-C₆-alkylcarbonyl, C₁-C₆-haloalkylcarbonyl, C₁-C₆-alkoxycarbonyl, C₁-C₆-alkylsulfonyl, C₁-C₆-haloalkylsulfonyl, phenylcarbonyl, phenylcarbonylmethyl, phenoxycarbonyl or phenylsulfonyl, the four last-mentioned substituents being unsubstituted or the phenyl ring being in each case partially or fully halogenated and/or having attached to it one to three of the following radicals: nitro, cyano, C₁-C₄-alkyl, C₁-C₄-haloalkyl, C₁-C₄-alkoxy, C₁-C₄-haloalkoxy;
R¹¹ is hydrogen, C₁-C₆-alkyl or C₁-C₆-haloalkyl;
or an agriculturally useful salt thereof.

2. A process for the preparation of 4-benzoylpyrazoles of the formula I as claimed in claim 1, which comprises acylating a pyrazole of the formula II (where R¹⁰ = H) where the variables R⁹ and R¹¹ have the meanings given under claim 1 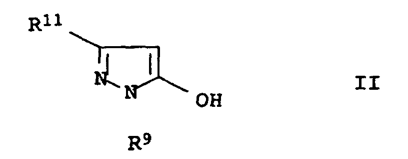 with an activated carboxylic acid IIIα or with a carboxylic acid IIIβ, 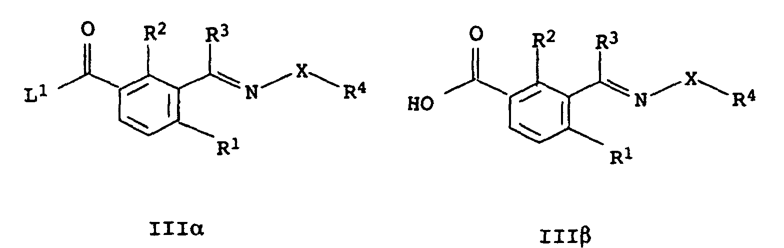 where the variables R¹ to R⁴ and X have the meanings given under claim 1 and L¹ is a nucleophilically displaceable leaving group, subjecting the acylation product to a rearrangement reaction, if appropriate in the presence of a catalyst, to give the compounds I (where R¹⁰ = H) and, if desired, to prepare 4-benzoylpyrazoles of the formula I where R¹⁰ ≠ H reacting the product with a compound of the formula V
L²―R¹⁰ V
(where R¹⁰≠ H)
where R¹⁰ has the meanings given under claim 1 with the exception of hydrogen and L² is a nucleophilically displaceable leaving group.

3. A composition comprising a herbicidally active amount of at least one 4-benzoylpyrazole of the formula I or of an agriculturally useful salt of I as claimed in claim 1 and auxiliaries conventionally used for the formulation of crop protection products.

4. A process for the preparation of herbicidally active compositions as claimed in claim 3, which comprises mixing a herbicidally active amount of at least one 4-benzoylpyrazole of the formula I or of an agriculturally useful salt of I as claimed in claim 1 with auxiliaries conventionally used for the formulation of crop protection products.

5. A method of controlling undesirable vegetation, which comprises allowing a herbicidally active amount of at least one 4-benzoylpyrazole of the formula I or of an agriculturally useful salt of I as claimed in claim 1 to act on plants, their environment and/or on seeds.

6. The use of a 4-benzoylpyrazole of the formula I or an agriculturally useful salt thereof as claimed in claim 1 as a herbicide.

## Revendications

1. 4-benzoyl-pyrazoles de formule I 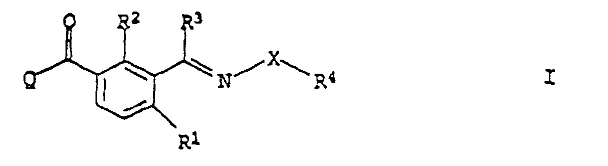 dans laquelle les variables ont la bignification suivante:
R¹, R² nitro, halogéno, cyano, rhodano, alkyle en C₁-C₆, halogénoalkyle en C₁-C₆, alcoxy(C₁-C₆)-alkyle(C₁-C₆), alcényle en C₂-C₆, alcynyle en C₂-C₆, -OR⁵ ou -S(O)ₙR⁶;
R³ hydrogène, cyano, alkyle en C₁-C₆, halogénoalkyle en C₁-C₆, -OR⁶, -SR⁶ ou -NR⁶R⁸;
R⁴ hydrogène, alkyle en C₁-C₆, cycloalkyle en C₃-C₆, alcényle en C₃-C₆ ou alcynyle en C₃-C₆, tandis que les quatre substituants mentionnés en dernier peuvent être partiellement ou totalement halogénés et/ou peuvent porter un à trois des groupes suivants:
hydroxy, mercapto, amino, cyano, R⁸, -OR⁸, =NOR⁸, -OCOR⁸, -CO₂R⁸, -COSR⁸, -OONR⁷R⁸, alkyliminooxy en C₁-C₄, alkyl(C₁-C₄)carbonyle, alcoxy(C₁-C₄)-alcoxy(C₂-C₆)carbonyle, hétérocyclyle, hétérocyclyloxy, phényle, benzyle, hétaryle, phénoxy, benzyloxy et hétaryloxy, tandis que les huit restes mentionnées en dernier peuvent à leur tour être partiellement ou totalement halogénés et/ou peuvent porter un à trois restes du groupe suivant:
nitro, cyano, alkyle en C₁-C₄, halogénoalkyle en C₁-C₄, alcoxy en C₁-C₄, halogénoalcoxy en C₁-C₄, alcoxycarbonyle en C₁-C₄; et tandis que le reste hétérocyclyle ou hétérocyclyloxy contient un à trois hétéroatomes choisis dans le groupe de l'oxygène, l'azote et le soufre, est mono- ou polycyclique à 3-7 chaînons, et est saturé ou partiellement insaturé;
et tandis que le reste hétaryle ou hétaryloxy mentionné ci-dessus contient, outre les chaînons de cycle hydrocarboné, également un à quatre atomes d'azote ou un à trois atomes d'azote et un atome d'oxygène ou de soufre, ou un atome d'oxygène, ou un atome de soufre, et est aromatique;
X oxygène ou NR⁷;
n 0, 1 ou 2;
R⁵ hydrogène, alkyle en C₁-C₆, halogénoalkyle en C₁-C₆, alcoxy(C₁-C₆)-alkyle(C₂-C₆), alcényle en C₃-C₆ ou alcynyle en C₃-C₆;
R⁶ Alkyle en C₁-C₆, halogénoalkyle en C₁-C₆, alcoxy(C₁-C₆)-alkyle(C₂-C₆), alcényle en C₃-C₆ ou alcynyle en C₃-C₆;
R⁷ hydrogène ou alkyle en C₁-C₆;
R⁸ Alkyle en C₁-C₆, halogénoalkyle en C₁-C₆, alcényle en C₃-C₆ ou alcynyle en C₃-C₆;
Q un pyrazole lié sur la position 4, de formule II 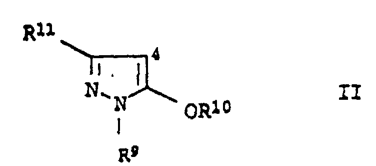 où
R9 désigne un alkyle en C₁-C₆, un halogénoalkyle en C₁-C₆, un phényle ou un phényle qui est partiellement ou totalement halogéné et/ou porte un à trois des restes suivants:
nitro, cyano, alkyle en C₁-C₄, halogénoalkyle en C₁-C₄, alcoxy en C₁-C₄, halogénoalcoxy en C₁-C₄;
R¹⁰ représente l'hydrogène, un alkyle en C₁-C₆, un halogénoalkyle en C₁-C₆, un alkyl(C₁-C₆)carbonyle, un halogénoalkyl(C₁-C₆)carbonyle, un alcoxy(C₁-C₆)carbonyle, un alkylsulfonyle en C₁-C₆, un halogénoalkylsulfonyle en C₁-C₆, un phénylcarbonyle, un phénylcarbonylméthyle, un phénoxycarbonyle ou un phénoxysulfonyle, tandis que les quatre substituants mentionnées en dernier sont non-substirués ou le cycle phényle est à chaque fois partiellement ou totalement halogéné et/ou porte un à trois des restes suivants:
nitro, cyano, alkyle en C₁-C₄, halogénoalkyle en C₁-C₄, alcoxy en C₁-C₄, halogénoalcoxy en C₁-C₄;
R¹¹ désigne l'hydrogène, un alkyle en C₁-C₆ ou un halogénoalkyle en C₁-C₆;
ainsi que leurs sels utilisables en agriculture.

2. Procédé pour la préparation de 4-benzoyl-pyrazoles de formule I selon la revendication 1, **caractérisé par le fait qu'**on acyle un pyrazole de formule II (avec R¹⁰ = H), dans laquelle les variables R⁹ et R¹¹ ont la signification indiquée dans la revendication 1, 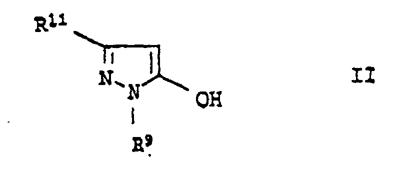 avec un acide carboxylique activé IIIα ou avec un acide carboxylique IIIβ, 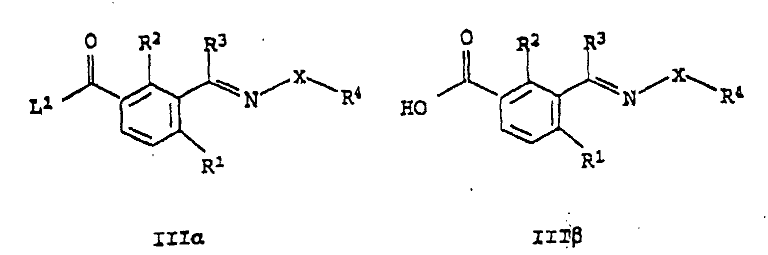 où les variables R¹ à R⁴ et X ont les significations indiquées dans la revendication 1 et L¹ désigne un groupe éliminable, déplaçable par voie nucléophile, et on transpose le produit d'acylation éventuellement en présence d'un catalyseur pour former le composé I (avec R¹⁰ = H), et si on le souhaite pour la préparation de 4-benzoylpyrazoles de formule I avec R¹⁰ ≠ H on fait réagir avec un composé de formule V,
L²―R¹⁰ V
(avec R10 ≠ H)
dans laquelle R¹⁰ a, à l'exception de l'hydrogène, la signification indiquée dans la revendication 1 et L² représente un groupe éliminable, déplaçable par voie nucléophile.

3. Produit contenant une quaritité à activité herbicide d'au moins un 4-benzoyl-pyrazole de formule I ou un sel de I utilisable en agriculture selon la revendication 1 et d'adjuvants classiques pour la formulation de produits phytosanitaires.

4. Procédé pour la préparation de produits à activité herbicide selon la revendication 3, **caractérisé par le fait qu'**on mélange une quantité à activité herbicide d'au moins un 4-benzoyl-pyrazole de formule I ou un sel de I utilisable en agriculture selon la revendication 1 et des adjuvants classiques pour la formulation de produits phytosanitaires.

5. Procédé pour la lutte contre la croissance indésirable des plantes, **caractérisé par le fait qu'**on fait agir une quantité à efficacité herbicide d'au moins un 4-benzoyl-pyrazole de formule I ou un sel de I utilisable en agriculture selon la revendication 1 sur des plantes, leur biotope et/ou des semences.

6. Utilisation des 4-benzoyl-pyrazoles de formule I et de leurs sels utilisables en agriculture selon la revendication 1 comme herbicides.
